# EUROPEAN PATENT SPECIFICATION

(11) **EP 0 474 767 B1**
(45) Date of publication and mention of the grant of the patent: **21.02.1996**
(21) Application number: 90909264.5
(22) Date of filing: 22.05.1990
(51) Int. Cl.: C07D 409/04, C07D 311/74, C07D 221/06, C07D 487/00, C07D 209/56, C07D 211/00, C07C 69/76, A01N 43/32, A01N 43/16, A01N 43/42, A01N 43/62

(54) **DOPAMINE AGONISTS**
DOPAMIN-AGONISTEN
AGONISTES DE DOPAMINE

(30) Priority: 31.05.1989 US 359448
(43) Date of publication of application: 18.03.1992
(73) Proprietor: ABBOTT LABORATORIES, Abbott Park,Illinois 60064-3500 (US)
(72) Inventor: SCHOENLEBER, Robert, W., Deerfield, IL 60015 (US); KEBABIAN, John, W., Lake Bluff, IL 60044 (US); MARTIN, Yvonne, C., Waukegan, IL 60087 (US); DeNINNO, Michael, P., Wildwood, IL 60030 (US); PERNER, Richard, J., Gurnee, IL 60031 (US); STOUT, David, M., Mettawa, IL 60048 (US); HSIAO, Chi-Nung, W., Libertyville, IL 60048 (US); DiDOMENICO, Stanley, Jr., Gurnee, IL 60031 (US); DeBERNARDIS, John, F., Lindenhurst, IL 60046 (US); BASHA, Fatima, Z., Lake Forest, IL 60045 (US); MEYER, Micheal, D., Lindenhurst, IL 60046 (US); DE, Biswanath, Vernon Hills, IL 60061 (US); EHRLICH, Paul, P., Evanston, IL 60201 (US); CAMPBELL, James, R., Ft. Sheridan, IL 60037 (US); MORTON, Howard, E., Gurnee, IL 60031 (US); LIJEWSKI, Linda, M., Oak Creek, WI 53154 (US)
(74) Representative: Modiano, Guido, Dr.-Ing.
(86) International application number: PCT/US90/02864
(87) International publication number: WO 90/15056

(56) References cited:
- EP-A- 0 321 968
- EP-A- 0 325 963
- FR-A- 2 407 212
- GB-A- 1 552 004
- GB-A- 2 093 837
- US-A- 4 473 586
- US-A- 4 618 683
- MEDICINAL RESEARCH REVIEWS, vol. 5, nr. 2, pages 145-229, ed. 1985.

## Description

### Cross Reference to Related Applications

This application is a continuation-in-part of copending U.S. application Serial Number 359,448 filed May 31, 1989.

### Technical Field

This invention relates to novel compounds which have pharmacological activity. The compounds of the invention are selective dopamine agonists useful for treating dopamine-related neurological, psychological, cardiovascular and behavioral disorders.

### Background of the Invention

Dopamine is an important neurotransmitter in the central nervous system (CNS), and also has several important roles in the peripheral nervous system such as in the control of supply of blood to the kidneys and in autonomic ganglion transmission.

It is now widely accepted that dopamine receptors in the CNS can be divided into two general categories, designated D-1 and D-2 receptors The division was originally based on biochemical and pharmacological differences between the two receptor types. Recently, further evidence which supports this division has come from study of the molecular biology of dopamine receptors in the CNS. The dopamine D-1 receptor is linked to the enzyme adenylate cyclase through a stimulatory G protein such that stimulation of this receptor by dopamine or a dopamine D-1 receptor agonist causes an increase in the production of 3',5'-cyclic adenosine monophosphate (cAMP). The D-2 receptor, on the other hand, also regulates important functional activity within the CNS, although the biochemical events which follow stimulation of this receptor by dopamine or a D-2 receptor agonist are not as well understood. Autoreceptors on dopaminergic neurons which have the pharmacological properties of D-2 receptors are thought to control the firing rate of these cells as well as the release of copamine from the nerve terminals It is also known that stimulation of the D-2 receptors in the intermediate lobe of the pituitary gland causes a decrease in CAMP production and that stimulation of the D-2 receptors on the mammotrophs of the anterior pituitary gland suppresses prolactin secretion. Dopaminergic neurons are also affected by and interact with other neurotransmitter systems in the CNS. For example, D-2 receptors on the cholinergic interneu- rons in the striatum (one of the components of the basal ganglia) regulate the release of acetylcholine from these cells.

Dopamine involvement has been proposed for several diverse neurological and psychological disorders. One disorder involving dopamine is Parkinson's Disease. Dopamine occurs at high concentration within the nerve terminals in the basal ganglia of the mammalian brain and in the early 1960's, the loss of striatal dopamine was established as a chemical marker of Parkinson's Disease. This deficiency is still thought to be primary to the etiology of the disease state.

L-DOPA (3,4-dihydroxyphenylalanine), which is used in conjunction with a peripheral aromatic amino acid decarboxylase inhibitor and often supplemented with anticholinergic agents, has been shown to be useful in the treatment of Parkinson's Disease. The response to L-DOPA is thought to be a result of the conversion of L-DOPA to dopamine within the striatum, and is linked to stimulation of both the D-1 and D-2 receptors.

The success of L-DOPA therapy has led to the testing of other compounds capable of mimicking the post-synaptic receptor actions of dopamine. Such direct-acting agents might offer the therapeutic advantages of greater potency, increased duration of action, or fewer side effects over L-DOPA. For example, bromocryptine, the direct-acting dopamine agonist most widely used in the treatment of Parkinson's Disease, lowers the amount of L-DOPA required to achieve the maximal therapeutic response and allows for a delay in the onset of L-DOPA therapy. However, the response to bromocryptine alone is not as great as that observed with L-DOPA.

Another disorder in which dopamine has been implicated is the psychosis schizophrenia. The psychoses are serious psychiatric illnesses characterized by abnormal behavior which may include delusions, hallucinations, violence, mania and serious long-lasting depression. Schizophrenia is the most common psychosis and involves disturbance of thought processes, hallucinations and loss of touch with reality. The theory of schizophrenia as a disease of the CNS was first formalized by Kraepelin and Bleuler in the early 1900's. It was not until chlorpromazine was discovered by Delay and Daniker in the early 1950's, however, that drug management of this disease was possible.

The pioneering work of Carlsson and others led to the now widely-held dopamine theory of schizophrenia. According to this theory, schizophrenia is caused by an excess of dopamine in the brain. Several lines of evidence support this hypothesis. For example, chronic abuse of stimulants such as amphetamines, known to enhance dopaminergic activity in the brain, can lead to a paranoid psychosis that is almost indistinguishable from classic paranoid schizophrenia. The mechanism-of-action proposed for drugs with anti-schizophrenic activity is the blockade by these compounds of the dopamine receptors, and consequently, the prevention of excess receptor stimulation. In the mid 1970's it was observed that virtually all of the currently used antipsychotic agents could displace radiolabeled haloperidol (a dopamine antagonist) from striatal dopamine receptors with a good correlation between average effective clinical dose and drug binding affinity.

Unfortunately, the currently. available antipsychotic agents frequently produce undesirable side-effects, the most common of which are the so-called extrapyramidal effects that include bizarre involuntary movements and Parkinson-like effects Sedation and hypotension are also common side effects. Because of these often severe side-effects and the high incidence of patients unresponsive to currently available drugs, more potent and selective agents are needed.

It is also recognized that depressive conditions and related affective disorders result from a reduction in the central nervous system of certain biogenic amine neurotransmitters such as dopamine (DA), noradrenaline (NA) and serotonin (5-HT). Affective disorders are characterized by changes in mood as the primary clinical manifestation. Disturbances of mood are the most common psychiatric disorders in adults with 18-23% of women and 8-11% of men experiencing at least one major depressive episode. Currently available antidepressant drugs work primarily by raising the levels of the biogenic amine neurotransmitters by either inhibition of the neuronal uptake of the neurotransmitters or inhibition of the metabolic enzymes responsible for converting the biogenic amines to inactive metabolites. Unfortunately there are major drawbacks to the use of currently available agents for treating affective disorders. For example, no antidepressant drug to date has proven to be superior to electroconvulsive shock therapy in the treatment of severe, suicidal depression. Other problems with the use of available drugs are delayed onset of activity, poor efficacy, anticholinergic effects at therapeutic doses, cardiotoxicity, convulsions and the danger of taking a fatal overdose. There also exists a large number of untreated individuals and treatment-resistant patients in need of effective therapy. A role for direct-acting dopamine agonists in antidepressant therapy has been suggested based on the effects observed for several dopamine agonists in various animal models used for predicting antidepressant activity such as the "mouse behavioral despair test".

A role for dopamine has been established in several other neurological functions such as cognitive function and attention mechanisms. Animal studies implicate dopamine in attention-related behaviors involving search and exploratory activity, distractibility, response rate, discriminability and the switching of attention. A therapeutic role in the treatment of cognitive impairment and attention deficit disorders has therefore been proposed and is under active investigation for compounds which mimic the receptor activity of dopamine.

Dopamine has been used in the treatment of shock, congestive heart failure and renal failure. Stimulation of the peripheral DA-1 receptors causes vasodilation, particularly in the renal and mesenteric vascular beds where large numbers of these receptors are found. The utility of dopamine has been limited, however, by its ability to cause vasoconstriction at higher concentrations, presumably due to its secondary effects on adrenergic receptors and by its emetic effects due to peripheral DA-2 stimulation. Agents selective for the peripheral DA-1 receptors may offer significant advantages over currently used treatments for these and other related disorders.

Published evidence suggests that dopamine also has a central role in the brain's reward system. For example, it has been reported that animals trained to selfadminister cocaine will increase their consumption of this drug after treatment with either a D-1 or a D-2 receptor antagonist. It was proposed that the animals would increase the amount of cocaine administered in order to maintain the elevated dopamine levels responsible for the drugs euphorigenic and reinforcing properties. The dopamine D-1 agonist SKF 38393 has been reported to decrease food intake by rats presumably by direct action of the drug on neural feeding mechanisms. Because of this interrelationship between dopamine and reward, dopaminergic agents could be useful for the treatment of substance abuse and other addictive behavior disorders including cocaine addiction, nicotine addiction and eating disorders.

Dopaminergic agents such as the compounds of the present invention that mimic the actions of dopamine and show selectivity for the different dopamine receptor subtypes are needed in order to obtain the anticipated physiological responses discussed above, separate from other possibly less desirable effects.

Relevant as background to the present invention is FR-A-2 407 212, which discloses anti-psychotic and hypotensive 1-(heterocyclic group-substituted)alkyl benzopyran compounds which may be substituted in the 5-, 6-_{;} 7- or 8-positions by, for example, methoxy or hydroxy, and in the 3-position by alkyl, aryl, cycloalkyl or spircycloalkyl. The heterocyclic group contains nitrogen atoms as the heteratoms and is attached to the alkyl group by one of the ring nitrogens.

GB-A-2 093 837 discloses 1-aminoalkyl-tetrahydronaphthalenes which may be substituted in the 5-, 6- or 7-positions by, for example, methoxy and hydroxy, and which are unsubstituted in the 3-position. Such compounds are useful as dopamine agonists.

EP-A-0 325 963, which is considered prior art under the terms of Article 54(3) and (4) EPC, discloses 1-aminomethyl-tetrahydronaphthalenes as alpha-2-adrenergic antagonists.

Medicinal Research Reviews, vol. 5, no 2, p. 145-229 (1985) discloses the compound 1-(aminomethyl)-5,6-dihydroxy-2-benzopyran as a dopamine agonist.

### Summary of the Invention

The compounds of the present invention are dopaminergic compounds represented by the following structural formula (I): 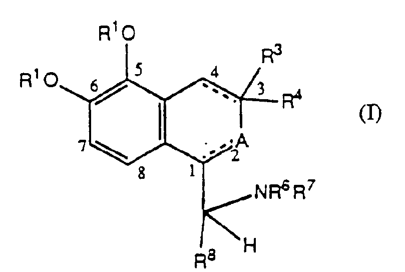 or a pharmaceutically acceptable salt, ester or amide thereof, wherein A is 0, S, CHR², CR² or C when A and R⁶ taken together form a nitrogen-containing 5-, 6- or 7-membered ring;

the dotted lines represent optional double bonds;
R^{l} is selected from hydrogen and a group which is readily cleavable in vivo;
R² is selected from hydrogen, C₁-C₁₂ alkyl and substituted C₁-C₆ alkyl;
R³ is selected from C₁-C₁₂ alkyl substituted C₁-C₆ alkyl as defined below, C₂-C₁₂ alkenyl, substituted C₂-C₁₂ alkenyl as defined below, C₂-C₁₂ alkynyl, substituted C₂-C₁₂ alkynyl as defined below, C₃-C₁₂ cycloalkyl, substituted C₃-C₁₂ cycloalkyl as defined below, carbocyclic C₅-C₁₀ aryl, substituted carbocyclic C₅-C₁₀ aryl as defined below, heterocycle as defined below, and substituted heterocycle as defined below,
R⁴ is selected from hydrogen and C₁-C₁₂ alkyl or, taken together with R³ and the carbon atom to which they are attached, forms a spirocycloalkyl ring of from 3 to 7 carbons;
R⁶ is selected from hydrogen, C₁-C₁₂ alkyl, substituted C₁-C₆ alkyl as defined below, C₂-C₁₂ alkenyl, substituted C₂-C₁₂ alkenyl as defined below, C₂-C₁₂ alkynyl, substituted C₂-C₁₂ alkynyl as defined below, C₃-C₁₂ cycloalkyl, substituted C₃-C₁₂ cycloalkyl as defined below, alkanoyl of from 1 to 8 carbons, naturally occurring amino acid and dipeptide formed from naturally occurring amino acids or, taken together with A when A is C, forms a nitrogen-containing 5-, 6- or 7-membered ring;
R⁷ is hydrogen or C₁-C₁₂ alkyl or, taken together with R⁶ or R⁸, forms a nitrogen-containing 5-, 6- or 7-membered ring, provided that when R⁶ is carbocyclic arylalkyl, R⁷ is not alkyl;
R⁸ is hydrogen or C₁-C₁₂ alkyl or, taken together with R⁶ or R⁷, forms a nitrogen-containing 5-, 6- or 7-membered ring or taken together with the catechol ring at the 8-position and the carbon atoms to which they are attached forms a 5- 6- or 7-membered ring;
wherein substituted C₁-C₆ alkyl is a C₁-C₆ alkyl group bearing 1-2 substituents selected from halogen, hydroxy, C₁-C₆ alkoxy, amino, C₁-C₆ alkylamino, C₃-C₁₂ cycloalkyl, heterocycle as defined below, substituted heterocycle as defined below, carbocyclic C₅-C₁₀ aryl, substituted carbocyclic C₅₋C₁₀ aryl as defined below, carbocyclic C₅-C₁₀ aryloxy, substituted carbocyclic C₅-C₁₀ aryloxy wherein substituted carbocyclic C₅-C₁₀ aryl is as defined below, or -NC(O)R¹¹ wherein R¹¹ is selected from hydrogen, C₁-C₆ alkyl, halo-(C₁-C₆ alkyl), heterocycle as defined below, substituted heterocycle as defined below, carbocyclic C₅-C₁₀ aryl, and substituted carbocyclic C₅-C₁₀ aryl as defined below;
wherein substituted C₂-C₁₂ alkenyl is a C₂-C₁₂ alkenyl group bearing a substituent selected from C₁-C₆ alkyl, C₃-C₁₂ cycloalkyl, carbocyclic C₅-C₁₀ aryl, benzyl, phenylethyl, amino, hydroxy, C₁-C₆ alkoxy and heterocycle as defined below,
wherein substituted C₂-C₁₂ alkynyl is a C₂-C₁₂ alkynyl bearing a substituent selected from C₁-C₆ alkyl, C₃-C₁₂ cycloalkyl, carbocyclic C₅-C₁₀ aryl, benzyl, phenylethyl, amino, hydroxy, C₁-C₁₂ alkylamino, C₁-C₆ alkoxy, and heterocycle as defined below;
wherein substituted C₃-C₁₂ cycloalkyl is a C₃-C₁₂ cycloalkyl group substituted by one or more C₁-C₁₂ alkyl, hydroxy, amino, C₁-C₁₂ alkylamino, C₁-C₁₂ aminoalkyl, mercapto, C₁-C₁₂ alkylthio or halogen groups,
wherein substituted carbocyclic C₅-C₁₀ aryl is a carbocyclic C₅-C₁₀ aryl substituted by one or more hydroxy, halogen, C₁-C₆ alkyl or C₁-C₆ alkoxy groups or by a phenyl group;
wherein heterocycle is a monocyclic 5- or 6-membered saturated or unsaturated group containing one or two ring heteroatoms selected from N. O and S, the remaining atoms being carbon, such as, for example, furyl, tetrahydrofuryl, thienyl, pyrryl, pyrrolidyl, pyridyl, piperidyl, pyrazinyl, pyrimidyl, piperazinyl, morpholinyl, thiomorpholinyl, pyrazolyl, imidazolyl, oxazolyl, oxazolidyl, isoxazolyl, isoxazolidyl and thiazolyl; and
wherein substituted heterocycle is a heterocycle as defined above substituted by halogen, C₁-C₆ alkyl or C₁-C₆ alkoxy.

The compounds of Formula I have the ability to act on dopamine receptors in the central and peripheral nervous systems and to mimic the activity of dopamine. The compounds of the present invention are, therefore, useful in the treatment of dopamine-related neurological, psychological and cardiovascular disorders as well as in the treatment of substance abuse and other addictive behavior disorders, cognitive impairment and attention deficit disorder.

### Detailed Description of the Invention

This invention relates to novel compounds which are selective dopamine agonists. More particularly, this invention relates to compounds of the following formula: 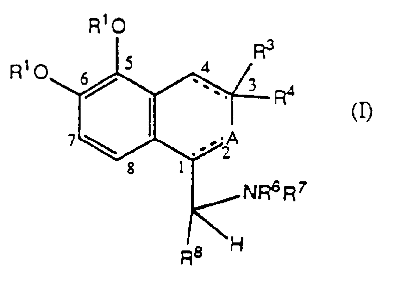 or a pharmaceutically acceptable salt, ester or amide thereof wherein A is 0, S, CHR², CR² or C when A and R⁶ taken together form a nitrogen-containing 5-, 6- or 7-membered ring and wherein R¹, R², R³, R⁴, R⁶, R⁷ and R⁸ are as defined above.

The dotted lines represent optional double bonds.

In one embodiment of the present invention, represented by formula (la), A is CR² and the dotted line between A and ring atom number 1 represents a bond: 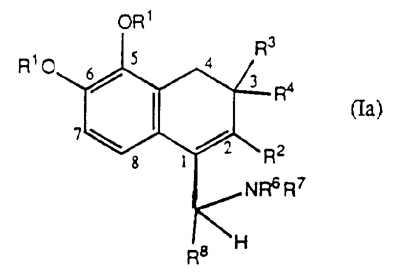 wherein R¹, R², R³, R⁴, R⁶, R⁷ and R⁸ are as defined above.

The preferred compounds of formula la are those in which R⁴ is hydrogen.

In another embodiment of the present invention, represented by formula (Ib), A is CH R²; 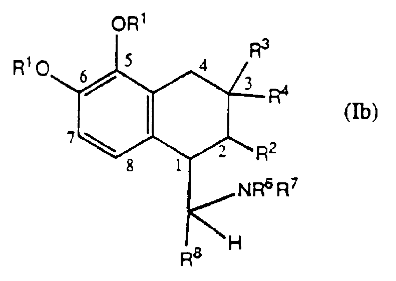 and R¹, R², R³, R⁴, R⁶, R⁷ and R⁸ are as defined above.

The preferred compounds of formula lb are those in which R⁴ is hydrogen.

Another embodiment of the present invention is represented by the formula (lc): 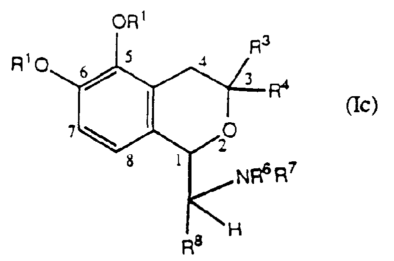 wherein R¹, R³, R⁴, R⁶, R⁷ and R⁸ are as defined above.

In another embodiment of the present invention, represented by formula (Id), R⁸ is H, and R⁶ and A taken together form a nitrogen-containing 5-, 6- or 7-membered ring: 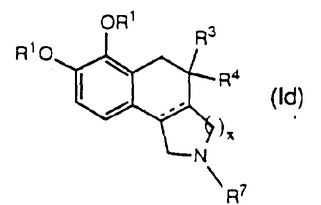 wherein x is 1, 2 or 3 and R¹, R³, R⁴ and R⁷ are as defined above.

In another embodiment of the present invention, represented by formula (le), A is CHR², R² is H and R⁸ taken together with position 8 of the catechol ring form a 5-, 6- or to 7-membered ring: 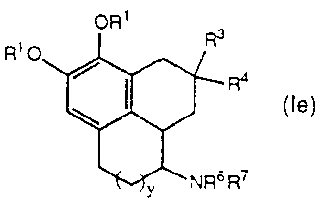 wherein y is 0, 1 or 2 and R¹ and R³, R⁴, R⁶ and R7 are as defined above.

The present invention also relates to pharmaceutical compositions comprising a therapeutically effective amount of the compound of Formula (I) and a pharmaceutically acceptable carrier or diluent.

The present invention also relates to the use of the compounds of Formula (I) in the treatment of dopamine related disorders.

The following compounds are representative of the preferred compounds of Formula (I):
1-Aminomethyl-5 ,6-dihydroxy-3-phenyl-3,4-dihydronaphthalene;
1-Aminomethyl-5,6-bis(acetoxy)-3-phenyl-3,4-dihydronaphthalene;
1 -Aminomethyl-5 ,6-bis(trimethylacetoxy)-3-phenyl-3,4-dihydronaphthalene;
[1R,3S]1-Aminomethyl-5,6-dihydroxy-3-phenyl-1 ,2,3,4-tetrahydronaphthalene;
1 -Aminomethyl-3-cyclohexyl-5,6-dihydroxy-3,4-dihydronaphthalene;
[1R,3S] 1-Aminomethyl-3-cyclohexyl-5,6-dihydroxy-1,2,3,4-tetrahydronaphthalene;
[1R,3S] 1-Aminomethyl-3-t-butyl-3,4-dihydro-5,6-dihydroxy-1 H-2-benzopyran;
[1R,3R] 1-Aminomethyl-3-n-butyl-3,4-dihydro-5;6-dihydroxy-1 H-2- benzopyran;
[1R,3S] 1-Aminomethyl-3,4-dihydro-5,6-dihydroxy-3-phenyl-1H-2-benzopyran;
[1R,3S] 1-Aminomethyl-3-cyclohexyl-3,4-dihydro-5,6-dihydroxy-1H-2-benzopyran;
[1R,3R] 1 -Aminomethyl-3-cyclopentylmethyl-3,4-dihydro-5,6-dihydroxy-1 H-2-benzopyran;
[1R,3S] 1-Aminomethyl-3-cyclooctyl-3,4-dihydro-5,6-dihydroxy-1 H-2- benzopyran;
[1R,3R] 1-Aminomethyl-3,4-dihydro-5,6-dihydroxy-3-ethyl-1 H-2-benzopyran;
Spiro[(1-aminomethyl-3,4-dihydro-5,6-dihydroxy-1 H-2-benzopyran)-3,1'-cyclohexane];
[1R,3S] 1-Aminomethyl-3,4-dihydro-5,6-dihydroxy-3-(4'-methoxyphenoxy)methyl-1H-2-benzopyran;
[1R^{*},3S^{*}] 1-Aminomethyl-3,4-dihydro-5,6-dihydroxy-3-phenyl-1 H-2-benzopyran;
[1R,3S] 1-Aminomethyl-3,4-dihydro-5,6-dihydroxy-3-phenoxymethyl- H-2-benzopyran;
[1R,3S] 1 -Aminomethyl-3,4-dihydro-5,6-dihydroxy-3-(2'-phenylphenoxy)methyl-1 H-2-benzopyran;
[1R,3S] 1-Aminomethyl-3-(4'-t-butylphenoxy)methyl H-2-benzopyran;
[1R,3S] 1-Aminomethyl-3-(4'-bromophenoxy)methyl-3,4-dihydro-5,6-dihydroxy-1 H-2-benzopyran;
[1 R,3S] 3-(1'-Adamantyl) -1-aminomethyl-3,4-dihydro-5,6-dihydroxy-1 H-2-benzopyran;
[1R,3R] 1-Aminomethyl-3-benzyl-3,4-dihydro-5,6-dihydroxy-1H-2-benzopyran;
[1R,3R] 1-Aminomethyl- 3,4-dihydro-5,6-dihydroxy-3-(2'phenyl)ethyl-1 H-2-benzopyran;
[1R,3S] 1-Aminomethyl-8-bromo-3,4-dihydro-5,6-dihydroxy-3-phenyl-1H-2-benzopyran;
[1R,3R]-Aminomethyl-3,4-dihydro-5,6-dihydroxy-3-n-octyl-1 H-2-benzopyran;
[1R,3R] 1-Aminomethyl-3,4-dihydro-5,6-dihydroxy-3-(1'-hex-5'-ene)-iH-2-benzopyran;
[1R,3S] 1-Aminomethyl-3,4-dihydro-5,6-dihydroxy-3-ethyl-1H-2-benzopyran;
[1R,3R] 1-Aminomethyl-3,4-dihydro-5,6-dihydroxy-3-n-hexyl-1H-2-benzopyran;
1R,3S] 1-Aminomethyl-3-(4'-bromophenyl)-3,4-dihydro-5,6-dihydroxy-1 H-2-benzopyran;
[1R,3S] 1-Aminomethyl-3,4-dihydro-5,6-dihydroxy-3-(3'-hydroxyphenyl)-1 H-2-benzopyran ;
[1R,3S] 3-Cyclohexyl-3,4-dihydro-5,6-dihydroxy-1-(N-methyl)aminomethyl-1 H-2-benzopyran;
[1 R,3S] 3-t-Butyl-3,4-dihydro-5,6-dihydroxy-1 -(N-methyl)aminomethyl-1 H-2-benzopyran;
[1R,3R] 3-n-Butyl-3,4-dihydro-5,6-dihydroxy-1-(N-methyl)aminomethyl-1H-2-benzopyran;
[1R,3S] 3-(1'-Adamantyl)-3,4-dihydro-5,6-dihydroxy-1-(N-methyl)aminomethyl-1H-2-benzopyran;
[1R,3S] 1-(N-Allyl)aminomethyl-3-cyclohexyl-3,4-dihydro-5,6-dihydroxy-1H-2-benzopyran;
[1R,3S] 3-Cyclohexyl-1-(N-cyclopropyl)aminomethyl-3,4-dihydro-5,6-dihydroxy-1 H-2-benzopyran ;
[1R,3S] 1-(N-benzyl)aminomethyl-3-cyclohexyl-3,4-dihydro-5,6-dihydroxy-1H-2-benzopyran;
[1R,3S] 1-,3-Bis(aminomethyl)-3,4-dihydro-5,6-dihydroxy-1 H-2-benzopyran;
[1R,3S] 1-Aminomethyl-3,4-dihydro-5,6-dihydroxy-3-hydroxymethyl-1 H-2-benzopyran ;
[1R,3S] 1-Aminomethyl-3,4-dihydro-5,6-dihydroxy-3-(N-piperidinyl)methyl- 1H-2-benzopyran;
[1R,3S] 5,6-Dihydroxy-3-phenyl-1- (2'R-pyrrolidinyl)-1,2,3,4-tetrahydronaphthalene,
[1R,3R] 5,6-Dihydroxy-3-phenyl-1-(2'R-pyrrolidinyl)-1,2,3,4-tetrahydronaphthalene;
5,6-Dihydroxy-1-(N-methyl)aminomethyl-3-phenyl-3,4-dihydronaphthalene;
[1R,3S] 5,6-Dihydroxy-1-(N-methyl)aminomethyl-3-phenyl-1,2,3,4-tetrahydronaphthalene;
1-Aminomethyl-5,6-dihydroxy-3-(3'-hydroxyphenyl)-3,4-dihydronaphthalene;
1-Aminomethyl-5,6-dihydroxy-3-(4'-hydroxyphenyl)-3,4-dihydronaphthalene;
[1R,3S] 1-Aminomethyl-5,6-dihydroxy-3-(3'-hydroxyphenyl)-1,2,3,4-tetrahydronaphthalene;
5,6-Bis(acetoxy)-1-(alanyl-alanyl)aminomethyl-3-phenyl-3,4-dihydronaphthalene;
5,6-Bis(acetoxy)-1-(y-glutamyl)aminomethyl-3-phenyl-3,4-dihydronaphthalene;
5 ,6-Bis(acetoxy)-1-(alanyl)aminomethyl-3-phenyl-3,4-dihydronaphthalene;
5,6-Bis(acetoxy)-1-(methionyl)aminomethyl-3-phenyl-3,4-dihydronaphthalene;
1-(Alanyl-alanyl)aminomethyl-5,6-bis(benzoyloxy)-3-phenyl-3,4-dihydronaphthalene;
[1R,2S] 1-Aminomethyl-5,6-dihydroxy-2-(2'-hydroxy-1'-ethyl)-3-phenyl-1,2,3,4-tetrahydronaphthalene; 1-Aminomethyl-5,6-dihydroxy-3-phenylnaphthalene;
(1R,3S] 1-Aminomethyl-3,4-dihydro-5,6-dihydroxy-3-(diphenyl)methyl-1 H-2-benzopyran;
[1R,3S] 1-Aminomethyl-3,4-dihydro-5,6-dihydroxy-3-(3'-methyl-2'-n-pentyl)-1H-2-benzopyran;
[1R,3R] 1-Aminomethyl-3,4-dihydro-5,6-dihydroxy-3-(1'-but-3'-ene)-1 H-2-benzopyran;
[1R,3S] 1-Aminomethyl-3,4-dihydro-5,6-dihydroxy-3-(6'-methyl-2'-hep-5'-ene)--1 H-2-benzopyran;
[1R,3S] 1-Aminomethyl-3-benzyloxymethyl-3,4-dihydro-5,6-dihydroxy-1H-2-benzopyran;
[1R,3S] 1-Aminomethyl-3-cyclooctyl-3,4-dihydro-5,6-dihydroxy-1H-2-benzopyran;
[1R, 8S, 9aR] 1-Amino-5,6-dihydroxy-2,3,7,8,9,9a-hexahydro-8-phenyl-phenalene;
[1S, 8S, 9aR] 1-Amino-5,6-dihydroxy-2,3,7,8,9,9a-hexahydro-8-phenyl-phenalene; 6,7-Dihydroxy-4-phenyl-2,3,4,5-tetrahydro-1H-benz[e] isoindole;
[1 R^{*}, 3S^{*}] 3-(1 '-Adamantyl)-1-aminomethyl-3,4-dihydro-5,6-dihydroxy-1 H-2-benzopyran;
1 -Aminomethyl-5,6-bis(benzoyloxy)-3-phenyl-3,4-dihydronaphthalene; and their pharmaceutically acceptable salts.

The following compounds are representative of the more preferred compounds of Formula (I):
1-Aminomethyl-5,6-dihydroxy-3-phenyl-3,4-dihydronaphthalene;
1-Aminomethyl-5,6-bis(acetoxy)-3-phenyl-3,4-dihydronaphthalene;
1-Aminomethyl-5,6-bis(trimethylacetoxy)-3-phenyl-3,4.dihydronaphthalene;
[1R,3S] 1-Aminomethyl-5,6-dihydroxy-3-phenyl-1,2,3,4-tetrahydronaphthalene;
1 -Aminomethyl-3-cyclohexyl-5,6-dihydroxy-3,4-dihydronaphthalene,
[1R,3S] 1-Aminomethyl-3-cyclohexyl-5,6-di hydroxy-1,2,3,4-tetrahydronaphthalene;
[1R,3S] 1-Aminomethyl-3-t-butyl-3,4-dihydro-5,6-dihydroxy-1 H-2-benzopyran ;
[1R,3R] 1-Aminomethyl-3-n-butyl-3,4-dihydro-5,6-dihydroxy-1 H-2-benzopyran ;
[1R,3S] 1-Aminomethyl-3,4-dihydro-5,6-dihydroxy-3-phenyl-1H-2-benzopyran;
[1R,3S] 1-Aminomethyl-3-cyclohexyl-3,4-dihydro-5,6-dihydroxy-1H-2-benzopyran;
[1R,3R] 1 -Aminomethyl-3-cyclopentylmethyl-3,4-dihydro-5,6-dihydroxy-1 H-2-benzopyran;
[1R,3S] 1-Aminomethyl-3-cyclooctyl-3,4-dihydro-5,6-dihydroxy-1 H-2- benzopyran;
[1R,3S] 1-Aminomethyl-3,4-dihydro-5,6-dihydroxy-3-(4'-methoxyphenoxy)methyl-1H-2-benzopyran;
[1R^{*},3S^{*}] 1-Aminomethyl-3,4-dihydro-5,6-dihydroxy-3-phenyl-1H-2-benzopyran;
[1R,3S] 1-Aminomethyl-3,4-dihydro-5,6-dihydroxy-3-phenoxymethyl-1H-2-benzopyran;
[1R,3S] 1-Aminomethyl-3-(4'-t-butylphenoxy)methyl-3,4-dihydro-5,6-dihydroxy-1H-2-benzopyran;
[1R,3S] 1-Aminomethyl-3-(4'-bromophenoxy)methyl-3,4-dihydro-5,6-dihydroxy-1H-2-benzopyran;
[1 R,3S] 3-(1'-Adamantyl) -1-aminomethyl-3,4-dihydro-5,6-dihydroxy-1 H-2-benzopyran;
[1R,3R] 1-Aminomethyl-3-benzyl-3,4-dihydro-5,6-dihydroxy-1 H-2-benzopyran;
[1R,3R] 1-Aminomethyl- 3,4-dihydro-5,6-dihydroxy-3-(2'phenyl)ethyl-1 H-2-benzopyran;
[1R,3S] 1-Aminomethyl-8-bromo-3,4-dihydro-5,6-dihydroxy-3-phenyl-1H-2-benzopyran;
[1R,3R] 1-Aminomethyl-3,4-dihydro-5,6-dihydroxy-3-n-octyl-1 H-2-benzopyran;
[1R,3R] 1 -Aminomethyl-3,4-dihydro-5,6-dihydroxy-3-(1'-hex-5'-ene)-1 H-2-benzopyran;
[1R,3R] 1-Aminomethyl-3,4-dihydro-5,6-dihydroxy-3-n-hexyl-1 H-2-benzopyran;
[1R,3S] 1-Aminomethyl-3-(4'-bromophenyl)-3,4-dihydro-5,6-dihydroxy-1 H-2-benzopyran;
[1R,3S] 1-Aminomethyl-3,4-dihydro-5,6-dihydroxy-3-(3'-hydroxyphenyl)-1H-2-benzopyran;
[1R,3S] 3-Cyclohexyl-3,4-dihydro-5,6-dihydroxy-1-(N-methyl)aminomethyl-1 H-2-benzopyran;
[1R,3S] 3-t-Butyl-3,4-dihydro-5,6-dihydroxy-1-(N-methyl)aminomethyl-1 H-2-benzopyran;
[1R,3R] 3-n-Butyl-3,4-dihydro-5,6-dihydroxy-1-(N-methyl)aminomethyl-1H-2-benzopyran;
[1R,3S] 3-(1'-Adamantyl)-3,4-dihydro-5,6-dihydroxy-1-(N-methyl)aminomethyl-1H-2-benzopyran;
[1R,3S] 1-(N-Allyl)aminomethyl-3-cyclohexyl-3,4-dihydro-5,6-dihydroxy-1H-2-benzopyran;
[1 R,3S] 3-Cyclohexyl-1-(N-cyclopropyl)aminomethyl-3,4-dihydro-5,6-dihydroxy-1 H-2-benzopyran;
[1R,3S]-(N-benzyl)aminomethyl-3-cyclohexyl-3,4-dihydro-5,6-dihydroxy-1 H-2-benzopyran;
[1R,3S] 1-,3-Bis(aminomethyl)-3,4-dihydro-5,6-dihydroxy-1 H-2-benzopyran;
[1R,3S] 1-Aminomethyl-3,4-dihydro-5,6-dihydroxy-3-hydroxymethyl-1 H-2-benzopyran;
[1R,3S] 1-Aminomethyl-3,4-dihydro-5,6-dihydroxy-3-(N-piperidinyl)methyl-1 H-2-benzopyran;
[1R,3S] 5,6-Dihydroxy-3-phenyl-1-(2'R-pyrrolidinyl)-1,2,3,4-tetrahydronaphthalene;
[1R,3R] 5,6-Dihydroxy-3-phenyl-1-(2'R-pyrrolidinyl)-1,2,3,4-tetrahydronaphihalene;
5,6-Dihydroxy-1-(N-methyl)aminomethyl-3-phenyl-3,4-dihydronaphthalene;
[1R,3S] 5,6-Dihydroxy-1-(N-methyl)aminomethyl-3-phenyl-1 ,2,3,4-tetrahydronaphthalene;
1-Aminomethyl-5,6-dihydroxy-3-(3'-hydroxyphenyl)-3 ,4-dihydronaphthalene ;
1-Aminomethyl-5,6-dihydroxy-3-(4'-hydroxyphenyl)-3,4-dihydronaphthalene; and
[1R,3S] 1-Aminomethyl-5,6-dihydroxy-3-(3'-hydroxyphenyl)-1,2,3,4-tetrahydronaphthalene; 1-Aminomethyl-5,6-dihydroxy-3-phenylnaphthalene;
[1R,3S] 1-Aminomethyl-3-benzyloxymethyl-3,4-dihydro-5,6-dihydroxy-1H-2-benzopyran;
[1R,3S] 1-Aminomethyl-3-cydooctyl-3,4-dihydro-5,6-dihydroxy-1H-2-benzopyran;
[1R,8S, 9aR] 1-Amino-5,6-dihydroxy-2,3,7,8,9,9a-hexahydro-8-phenyl-phenalene;
[1S, 8S, 9aR] 1-Amino-5,6-dihydroxy-2,3,7,8,9,9a-hexahydro-8-phenyl-phenalene; 6,7-Dihydroxy-4-phenyl-2,3,4,5-tetrahydro-1 H-benz[e]isoindole;
[1R*, 3S^{*}] 3-(1 '-Adamantyl)-1-aminomethyl-3,4-dihydro-5,6-dihydroxy-1 H-2-benzopyran; 1-Aminomethyl-5,6-bis(benzoytoxy)-3-phenyl-3,4-dihydronaphthalene; and their pharmaceutically acceptable salts.

The following compounds are representative of the most preferred compounds of the present invention:
[1R,3S] 3-(1'-Adamantyl) -1-aminomethyl-3,4-dihydro-5,6-dihydroxy-1 H-2-benzopyran;
[1 R^{*}, 3S^{*}] 3-(1 '-Adamantyl)-1-aminomethyl-3,4-dihydro-5,6-dihydroxy-1 H-2-benzopyran;
[1 R,3S] 3-(1'-Adamantyl)-3,4-dihydro-5,6-dihydroxy-1-(N-methyl)aminomethyl-1H-2-benzopyran;
[1R,3R] 1-Aminomethyl-3-cyclopentylmethyl-3,4-dihydro-5,6-dihydroxy- 1 H-2-benzopyran;
[1R ,3S] 1-Aminomethyl-3-cyclooctyl-3,4-dihydro-5,6-dihydroxy-1 H-2- benzopyran;
[lR,3R] 1-Aminomethyl-3-n-butyl-3,4-dihydro-5,6-dihydroxy-1H-2-benzopyran;
[1R,3R] 3-n-Butyl-3,4-dihydro-5,6-dihydroxy-1-(N-methyl)aminomethyl-1 H-2-benzopyran;

Certain compounds of this invention exist in optically active forms. The pure d isomers and pure / isomers, as well as mixtures thereof including the racemic mixtures, are contemplated by this invention. Additional asymmetric centers may be present in a substituent such as an alkyl group. All such isomers as well as mixtures thereof are intended to be within the scope of this invention. In particular, stereochemistry of the substituents at the 1 and 3 positions, as shown in Formula (I), can independently be either axial or equatorial unless specifically noted otherwise.

The term "administration" of the dopaminergic agent or composition, as used herein, refers to systemic use as when taken orally, parenterally, by inhalation spray, by nasal, rectal or buccal routes, or topically in dosage form unit formulations containing conventional nontoxic pharmaceutically acceptable carriers, adjuvants and vehicles as desired.

The term "affective disorder" as used herein refers to disorders that are characterized by changes in mood as the primary clinical manifestation, for example, depression.

The following structures in the definition of the compounds of the invention: 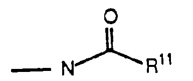 in which R¹¹ is selected from hydrogen, a C₁-C₆ alkyl group, a C₁-C₆ haloalkyl group, a heterocycle, substituted heterocycle, a carbocyclic aryl group or a substituted carbocyclic aryl group are partly exemplified in Table 7.

The term "C₂-C₁₂ alkenyl" is used herein to mean straight or branched chain radicals of two to twelve carbon atoms containing at least one carbon-to-carbon double bond. Representative of such radicals are ethenyl, n-propenyl, isopropenyl, n-butenyl, isobutenyl, 2-ethylhexenyl, n-octenyl and 2,4-dimethylpentenyl.

The term "C₁-C₁₂ alkyl" is used herein to mean straight or branched chain radicals of one to twelve carbon atoms. Representative of such radicals are methyl, ethyl, n-propyl, isopropyl, n-butyl, sec-butyl, isobutyl, t-butyl, 2-ethylhexyl, n-octyl and 2,4-dimethylpentyl.

The term "C₂-C₁₂ alkynyl" is used herein to mean straight or branched chain radicals of two to twelve carbon atoms containing at least one carbon-to-carbon triple bond. Representative of such radicals are ethynyl, n-propynyl, butynyl, 3-ethylhexynyl, n-octynyl and 4-methylpentynyl.

The terms "amino acid" and "dipeptide" refer to a single a-amino acid or two amino acids joined by amide (peptide) bonds. The amino acids are naturally occurring amino acids such as valine, glycine, norvaline, alanine, glutamic acid, glutamine, aspartic acid, leucine, isoleucine, proline, methionine, or phenylalanine.

If not specified, amino acid substituents are optically active and have the L configuration. The term "antipsychotic agent" as used herein refers to drugs used extensively in the symptomatic management of all forms of schizophrenia, organic psychosis, the manic phase of manic depressive illness and other acute idiopathic illnesses and occasionally used in depression or in severe anxiety.

The term "attention deficit disorder" refers to a recently classified pediatric neuropsychiatric disorder characterized by inattention, impulsivity, distractibility and sometimes hyperactivity, which replaces the less formal diagnoses of hyperactivity syndrome, hyperkinetic syndrome, minimal brain dysfunction and specific learning disability. The disorder is prevalent among pre-adolescent children and is reflected in poor school performance and social behavior and has been described in experimental reports of impaired perceptual, cognitive and motor function.

The term "C₅-Cᵢₒ carbocyclic aryl" as used herein refers to aromatic radicals having five to ten carbon atoms in a single ring system. Further, the single ring system may be substituted to form a multiple fused ring system. Representative examples of aryl substituents are phenyl, 1-naphthyl and 2-naphthyl.

Representative C₅-C₁₀ carbocyclic aryl-substituted alkyl groups include benzyl and phenylethyl groups.

The term "C₅-C₁₀ carbocyclic aryloxy" refers to C₅-C₁₀ carbocyclic aryl groups as defined above linked through oxygen by an ether bond. Examples of such groups include phenoxy and benzyloxy. Examples of substituted carbocyclic aryloxy are halo-substituted phenoxy, hydroxy-substituted phenoxy, lower alkyl substituted phenoxy and phenyl substituted phenoxy.

The term "catechol-protecting groups" as used herein refers to groups used to derivatize catechol hydroxyl oxygen atoms in order to prevent undesired reactions or degradation during a synthesis. The term "protecting group" is well known in the art and refers to substituents on functional groups of compounds undergoing chemical transformation which prevent undesired reactions and degradations during a synthesis; see, for example, TH. Greene, "Protective Groups in Organic Synthesis", John Wiley & Sons, New York (1981 These derivatizing groups can be selected from phenol-protecting groups or they may be selected from those groups which are particularly suitable for the protection of catechols because of the proximity of the two hydroxyl functions on the catechol ring. Commonly used catechol-protecting groups include dimethyl ethers, dibenzyl ethers, cyclohexylidene ketals, methylene acetals, acetonide derivatives, diphenylmethylene ketals, cyclic borate esters, cyclic carbonate esters, cyclic carbamates and the like.

The term "cognitive impairment" refers to a deficiency in any of the aspects of the cognitive (information processing) functions of perceiving, thinking and remembering.

The term "cycloalkyl" as used herein refers to a three- to twelve-carbon monocyclic, bicyclic or tricyclic cyclic group such as cyclopentane, cyclohexane, cycloheptane, cyclooctane, cyclopropane, bicycloheptane, bicyclooctane, adamantane, norbornane, norbornene, camphene and pinene.

The term "dopamine-related cardiovascular disorders" as used herein refers to conditions which can be reversed or improved by administration of dopamine or a dopaminergic agent, either alone or in combination therapy with other classes of cardiovascular agents. The usefulness of dopaminergic agents in cardiovascular diseases, for example in the treatment of shock and congestive heart failure, is based on the known, but incompletely understood, role of dopamine in the cardiovascular system, especially the effects of dopamine on the heart and the ability of dopamine to produce vasoconstriction while maintaining blood flow through renal and mesenteric beds. Also included are other related, potential uses for dopaminergic agents which, because the role of dopamine in the cardiovascular system is presently incompletely defined, are still under investigation, for example use in renal failure.

The term "dopamine-related neurological and psychological disorders" as used herein refers to behavioral disorders, such as psychoses and addictive behavior disorders; affective disorders, such as major depression; and movement disorders such as Parkinson's Disease, Huntington's Disease and Gilles de la Tourette's syndrome; which have been linked, pharmacologically and/or clinically, to either insufficient or excessive functional dopaminergic activity in the CNS. Also included are miscellaneous indications for which dopaminergic agents have been found to be clinically useful. Examples of such indications are disorders characterized by vomiting, such as uremia. gastroenteritis, carcinomatosis, radiation sickness, and emesis caused by a vanety of drugs; intractable hiccough and alcoholic hallucinosis.

The term "fused" is used herein to mean two cyclic groups having at least two atoms in common to both rings.

The term "haloalkyl" refers to a lower alkyl group, as defined below, bearing at least one halogen substituent, for example chloroethyl and trifluoromethyl.

The term "halogen" refers to bromo (Br), chloro (CI), fluoro (F) and iodo (I).

The term "heterocycle" refers to a monocyclic 5- or 6-membered saturated or unsaturated group containing one to two heteroatoms selected from N, O and S the remaining atoms being carbon Examples of heterocycles include furan, tetrahydrofuran, thiophene, pyrrole, pyrrolidine, pyridine, piperidine, pyrazine, pyrimidine, piperazine, morpholine, thiomorpholine, pyrazole, imidazole, oxazole, oxazolidine, isoxazole, isoxazolidine and thiazole.

The term "C₁-C_{S} alkoxy" refers to C₁-C_{S} alkyl group, which is bonded through an oxygen atom. Examples of C₁-C_{S} alkoxy groups are methoxy, ethoxy and t-butoxy.

The term "Ci-C6 alkyl" refers to branched or straight chain alkyl groups comprising one to six carbon atoms, including, but not limited to, methyl, ethyl, propyl, isopropyl, n-butyl, t-butyl and neopentyl.

"Normal dopamine levels" are those levels of dopamine that are found in the brains of control subjects and are usually measured as levels of the dopamine metabolites homovanillic acid (3-methoxy-4-hydroxyphenylacetic acid) and 3,4-dihydroxyphenylacetic acid. Abnormal dopamine levels are those levels that are not within the range of dopamine levels found in the brains of control subjects.

The term "parenteral" as used herein includes intravenous, intramuscular, intraperitoneal, intrasternal, subcutaneous and intraarticular injection and infusion techniques.

By "pharmaceutically acceptable" it is meant those salts, amides and esters which are, within the scope of sound medical judgement, suitable for use in contact with the tissues of humans and lower animals without undue toxicity, irritation, allergic response and the like, and are commensurate with a reasonable benefit/risk ratio, effective for their intended use in the treatment of psychological, neurological, cardiovascular and addictive behavior disorders. Pharmaceutically acceptable salts are well known in the art. For example, S. M Berge, et al. describe pharmaceutically acceptable salts in detail in J. Pharmaceutical Sciences, 66: 1 - 19, 1977. The salts can be prepared in situ during the final isolation and purification of the compounds of Formula (I), or separately by reacting the free base function with a suitable organic acid. Representative acid addition salts include hydrochloride, hydrobromide, sulfate, bisulfate, acetate, oxalate, valerate, oleate, palmitate, stearate, laurate, borate, benzoate, lactate, phosphate, toluenesulfonate, methanesulfonate, citrate, maleate, fumarate, succinate, tartrate, ascorbate, glucoheptonate, lactobionate, lauryl sulfate salts and the like. Representative alkali or alkaline earth metal salts include sodium, calcium, potassium, magnesium salts and the like. Examples of pharmaceutically acceptable, nontoxic amides of the compounds of Formula I include amides derived from C1 to C6 alkyl carboxylic acids wherein the alkyl groups are straight or branched chain, aromatic carboxylic acids such as derivatives of benzoic acid and heterocyclic carboxylic acids such as furan-2-carboxylic acid or nicotinic acid. Amides of the compounds of Formula I may be prepared according to conventional methods. It is understood that amides of the compounds of the present invention include amino acid and polypeptide derivatives of the amines of Formula I.

As used herein, the term "pharmaceutically acceptable carriers" means a non-toxic, inert solid, semi-solid or liquid filler, diluent, encapsulating material or formulation auxillary of any type. Some examples of the materials that can serve as pharmaceutically acceptable carriers are sugars, such as lactose, glucose and sucrose; starches such as corn starch and potato starch; cellulose and its derivatives such as sodium carboxymethyl cellulose, ethyl cellulose and cellulose acetate; powdered tragacanth; malt; gelatin; talc; excipients such as cocoa butter and suppository waxes; oils such as peanut oil, cottonseed oil, safflower oil, sesame oil, olive oil, com oil and soybean oil; glycols, such as propylene glycol; polyols such as glycerin, sorbitol, mannitol and polyethylene glycol; esters such as ethyl oleate and ethyl laurate; agar; buffering agents such as magnesium hydroxide and aluminum hydroxide; alginic acid; pyrogen-free water; isotonic saline; Ringer's solution; ethyl alcohol and phosphate buffer solutions, as well as other non-toxic compatible substances used in pharmaceutical formulations. Wetting agents, emulsifiers and lubricants such as sodium lauryl sulfate and magnesium stearate, as well as coloring agents, releasing agents, coating agents, sweetening, flavoring and perfuming agents, preservatives and antioxidants can also be present in the composition, according to the judgement of the formulator. Examples of pharmaceutically acceptable antioxidants include water soluble antioxidants such as ascorbic acid, cysteine hydrochloride, sodium bisulfite, sodium metabisulfite, sodium sulfite, and the like; oil soluble antioxidants such as ascorbyl palmitate, butylated hydroxyanisole (BHA), butylated hydroxytoluene (BHT), lecithin, propyl gallate, alpha-tocopherol and the like; and the metal chelating agents such as citric acid, ethylenediamine tetraacetic acid (EDTA), sorbitol, tartaric acid, phosphoric acid and the like.

The term "readily cleavable group" is used herein to mean substituents which are rapidly cleaved in vivo, for example by hydrolysis in blood, to yield the parent compounds of the Formula (I). Readily cleavable groups include those substituents commonly referred to as prodrug moieties. T. Higuchi and V Stella provide a thorough discussion of the prodrug concept in "Pro-drugs as Novel Delivery Systems", Vol 14 of the A.C.S. Symposium Series, American Chemical Society (1975). Examples of readily cleavable groups include acetyl, trimethylacetyl, butanoyl, methyl succinoyl, t-butyl succinoyl, ethoxycarbonyl, methoxycarbonyl, benzoyl and 3-aminocyclohexylidenyl.

The term "spirocycloalkyl" is used herein to mean two cycloalkyl groups bonded to each other in such a way that a single carbon atom is common to both rings.

The term "substance abuse" is used herein to mean periodic or continued self-administration of psychoactive substances in the absence of medical indications and despite the presence of persistent or recurrent social, occupational, psychological or physical problems that the person knows are caused by or may be exacerbated by continued use of the substance.

By a "therapeutically effective amount" of the dopaminergic agent is meant a sufficient amount of the compound to treat dopamine-related disorders at a reasonable benefit/risk ratio applicable to any medical treatment. It will be understood, however, that the total daily usage of the compounds and compositions of the present invention will be decided by the attending physician within the scope of sound medical judgement. The specific therapeutically effective dose level for any particular patient Will depend upon a variety of factors including the disorder being treated and the severity of the disorder; activity of the specific compound employed; the specific composition employed; the age, body weight, general health, sex and diet of the patient; the time of administration, route of administration, and rate of excretion of the specific compound employed; the duration of the treatment; drugs used in combination or coincidently with the specific compound employed; and like factors well known in the medical arts.

The total daily dose of the compounds of this invention administered to a host in single or in divided doses can be in amounts, for example, from 0.01 to 25 mg/kg body weight or more usually from 0.1 to 15 mg/kg body weight. Single dose compositions may contain such amounts or submultiples thereof to make up the daily dose. In general, treatment regimens according to the present invention comprise administration to a patient in need of such treatment from about 10 mg to about 1000 mg of the compound(s) of this invention per day in multiple doses or in a single dose of from 10 mg to 1000 mg.

The compounds of the present invention may be administered alone or in combination or in concurrent therapy with other agents which effect the dopaminergic system such as L-dopa, amantadine, apomorphine or bromocryptine; and with cholinergic agents, for example, benztropine, biperiden, ethopromazine, procyclidine, trihexylphenidyl and the like. The compounds of the present invention may also be co-administered with agents, for example enzyme inhibitors, which block their metabolic transformation outside the CNS.

This invention also provides pharmaceutical compositions in unit dosage forms, comprising a therapeutically effective amount of a compound (or compounds) of this invention in combination with a conventional pharmaceutical carrier.

Injectable preparations, for example, sterile injectable aqueous or oleaginous suspensions may be formulated according to the known art using suitable dispersing or wetting agents and suspending agents. The sterile injectable preparation may also be a sterile injectable solution, suspension or emulsion in a nontoxic parenterally acceptable diluent or solvent, for example, as a solution in 1,3-butanediol. Among the acceptable vehicles and solvents that may be employed are water, Ringer's solution, U.S.P. and isotonic sodium chloride solution. In addition, sterile, fixed oils are conventionally employed as a solvent or suspending medium. For this purpose any bland fixed oil can be employed including synthetic mono- or diglycerides. In addition, fatty acids such as oleic acid are used in the preparation of injectables.

The injectable formulation can be sterilized, for example, by filtration through a bacteria-retaining filter, or by incorporating sterilizing agents in the form of sterile solid compositions which can be dissolved or dispersed in sterile water or other sterile injectable medium just prior to use.

In order to prolong the effect of a drug, it is often desirable to slow the absorption of a drug from subcutaneous or intramuscular injection. The most common way to accomplish this is to inject a suspension of crystalline or amorphous material with poor water solubility The rate of absorption of the drug becomes dependent on the rate of dissolution of the drug which is, in turn, dependent on the physical state of the drug, for example, the crystal size and the crystalline form. Another approach to delaying absorption of a drug is to administer the drug as a solution or suspension in oil. Injectable depot forms can also be made by forming microcapsule matrices of drugs and biodegradable polymers such as polylactide-polyglycolide. Depending on the ratio of drug to polymer and the composition of the polymer, the rate of drug release can be controlled. Examples of other biodegradable polymers include polyorthoesters and polyanhydrides. The depot injectables can also be made by entrapping the drug in liposomes or microemulsions which are compatible with body tissues

Suppositories for rectal administration of the drug can be prepared by mixing the drug with a suitable nonirritating excipient such as cocoa butter and polyethylene glycol which are solid at ordinary temperature but liquid at the rectal temperature and will therefore melt in the rectum and release the drug.

Solid dosage forms for oral administration may include capsules, tablets, pills, powders, prills and granules. In such solid dosage forms the active compound may be admixed with at least one inert diluent such as sucrose, lactose or starch. Such dosage forms may also comprise, as is normal practice, additional substances other than inert diluents, e.g., tableting lubricants and othertableting aids such as magnesium stearate and microcrystalline cellulose. In the case of capsules, tablets and pills, the dosage forms may also comprise buffering agents. Tablets and pills can additionally be prepared with enteric coatings and other release-controlling coatings.

Solid compositions of a similar type may also be employed as fillers in soft and hard-filled gelatin capsules using such exipients as lactose or milk sugar as well as high molecular weight polyethylene glycols and the like.

Liquid dosage forms for oral administration may include pharmaceutically acceptable emulsions, microemulsions, solutions, suspensions, syrups and elixirs containing inert diluents commonly used in the art such as water. Such compositions may also comprise adjuvants, such as wetting agents; emulsifying and suspending agents; sweetening, flavoring and perfuming agents.

If desired, the compounds of the present invention can be incorporated into slow release or targeted delivery systems such as polymer matrices, liposomes and microspheres. They may be sterilized, for example, by filtration through a bacteria-retaining filter, or by incorporating sterilizing agents in the form of sterile solid compositions which can dissolve in sterile water, or some other sterile injectable medium immediately before use.

The active compounds can also be in micro-encapsulated form with one or more excipients as noted above. The solid dosage forms of tablets, dragees, capsules, pills, and granules can be prepared with coatings and shells such as enteric coatings and other coatings well known in the pharmaceutical formulating art. They may optionally contain opacifying agents and can also be of a composition that they release the active ingredient(s) only, or preferably, in a certain part of the intestinal tract, optionally in a delayed manner. Examples of embedding compositions which can be used include polymeric substances and waxes.

Dosage forms for topical or transdermal administration of a compound of this invention further include ointments, pastes, creams, lotions, gels, powders, solutions, sprays, inhalants or patches. The active component is admixed under sterile conditions with a pharmaceutically acceptable carrier and any needed preservatives or buffers as may be required. Ophthalmic formulations, ear drops, eye ointments, powders and solutions are also contemplated as being within the scope of this invention.

The ointments, pastes, creams and gels may contain, in addition to an active compound of this invention, excipients such as animal and vegetable fats, oils, waxes, paraffins, starch, tragacanth, cellulose derivatives, polyethylene glycols, silicones, bentonites, silicic acid, talc and zinc oxide, or mixtures thereof.

Powders and sprays can contain, in addition to the compounds of this invention, excipients such as lactose, talc, silicic acid, aluminum hydroxide, calcium silicates and polyamide powder, or mixtures of these substances. Sprays can additionally contain customary propellants such as chlorofluorohydrocarbons.

Transdermal patches have the added advantage of providing controlled delivery of a compound to the body. Such dosage forms can be made by dissolving or dispersing the compound in the proper medium. Absorption enhancers can also be used to increase the flux of the compound across the skin. The rate can be controlled by either providing a rate controlling membrane or by dispersing the compound in a polymer matrix or gel.

In general, the compounds of this invention are synthesized by reaction schemes I through XVI as illustrated below. It should be understood that RL R⁸ as used herein correspond to the R groups identified by Formula (I). The oxygens of the catechol groups can be derivatized with "protecting groups" or "leaving groups" which are known in the art and can be prepared by conventional methods. These derivatizing groups can be selected from among phenol derivatives and derivatives which are suitable to catechols because of the proximity of the two hydroxyl functions. Commonly used phenol derivatives are ethers, for example alkyl, alkenyl, and cycloalkyl ethers (such as methyl, isopropyl, t-butyl, cyclopropylmethyl, cyclohexyl, allyl ethers and the like); alkoxyalkyl ethers such as methoxymethyl or methoxyethoxymethyl ether and the like; alkylthioalkyl ethers such as methylthiomethyl ether; tetrahydropyranyl ethers, arylalkyl ethers (such as benzyl, o-nitrobenzyl, 9-anthrylmethyl, 4-picolyl ethers and the like); trialkylsilyl ethers such as trimethylsilyl, triethylsilyl, t-butyldimethylsilyl ethers and the like; alkyl esters such as acetates, propionates, n-butyrates, isobutyrates, trimethylacetates, benzoates and the like; substituted alkyl esters such as 3-(methoxycarbonyl)propionate, 3-aminopro- pionate, 3-(t-butoxycarbonyl)propionate and the like; carbonates such as methyl ethyl, 2,2,2-trichloroethyl, vinyl, benzyl and the like; carbamates such as methyl, isobutyl, phenyl, benzyl, dimethyl, and the like; and sulfonates such as methanesulfonate, trifluoromethanesulfonate, toluenesulfonate and the like. Commonly used catechol derivatives include cyclic acetals and ketals such as methylene acetal, acetonide derivatives, cyclohexylidene ketal, diphenylmethylene ketal and the like; cyclic esters such as borate esters, cyclic carbonate esters and the like.

The condensation of amino groups (such as those present in the certain of the compounds of this invention) with amino acids and peptides may be effected in accordance with conventional condensation methods such as the azide method, the mixed acid anhydride method, the DCC (dicyclohexylcarbodiimide) method, the active ester method ( p-nitrophenyl ester method, N-hydroxysuccinic acid imide ester method, cyanomethyl ester method and the like), the Woodward reagent K method, the DCC-HOBT (1-hydroxy-benzotriazole) method and the like. Classical methods for amino acid condensation reactions are described in "Peptide Synthesis" Second Edition, M. Bodansky, YS. Klausner and M.A. Ondetti (1976).

As in conventional peptide synthesis, branched chain amino and carboxyl groups at alpha and omega positions in amino acids may be protected and deprotected if necessary. The protecting groups for amino groups which can be used involve, for example, benzyloxycarbonyl (Z), o-chloro-benzyloxycarbonyl ((2-CI)Z), p-nitrobenzyloxycarbonyl (Z(N02)), p-methoxybenzyloxycarbonyl (Z(OMe)), t-butoxycarbonyl (Boc), t-amyloxycarbonyl (Aoc), isobornealoxycarbonyl, adamantyloxycarbonyl (Adoc), 2-(4-biphenyl)-2-propyloxy carbonyl (Bpoc), 9-fluorenylmethoxycarbonyl (Fmoc), methylsul- fonylethoxy carbonyl (Msc), trifluoroacetyl, phthalyl, formyl, 2-nitrophenylsulfenyl (Nps), diphenylphosphinothioyl (Ppt) and dimethylphosphinothioyl (Mpt).

The examples of protecting groups for carboxyl groups involve, for example, benzyl ester (OBn), cyclohexyl ester, 4-nitrobenzyl ester (OBnN02), t-butyl ester (OtBu), 4-pyridylmethyl ester (OPic) and the like.

In the course of the synthesis of certain of the compounds of the present invention, specific amino acids having functional groups other than amino and carboxyl groups in the branched chain such as arginine, cysteine, serine and the like may be protected, if necessary, with suitable protecting groups. It is preferable that, for example, the guanidino group (N^{G}) in arginine may be protected with nitro, p-toluenesulfonyl (Tos), benzyloxycarbonyl (Z), adamantyloxycarbonyl (Adoc), p-methoxybenzenesulfonyl, 4-methoxy-2,6-dimethyl-benzenesulfonyl (Mts) and the like, and the thiol group in cysteine may be protected with benzyl, p-methoxybenzyl, triphenylmethyl, acetomidomethyl, ethylcarbamyl, 4-methylbenzyl (4-MeBn, 2,4,6,-trimethylbenzyl (Tmb) and the like, and the hydroxy group in serine may be protected with benzyl (Bn), t-butyl, acetyl, tetrahydropyranyl (THP) and the like.

### Scheme IA

The compounds of Formula I A and I Bare synthesized by the method discussed herein. 2, 3-Dihydroxybenzaldehyde (which has the two catechol hydroxy groups protected by, for example, alkyl groups preferably methyl groups) and a substituted acetic acid derivative, such as phenyl acetic acid, are condensed in the presence of a dehydrating agent, such as acetic anhydride, and a proton acceptor such as triethylamine (TEA) to give compound 2. The carboxylic acid (or acid derivative such as the methyl or ethyl ester) of compound 2 is reduced by a reducing agent such as lithium aluminum hydride (LAH) preferably in an ether solvent such as tetrahydrofuran (THF). The leaving group ability of the hydroxyl group of compound 3 is enhanced by derivatizing it with, for example, methanesulfonyl chloride, in the presence of a proton acceptor such as TEA, and it is then converted to the cyano compound 4 by nucleophilic displacement with a salt of cyanic acid such as sodium cyanide in a polar solvent such as dimethyl sulfoxide (DMSO). The cyano group is hydrolyzed to the corresponding carboxylic acid group under basic conditions using, for example, aqueous sodium hydroxide, and the naphthalenone derivative (compound 5) is prepared by intramolecular acylation of the protected catechol ring using a dehydrating agent such as polyphosphoric acid or methanesulfonic acid/ trifluoroacetic acid. Compound 5 is converted to the corresponding cyanohydrin by treatment with a nucleophilic cyano derivative such as trimethylsilylcyanide and the cyano alcohol is reduced to the amine (compound 6) by treatment with a reducing agent such as LAH, preferably in a ether solvent such as diethyl ether. The 1-hydroxyl group is eliminated from compound 6 by heating it under acidic conditions, e.g. in isopropyl alcohol saturated with hydrochloric acid, to produce the dihydronaphthalene derivative (compound 7) Compound I A is produced when the catechol hydroxyl groups of compound 7 are deprotected with, for example, boron tribromide or boron trichloride in an inert solvent such as dichloroethane or methylene chloride. Compound 7 is also hydrogenated to the corresponding tetrahydronaphthalene derivative in the presence of a catalyst such as palladium or platinum on carbon and then deprotected with e.g. boron tribromide or boron trichloride to produce 1 B. In the preferred embodiments of compounds I A and I B, R³ is phenyl or cyclohexyl and X is bromo or chloro.

### Scheme I B

The compounds of Formula I are alternately synthesized by the method discussed herein. 2,3-Dihydroxybenzaldehyde with both the catechol hydroxyl protected as described in Example I and the aldehyde group derivatized as its dithiane is treated with a base such as n-butyl lithium to generate the anion (compound 8), and condensed with an alpha-beta unsaturated acid derivative such as ethyl cinnamate in the presence of dimethyl-2-imidizolidinone to produce compound 9. The dithiane group is removed from compound 9 by treatment with hydrogen in the presence of a catalyst such as Raney nickel and converted to compound 5 as described in Scheme IA. Compound 5 is further converted to IA and IB as described in Scheme lA.

### Scheme II

The compounds of Formulas II A and II Bare prepared by the method illustrated in Scheme II. The naphthalenones of Formula 5 are treated sequentially with a suitable base such as lithium bis(trimethylsilyl)amide and a haloacetic acid ester, for example ethyl bromoacetate, to afford the compounds of Formula 10. Compounds of Formula 10 are converted to compounds of Formula 11 by treatment with diethylaluminum cyanide under anhydrous conditions, followed by cyclization in aqueous mineral acid, for example aqueous hydrochloric acid. Compounds of Formula 11 are reduced using a suitable reagent such as LAH followed by elimination with an acid such as hydrogen chloride in isopropanol to afford compounds of Formula 12. Compounds of Formula 12 are treated with a suitable reagent for removal of the catechol protecting groups, for example boron tribromide, to yield the compounds of Formula II A.

Alternately, compounds of Formula 11 are converted to compounds of Formula 13 by treatment with a mineral acid in anhydrous alcohol. The compounds of Formula 13 are, in turn, converted to compounds of Formula 14 by treatment with magnesium followed by aqueous mineral acid and reduction using a suitable reagent such as LAH. The compounds of Formula 14 are then treated with a suitable reagent for the removal of the catechol protecting groups, for example boron tribromide to afford the compounds of Formula II B.

### Scheme III

Compounds of Formulas III A - III E are synthesized by the methods illustrated in Scheme III. Naphthalenones of Formula 5 are alkylated to afford the compounds of Formula 15 by treatment with a suitable base(for example lithium bis(trimethylsilyl)amide and a suitable alkylating agent such as allyl bromide. The compounds of Formula 15 are converted to the compounds of Formula 16 by sequential treatment with trimethylsilyl cyanide and a suitable reducing agent such as LAH. The compounds of Formula 16 are, in turn, cyclized to the compounds of Formula 17 by treatment with a suitably reactive carbonic acid derivative such as 1,1'-carbonyldiimidazole.

Compounds of Formula III A are prepared by reduction of compounds of Formula 17 with a suitable reagent (for example by hydrogenation using a suitable catalyst such as palladium on carbon), followed by treatment with a suitable reagent for removal of the catechol protecting groups such as boron tribromide resulting in simultaneous elimination of carbon dioxide to afford the desired amines

Compounds of Formula III B are prepared by hydrogenation of compounds of Formula 17 using a suitable catalyst such as palladium hydroxide on carbon, followed by treatment with a suitable reagent for removal of the catechol protecting groups such as boron tribromide.

Compounds of Formulas III C, III D and III E are prepared from compounds of Formula 20. Compounds of Formula 17 are converted to compounds of Formula 20 by hydroboration/oxidation under standard conditions. Compounds of Formula 20 are converted to compounds of Formula III by treatment with 3 equivalents of boron tribromide. Compounds of Formula 20 are converted to compounds of Formula III D by treatment with 4.5 equivalents of boron tribromide. Compounds of Formula 20 are converted to compounds of Formula III E by reductive opening of the oxazolidinone ring followed by treatment with a suitable reagent for removal of the catechol protecting groups such as boron tribromide.

### Scheme IV

The compounds of Formula IV A, IV B and IV C are synthesized by the method discussed herein. A catechol (compound 22 wherein R¹ is selected from alkyl groups such as methyl or both R¹ groups together form a spiro cycloalkyl group such as cyclohexyl) is reacted in the presence of a base, such as n-butyl lithium, with an epoxide such as compound 23 (wherein R⁴ is hydrogen and R³ is preferably selected from cyclohexyl, phenyl, ethyl, p-methoxyphenoxymethyl, phenoxymethyl, o-phenylphenoxymethyl, p-t-butylphenoxymethyl, p-bromophenoxymethyl, adamantyl, benzyl, phenylethyl, n-octyl, n-hexyl, 1-hex-5-enyl, n-decyl, t-butyl or benzyloxymethyl; or R³ and R⁴ together form a spiro cycloalkyl group such as cyclohexyl) to produce compound 24.

Compound 24 can be oxidized to the corresponding ketone with an oxidizing agent such as pyridinium chlorochromate (PCC) and the resultant ketone can be stereoselectively reduced with, for example, B-chlorodiisopinocampheylborane (as described in Example 46) to give the optically active isomers of compound 24.

Compound 24 is condensed with a bromoaldehyde such as bromoacetaldehyde dimethyl acetal or 3-bromopropi- onaldehyde dimethyl acetal to form the substituted benzopyran derivative 26. Compound 26 is converted to compound 27 by treatment with a nucleophilic azide such as lithium azide in a polar solvent such as dimethyl formamide, followed by reduction of the azido compound , for example with LAH. Compound 27 is converted to IV A by generation of the amine salt in acidic solution and deprotection of the catechol hydroxyl groups in acid solution. Compound 27 is converted to compound IV B by treatment with ethyl formate followed by reduction with, for example LAH and generation of the amine salt with deprotection of the catechol hydroxyl groups in acidic solution. Compound 26 is converted to IV C by treatment with an amine such as allyl amine, cyclopropylamine, benzylamine, phenethylamine or pyrrolidine, followed by deprotection of the catechol hydroxyl groups and generation of the amine salt in acidic solution. In the case wherein the epoxide 23 is substituted with a benzyloxymethyl group (i.e. R³ = benzyloxymethyl), R³ is further elaborated as shown in Scheme V

### Scheme V

The compounds of Formula V A, V B, V C, V D and V E are synthesized by the methods illustrated in Scheme V R¹ is defined in Scheme IV. Compound of Formula 28 are alkylated with an epoxide of Formula 29 (wherein Bn is benzyl, z is an integer from 0 to 6, and R¹² is a substituent selected from the group R³) to afford compounds of Formula 30. Compounds of Formula 30 are condensed with N-formylaminoacetaldehyde dimethyl acetal in the presence of a catalyst selected from boron trifluoride etherate, zinc triflate, trimethylsilyl triflate, methanesulfonic acid, p-toluenesulfonic acid and polyphosphoric acid to afford the isochromans of Formula 31. The formyl group is removed and replaced with a t-butyloxycarbonyl protecting group and the hydroxy group is deprotected preferably by hydrogenolysis to afford the compounds of Formula 32.

Compounds of Formula V A are prepared by removal of the amino and catechol protecting groups from the compounds of Formula 32 in acidic solution. Compounds of Formula V B are prepared from the compounds of Formula 32 by the following sequence of reactions: activation of the hydroxymethyl group, for example by reaction with methanesulfonyl chloride; displacement with a nucleophilic azide such as lithium azide to give the azidomethyl compound; followed by reduction of the azido group to give the compounds of Formula 33 and deprotection of the amine and the catechol hydroxyls with an acid such as hydrochloric acid in alcohol.

Compounds of Formula V C are prepared from the compounds of Formula 33 by acetylation of the free amino group followed by simultaneous removal of the amine and catechol protecting groups in acidic solution.

Alternately, compounds of Formula 31 are converted to compounds of Formula 34 by hydrogenolysis followed by activation of the hydroxymethyl group, for example by reaction with methanesulfonyl chloride and displacement with a nucleophilic azide such as lithium azide. The compounds of Formula 34 are, in turn, converted to compounds of Formula V D by reduction of the formyl group and the azido group followed by removal of the catechol protecting groups in acidic solution. The compounds of Formula 34 are also converted to the compounds of Formula V E by reduction of the azido group, formylation of the free amino, simultaneous reduction of both formyl groups to methylamino groups and treatment with a suitable reagent for the removal of the catechol protecting groups.

Alternately, compounds of Formula 32 are converted to V F by activation of the 3-hydroxymethyl group, for example by reaction with methanesulfonyl chloride followed by displacement with a nucleophilic amine, NHR⁹R¹⁰, in which R⁹ and R¹⁰ are independently selected from H and lower alkyl or R⁹ and R¹⁰ together form a ring containing a nitrogen atom such as pyrrolinyl or piperidinyl or morpholino, followed by deprotection of the amino group and the catechol hydroxyls in acidic solution.

### Scheme VI

According to reaction Scheme VIA, compounds of Formula 35, wherein R^{l} is as defined in Scheme IV, are converted to compounds of Formula 36 by treatment with oxalyl chloride followed by treatment with O-methyl N-methyl hydroxylamine. Compounds of Formula 36 reacted with furan in the presence of a suitable base such as n-butyl lithium to afford the compounds of Formula 37. The furan ring and the ketone are then reduced, for example by hydrogenation using a suitable catalyst such as palladium on carbon to afford the compounds of Formula 24. Compounds of Formula 24 are valuable intermediates and can be converted to the isochroman and isothiochroman compounds of the present invention by any of the methods shown in Schemes lV, V, VI and VIII.

According to Scheme VI B, compounds of Formula 38, wherein R⁷ is as defined in Scheme lV, are converted to compounds of Formula 24 by treatment with lithium acetylide-ethylenediamine complex in a polar solvent such as DMSO. Compounds of Formula 24 are valuable intermediates and can be converted to the isochroman and isothiochroman compounds of the present invention by any of the methods shown in Schemes IV, V, VII and VII I.

### Scheme VII

Compounds of Formulas VII A - VII D are synthesized by the methods illustrated in Scheme VII. Compounds of Formula 41 are prepared by oxidation of Compounds of Formula 24 with a suitable reagent such as pyridinium chlorochromate. Compounds of Formula 41 are treated with formaldehyde in the presence of a suitable base such as sodium hydroxide to afford the hydroxymethyl compounds of Formula 42. The compounds of Formula 42 are, in turn, reacted with a reagent suitable for protection of the hydroxyl group, for example (1,1 -dimethyl)ethyldiphenylsilyl chloride in the presence of a suitable base, for example dimethylaminopyridine (DMAP), followed by treatment with a suitable reducing agent such as sodium borohydride to afford the compounds of Formula 43. The compounds of Formula 43 are converted to the isochromans of Formula 44 by treatment with N-formylaminoacetaldehyde dimethyl acetal. The formyl group is reduced with a suitable reagent such as LAH and the hydroxy group is treated with an appropriate reagent for removal of the silyl protecting group, for example tetra-n-butylammonium fluoride, and finally the catechol hydroxyl groups are deprotected to afford the compounds of Formula VII A.

The compounds of Formula VI Bare prepared by treatment of the compounds of Formula 44 with a suitable reagent for the removal of the silyl protecting group, followed by simultaneous hydrolysis of the formyl group and the catechol hydroxy protecting groups.

Alternately, the compounds of Formula 43 are cyclized to the compounds of Formula 45 by condensation with bromoacetaldehyde dimethyl acetate followed by displacement of the bromine atom with a nucleophilic azido compound such as lithium azide to afford the compounds of Formula 45. The compounds of Formula 45 are converted to the compounds of Formula Vll C by deprotection followed by activation of the hydroxymethyl group, for example by reaction with methanesulfonyl chloride, and displacement with a nucleophilic azide such as lithium azide to give the azidomethyl compound, followed by reduction of both azido groups with LAH and deprotection of the catechol hydroxyls with an acid such as hydrochloric acid in alcohol. The compounds of Formula 45 are converted to the compounds of Formula VII D by the following series of reactions: 1) reduction of the azido group with a suitable reagent, for example LAH, 2) protection of the amino group with, for example a t-butylcarbonyl group, 3) deprotection and 4) activation of the hydroxy group by treatment with a suitable reagent, for example methanesulfonyl chloride, 5) displacement of the activated hydroxy group with a nucleophilic azido compound such as lithium azide, 6) reduction of the azido group with a suitable reagent such as LAH, 7) acetylation of the free amino group, 8) removal of the amino protecting group and 8) removal of the catechol hydroxy protecting groups.

### Scheme VIII

The compounds of Formula VIII are synthesized by the methods illustrated in Scheme VIII. Intermediates of Formula 24 are converted to the corresponding thio compounds of Formula 40 by treatment with triphenylphosphine and diiso- propylazodicarboxylate, followed by treatment with thioacetic acid to afford an intermediate thiolacetate which was reduced using a suitable reagent such as LAH. The compounds of Formula 40 are valuable intermediates and are converted to the compounds of Formula VIII and the thio equivalents of compounds IV, V, VI and VII by the methods illustrated in Schemes IV - VI I.

### Scheme IX

The compounds of Formula IX A and IX B are synthesized by the method discussed herein. R¹ R² and R³ are defined in Scheme I. Compound 5 is converted to the cyanohydrin by treatment with a nucleophilic cyano derivative such as trimethylsilyl cyanide in the presence of a catalyst such as aluminum trichloride. The cyanohydrin is dehydrated to the α, β, -unsaturated nitrile by treatment with a dehydrating agent such as TFA/p-toluenesulfonic acid and the unsaturated nitrile reduced to the saturated nitrile (compound 46) by treatment with a reducing agent such as sodium borohydride. The nitrile group is hydrolyzed to a carboxylic acid group (compound 47) and the acid converted to the N-methoxy-N-methyl amide 48 by sequential treatment with a chlorinating agent, such as oxalyl chloride, to generate the acid chloride, and N-methoxymethylamine. Compound 48 is converted to a mixture of the diastereomeric pyrrolidinyl derivatives 49 and 50 by treatment with 2,2,5,5-tetramethyl-1-aza-2,5-disilacydopentane-1-propyl magnesium bromide followed by reduction with a reducing agent such as sodium borohydride, and the diastereomers are separated chromatographically. The separated isomers 49 and 50 are converted to IX A and IX B, respectively, by treatment with boron trihalide, preferably boron tribromide.

### Scheme X

The compounds X A and X B are synthesized by the method discussed herein. R^{1 -} R³ are defined in Scheme I. Compound 5 is converted to compound 51 by treatment with dimethyl succinate in the presence of a base such as potassium t-butoxide. Compound 51 is reduced to the corresponding 1,2,3,4-tetrahydronaphthalene and the tricyclic ring system is formed by treating compound 51, with a dehydrating agent such as polyphosphoric acid. Four isomeric products are obtained. Two of the isomers, compounds 52 and 53, are carried on to X A and X B, respectively Reduction of the 3-keto group of compounds 52 and 53 with, for example hydrogen in the presence of a catalyst such as palladium on carbon support is followed by hydrolysis of the ester in basic solution to give compounds 54 and 55, respectively. Compounds 54 and 55 are each treated with diphenylphosphoryl azide and benzyl alcohol in the presence of a base such as triethylamine to give the carbobenzyloxy protected amino derivatives, which are deprotected by hydrogenolysis using, for example, palladium on carbon support as a catalyst, and demethylation using, for example, boron tribromide to give X A and X B

### Scheme XI

The compounds of Formula XI are synthesized by the method described herein. R^{1 -} R³ are defined in Scheme I. Compound 5 is converted to the a-bromoketone by treatment with a brominating agent such as phenyltrimethylammonium tribromide The bromide undergoes nucleophilic displacement, for example, with the anion of thiophenol to give the a-thiophenylketone compound 56. The ketone is reduced to the alcohol with a reducing agent such as sodium borohydride and the hydroxy group is eliminated with a dehydrating agent such as p-toluenesulfonic acid to give the thio-enolether. The sulfur atom of the thio-enolether is oxidized to the sulfoxide with an oxidizing agent such as mCPBA to give compound 57. The amine component is made by a nucleophilic displacement on chloromethyltrimethylsilane by an amine (compound 58), such as benzylamine. The imine is formed by treatment of the amine with an aldehyde such as formaldehyde and then an alcohol such as methanol, is added to form the alkoxymethyl amine compound 60. Compound 60 is then reacted with the sulfoxide (compound 57) in the presence of an acid, such as TFA to generate the azomethine ylid in situ which traps the activated double bond of the a, β-unsaturated sulfoxide to give a 1,3-dipolar addition adduct which, on heating, spontaneously undergoes elimination to give the cyclization/elimination product, compound 61. The nitrogen can be deprotected by treatment with an acylating agent, such as 1-chloroethylchloroformate followed by acyl group removal with a nucleophile, such as methanol to give compound 62. The catechol is deprotected by treatment with a boron trihalide, preferably boron tribromide to give XI.

### Scheme XII

The compounds of Formulas XII A, XI I B and XII C are synthesized by the methods described herein. R¹ and R³ are defined in Scheme I. Compounds of the Formula 12 are reduced by catalytic hydrogenation using a suitable catalyst such as palladium hydroxide to afford the compounds of Formula 63. The compounds of Formula 63 are treated with a suitable reagent for protecting the amino group, for example benzyloxycarbonyl chloride, followed by a suitable reagent for activating the hydroxyl group such as methanesulfonyl chloride to afford the compounds of Formula 64. The compounds of Formula 64 are, in turn, cyclized by treatment with a suitable base for example sodium hydride in DMF and deprotected with acid, for example by treatment with hydrogen bromide in acetic acid, to afford the compounds of Formula XII B.

Alternately, the compounds of Formula 12 are converted to the compounds of Formula 65 by treatment with a suitable reagent for protecting the amino group, for example benzyloxycarbonyl chloride, followed by a suitable reagent for activating the hydroxyl group such as methanesulfonyl chloride to afford the compounds of Formula 65. The compounds of Formula 65 are, in turn, cyclized by treatment with a suitable base for example sodium hydride in DMF and deprotected with acid, for example by treatment with hydrogen bromide in acetic acid, to afford the compounds of Formula XII A.

The compounds of Formula 11 are treated with a suitable reducing agent such as LAH to afford the compounds of Formula 66. The compounds of Formula 66 are treated with an appropriately reactive carbonic acid derivative, for example carbonyl diimidazole to afford the oxazolidinones of Formula 67. The compounds of Formula 67 are reduced by catalytic hydrogenation using a suitable catalyst such as palladium hydroxide to afford the compounds of Formula 68. The compounds of Formula 68 are treated with a suitable reagent for protecting the amino group, for example benzyloxycarbonyl chloride, followed by a suitable reagent for activating the hydroxyl group such as methanesulfonyl chloride to afford the compounds of Formula 69. The compounds of Formula 69 are, in turn, cyclized by treatment with a suitable base for example sodium hydride in DMF and deprotected with acid, for example by treatment with hydrogen bromide in acetic acid, to afford the compounds of Formula XII C.

### Scheme XIII

The compounds of Formulas XIII A and XIII B are synthesized by the methods described herein. R¹ and R³ are defined in Scheme I. The compounds of Formula 20 are treated with a suitable acid such as hydrogen chloride in a suitable solvent, for example isopropyl alcohol or ethyl alcohol or diethyl ether, in order to open the oxazolidinone ring with the elimination of carbon dioxide. The resultant amino alcohols are treated with a suitable reagent for protecting the amino group, for example benzyloxycarbonyl chloride, followed by a suitable reagent for activating the hydroxyl group such as methanesulfonyl chloride to afford the compounds of Formula 70. The compounds of Formula 70 are, in turn, cyclized by treatment with a suitable base for example sodium hydride in DMF and deprotected with acid, for example by treatment with hydrogen bromide in acetic acid, to afford the compounds of Formula XIII A.

The compounds of Formula 21 are treated with a suitable reagent for protecting the amino group, for example benzyloxycarbonyl chloride, followed by a suitable reagent for activating the hydroxyl group such as methanesulfonyl chloride to afford the compounds of Formula 71. The compounds of Formula 71 are, in turn, cyclized by treatment with a suitable base for example sodium hydride in DMF and deprotected with acid, for example by treatment with hydrogen bromide in acetic acid, to afford the compounds of Formula XIII B.

### Scheme XIV

The compounds of Formulas XIV A and XIV B are synthesized by the methods described herein. Compounds of Formula 5 are converted to compounds of Formula 72 by treatment with dimethyl malonate in the presence of a base such as potassium t-butoxide. The tricyclic ring system is formed by treating a compound of Formula 72, with an acid such as polyphosphoric acid, followed by reduction of the keto group with, for example, triethylsilane in trifluoroacetic acid to afford a compound of Formula 73. Hydrolysis of the ester group in basic solution affords compounds of Formula 74. Compounds of Formula 74 are treated with diphenylphosphoryl azide and benzyl alcohol in the presence of a base such as triethylamine to give the carbobenzyloxy protected amino derivatives, which are deprotected along with the catechol hydroxyl groups using, for example, hydrogen bromide in acetic acid to give XIV A.

Alternately the compounds of Formula 72 are reduced by catalytic hydrogenation using a suitable catalyst such as palladium hydroxide to afford the compounds of Formula 75. The compounds of Formula 75 are, in turn, converted to the compounds of Formula XIV B by the same series of chemical tranformations described above for the conversion of compounds of Formula 72 to compounds of Formula XIV A.

### Scheme XV

The compounds of Formulas XV A and XV B are synthesized by the methods described herein. R^{l} and R³ are defined in Scheme I. The compounds of Formula 5 are converted to compounds of Formula 51 by treatment with dimethyl succinate in the presence of a base such as potassium t-butoxide. The compounds of Formula 51 are, in turn, treated with a suitable reducing agent for reducing the acid, for example borane, to afford the corresponding hydroxy compounds of Formula 78. The compounds of Formula 78 are treated with a suitable reagent to activate the hydroxyl group, for example methanesulfonyl chloride, followed by displacement with a nucleophic cyano derivative such as sodium cyanide to afford the compounds of Formula 79. The tricyclic ring structure is formed by an intramolecular Houben-Hoesch reaction using hydrogen chloride and zinc dichloride to give the compounds of Formula 80. Reduction of the keto group using, for example, triethylsilane in trifluoroacetic acid, followed by hydrolysis of the ester group in basic solution affords compounds of Formula 81. Compounds of Formula 81 are treated with diphenylphosphoryl azide and benzyl alcohol in the presence of a base such as triethylamine to give the carbobenzyloxy protected amino derivatives, which are deprotected along with the catechol hydroxyls using, for example, hydrogen bromide in acetic acid, to give XV A.

Alternately the compounds of Formula 78 are reduced by catalytic hydrogenation using a suitable catalyst such as palladium hydroxide to afford the compounds of Formula 82. The compounds of Formula 82 are, in turn, converted to the compounds of Formula XV B by the same series of chemical tranformations described above for the conversion of compounds of Formula 78 to compounds of Formula XIV A.

### Scheme XVI

The process illustrated in Reaction Scheme XVI is a novel and practical method for ring closure in the synthesis of the isochroman and thioisochroman compounds of the present invention. Compounds of the Formula 24 are condensed with either N-formylamino-acetaldehyde dimethyl acetal or 3-(N-formylamino)-propionaldehyde dimethyl acetal in the presence of an acid catalyst to afford the compounds of Formula XVI. The reaction is carried out in an inert solvent, for example a chlorinated solvent such as methylene chloride or 1,2-dichloroethane, an ether solvent such as diethyl ether or THF, or a polar aprotic solvent such as acetonitrile. The reaction is carried out in the temperature range of from about 0°C to about 100°C. The preferred reaction temperature is determined by the choice of solvent, the choice of catalyst and the amount of catalyst present. In general, reactions in chlorinated solvents are carried out at lower temperatures than reactions in more polar solvents and larger amounts of catalyst require lower reaction temperatures.The formylamino reagent (compounds of Formula86) is present in the reaction mixture at from about 1 to about 4 equivalents, preferably from about 1.5 to about 2.0 equivalents. The catalyst is preferably selected from boron trifluoride etherate, trimethylsilyl triflate, zinc triflate, polyphosphoric acid, methanesulfonic acid and p-toluene sulfonic acid. The amount of catalyst present in the reaction mixture depends on the catalyst used, the solvent and reaction temperature. Generally, the catalyst is present in the range of from about 1 mole % to about 3 equivalents. Most preferably the reaction is carried with either 1 mole % trimethylsilyl triflate in refluxing acetonitrile or with 5 mole % boron trifluoride etherate in refluxing acetonitrile. The reactions are monitored by TLC analysis to determine the optimum reaction time for good yields with minimum product degradation and this time varies with choice of solvent, catalyst and reaction temperature.

The compounds of Formula XVI are valuable intermediates in the synthesis of the compounds of Formula I in which A is an oxygen or a sulfur atom (isochromans and thioisochromans). Compounds of Formula XVI can be either hydrolyzed or reduced to give certain compounds of Formula I directly. Alternately, the formyl group can be replaced with an amino protecting group and the intermediate further modified to give other compounds of Formula I, for example as illustrated in Scheme V below.

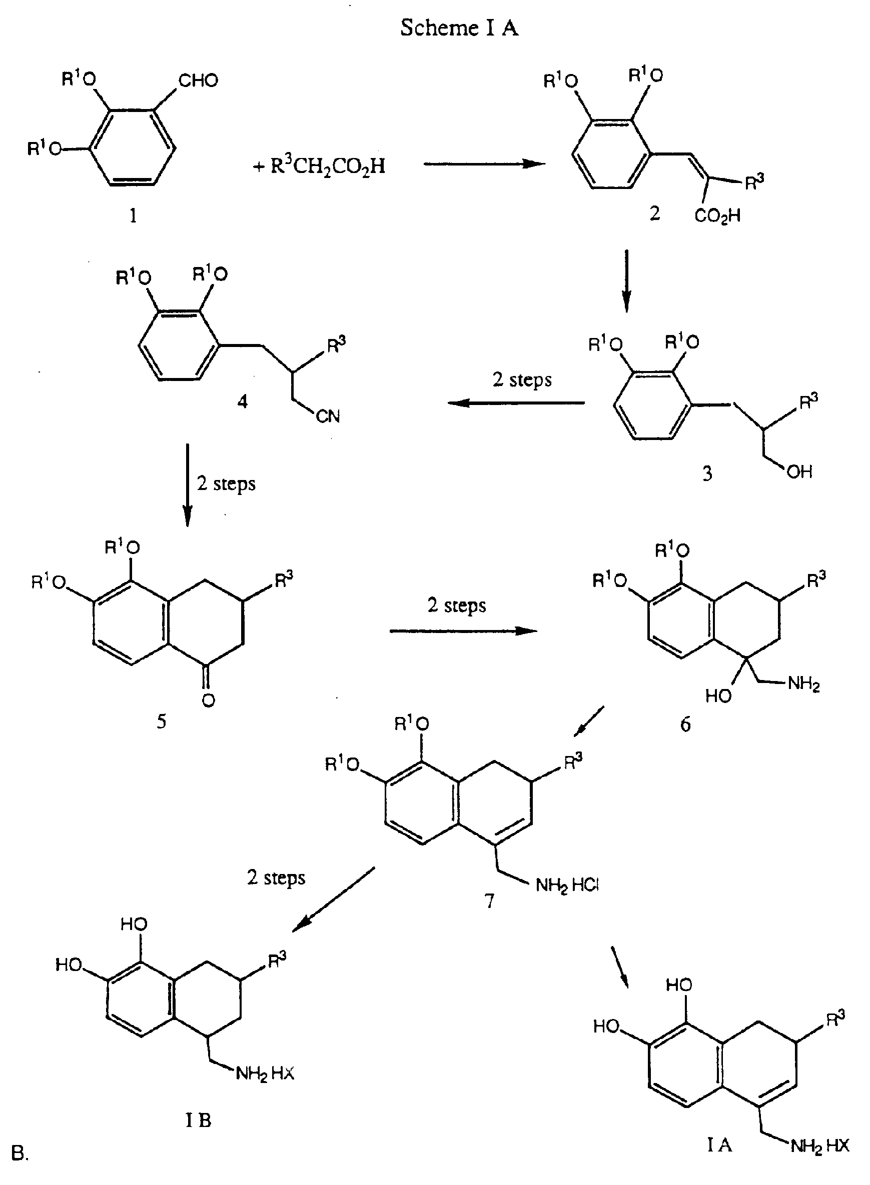

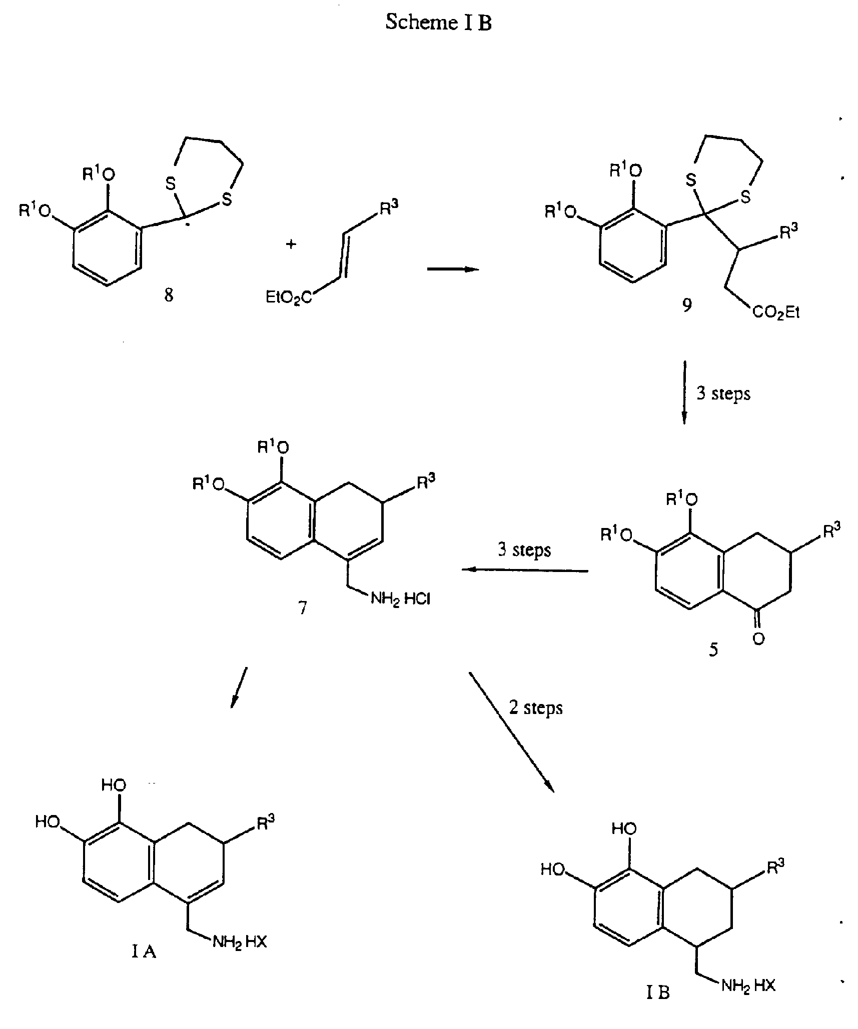

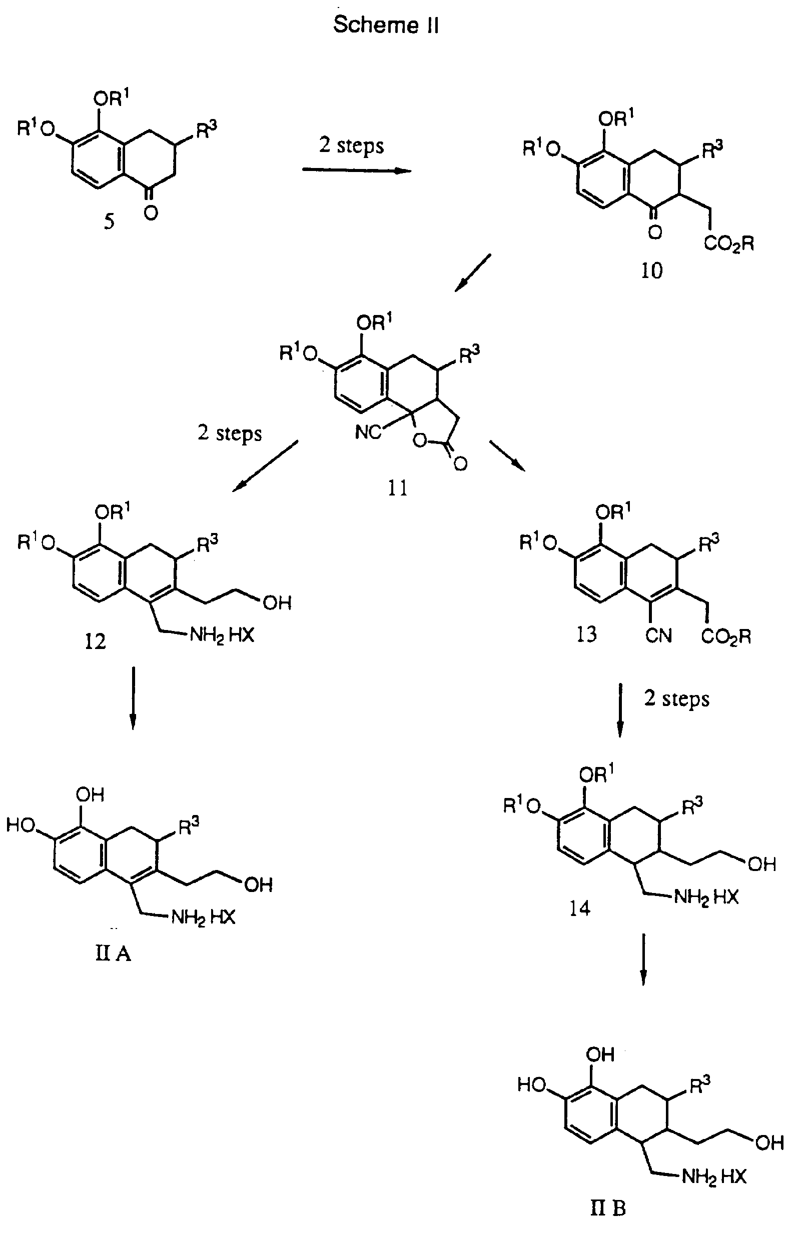

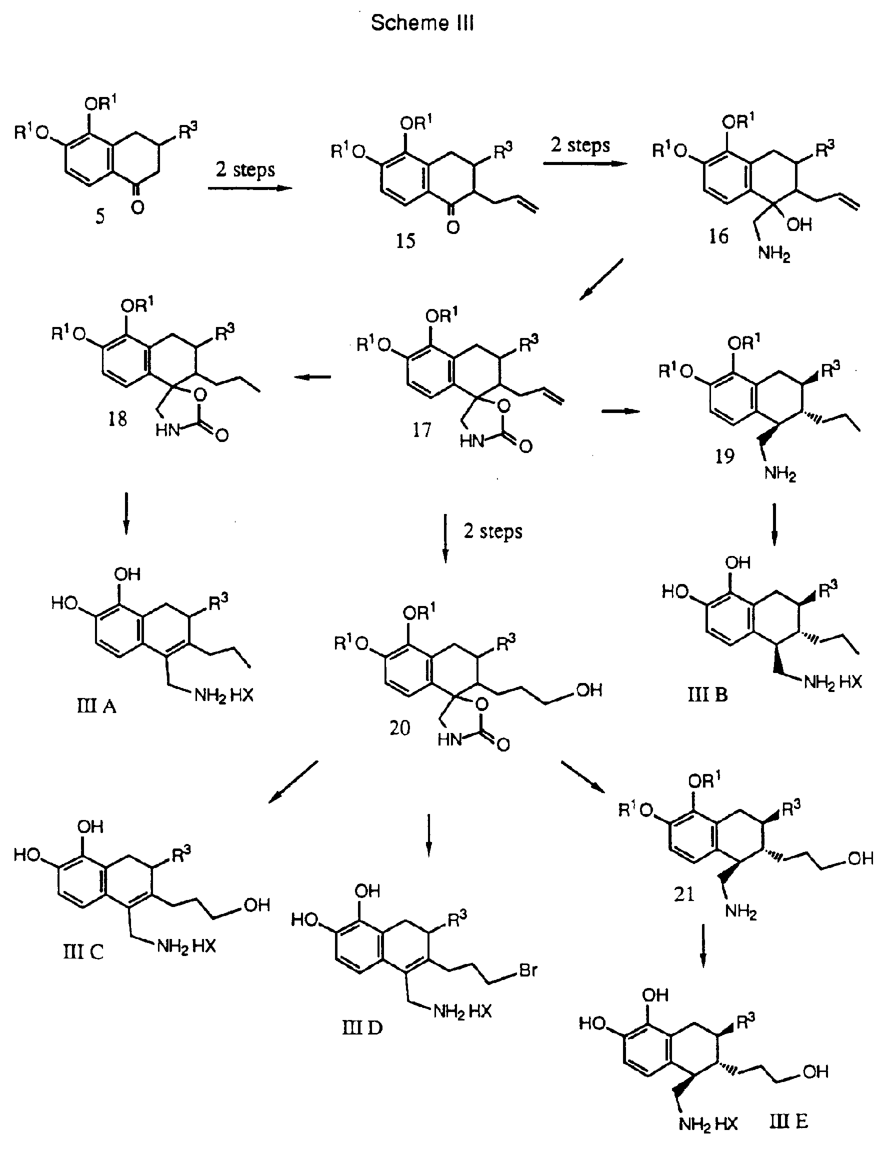

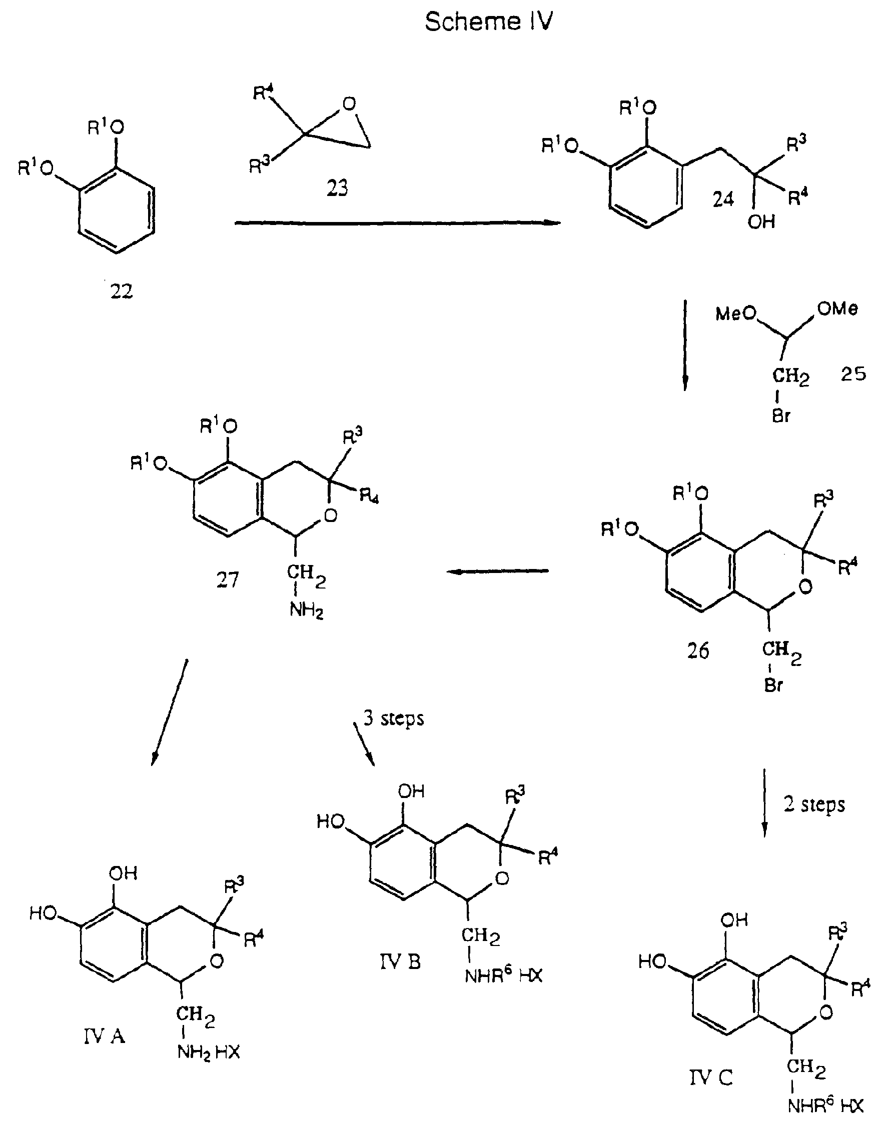

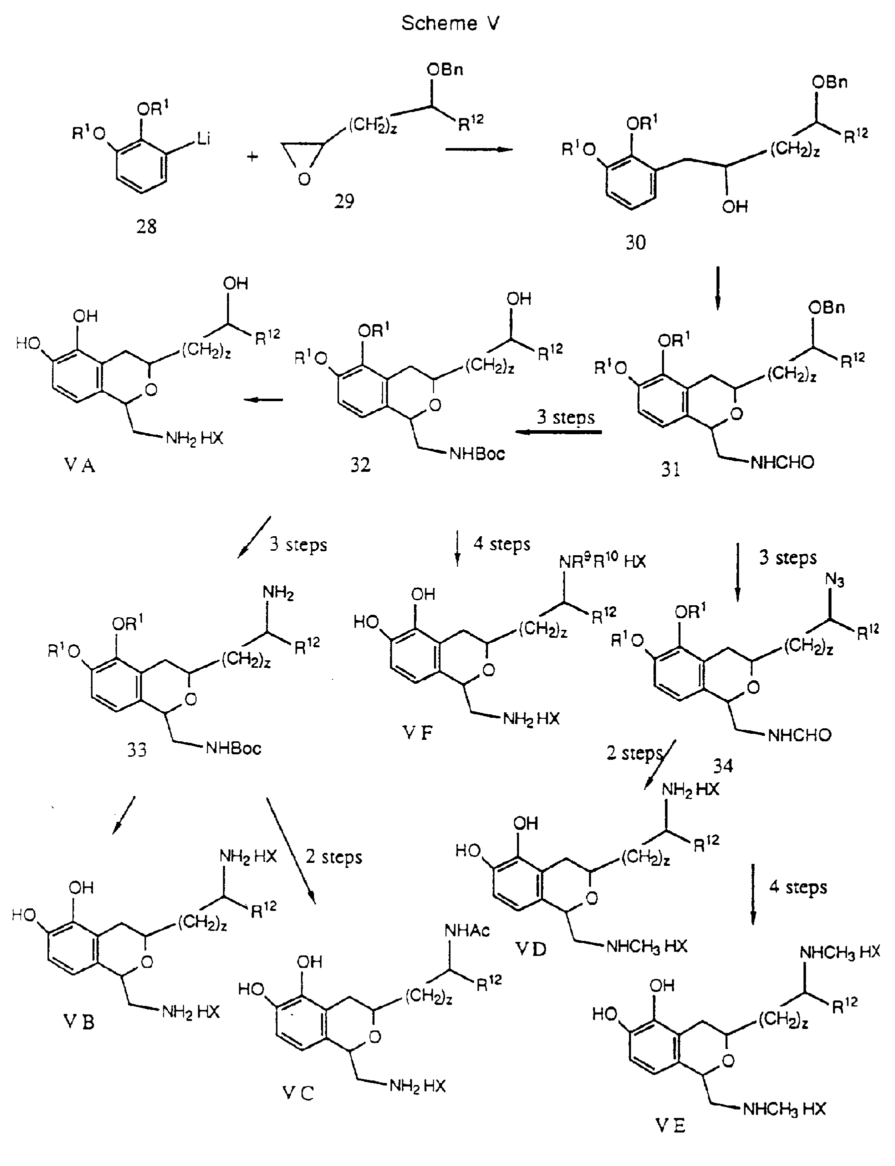

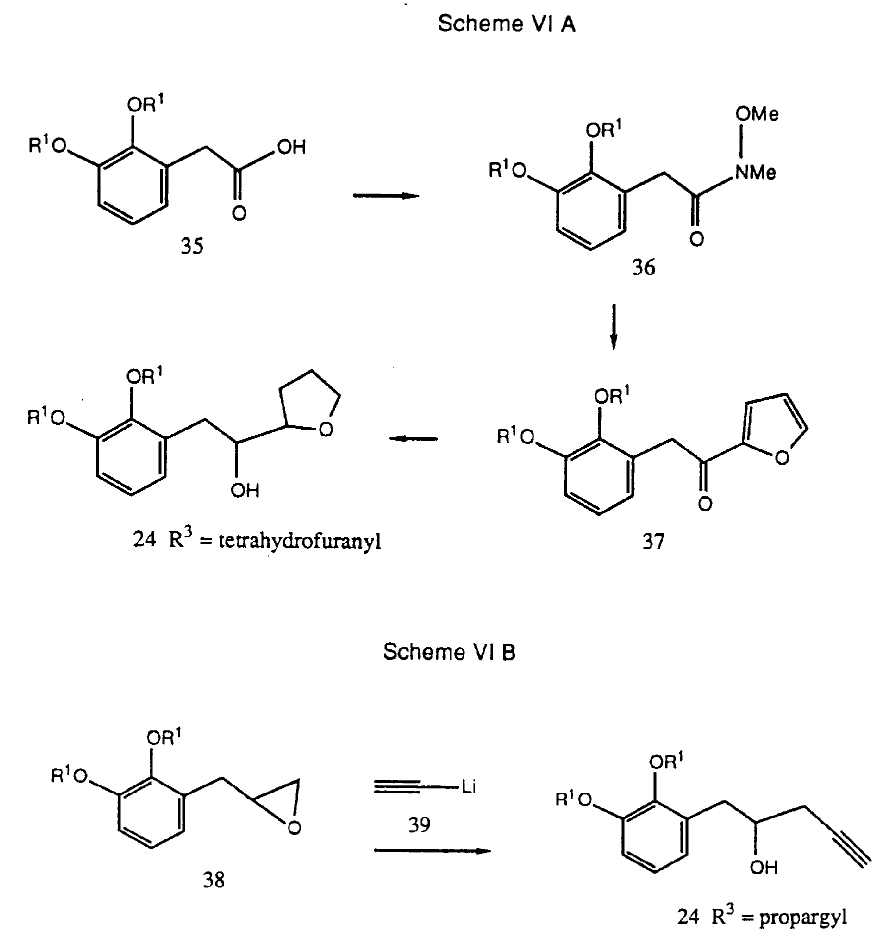

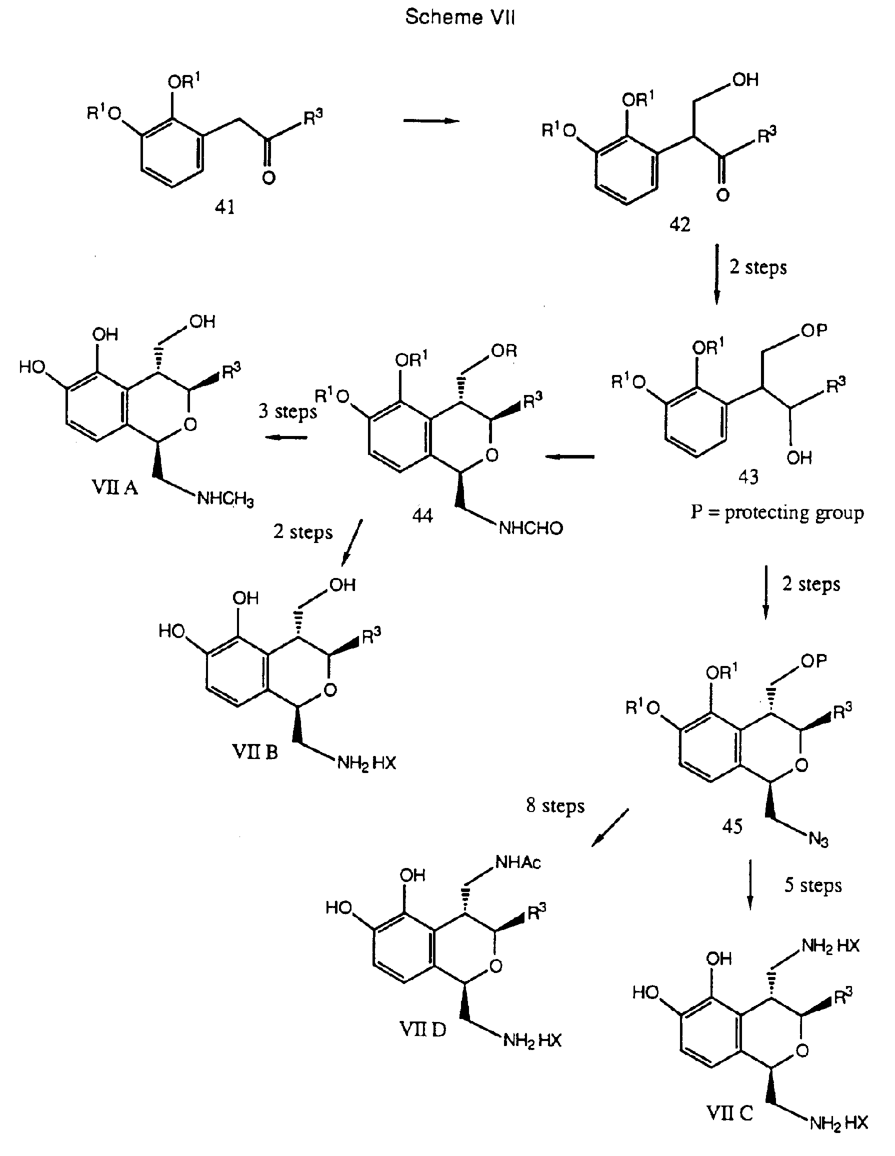

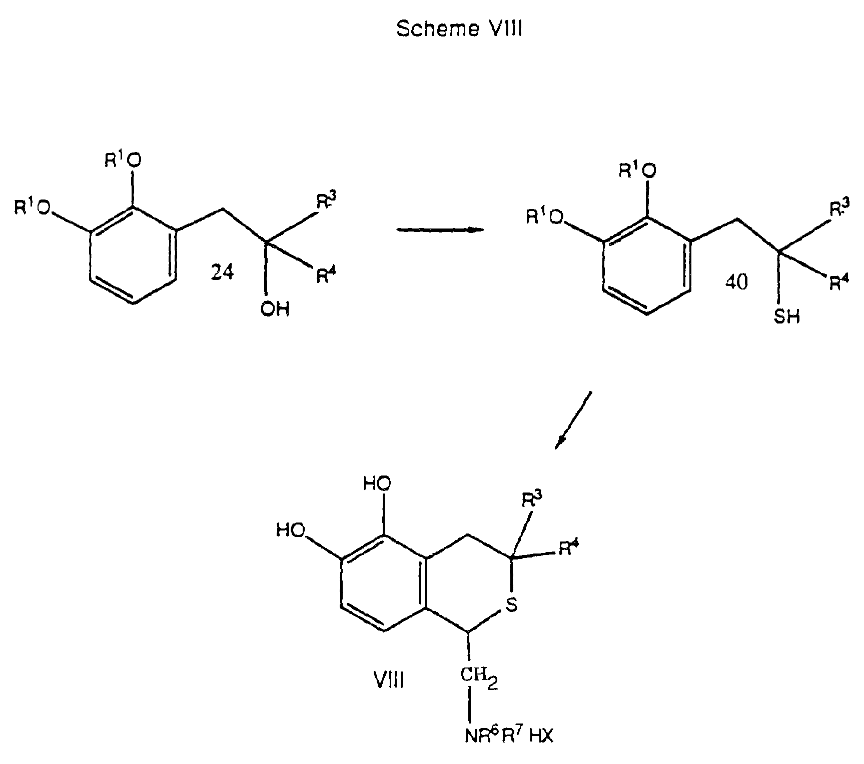

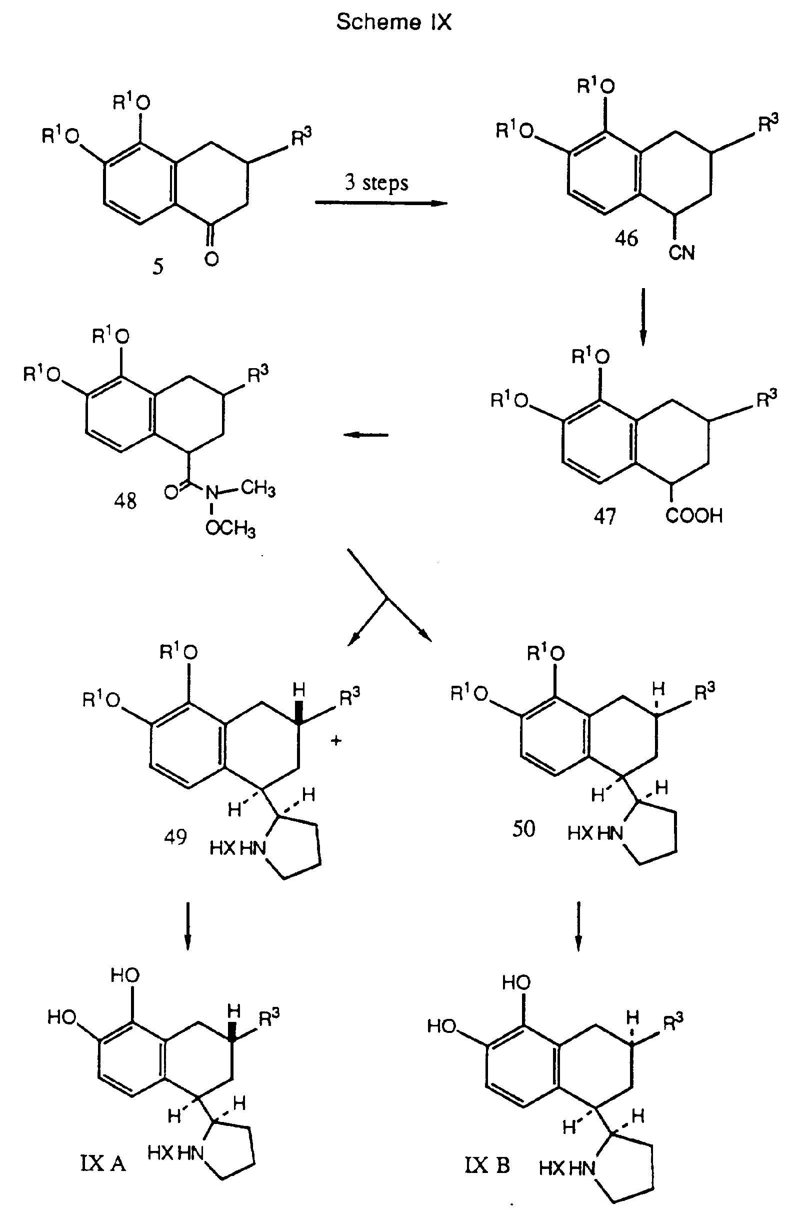

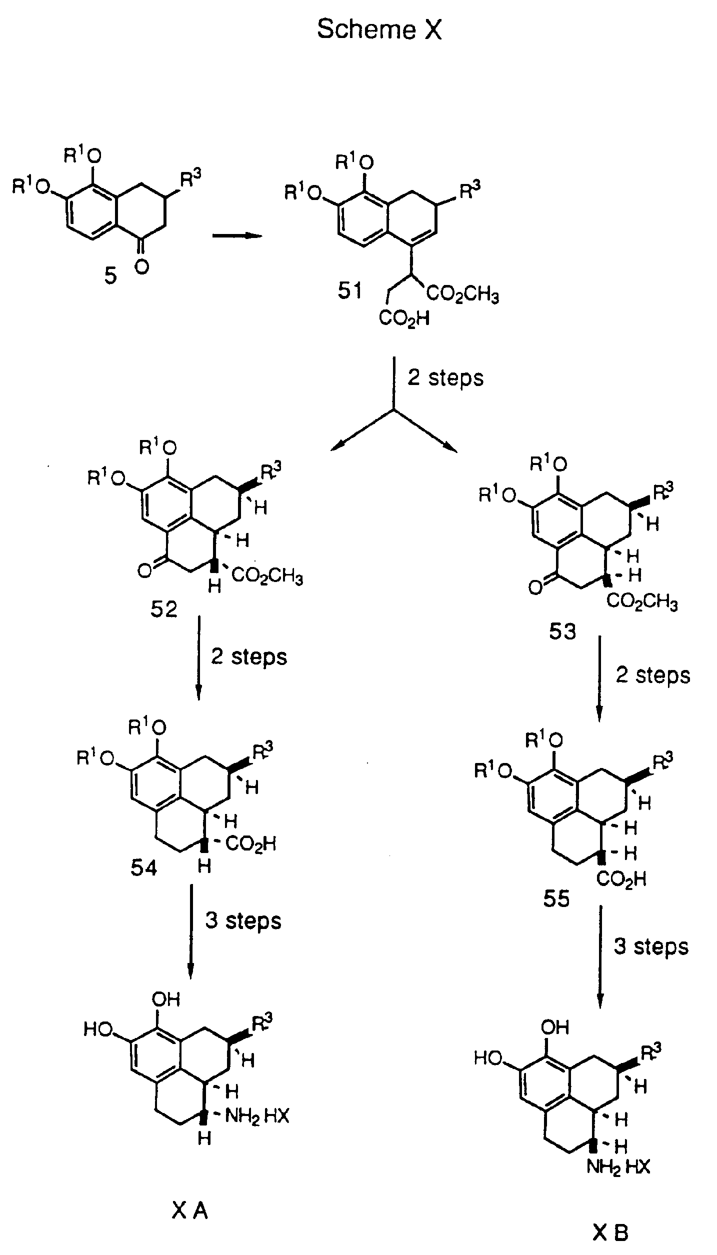

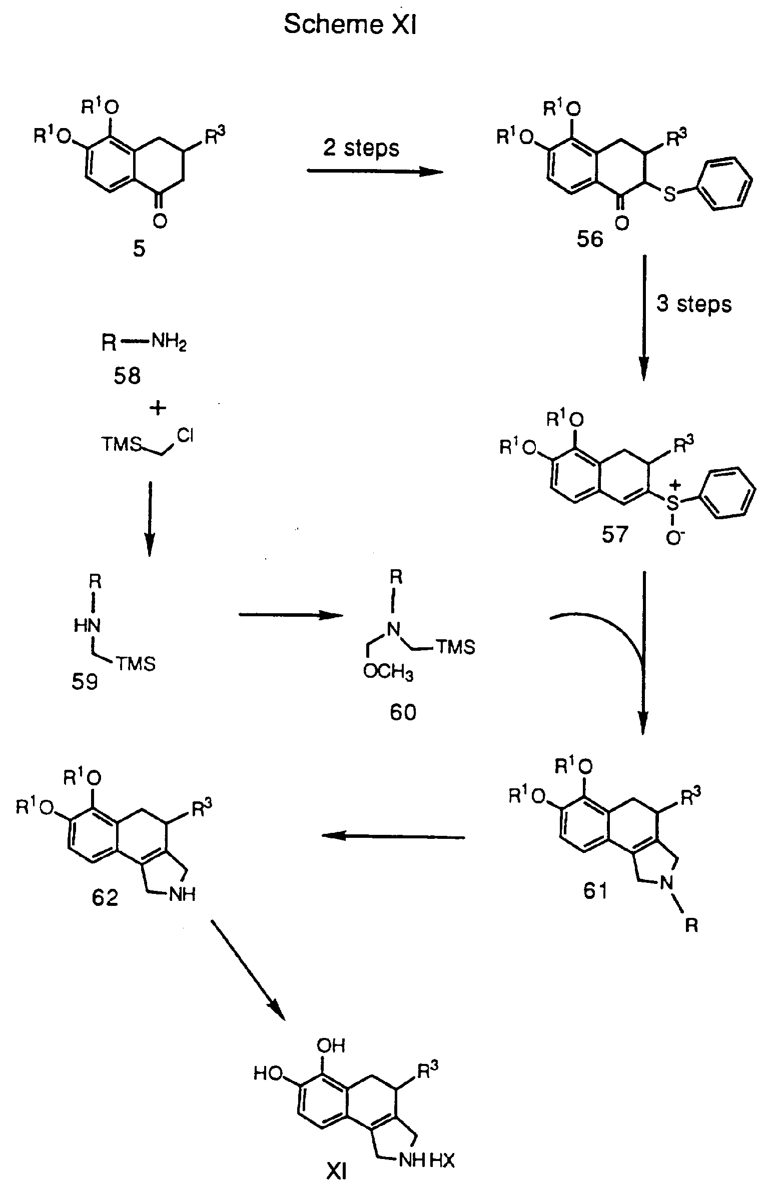

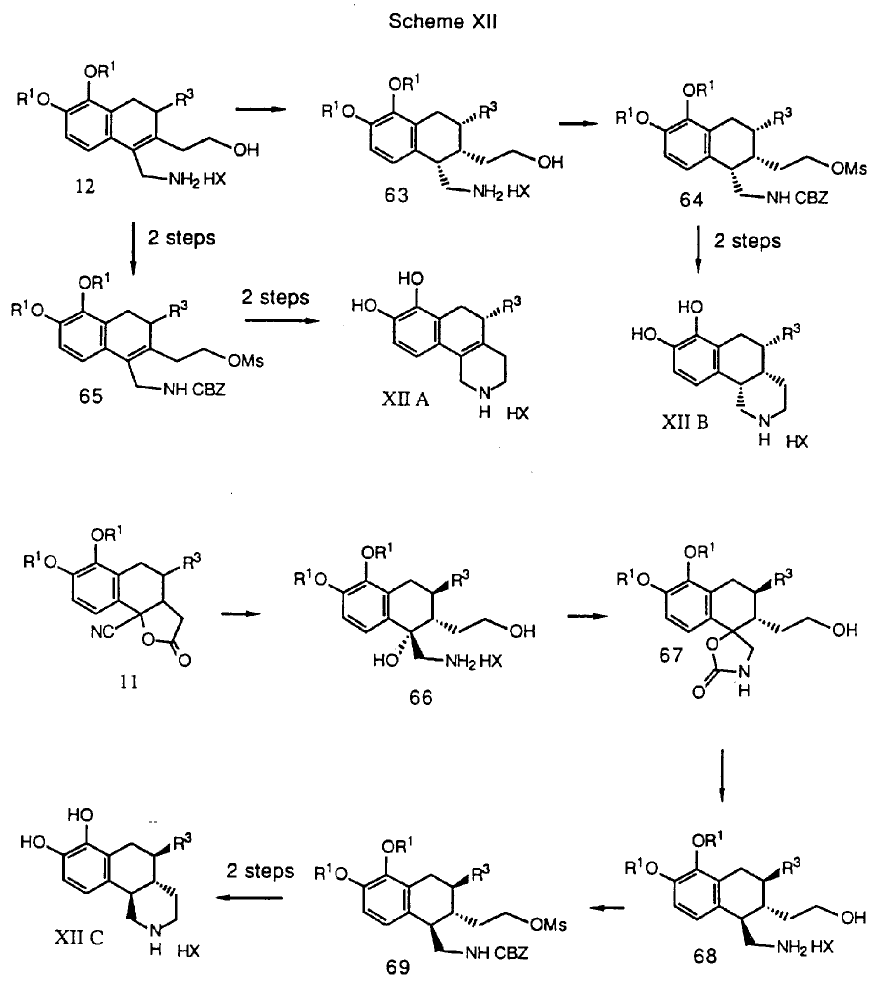

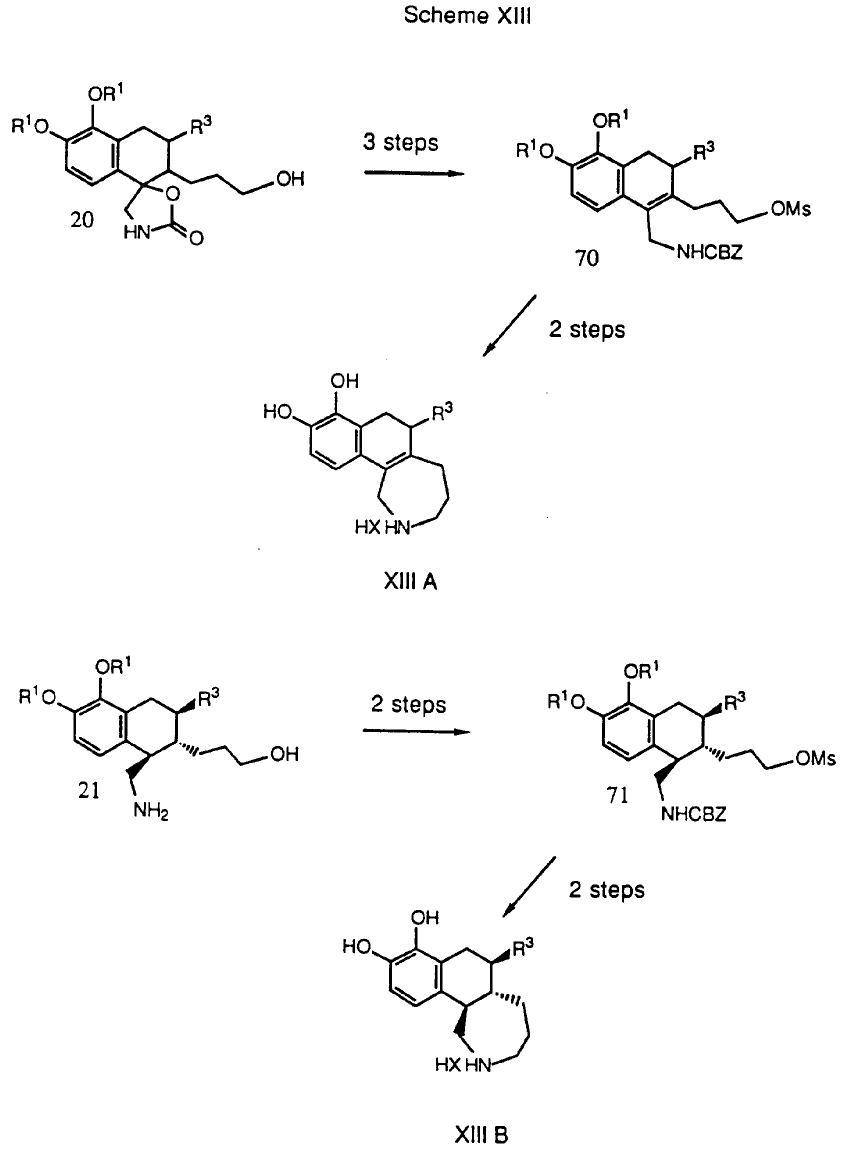

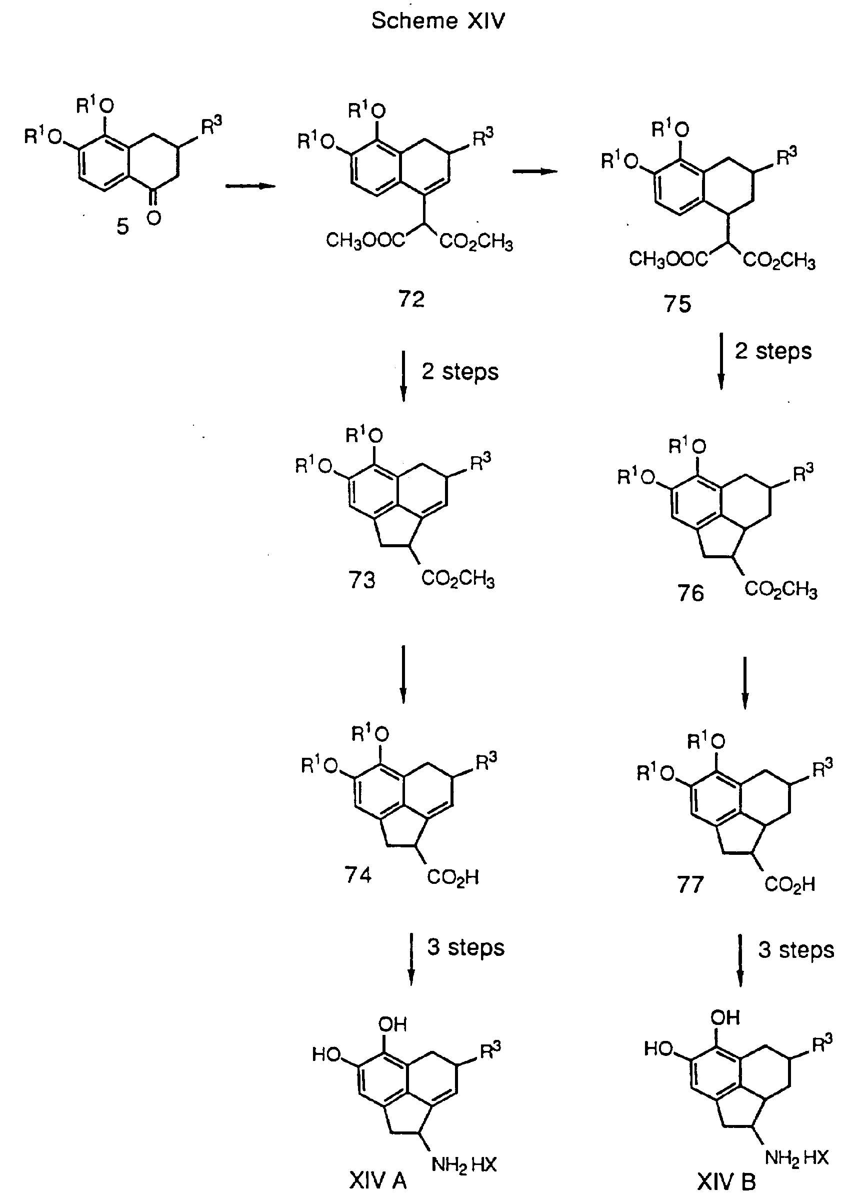

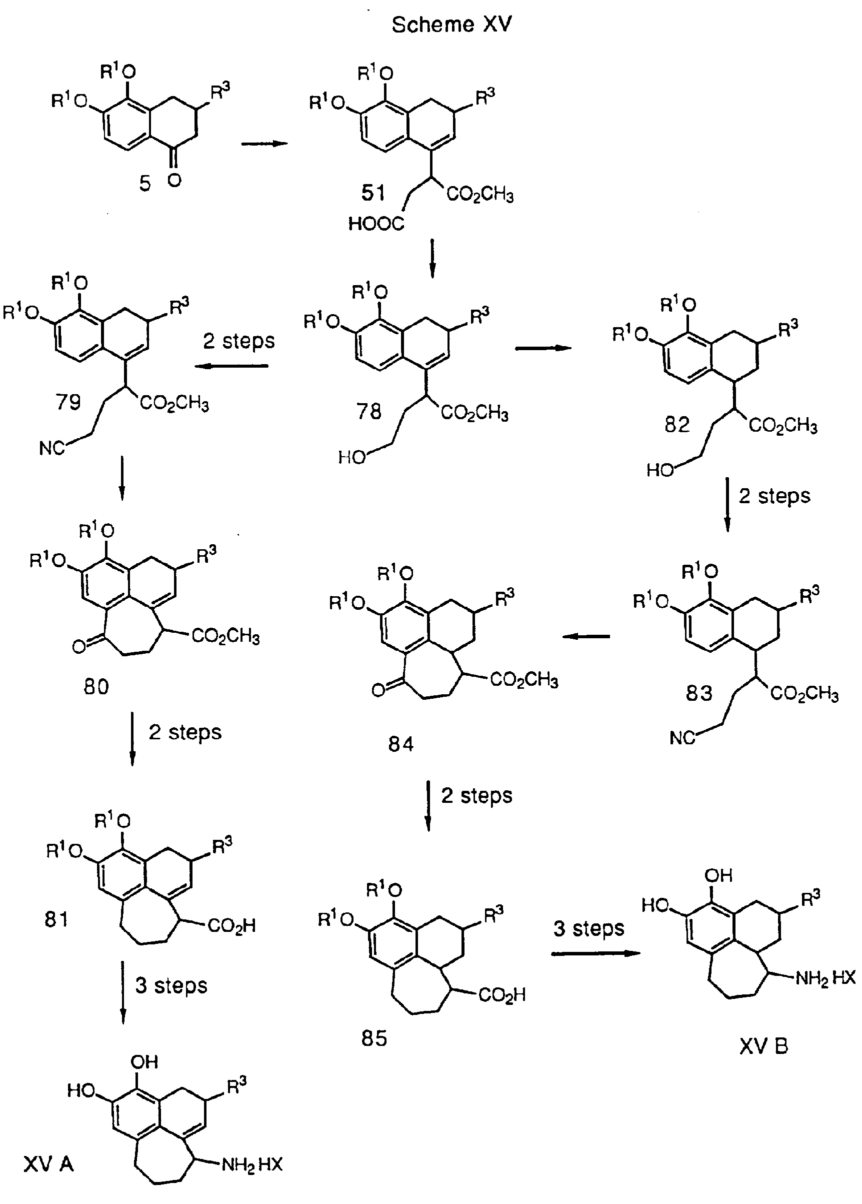

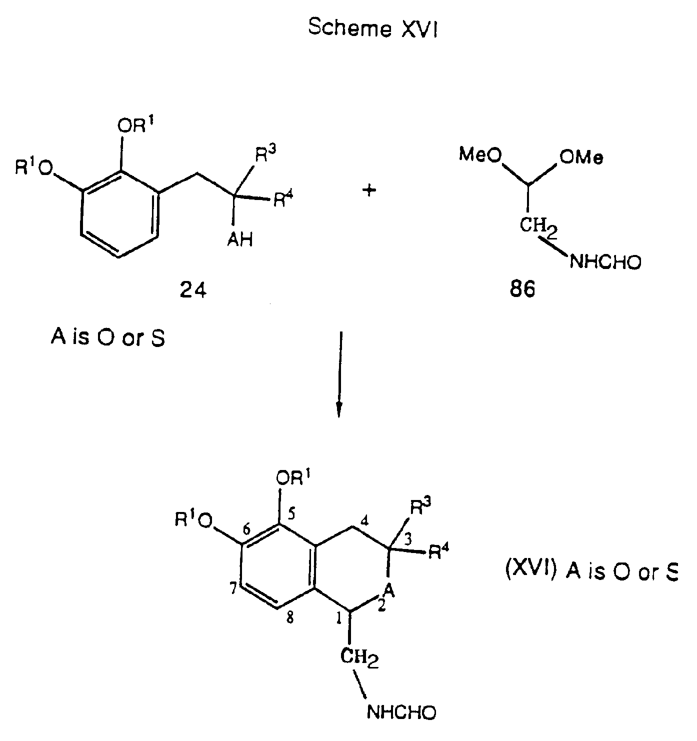

### Example 1

### 5,6-Dimethoxy-3-phenyl-1,2,3,4-tetrahydronaphthalen-1-one

### Method A

### Step 1: (E.Z)-3-(2',3'-Dimethoxyphenyl)-2-phenylpropenoic acid

A solution of 202 g (1.21 mol) of 2,3-dimethoxybenzaldehyde (commercially available from Aldrich Chemical Co.), 200 g (1.47 mol) of phenyl acetic acid (commercially available from Aldrich Chemical Co.), 600 mL of acetic anhydride and 204 mL (1.46 mol) of triethylamine (TEA) was heated at reflux temperature for 24 h. The reaction mixture was allowed to cool to ambient temperature and 1 L of water was added, followed by the addition of 2 L of ethyl acetate and another 4 L of water. The layers were separated and the organic layer was extracted with saturated aqueous sodium bicarbonate solution. The combined aqueous layers were acidified with concentrated hydrochloric acid and extracted with 4 L of ethyl acetate. The ethyl acetate solution was dried over anhydrous magnesium sulfate, filtered and concentrated in vacuo to give 250 g (72% yield) of the title compound as a 30/70 mixture of the E and Z isomers, m.p. 115-160°C; DCI MS: 285 (M+H)⁺, 302 (M+NH₄)⁺

### Step 2: 3-(2',3'Dimethoxyphenyl)-2-phenylpropanol

A solution of 15 g (395 mmol) of lithium aluminum hydride (LAH) in 500 mL of tetrahydrofuran (THF) was cooled to 0°C. A solution of (E,Z)-3-(2',3'-dimethoxyphenyl)-2-phenylpropenoic acid (50 g, 176 mmol), from step 1, in 100 mL of THF was added to the LAH solution dropwise over a 30 min period. The reaction mixture was heated at reflux temperature for 2 hand then cooled to 0°C . The reaction was quenched by the sequential addition of 15 mL of water, 15 mL of 15% aqueous sodium hydroxide solution and 45 mL of water. The precipitate was filtered and the filtrate concentrated in vacuo to give 46.6 g (97% yield) of the title compound as an oil; 1H NMR (CDCI₃) δ 1.8-.19 (m, 1H), 2.1-2.2 (m, 1 H), 2.7-2.95 (m, 1 H), 3.0-3.15 (m, 2H), 3.7-3.8 (m, 1 H), 3.8 (s, 3H), 3.83 (s, 3H), 6.63 (d, 1 H), 6.75 (d, 1H), 6.9 (t, 1 H), 7.15-7.4 (m,5H).

### Step 3: 3-(2,3'-Dimethoxyphenyl)-2-phenylpropane 1-methanesulfonate

3-(2',3'-Dimethoxyphenyl)-2-phenylpropanol (41.5 g, 152 mmol), from step 2, and 30.5 g (301 mmol) of TEA were dissolved in 300 mL of THF. Methanesulfonyl chloride (34.5 g, 301 mmol) was added slowly to this solution at 0°C. The reaction mixture was allowed to warm to ambient temperature. After stirring the reaction mixture for 1 h at ambient temperature, it was diluted with 300 mL of diethyl ether and washed with water, dried over anhydrous magnesium sulfate, filtered and concentrated to give 40.8 g (76% yield) of 3-(2',3'-dimethoxyphenyl)-2-phenylpropane 1-methanesulfonate as an oil; 1 H NMR (CDCI₃) δ 2.7 (s, 3H), 2.96 (dd, 1 H), 3.1 (dd, 1 H), 3.35-3.45 (m, 1 H), 3.78 (s, 3H), 3.82 (s, 3H), 4.35 (m, 2H), 6.62 (dd, 1 H), 6.77 (dd, 1 H), 6.9 (t, 1 H), 7.2-7.35 (m, 5H).

### Step 4: 4-(2',3'-Dimethoxyphenyl)-3-phenylbutanenitrile

3-(2',3'-Dimethoxyphenyl)2-phenylpropane 1-methanesulfonate (40.5 g, 116 mmol), from step 3, and 17 g (347 mmol) of sodium cyanide were dissolved in 100 mL of dimethyl sulfoxide (DMSO) and the resultant solution was heated to 80°C. After being stirred at 80°C for 18 h, the reaction mixture was allowed to cool to ambient temperature, diluted with ethyl acetate and washed sequentially with water and brine. The solvents were removed in vacuo to give 25 g (77% yield) of the title compound as an oil; ¹ H NMR (CDCI₃) 8 2.56 (d, 2H), 3.02 (d, 1 H), 3.05 (d, 1 H), 3.25-3.35 (m, 1 H), 3.72 (s, 3H), 3.75 (s, 3H), 6.65 (dd, 1 H), 6.8 (dd, 1 H), 6.93 (t, 1 H), 7.2-7.4 (m, 5H)

### Step 5: 4-(2',3'-Dimethoxyphenyl)-3-phenylbutyric acid

4-(2',3'-Dimethoxyphenyl)-3-phenylbutanenitrile (20 g, 71 mmol), from Step 4, was dissolved in 1.5 L of ethanol. Sodium hydroxide (20 g, 0.5 mol) and 200 mL of water were added and the reaction mixture was heated at reflux temperature for 24 h. The solvent was removed in vacuo and 1 L of water plus 1 L of methylene chloride were added to the residue. The layers were separated and the organic layer discarded. The aqueous layer was acidified with concentrated hydrochloric acid and extracted with 3 L of ethyl acetate. The ethyl acetate solution was dried over anhydrous sodium sulfate, filtered and concentrated in vacuo to give 21 g (98% yield) of the title compound as an oil; ¹ H NMR (CDCI₃) δ 2.6-2.7 (m, 2H), 2.9 (d, 2H), 3.4-3.5 (m, 1 H), 3.72 (s, 3H), 3.82 (s, 3H), 6.6 (dd, 1 H), 6.73 (dd, 1 H), 6.88 (t, 1H), 7.1-7.3 (m, 5H).

### Step 6: 5,6-Dimethoxy-3-phenyl-1 ,2,3,4-tetrahydronaphthalen-1-one

4-(2',3'-Dimethoxyphenyl)-3-phenylbutyric acid (37 g, 123 mmol), from Step 5, was added dropwise to 200 g of polyphosphoric acid heated to 100°C. The resultant mixture was stirred and heated at 100°C for 0.25 h. A mixture of 100 g of ice and 200 mL of water was added to the reaction mixture. The precipitate which formed was filtered, washed with 3 X 75 mL of water and dissolved in 300 mL of methylene chloride. The methylene chloride solution was dried over anhydrous magnesium sulfate, filtered and concentrated to give 28 g (81 % yield) of 5,6-dimethoxy-3-phenyl-1,2,3,4-tet- rahydronaphthalen-1 -one, m.p; 127-128°C; ¹H NMR (CDCI₃) δ 2.75-3.0 (m, 3H), 3.3-3.5 (m, 2H), 3.8 (s, 3H), 3.95 (s, 3H), 6.93 (d, 1 H), 7.25-7.4 (m, 5H), 7.9 (d, 1 H).

### Method B

### Step 1: 2-(2',3'-Dimethoxyphenyl)-1,3-dithiane

A solution of 49.5 g (298 mmol) of 2,3-dimethoxybenzaldehyde and 48.4 g (447 mmol) of propane-1,3-dithiol in 800 mL of methylene chloride was cooled to 0°C. Boron trifluoride etherate (7.5 mL, 61 mmol) was added dropwise to the cooled solution and the reaction mixture was stirred at 0°C for 0.5 h, then at ambient temperature for 18 h. The methylene chloride solution was washed with 2 X 200 mL of 10% aqueous sodium hydroxide solution, 200 mL of water and 100 mL of brine, dried over anhydrous sodium sulfate and concentrated in vacuoto give 75 g (98% yield) of 2-(2',3'-dimethoxyphenyl)-1,3-dithiane, m.p. 119-120°C; ¹ H NMR (CDCI₃) δ 1.8-2.0 (m, 1 H), 2.1-2.25 (m, 1 H), 2.86 (t, 1 H), 2.91 (t, 1 H), 3.05-3.2 (m, 2H), 3.83 (s, 3H), 3.91 (s, 3H), 5.68 (s, 1 H), 6.86 (dd, 1 H), 7.07 (t, 1 H), 7.19 (dd, 1 H).

### Step 2: Ethyl 4-(2'.3'-dimethoxyphenyl)-4-(1",3"-dithiane)-3-phenylbutyrate

A solution of 2-(2',3'-dimethoxyphenyl)-1,3-dithiane (57 g, 222 mmol), from Step 1, in 273 mL of dry THF was cooled to -78°C in a dry ice/acetone bath. To this solution was added 92.2 mL of a 2.5 M solution of n-butyl lithium in hexane. After the addition was complete the reaction mixture was stirred for 0.75 h at -78°C. 1,3-Dimethyl-2-imidazolidinone (75 mL, 686 mmol), commercially available from Aldrich Chemical Company, was added to the reaction mixture in one portion, followed by 39 g (221 mmol) of ethyl cinnamate (commercially available from Aldrich Chemical Company) added dropwise. The reaction mixture was stirred for 1 h at -78°C then quenched with 50 mL of 10% aqueous acetic acid and allowed to warm to 0°C. The reaction mixture was diluted with 150 mL of diethyl ether and the layers separated. The organic layer was washed with 2 X 100 mL of saturated aqueous sodium bicarbonate solution, 100 mL of water and 100 mL of brine, dried over anhydrous magnesium sulfate, filtered and concentrated to give a crude oily product. The crude product was crystallized from ethyl acetate/hexane to give 32 g (48% yield) of the title compound, m.p. 125-126°C. A second crop of crystals yielded an additional 11 g (total yield 59%) of ethyl 4-(2',3'-dimethoxyphenyl)-4-(1",3"-dithiane) -3-phenylbutyrate, m.p. 124.5-125°C; ¹H NMR (CDCL₃) 6 0.8 (t, 3H), 1.75-1.9 (m, 2H), 2.5-2.85 (m, 4H), 3.05-3.25 (m, 2H), 3.7-3.95 (m, 2H), 3.88 (s, 3H), 4.0 (s, 3H), 4.45-4.5 (m, 1H), 6.8-6.9 (m, 2H), 7.0-7.4 (m, 6H).

### Step 3: Ethyl 4-(2',3'-dimethoxyphenyl)-3-phenylbutyrate

Ethyl 4-(2',3'-dimethoxyphenyl)-4-(1 ",3"-dithiane)-3-phenylbutyrate (14.5 g, 39 mmol), from Step 2, and 145 g Raney nickel and 300 mL of absolute ethanol were mixed together and heated at reflux temperature under 1 atmosphere of hydrogen for 3.25 h. The stirring was stopped and the mixture was allowed to cool slightly before the solvent was decanted from the catalyst. An additional 300 mL of absolute ethanol was added to the catalyst and the mixture stirred and heated to reflux. The stirring was again stopped and the reaction mixture was allowed to cool slightly before the solvent was decanted from the catalyst. The combined supernatants were filtered through Celite® filter aid and concentrated in vacuo to give 10.8 g (97% yield) of ethyl 4-(2',3'-dimethoxyphenyl)-3-phenylbutyrate as a clear oil; ¹ H NMR (CDC1₃) 8 1.11 (t, 3H), 3.07 (dd, 1 H), 3.35 (dd, 1 H), 3.81 (s, 3H), 3.84 (s, 3H), 3.9-4.1 (m, 3H), 6.65 (dd, 1 H), 6.77 (dd, 1 H), 6.88 (t, 1 H), 7.2-7.4 (m, 5H).

### Step 4: 4-(2',3'-Dimethoxyphenyl)-3-phenylbutyric acid

Ethyl 4-(2',3'-dimethoxyphenyl)-3-phenylbutyrate (40.3 g, 123 mmol), from Step 3, was dissolved in 400 mL of methanol and 62 mL of 3 M aqueous sodium hydroxide solution was added in one portion. The reaction mixture was stirred at ambient temperature for 18 h. The reaction mixture was concentrated and the residue was partitioned between 300 mL of diethyl ether and 200 mL of water. The layers were separated and the aqueous layer was adjusted to pH 6 with 6 M aqueous hydrochloric acid solution and extracted with 3 X 200 mL of diethyl ether. The organic layers were combined, washed with brine, dried over anhydrous magnesium sulfate, filtered and concentrated in vacuo to give 37 g (100% yield) of the title compound as a colorless oil. The 1H NMR spectrum was identical to the spectrum reported for the product of Step 5 of Method A, Example 1.

### Step 5: 5,6-Dimethoxy-3-phenyl-1,2,3,4-tetrahydronaphthalen-1-one

4-(2',3'-Dimethoxyphenyl)-3-phenylbutyric acid (13.3 g, 44.3 mmol), from Step 4, was treated with 14 mL (216 mmol) of methanesulfonic acid and 200 mL of trifluoroacetic acid at 60°C for 1.5 h. After cooling the reaction mixture, the trifluoroacetic acid was removed in vacuo and ice water was added to the residue. Methylene chloride was added and the layers were separated. The organic layer was washed with 1 N aqueous sodium hydroxide solution, water and brine, dried over anhydrous magnesium sulfate, filtered and concentrated under reduced pressure. The residue was recrystallized three times from methanol to give 9.6 g (77% yield) of 5,6-dimethoxy-3-phenyl-1,2,3,4-tetrahydronaphtha- len-1-one, m.p. 126-128°C; ¹H NMR spectrum was identical to the spectrum reported for the product of Step 6 of Method A, Example 1.

### Example 2

### 1 -Aminomethyl-5,6-dihydroxy-3-phenyl-3.4-dihydronaphthalene

### Step 1: 1-Aminomethyl-5,6-dimethoxy-1-hydroxy-3-phenyl-1,2,3,4-tetrahydronaphthalene

5,6-Dimethoxy-3-phenyl-1,2,3,4-tetrahydronaphthalen-1-one (14.6 g, 51.7 mmol), from Example 1, 24 mL of acetonitrile, 10.3 g (104 mmol) of trimethylsilylcyanide, commercially available from Aldrich Chemical Company, and 100 mg of aluminum chloride were mixed together and heated at reflux temperature for 2.5 h. The reaction mixture was cooled and concentrated The residue was added dropwise to a solution of 4.3 g (113 mmol) of lithium aluminum hydride in 101 mL of diethyl ether. After the reaction mixture was heated at reflux temperature for 2.5 h, 4.3 mL of water was added dropwise, followed by 4.3 mL of 15% aqueous sodium hydroxide solution, followed by a second 4.3 mL of water. The reaction mixture was stirred until a granular precipitate formed. The solid was filtered and washed with 3 X 80 mL of methylene chloride. The filtrate was concentrated and the resultant solid was triturated with ethyl acetate/hexane to give 11.9 g (73% yield) of the title compound, m.p. 175-176°C; 1 H NMR (dₛ-DMSO) 8 2.03 (t, 1 H), 2.28 (dt, 1 H), 2.65 (dd, 1 H), 2.83 (dd, 1 H), 2.95-3.1 (m, 2H), 3.28 (dd, 1 H), 3.75 (s, 3H), 3.86 (s, 3H), 6.87 (d, 1 H), 7.2-7.4 (m, 6H).

### Step 2; 1-Aminomethyl-5,6-dimethoxy-3-phenyl-3,4-dihydronaphthalene hydrochloride

1-Aminomethyl-5,6-dimethoxy-1-hydroxy-3-phenyl-1,2,3,4-tetrahydronaphthalene (11.5 g, 37 mmol), from Step 1, was heated at reflux temperature in 300 mL of isopropyl alcohol saturated with hydrochloric acid for 2 h. The resultant solution was concentrated and the solid residue was triturated with hot toluene to give 10.6 g (98% yield) of 1-aminomethyl-5,6-dimethoxy-3-phenyl-3,4-dihydronaphthalene hydrochloride, m.p. 189.5-190°C; ¹H NMR (d₆-DMSO) 8 2.78 (dd, 1 H), 3.11 (dd, 1 H), 3.2-3.4 (m, 2H + H₂0), 3.6 (s, 3H), 3.81 (s, 3H), 3.93 (d, 1 H), 6.1 (d,1 H), 6.93 (d, 1 H), 7.12 (d, 1 H), 7.2-7.4 (m, 5H).

### Step 3: 1-Aminomethyl-5,6-dihydroxy-3-phenyl-3,4-dihydronaphthalene hydrobromide (Example 2A)

1-Aminomethyl-5,6-dimethoxy-3-phenyl-3,4-dihydronaphthalene hydrochloride (6.0 g, 20.2 mmol), from Step 2, was suspended in 200 mL of methylene chloride and boron tribromide ( 90.5 mL of 1 M solution of BBr₃ in methylene chloride) was added dropwise while the reaction mixture was being cooled (to -78°C) in a dry ice/acetone bath. The reaction mixture was warmed to 0°C and stirred for 0.5 h, then again cooled to -78°C in a dry ice/acetone bath. Methanol (50 mL) was added dropwise to the reaction mixture, which was allowed to warm to ambient temperature then concentrated in vacuo. Methanol was added to the residue and the solution was reconcentrated. This residue was dissolved in a small amount of methanol and the methanol solution was added to 700 mL of diethyl ether. The precipitate which formed was filtered, washed with diethyl ether and recrystallized from methanol/ether to give 2.5 g (45% yield) of the title compound, m.p. 223-225°C. 1H NMR (d₆-DMSO) δ 2.68 (dd, 1 H), 3.09 (dd, 1 H), 3.6-3.7 (m, 1 H), 3.9 (s, 2H), 5.97 (d, 1 H), 6.69 (m, 2H), 7.2-7.35 (m, 5H), 8.1 (br s, 3H), 8.4 (s, 1 H), 9.5 (s, 1 H).

### Step 4: 1-Aminomethyl-5,6-dihydroxy-3-phenyl-3,4-dihydronaphthalene hydrochloride (Example 2B)

A slurry of 10 g (25 mmol) of 1-aminomethyl-5,6-dimethoxy-3-phenyl-3,4-dihydronaphthalene hydrochloride, from Step 2, in 150 mL of 1,2-dichloroethane was cooled to 10°C under a nitrogen atmosphere. Boron trichloride was passed through the reaction mixture until 27 g (230 mmol) had been added. The reaction mixture was allowed to warm to ambient temperature and stirred for 18 h. The reaction mixture was then cooled in ice and 100 mL of methanol was added dropwise. The reaction mixture was again allowed to warm to ambient temperature and concentrated in vacuo. Twice, 500 mL portions of methanol were added to the residue and it was reconcentrated. The resultant foam was dissolved in 40 mL of ethanol, filtered and the solution was treated with 40 mL of methylene chloride and 80 mL of heptane. Off-white crystals of 1 -aminomethyl-5,6-dihydroxy-3-phenyl-3,4-dihydronaphthalene hydrochloride (5.1 g, 56% yield) were collected by filtration, m.p. 204-205°C. The ¹ H NMR spectrum was identical to the spectrum for the product of Example 2A.

### Example 3

### 5,6-Bis(acetoxy)-1-aminomethyl-3-phenyl-3,4-dihydronaphthalene hydrochloride

A suspension of 7.6 g (25 mmol) of 1-aminomethyl-5,6-dihydroxy-3-phenyl-3,4-dihydronaphthalene hydrochloride (Example 2B) in 400 mL of acetic anhydride saturated with anhydrous hydrogen chloride was stirred at ambient temperature for 48 h. Approximately 2 L of diethyl ether was added and a solid was collected by filtration and washed with diethyl ether. Crystallization of the crude material (6.7 g) was achieved by dissolving the powder in 400 mL of hot ethanol, adding 100 mL of water, filtering the solution hot and allowing it to cool. The white crystals which formed were filtered and dried to give 2.8 g (29% yield) of 5,6-bis(acetoxy)-1-aminomethyl-3-phenyl-3,4-dihydronaphthalene hydrochloride, m.p. 207-208°C. ¹ H NMR (dₛ-DMSO) 8 2.28 (s, 6H), 2 62 (dd, 1 H), 2 95 (dd, 1 H), 3.7-3.8 (m, 1 H), 3.97 (s, 2H), 6.25 (d, 1 H), 7.19 (d, 1 H), 7.2-7.4 (m, 6H), 8.41 or s, 3H).

### Example 4

### 5,6-Bis(acetoxyl-1-(alanyl-alanyl)aminomethyl-3-phenyl-3,4-dihydronaphthalene hydrochloride (general procedure for preparation of amino prodruqs)

### Step 1: 5,6-Bis(acetoxy)-1 (N-t-butoxycarbonyl-alanyl-alanyl)aminomethyl-3-phenyl-3,4-dihydronaphthalene

N-t-Butoxycarbonyl-alanyl-alanine (BocAla-Ala) (2.01 g, 7.74 mmol) was added to a stirred solution of 1-aminomethyl-5,6-bis(acetoxy)-3-phenyl-3,4-dihydronaphthalene hydrochloride (3 g, 7.74 mmol), the product of Example 3, in 35 mL of DMF. The resultant solution was cooled to 0°C. To the cold solution was added 1.56 g (8.13 mmol) of 1-(3-dimethylaminopropyl)-3-ethylcarbodiimide hydrochloride (EDCI), followed approximately 10 minutes later by 1.1 g (8.13 mmol) of 1-hydroxybenzotriazole hydrate (HOBT) and, after allowing the HOBT to dissolve, by 1.8 g (16 mmol) of 4-methylmorpholine (NMM). The resultant solution was stirred for 3 h at 0°C and then stirred overnight at ambient temperature. The reaction mixture was then diluted with 100 mL of water and the resultant milky mixture was extracted with 3 X 75 mL of ethyl acetate. The combined organic layers were washed successively with 50 mL of 1 M aqueous phosphoric acid solution, 50 mL of saturated aqueous sodium bicarbonate solution, 2 X 50 mL of water and 50 mL of brine. The organic layer was then dried over anhydrous magnesium sulfate, filtered and concentrated in vacuo to a light yellow colored foam. The foam (5.06 g) was purified by flash chromatography using C₁₈ ODS (C₁₈ -octadecylsilane) on silica as the solid phase and a 50% solution of 1% aqueous trifluoroacetic acid (TFA) in acetonitrile as the eluent to give 2.03 g (44% yield) of the title compound as a light yellow colored solid, m.p. 114-116°C; ¹H NMR (CDCI₃) δ 1.23 (q, 3H), 1.35 (q, 3H), 1.42 (s, 9H), 2.23 (s, 3H), 2.28 (s, 3H), 2.70 (m, 1 H), 2.95 (dd, 1 H), 3.68 (m, 1 H), 4.05 (q, 1 H), 4.2-4.47 (m, 3H), 6.01 (d, 1 H), 7.04 (d, 1 H), 7.17 (d, 1 H), 7.26 (m, 5H).

### Step 2: 5,6-Bis(acetoxy)-1-(alanyl-alanyl)aminomethyl-3-phenyl-3,4-dihydronaphthalene hydrochloride

5,6-Bis(acetoxy)-1-(N-t-butoxycarbonyl-alanyl-alanyl)aminomethyl-3-phenyl-3_{;}4-dihydronaphthalene hydrochloride (2.00 g, 3.36 mmol) from Step 1 was dissolved in 25 mL of diethyl ether The resultant solution was cooled and saturated with hydrogen chloride. The solution was stirred at ambient temperature for 3 h. The precipitate was filtered and washed thoroughly with dry diethyl ether. The solid was dried overnight at 60°C in vacuo to give 1.69 g (95% yield) of the title compound as an off-white solid, m.p 145-161 °C (dec); ¹H NMR (CDCI₃) δ 1.35 (dd, 3H), 1.41 (m, 3H), 2.23 (s, 3H), 2.26 (s, 3H), 2.70 (dt, 1 H), 2.95 (dd, 1 H), 3.69 (br s, 1 H), 3.89 (dq, 1 H), 4.17-4.45 (m, 3H), 6.11 (d, 1 H), 7.07 (d, 1H), 7.25 (m, 6H), 8.27 (m, 1H). Analysis calculated for C₂7H₃₃N₃06+1.3HCl: C, 59.73; H, 6.37; N, 7.74. Found: C, 59 94; H, 6.08; N, 7.64.

### Examples 5-14

Following the procedures described in Example 4 using the appropriate aminomethyl compound of Formula I with both catechol hydroxyl group protected as shown in the table and the appropriate (D) or (L) amino acid or peptide having the N-terminal amino group protected preferably as a carbamate, and more preferably as the t-butoxycarbonyl derivative, Examples 5 - 14 were prepared as disclosed in Table 1.

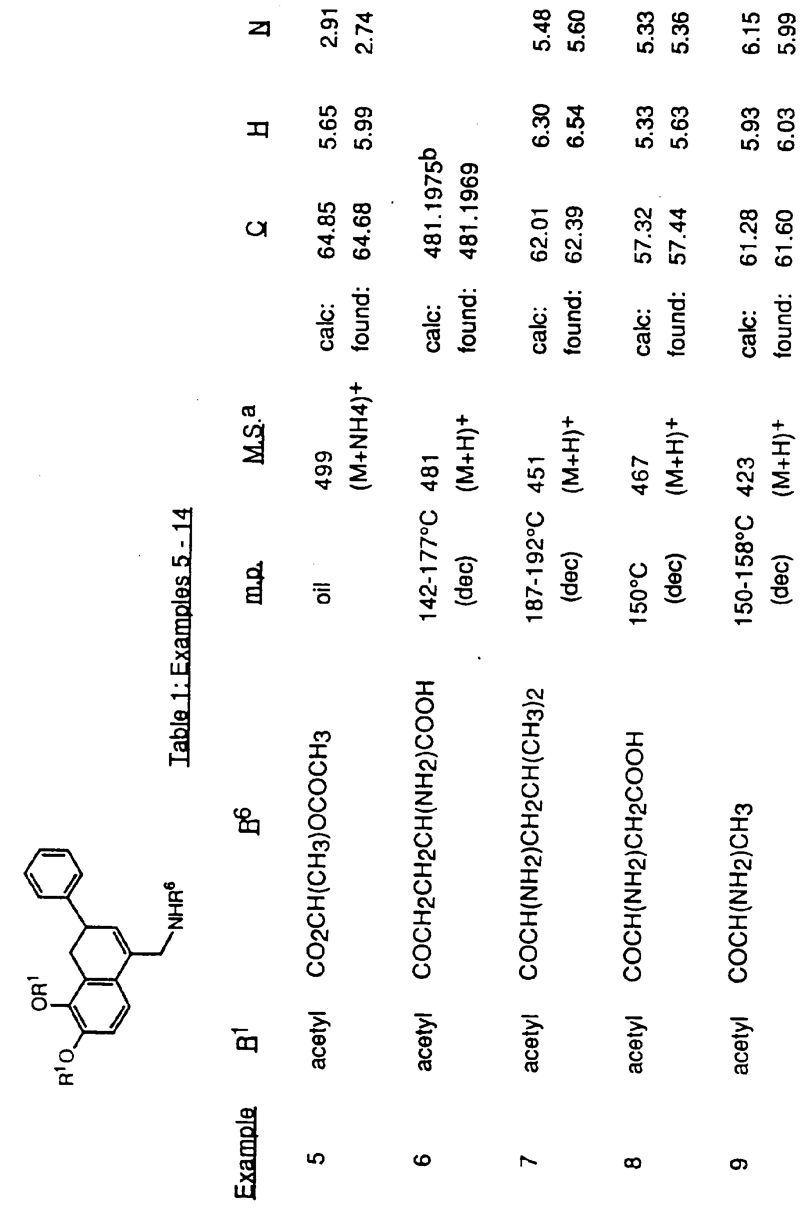

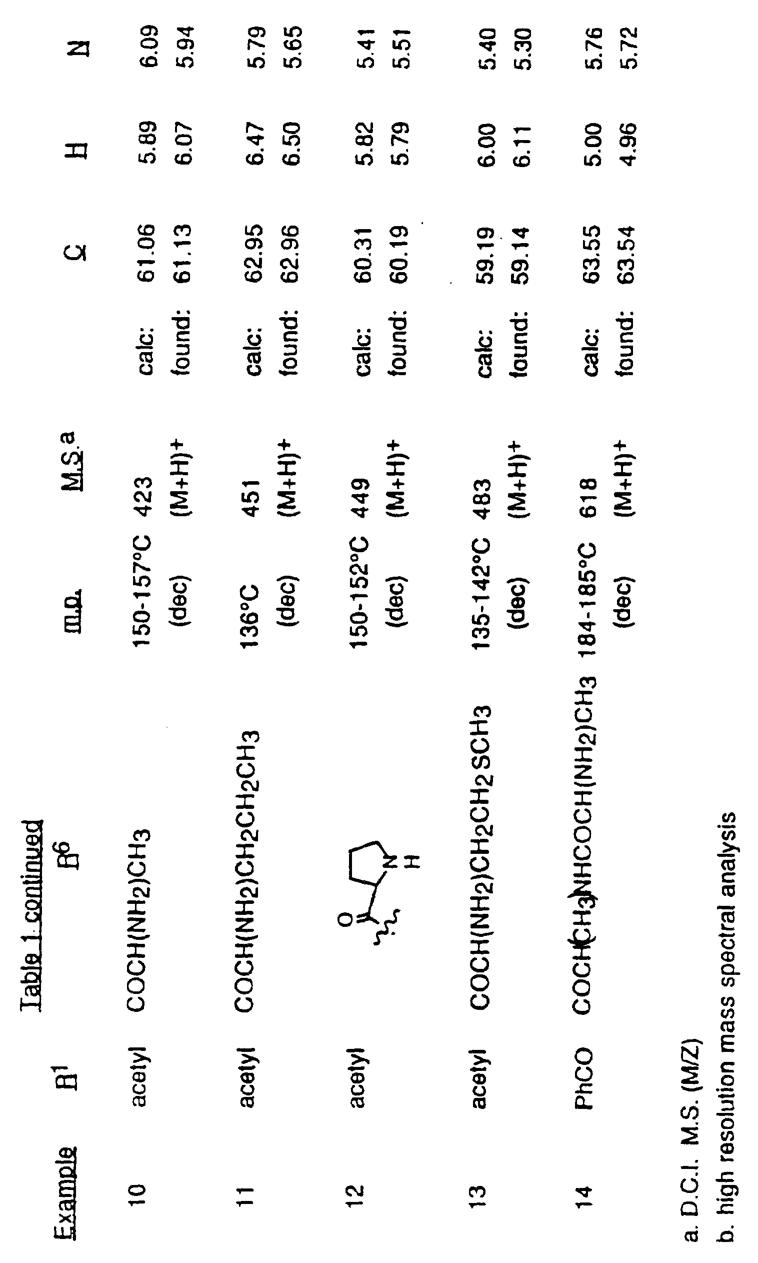

### Example 15

### 1-Aminomethyl-5,6-bis(trimethylacetoxy) -3-phenyl-3,4-dihydronaphthalene-hydrochloride

### Step 1: N-t-Butyloxycarbonyl-1-aminomethyl-5,6-dihydroxy-3-phenyl-3,4-dihydronaphthalene

Triethylamine (7 mL) was added to a solution of 15 g (56 mmol) of 1-aminomethyl-5,6-dihydroxy-3-phenyl-3,4-dihydronaphthalene hydrochloride, from Example 2 in 100 mL of dimethylformamide (DMF). The solution was cooled to 0°C and a solution of di-t-butyl-dicarbonate (18 g, 82.5 mmol) in 50 mL of DMF was added over a period of 1 h. After the addition was complete, 250 mL of water was added to the reaction mixture and it was extracted with ethyl acetate. The combined organic layers from the extraction were washed with 1 N hydrochloric acid solution and brine, dried over anhydrous magnesium sulfate, filtered and concentrated in vacuo. The brown residue was triturated with boiling hexanes to give 16.7 g (99% yield) of the title compound as an off-white solid, m.p 175-177°C, ¹ H NMR δ 1.45 (s, 9H), 2.74 (dd, 1 H), 3.19 (dd, 1 H), 3.6-3.7 (m, 1 H), 4.1-4.25 (m, 2H), 4.71 (br s, 1 H), 5.4 (br s, 1 H), 5.88 (d, 1 H), 6.0 (br s, 1 H), 6.68 (s, 2H), 7.2-7.35 (m, 5H).

### Step 2: N-t-Butyloxycarbonyl-1 aminomethyl-5.6-bis(trimethylacetoxy)-3-phenyl-3,4-dihydronaphthalene

N-t-Butyloxycarbonyl-1-aminomethyl-5,6-dihydroxy-3-phenyl-3,4-dihydronaphthalene (3 g, 8.16 mmol), from Step 1, and 11 mL of triethylamine were combined and cooled to 0°C. A solution of trimethylacetyl chloride (2.1 mL, 17 mmol) in 13 mL of dioxane was added to the cooled solution dropwise. The reaction mixture was allowed to warm to ambient temperature and stirred at ambient temperature for 2 h. Water (25 mL) was added to the reaction mixture and the pH was adjusted to 4 with concentrated phosphoric acid. The reaction mixture was extracted with diethyl ether. The combined ether extracts were washed with aqueous saturated sodium bicarbonate solution, water and brine, dried over anhydrous magnesium sulfate, filtered and concentrated in vacuo to give 4.26 g (89% yield) of N-t-butyloxycarbonyl-1-aminomethyl-5,6-bis(trimethylacetoxy)-3-phenyl-3,4-dihydronaphthalene as an off-white solid, m.p. 64-69°C; ¹H NMR (CDCI₃) δ 1.3 (s, 9H), 1.34 (s, 9H), 1.45 (s, 9H), 2.68 (dd, 1 H), 2.93 (dd, 1 H), 3.6-375 (m, 1 H), 4.1-4.3 (m, 2H), 4.63 (br s, 1 H), 6.03 (d, 1 H), 6.98 (d,1 H), 7.15-7.35 (m, 6H).

### Step 3: 1-Aminomethyl-5,6-bis(trimethylacetoxyl-3-phenyl-3,4-dihydronaphthalene hydrochloride

N-t-Butyloxycarbonyl-1-aminomethyl-5,6-bis(trimethylacetoxy)-3-phenyl-3,4-dihydronaphthalene (14 g, 26 mmol), from Step 2, was dissolved in 75 mL of dioxane and saturated with anhydrous hydrogen chloride. The reaction mixture was stirred for 2 h and concentrated in vacuo. The solid residue was dissolved in a minimum amount of methanol and the methanol solution was added dropwise to an excess amount (500 mL) of diethyl ether. The precipitate was filtered, washed with diethyl ether and dried to give 9.1 g (90% yield) of 1-aminomethyl-5,6-bis(trimethylacetoxy)-3-phenyl-3,4-dihydronaphthalene hydrochloride as a white powder, m.p. 210-212°C; ¹H NMR (d₆-DMSO) 8 1.25 (s, 9H), 1.28 (s, 9H), 2.61 (dd, 1H), 2.89 (dd, 1H), 3.75-3.85 (m, 1H), 3.99 (s, 2H), 6.28 (d, 2H), 7.15 (d, 1H), 7.2-7.35 (m, 5H), 7.37 (d, 1H), 8.37 (br s, 3H).

### Example 16

### 1-Aminomethyl-5,6-bis(benzoyloxy)-3-phenyl-3,4-dihydronaphthalene hydrochloride

Following the procedures described in Example 15, replacing trimethylacetyl chloride with benzoyl chloride the title compound was prepared, m.p. 173-182°C; ¹H NMR (CD₃0D) 8 2.88 (t, 1H), 3.05 (dd, 1H), 3.65-3.8 (m, 2H), 4.0-4.1 (br s, 1 H+CH30H), 6.35 (d, 1 H), 7.05-7.35 (m, 10H), 7.35-7.55 (m, 3H), 7.9-8.0 (m, 4H), 8.5-8.65 (br s, 3H).

### Example 17

### [1 R,3S) 1-Aminomethyl-5,6-dihydroxy-3-phenyl-1,2,3,4-tetrahydronaphthalene hydrobromide

### Step 1: [1R,3S) 1-Aminomethyl-5,6-dimethoxy-3-phenyl-1,2,3,4-tetrahydronaphthalene hydrochloride

To 0.2 g (0.67 mmol) of 1-aminomethyl-5,6-dimethoxy-3-phenyl-3,4-dihydronaphthalene hydrochloride, from Step 2, of Example 2, was added 0.05 g of 10% palladium supported on carbon. The reaction mixture was sealed under hydrogen and stirred overnight at ambient temperature.The reaction mixture was flushed with nitrogen before it was filtered through Celite® filter aid and washed with 15 mL of absolute ethanol and 15 mL of methylene chloride The filtrate was concentrated to give 0.2 g (100% yield) of [1R,3S] 1-aminomethyl-5,6-dimethoxy-3-phenyl-1,2,3,4-tetrahydronaphthalene hydrochloride, m.p. 230-231°C; ¹ H NMR (dₛ-DMSO) 8 2.15-2.25 (m, 1 H), 2.5-2.65 (m, 1 H), 2.8-2.95 (m, 2H), 3.0-3.1 (m, 1 H), 3.1-3.4 (m, 1 H), 3.45-3.5 (m, 1 H), 3.66 (s, 3H), 3.78 (s, 3H), 6.95 (d, 1 H), 7.12 (d, 1 H), 7.2-7.3 (m, 1 H), 7.35-7.45 (m, 4H), 8.0 (br s, 3H).

### Step 2: [1R,3S] 1-Aminomethyl-5,6-dihydroxy-3-phenyl-1,2,3,4-tetrahydronaphthalene hydrobromide

[1R,3S] 1-Aminomethyl-5,6-dimethoxy-3-phenyl-1,2,3,4-tetrahydronaphthalene hydrochloride (0.2 g, 0.67 mmol), from Step 1, was suspended in 13 mL of methylene chloride and the suspension was cooled to 78°C in a dry ice/acetone bath. Boron tribromide (3 mL of a 1 M solution in methylene chloride, 3 mmol) was added and the reaction mixture was allowed to warm to ambient temperature, kept at ambient temperature for 1.5 h then cooled to -78°C. Methanol (3 mL) was added to the reaction mixture and it was again allowed to warm to ambient temperature then concentrated in vacuo. The residue was redissolved in methanol and reconcentrated. The residue was again redissolved in methanol and the methanol solution was added to a large excess of diethyl ether. The resultant precipitate was filtered and recrystallized from ethanol/diethyl ether to give 0.14 g (64% yield) of the title compound as a white powder, m.p. 256-259°C; 1H NMR (d₆-DMSO) δ 1.63 (q, 1 H), 2.1-2.25 (m, 1 H), 2.4-2.5 (m, 1 H), 2.75-2.95 (m, 2H), 3.02 (dd, 1 H), 3.15-3.3 (m, 1 H), 3.4-3.5 (m, 1 H), 6.68 (s, 2H), 7.2-7.3 (m, 1 H), 7.3-7.4 (m, 4H), 7.8 (br s, 3H), 8.2 (br s, 1 H), 9.1 (brs, 1 H).

### Example 18

### 1-Aminomethyl-3-cyclohexyl-5,6-dihydroxy-3,4-dihydronaphthalene hydrobromide

### Step 1: Ethyl 3-cyclohexylpropenoate

Sodium hydride (2.6 g, 108 mmol) was added to 100 mL of THF and 19.8 mL (98.9 mmol) of triethylphosphonoa- cetate, commercially available from Aldrich Chemical Company, was added dropwise at 0°C. The reaction mixture was stirred for 1 h at ambient temperature and 12.1 mL (99.9 mmol) of cyclohexanecarboxaldehyde, commercially available from Aldrich Chemical Company, was added. The reaction mixture was heated at reflux temperature for 15 min then cooled and filtered. The filtrate was concentrated under reduced pressure and the product was distilled at 140°C (15 Torr) to give 15.2 g (84% yield) of ethyl 3-cyclohexylpropenoate as a clear liquid; ¹H NMR (CDCI₃) 6 1.1-1.4 (m, 6H), 1.3 (t, 3H), 1.6-1.8 (m, 5H), 2.05-2.2 (m, 1 H), 4.2 (q, 2H), 5.75 (d, 1 H), 6.92 (d, 1 H).

### Step 2: 1-Aminomethyl-3-cyclohexyl-5,6-dimethoxy-3,4-dihydronaphthalene hydrochloride

2-(2',3'-Dimethoxyphenyl)-1,3-dithiane, from Step 1 of Example 1, Method B, and ethyl 3-cyclohexylpropenoate, from Step 1 of this Example, were condensed as described in Step 2 of Example 1, Method B. The adduct was treated with Raney nickel and sodium hydroxide to give the corresponding acid. The acid was cyclized with polyphosphoric acid as described in Step 6 of Example 1, Method A, to give 3-cyclohexyl-5,6-dimethoxy-1,2,3,4-tetrahydronaphthalen-1 -one. This ketone was treated with trimethylsilylcyanide in the presence of aluminum chloride and reduced with lithium aluminum hydride as described in Step 1 of Example 2 to give 1-aminomethyl-5,6-dimethoxy-1-hydroxy-3-cyclohexyl-1,2,3,4-tetrahydronaphthalene. The hydroxy group was eliminated by treatment with anhydrous hydrogen chloride in isopropyl alcohol as described in Step 2 of Example 2 to give 1-aminomethyl-3-cyclohexyl-5,6-dimethoxy-3,4-dihydronaphthalene hydrochloride, m.p. 178-179°C; ¹H NMR (d⁶⁻DMSO) 8 1.0-1.4 (m, 7H), 1.5-1.9 (m, 6H), 2.0-2.2 (m, 1 H), 2.5 (dd, 1 H), 2.7 (dd, 1 H), 3.6 (s, 3H), 3.81 (s, 3H), 5.8 (d, 1 H), 6.6 (m, 2H).

### Step 3: 1-Aminomethyl-3-cyclohexyl-5,6-dihydroxy-3,4-dihydronaphthalene hydrobromide

1 -Aminomethyl-3-cyclohexyl-5,6-dimethoxy-3,4-dihydronaphthalene hydrochloride (2.7 g, 8.9 mmol), from Step 2, was dissolved in 72 mL of methylene chloride and cooled to -78°C. Boron tribromide (36 mL of a 1 M solution in methylene chloride) was added and the reaction mixture was warmed to 0°C for 1 h. The reaction mixture was cooled again to -78°C and 30 mL of methanol was added. After stirring at ambient temperature for 1 h, the reaction mixture was concentrated, diluted with methanol and reconcentrated. The residue was dissolved in methanol and the methanol solution was added dropwise to an excess amount of diethyl ether. The precipitate was filtered and recrystallized from etha- nol/etherto give 2.2 g ( 79% yield) of the title compound, m.p. 212-213°C; ¹H NMR (d₆-DMSO) δ 1.0-1;4 (m, 7H), 1.55-1.9 (m, 6H), 2.05-2.15 (m, 1 H), 2.47 (dd, 1H),2.74(dd, 1H), 5.83 (d, 1 H), 6.60 (m, 2H).

### Example 19

### [1R,3S] 1-Aminomethyl- 3-cyclohexyl-5,6-dihydroxy-1,2,3,4-tetrahydronaphthalene hydrobromide

### Step 1: [1R,3S] 1-Aminomethyl-3-cyclohexyl-5,6-dimethoxy-1,2,3,4-tetrahydronaphthalene hydrochloride

1 -Aminomethyl-3-cyclohexyl-5,6-dimethoxy-3,4-dihydronaphthalene hydrochloride (1 g, 3.3 mmol), from Step 2 of Example 18, was dissolved in 20 mL of ethanol and 0.25 g of 10% palladium on carbon was added to the ethanol solution. The reaction mixture was sealed under one atmosphere of hydrogen and shaken at ambient temperature for 24 h. The reaction mixture was filtered to remove the catalyst and concentrated to give 1 g (100% yield) of the title compound, m.p. 282-283°C; ¹H NMR (d₆-DMSO) δ 1.0-1.5 (m, 8H), 1.5-1.9 (m, 5H), 2.0-2.2 (m, 2H), 2.7-3.1 (m, 3H), 3.3-3.4 (m, 1 H), 3.8 (s, 3H), 3.9 (s, 3H), 6.4-6.8 (m, 2H).

### Step 2: [1R,3S] 1-Aminomethyl-3-cyclohexyl-5,6-dihydroxy-1,2,3,4-tetrahydronaphthalane hydrobromide

[1R, 3S] 1-Aminomethyl-3-cyclohexyl-5,6-dimethoxy-1,2,3,4-tetrahydronaphthalene hydrochloride (0.7 g, 2.3 mmol), from Step 1, was suspended in 20 mL of methylene chloride at -78°C. Boron tribromide (9.7 mL of a 1 M solution in methylene chloride, 9.7 mmol) was added and the reaction mixture was allowed to warm to ambient temperature. After stirring at ambient temperature for 1 h, the reaction mixture was cooled to -78°C and 10 mL of methanol was added. The reaction mixture was again allowed to warm to ambient temperature and stirred at ambient temperature for 1 h. The solvent was removed in vacuo and methanol was added tothe residue. The methanol solution was concentrated and the residue dissolved in a minimal amount of methanol and added dropwise to a large excess of diethyl ether. The precipitate was filtered and recrystallized from ethanol/diethyl ether to give 0.48 g (65% yield) of [1R,3S] 1-aminomethyl-3-cyclohexyl-5,6-dihydroxy-1,2,3,4-tetrahydronaphthalene hydrobromide, m p. 203-204°C; ¹H NMR (d₆-DMSO) 8 0.9-1.5 (m, 8H), 1.6-1.9 (m, 5H), 2.0-2.1 (m, 2H), 2.7-3.0 (m, 3H), 3.3-3.4 (m, 1 H), 6.5-6.7 (m, 2H).

### Examples 20 - 22

Following the synthesis outlined in Example 18, using the appropriate aldehyde, Examples 20 - 22 were made, as their hydrobromide salts, as disclosed in Table 2. The structure of each was confirmed by melting point (m.p.), elemental analysis and mass spectra as designated. Example 22, as disclosed in Table 2, was prepared, using the appropriate aldehyde, as described in Examples 18 and 19, as its hydrobromide salt. The structure was confirmed by melting point (m.p.), elemental analysis and mass spectra as designated.

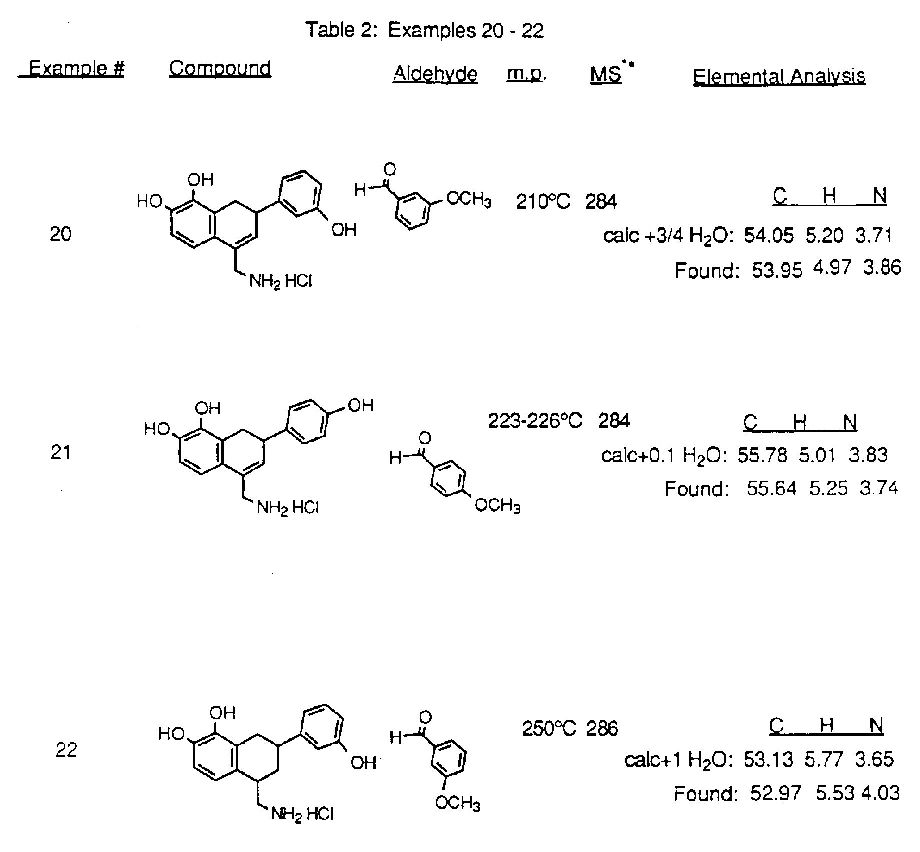

### Example 23

### 1-Aminomethyl-5,6-dihydroxy-2-(2'-hydroxy-1'-ethyl)-3-phenyl-3,4-dihydronaphthalene hydrobromide

### Step 1: 2-(Carboethoxy)methyl-5,6-dimethoxy-3-phenyl-1,2,4-tetrahydronaph-thalen-1-one

To a solution of 5,6-dimethoxy-3-phenyl-1,2,3,4-tetrahydronaphthalen-1-one (5 g, 17.7 mmol, 1.0 equivalents), the product of Example 1, in 150 mL of dry THF cooled to -78°C, was added 19.5 mL of lithium bis(trimethylsilyl)amide (1 M solution in THF, 19.5 mmol, 1.1 equivalent). The resultant solution was stirred at -78°C for 1 h and then ethyl bromoacetate (2.2 mL, 19.5 mmol, 1.1 equivalent) was added in one portion. The reaction solution was then allowed to warm to ambient temperature and was stirred for 3 h. The reaction was quenched by the addition of 50 mL of saturated ammonium chloride solution. The resultant light yellow colored THF layer was separated and evaporated to an oil. The oil was taken up into 200 mL of methylene chloride and the methylene chloride solution was washed with 2 X 50 mL of water and 50 mL of brine, dried over anhydrous magnesium sulfate, filtered and concentrated in vacuo. The residue was chromatographed on silica gel eluted with ethyl acetate/hexane (1:6) to give 5.4 g (83% yield) of the title compound as a white solid; 1 H NMR (CDCI₃) 8 1.2 (t, 3H, J=7.5 Hz), 2.45 (m, 2H), 3.03 (m, 1 H), 3.30 (m, 2H), 3.43 (m, 1 H), 3.78 (s, 3H), 3.93 (s, 3H), 4.07 (m, 2H), 6.92 (d, 1 H, J=9.0 Hz), 7.32 (m, 5H), 7.89 (d, 1 H, J=9.0 Hz). Analysis calculated for C₂₂H₂₄O₅ C, 71.72; H, 6.57. Found: C, 71.39; H, 6.63.

### Step 2: 9b-Cyano-6,7-dimethoxy-2-oxo-4-phenyl-2,3,3a,4,5,9b-hexahydronaphtho[1.2b]furan

To a solution of 2-(carboethoxy)methyl-5,6-dimethoxy-3-phenyl-1,2,3,4-tetrahydronaphthalen-1-one (1.0 g, 2.7 mmol, 1.0 equivalent) from Step 1, in 10 mL of anhydrous toluene at ambient temperature was added 5.4 mL of diethylaluminum cyanide (1 M solution in toluene, 5.4 mmol, 2.0 equivalents) The resultant solution was stirred at ambient temperature for 1 h and then poured with vigorous stirring into a mixture of 15 mL of concentrated hydrochloric acid and 70 mL of ice water. The organic layer was diluted with 75 mL of methylene chloride and then separated from the aqueous layer. The organic layer was washed with 25 mL of 2 M anhydrous hydrochloric acid solution, 25 mL of water and 25 mL of brine, dried over anhydrous magnesium sulfate, filtered and concentrated to give 1 g (105% crude yield) of the title compound as an oily residue. The crude product was carried on to the next step without purification; ¹H NMR (CDCI₃) δ 2.45 (d, 1 H, J=18.0 Hz), 2.72 (m, 2H), 3.05 (m, 1 H), 3.23 (m, 1 H), 3.37 (m, 1 H), 3.79 (s, 3H), 3.94 (s, 3H), 7.0 (d, 1 H), 7.30 (m, 5H), 7.52 (d, 1 H, J=9.0 Hz).

### Step 3: 1-Aminomethyl-5,6-dimethoxy-2-(2'-hydroxy-1'-ethyl)-3-phenyl-3,4-dihydronaphthalene hydrochloride

To a stirred solution of 9b-cyano-6,7-dimethoxy-2-oxo-4-phenyl-2,3,3a,4,5,9b-hexahydronaphtho[1,2b]furan (1.0 g, 2.86 mmol, 1.0 equivalent), from Step 2, in 30 mL of anhydrous THF, was added lithium aluminum hydride (0.22 g, 5.7 mmol, 2.0 equivalents) from a solid addition funnel. After the addition was complete, the reaction mixture was heated at reflux for 3 h. The reaction mixture was then cooled, poured into a 250 mL Erlenmeyer flask and was diluted with 50 mL of anhydrous THF The reaction was quenched by the addition of excess sodium sulfate decahydrate. The resultant suspension was filtered through Celite® filter aid and the filter cake was washed with 100 mL of hot THF. The filtrate was concentrated to a light amber colored foam. This foam was dissolved in a solution of 3 M anhydrous hydrochloric acid in isopropanol and the resultant solution was heated at reflux for 18 h. The solution was then concentrated in vacuo. The residue was triturated with methylene chloride/diethyl ether (1: 1) to give 250 mg (25% yield) of the title compound; ¹H NMR (CDC1₃) δ 2.03 (m, 1 H), 2.63 (m, 1 H), 3.12 (m, 2H), 3.37 (s, 3H), 3.52 (m, 3H), 3.71 (s, 3H), 3.80 (m, 1 H), 4.18 (m, 2H), 5.28 (br s, 1 H), 6.23 (d, 1 H, J=8 Hz), 6.95-7.4 (m, 6H). Analysis calculated for C₂₁ H₂₆Cl N0₃+1.5 H₂O: C, 54.16; H, 6.40; N, 2.87. Found: C,54.15; H, 6.01; N,3.01.

### Step 4: 1-Aminomethyl-5.6-dihydroxy-2-(2'-hydroxy-1'-ethyl)-3-phenyl-3,4-dihydronaphthalene hydrobromide

To a solution of 1-aminomethyl-5,6-dimethoxy-2-(2'-hydroxy-1'-ethyl)-3-phenyl-3,4-dihydronaphthalene hydrochloride (60 mg, 0.177 mmol, 1.0 equivalent), from Step 3, in 2 mL of anhydrous methylene chloride cooled to -78°C was added 350 µL of boron tribromide (1.0 M solution in methylene chloride, 0.35 mmol, 2.0 equivalents) The resultant solution was allowed to warm to ambient temperature gradually over a period of 2 h and then it was again cooled to -78°C and quenched by the addition of 20 mL of anhydrous methanol. The solid was filtered and recrystallized from a mixture of methanol, methylene chloride and diethyl ether to give 50 mg (80% yield) of the title compound as a white powder, m.p. 252-255°C; DCI MS M/Z: 312 (M+H)⁺; I.R. (KBr): 3400, 1600, 1490, 1280, 1200, 700 cm⁻¹ ; ¹H NMR (CD₃0D) δ 2.22 (m, 1 H), 2.75 (m, 1 H), 2.93 (m, 1 H), 3.30 (m, 1 H), 3.70 (m, 3H), 4.13 (d, 1 H, J=12 Hz), 4.28 (d, 1 H, J=13.5 Hz), 6.70 (d, 1H, J=₇.₅ Hz), 6.85 (d, 1H, J=7.5 Hz), 7.10 (m, 5H). Analysis calculated for C₁₉H₂₂BrNO₃+0.5 CH₂Cl₂: C, 53.87; H, 5.33; N, 3.22. Found: C, 53.54; H, 5.24; N, 3.21.

### Example 24

### [1R,2S] 1-Aminomethyl-5,6-dihydroxy-2-(2'-hydroxy-1'-ethyl)-3-phenyl-1,2,3,4-tetrahypronaphthalene formic acid salt

### Step 1: [1R,2S] 2-(Carboethoxy)methyl-1-cyano-5,6-dimethoxy-3-phenyl-3,4-dihydronaphthalene

9b-Cyano-6,7-dimethoxy-2-oxo-4-phenyl-2,3,3a,4,5,9b-hexahydronaphtho[1,2b]furan (5.0 g, 14.31 mmol, 1.0 equivalents), the product of Step 2 of Example 23, was dissolved in 120 mL of a solution of 2 M anhydrous hydrochloric acid in ethanol and the resultant solution was heated at reflux for 2 h. The solvent was evaporated in vacuo and the residue was dissolved in 120 mL of methylene chloride. The methylene chloride solution was washed with 2 X 25 mL of saturated aqueous sodium bicarbonate solution, 25 mL of water and 25 mL of brine, dried over anhydrous magnesium sulfate, filtered and concentrated in vacuo to give 5 g (93% yield) of the title compound as a colorless oil; ¹ H NMR (CDCl₃) 8 1 22 (t, 3H, J=7.5 Hz), 3.24 (m, 3H), 3.54 (s, 3H), 3.70 (d, 1H, J=15 Hz), 3.58 (s, 3H), 3.90 (m, 1 H), 4.08 (m, 2H), 6.82 (d, 1 H, J=₇.0 Hz), 7.03 (m, 2H), 7.20 (m, 3H), 7.30 (d, 1 H, J=₇.0 Hz).

### Step 2: [1R.2S] 1 -Aminomethyl-5,6,dimethoxy-2-(2'-hydroxy-1'-ethyl)-3-phenyl-1,2,3,4-tetrahydronaphthalene

To a stirred solution of 2-(carboethoxy)methyl-1 -cyano-5,6-dimethoxy-3-phenyl-3,4-dihydronaphthalene (2 g, 5.3 mmol, 1.0 equivalent), from Step 1, in 50 mL of anhydrous methanol at ambient temperature, was added 5.13 g (212 mmol, 40 equivalents) of magnesium powder. The resultant mixture was stirred at ambient temperature for 2 h and then it was cooled to 0°C. The reaction was quenched by the slow addition of 150 mL of 2 N aqueous hydrochloric acid solution. The aqueous solution was extracted with 4 X 50 mL of methylene chloride. The combined organic layers were washed with 50 mL of water and 50 mL of brine, dried over anhydrous magnesium sulfate, filtered and concentrated to a colorless oil. The oil was dissolved in 75 mL of anhydrous THF To this solution at 0°C was added 0.4 g (10.5 mmol, 2.0 equivalents) of lithium aluminum hydride. The resultant mixture was heated at reflux for 4 h and then diluted with 100 mL of anhydrous THF. The reaction was quenched by the addition of sodium sulfate decahydrate. The reaction mixture was then filtered through Celite® filter aid and the filter cake washed thoroughly with hot THF. The filtrate was concentrated in vacuo. The residue was chromatographed on silica gel eluted with methylene chloride/methanol/ ammonium hydroxide (89:9;1) to give 1.15 g (63% yield) of the title compound as a 1:1 mixture of diastereomers; ¹H NMR (CDCl₃) 6 1.15-1.5 (m, 4H), 1.67 (m, 1 H), 1.90 (m, 1 H), 2.20 (m, 1 H), 2.35 (m, 1 H), 2.45 (m, 1 H), 2.55-3.05 (m, 6H), 3.18 (m, 4H), 3.50 (m, 4H), 3.73 (s, 3H), 3.75 (m, 1H), 3.83 (s, 3H), 3.87 (s, 6H), 6.80 (m, 2H), 6.92 (m, 2H), 7.2-7.4 (m, 10H)

### Step 3: [1R.2S]1-Aminomethyl-5.6-dihydroxy-2-(2'-hydroxy-1'-ethyl)-3-phenyl-1.2.3.4-tetrahydronaphthalene formic acid salt

To a stirred solution of 1 -aminomethyl-5,6-dimethoxy-2-(2'-hydroxy- 1'-ethyl)-3-phenyl-1,2,3,4-tetrahydronaphthalene (0.6 g 1.76 mmol, 1.0 equivalents), from Step 2, in 18 mL of anhydrous methylene chloride cooled to -78°C, was added 3.52 mL of boron tribromide (1M solution in methylene chloride, 3.52 mmol, 2.0 equivalents). The resultant reaction mixture was stirred at -78°C for 1 h and then it was allowed to warm to ambient temperature. The reaction mixture was stirred at ambient temperature for 1 h and cooled again to -78°C. The reaction was quenched by the addition of 50 mL of anhydrous methanol. The resultant solution was stirred at ambient temperature for 1 h and then concentrated in vacuo. The residue was chromatographed on silica gel eluted with ethyl acetate/formic acid/water (18:1:1) to give the title compound as a light tan colored powder, m.p. 190°C (dec); DCI MS M/Z: 312 (M+H)⁺; I.R. (KBr): 3400, 3240, 1600, 1490, 1290, 1050, 700 cm⁻¹; ¹H NMR (CD₃OD) δ 1.21 (m, 1H), 1.50 (m, 2H), 1.67 (m,1 H),2.15(m, 1 H), 2.22 (m, 1 H), 2.53 (m, 1 H), 2.63 (m, 1 H), 3.10 (m, 8H), 3.43 (m, 2H), 3.50 (m, 2H), 7.6 (m, 2H), 7.72 (m, 2H), 7.23 (m, 4H), 7.35 (m, 6H), 8.51 (s, 2H). Analysis calculated for C₁₉H₂₃NO₃+2.5 HC0₂H: C, 56.96; H, 6.37; N, 2.45. Found: C, 56.72; H, 6.08; N, 3.30.

### Example 25

### 1-Aminomethyl-5,6-dihydroxy-3-phenyl-2-n-propyl-3,4-dihydronaphthalene hydrobromide

### Step 1: 5,6-Dimethoxy-3-phenyl-2-(1-prop-2-enyl)-1,2,3,4-tetrahydronaphthalen-1-one

To a solution of 5,6-dimethoxy-3-phenyl-1,2,3,4-tetrahydronaphthalen-1-one (5 g, 17.7 mmol, 1.0 equivalents), the product of Example 1, in 70 mL of dry THF cooled to -78°C under a nitrogen atmosphere, was added 19.5 mL of lithium bis(trimethylsilyl)amide (1 M solution in THF, 19.5 mmol, 1.1 equivalent). The resultant solution was stirred at -78°C for 0.75 h and then allyl bromide (2.25 g, 18.6 mmol) was added in one portion. The reaction mixture was allowed to slowly warm to ambient temperature. After stirring at ambient temperature for 16 h, the reaction was quenched by the addition of 200 mL of 2 M aqueous ammonium chloride solution and the mixture was extracted several times with ethyl acetate/hexane (1:1). The combined organic layers were washed with 200 mL of water, dried over anhydrous magnesium sulfate, filtered and concentrated to give 6.7 g of an oil. The oil was purified by flash chromatography on silica gel eluted with 10% ethyl acetate in hexane to give 2.4 g (42% yield) of the title compound as a 2:1 mixture of diastereomers; DCI MS M/Z: 323 (M+H)⁺; ¹H NMR (CDCl₃) 8 2.05-2.19 (m, 3H), 2.4-2.52 (m, 1 H) 2.65-2.75 (m, 2H), 2.85-3.00 (m, 5H), 3.20-3.42 (m, 7H), 3.65-3.72 (m, 1 H), 3.77 (s, 6H), 3.80 (s, 2H), 3.94 (s, 6H), 3.96 (s, 3H), 4.80-5.00 (m, 6H), 5.65-5.80 (m, 3H), 6.92 (d, 2H, J=9 Hz), 6.94 (d, 1H, J=9 Hz), 7.18-7.40 (m, 15H), 7.89 (d, 2H, J=9 Hz), 7.92 (d, 1H, J=9 Hz)

### Step 2: 1-Aminomethyl-5,6-dimethoxy-1-hydroxy-3-phenyl-2-(1-prop-2-enyl)-1,2.3,4-tetrahydronaphthalene

To a solution of 5,6-dimethoxy-3-phenyl-2-(1-prop-2-enyl)-1,2,3,4-tetrahydronaphthalen-1-one (15.9 g 49.4 mmol) in 100 mL of THF at ambient temperature, was added 7.0 mL of lithium cyanide (0.5 M in DMF, 49.4 mmol) followed by 9.8 g (98.8 mmol) of trimethylsilylcyanide (TMSCN). The resultant mixture was stirred for 24 h and then solvents and excess TMSCN were removed under reduced pressure. The residue was taken up in 100 mL of THF. The THF solution was added dropwise over a 40 min period to a suspension of (4.2 g, 110 mmol) of lithium aluminum hydride in 100 mL of refluxing THF under a nitrogen atmosphere. The resultant mixture was heated at reflux for 6 h, cooled to ambient temperature and diluted with THF The reaction was quenched by the portion-wise addition of sodium sulfate decahydrate. The addition was complete when the evolution of hydrogen gas was no longer observed. The mixture was filtered through Celite® filter aid and the filtrate concentrated to give 21 g of a dark yellow colored oil. The oil was purified by flash chromatography on silica gel eluted with 1% concentrated ammonium hydroxide in ethyl acetate to give 10.01 g (57% yield) of the title compound as a mixture of diastereomers; DCI MS M/Z: 323 (M+H)⁺.

### Step 3: 5-[5',6'-Dimethoxy-3'-phenyl-2'-(1-prop-2-enyl)-1',2',3',4'-tetrahydro-1'-naphthyl)-2-oxo-1,3-oxazolidine

To a solution of 1-aminomethyl-5,6-dimethoxy-1-hydroxy-3-phenyl-2-(1-prop-2-enyl)-1,2,3,4-tetrahydronaphthalene (3.98 g, 11.3 mmol), from Step 2, in 50 mL of acetonitrile at ambient temperature was added 2.3 g (14 mmol) of 1,1'-carbonyldiimidazole. The resultant solution was stirred for 6 h. Aqueous hydrochloric acid (200 mL of a 0.5 M solution) was added and the resultant mixture was extracted with 3X100 mL of diethyl ether. The combined organic layers were dried over anhydrous magnesium sulfate, filtered and concentrated to give 5.7 g of a yellow colored oil. The oil was purified by flash chromatography on silica gel eluted with 15% ethyl acetate in methylene chloride to give 4.21 g (98% yield) of the title compound; DCI MS M/Z: 380 (M+H)⁺, 397 (M +NH4)+; ¹H NMR (CDCI₃) 8 2.03-2.23 (m, 2H), 2.54-2.64 (m, 1H) 2.73-2.97 (m, 2H), 3.15-3.25 (m, 1H), 3.47 (d, 1H, J=9 Hz), 3.75 (s, 2H), 3.8 (s,1/2H), 3.88 (s, 2H), 3.89 (s, 1 H), 3.92 (d, 1 H, J=9 Hz), 4.75-4.93 (m, 2H), 5.30-5.32 (m, 1/2H), 5.37 -5.42 (m, 1 H), 5.02-5.18 (m, 1 H), 6.87-6.93 (m, 1H), 7.17-7.40 (m, 6H).

### Step 4: 5-[5',6'-Dimethoxy-3'-phenyl-2'-n-propyl-1',2',3',4'-tetrahydro-1'-naphthyl]-2-oxo-1,3-oxazolidine

To a solution of 205 mg (0.54 mmol) of 5-[5',6'-dimethoxy-3'-phenyl-2'-(1-prop-2-enyl)-1',2',3',4'-tetrahydro-1'-naphthyl]-2-oxo-1 ,3-oxazolidine (the product of Step 3 of Example 25) in 50 mL of ethyl alcohol at ambient temperature under a nitrogen atmosphere, was added 205 mg of 10% palladium on carbon. The reaction flask was purged with hydrogen and the reaction mixture was stirred for 8 h. The catalyst was removed by filtration and the filtrate was concentrated in vacuo to give a white solid. The solid was purified by flash chromatography on silica gel eluted with ethyl acetate to give 77 mg (37% yield) of the title compound; DCI MS M/Z: 382 (M+H)⁺, 399 (M +NH₄)⁺; 1 H NMR (CDCl₃) δ 0.65 (t, 3H, J=₇.₅ Hz), 0.89-1.01 (m, 1 H), 1.08-1.27 (m, 2H), 1.37-1.49 (m, 1 H), 2.32-2.40 (m, 1 H), 2.68-2.79 (m, 1 H)2.96 (dd, 1 H, J=18 Hz, 10.5 Hz), 3.16 (dd, 1 H, J=18 Hz, 6 Hz), 3.48 (d, 1 H, J=12 Hz), 3.72 (s, 3H), 3.87 (d, 1 H, J=12 Hz), 3.88 (s, 3H), 5.28-5.32 (m, 1 H), 6.88 (d, 1H, J=9 Hz), 7.19 (d, 1 H, J=9 Hz), 7.23-7 38 (m, 5H)

### Step 5: 1-Aminomethyl-5,6-dihydroxy-3-phenyl-2-n-propyl-3,4-dihydronaphthalene hydrobromide

To a solution of 68 mg (0.18 mmol) of 5-[5',6'-dimethoxy-3'-phenyl-2'-n-propyl-1',2',3',4'-tetrahydro-1'-naphthyl] -2-oxo-1,3-oxazolidine, from Step 4, in 2 mL of methylene chloride at -78°C under a nitrogen atmosphere, was added 0.5 mL of a 1 M solution of boron tribromide in methylene chloride. The resultant solution was stirred at -78°C for 45 min, warmed to ambient temperature, and then stirred for 4 h. The reaction mixture was then cooled to -78°C and 50 mL of cold methanol was added. The resultant solution was concentrated in vacuo. The residue was chased with 2 X 50 mL of methanol and then dried in vacuo to give a tan colored glass. The glass was dissolved in methanol and precipitated by the addition of methylene chloride and diethyl ether to give 39 mg (56% yield) of the title compound; DCI MS M/Z; 310 (M+H)⁺; 327 (M +NH₄)⁺; I.R. (KBr): 3410, 3230, 2960, 1600, 1490, 1285; 1185, 805, 695 cm⁻¹; ¹H NMR (CD₃0D) δ 0.93 (t, 3H, J=7.5 Hz), 1.36-1.56 (m, 2H), 2.03-2.16 (m, 1H), 2.35-2.48 (m, 1H), 2.92 (dd, 1H, J=16 Hz, 7 Hz), 3.26 (dd, 1 H J=16 Hz, 3 Hz), 4.13 (d 1 H, J=15 Hz), 4.22 (d, 1 H J=15 Hz), 6.70 (d, 1 H, J=9 Hz), 6.76 (d, 1 H, J=9 Hz), 7.0.1-7.16 (m, 5H). Analysis calculated for C₂₀H₂₄BrNO₂+O.7H₂O: C, 59.62; H, 6.35; N, 3.48. Found: C, 59.48; H, 6.27; N, 3.48.

### Example 26

### [1 R,3R] 1-Aminomethyl-5,6-dihydroxy-3-phenyl-2-n-propyl-1,2,3,4-tetrahydronaphthalene formic acid salt

### Step 1: [1R,3R] 1-Aminomethyl-5,6-dimethoxy-3-phenyl-2-n-propyl-1,2,3,4-tetrahydronaphthalene

To a solution of 225 mg (0.59 mmol) of 5-[5',6'-dimethoxy-3-phenyl-2'-(1-prop-2-enyl)-1',2',3',4'-tetrahydro-1'-naphthyl]-2-oxo-1,3-oxazolidine, the product of Step 3 of Example 25, in 50 mL of ethyl alcohol at ambient temperature under a nitrogen atmosphere, was added 225 mg of 20% palladium hydroxide on carbon. The reaction flask was purged with hydrogen and the reaction mixture was stirred for 24 h at ambient temperature. The catalyst was removed by filtration and the filtrate was concentrated in vacuo to give 113 mg of a colorless solid. The solid was purified by flash chromatography on silica gel eluted with concentrated ammonium hydroxide/methanol/ethyl acetate/methylene chloride (1:3:50:50) to give 63 mg (31% yield) of the title compound; DCI MS M/Z: 340 (M+H)⁺; ¹H NMR (CDCI₃) 8 0.75 (t, 3H, J=7.5 Hz), 1.12-1.38 (m, 4H), 1.90-1.99 (m, 1H), 2.40-2.50 (m, 1 H), 2.60 (dd, 1H, J=15 Hz, 12 Hz), 2.73-2.87 (m, 2H), 2.93-3.02 (m, 1 H), 3.14 (dd, 1 H, J=₁₅ Hz, 3 Hz), 3.74 (s, 3H), 3.86 (s, 3H), 6.79 (d, 1 H, J=9 Hz), 6.95 (d, 1 H, J=9 Hz), 7.20-7.40 (m, 5H).

### Step 2: [1 R,3R] 1-Aminomethyl-5,6-dihydroxy-3-Phenyl-2-n-propyl-1,2,3,4-tetrahydronaphthalene formic acid salt

To a solution of 75 mg (0.22 mmol) of [1R,3R] 1-aminomethyl-5,6-dimethoxy-3-phenyl-2-n-propyl-1,2,3,4-tetrahydronaphthalene, from Step 1, in 3 mL of methylene chloride at -78°C under a nitrogen atmosphere, was added boron tribromide (0.7 mL of a 1 M solution in methylene chloride, 0.7 mmol). The resultant solution was stirred at -78°C for 40 min, warmed to ambient temperature and then stirred for 4 h at ambient temperature. The reaction mixture was cooled to -78°C and the reaction was quenched by the addition of cold methanol. The resultant solution was concentrated in vacuo. The residue was chased with methanol (2 X 50 mL) and purified by flash chromatography on silica gel eluted with formic acid/water/ethyl acetate (1:1:18) to give 57 mg (73% yield) of the title compound; DCI MS M/Z: 312 (M+H)+; I.R. (KBr): 3420, 1600, 1380, 1350, 765, 700 cm⁻¹; ¹H NMR (CD₃OD) δ0.79 (t, 3H, J=6 Hz), 1.17-1.44 (m, 4H), 1.93-2.02 (m, 1 H), 2.47-2.57 (m, 1 H), 2.58-2.68 (m, 1 H), 2.96-3.13 (m, 4H), 5.62 (d, 1 H, J=8 Hz), 6.72 (d, 1 H, J=8 Hz), 7.19-7.37-7.40 (m, 5H), 8.53 (br s, 1.5H). Analysis calculated for C₂₁ H₂₇NO₄+0.6HBr+0.7 HCO₂H: C, 59.48; H, 6.67; N, 3.20. Found: C, 59.52; H, 6.45; N, 3.40.

### Example 27

### 1 -Aminomethyl-5,6-dihydroxy-2-(3'-hydroxy-1'-propyl)-3-phenyl-3.4-dihydronaphthalene formic acid salt

### Step 1: 5-[5',6'-Dimethoxy-2'-(3-hydroxy-1-n-propyl)-3'-phenyl-1',2',3',4'-tetrahydro-1'-naphthyl] -2-oxo-1,3-oxazolidine

To a solution of 1 02 g (2.6 mmol) of 5-[5',6'-dimethoxy-2'-(1-prop-2-enyl)-3'-phenyl-1',2',3',4'-tetrahydro-1'-naphthyl] -2-oxo-oxazolidine, the product of Step 3 of Example 25, in 8 mL of THF at -78°C under nitrogen, was added 2.3 mL of a 1 M solution of borane in THF (2.3 mmol). The reaction mixture was heated to ambient temperature and stirred at ambient temperature for 4 h. At this time 9 mL of 3M aqueous sodium hydroxide solution was added, followed by 9 mL of 30% aqueous hydrogen peroxide solution and then 10 mL of THF The resultant mixture was stirred for 5 h and excess 10% aqueous sodium hydrogen sulfite was added. The resultant mixture was added to 1 M aqueous hydrogen chloride solution and the aqueous mixture was extracted with ethyl acetate. The organic layer was dried over anhydrous magnesium sulfate, filtered and concentrated to give 1 g of a white solid. The solid was purified by flash chromatography on silica gel eluted with ethyl acetate/methylene chloride (1: 1) to give 359 mg, (35% yield) of the title compound; DCI MS M/Z: 398 (M+H)⁺, 415 (M +NH4)+; 1 H NMR (CDCl₃) δ 1.13-1,43(m, 4H), 2.33-2.42 (m, 1 H), 2.70-2.82 (m, 1 H), 3.18 (dd, 1H, J=15 Hz, 6 Hz), 3.31-3.46 (m, 2H), 3.48 (d, 1H, J=8 Hz), 3.73 (s, 3H), 3.88 (s, 3H), 3.89 (d, 1H, J=8 Hz), 5.43 (br s, 1 H), 6.89 (d, 1 H, J=9 Hz), 7.19 (d, 1 H, J=9 Hz), 7.24-7.40 (m, 5H).

### Step 2: 1-Aminomethyl-5,6-dihydroxy-2-(3'-hydroxy-1'-propyl)-3-phenyl-3,4-dihydronaphthalene formic acid salt

To a solution of 200 mg (0.5 mmol) of 5-[5',6'-dimethoxy-2'-(3-hydroxy-1-n-propyl)-3'-phenyl-1',2',3',4'-tetrahy- dro-1'-naphthyl]-2-oxo-1,3-oxazolidine, from Step 1, in 6 mL of methylene chloride at -78°C under a nitrogen atmosphere, was added 1.5 mL of a 1 M solution of boron tribromide in methylene chloride (1.5 mmol). The resultant solution was stirred at -78°C for 8 h and then it was stirred at -30°C for 14 h. The reaction mixture was cooled to -78°C and cold methanol was added to quench the reaction. The resultant solution was concentrated in vacuo and the residue was chased with 2 X 50 mL of methanol. The residue was then purified by flash chromatography on silica gel eluted with formic acid/water/ethyl acetate (1:1:18) to give 47 mg (25% yield) of the title compound; FAB MS M/Z: 326 (M+H)⁺, 348 (M +Na)⁺; I.R. (KBr): 3390, 2930, 1618, 1350, 1290, 770, 700 cm⁻¹; ¹H NMR (CD₃OD) δ 1.70 (m, 2H), 2.20 (m, 1H), 2.54-2.67 (m, 1H), 2.93 (dd, 1H J=15 Hz, 7.5 Hz), 3.27 (m, 1 H), 3.54 (m, 2H), 3.67 (m, 1H), 4.17 (d, 1 H, J=14 Hz), 4.25 (d, 1H, J=14 Hz), 6.70 (d, 1H, J=7.5 Hz), 7.02-7.16 (m, 5H), 8.47 (br s, 2H). Analysis calculated for C₂₁H₂₅rNO₅+0.7HBr+0.9 HCO₂H: C, 56.03; H, 5.90; N, 2.98. Found: C, 56.18; H, 5.64; N, 2.97.

### Example 28

### 1-Aminomethyl-2-(2'-bromo-1'-propyl)-5,6-dihydroxy-3-phenyl-3,4-dihydronaphthalene hydrobromide

To a solution of 85 mg (0.21 mmol) of 5-[5',6'-dimethoxy-2'-(3-hydroxy-1-n-propyl)-3'-phenyl-1',2',3',4'-tetrahy- dro-1'-naphthyl]-2-oxo-1,3-oxazolidine, the product of Step 1 of Example 27, in 3 mL of methylene chloride at -78°C under a nitrogen atmosphere, was added 0.9 mL of a 1 M solution of boron tribromide in methylene chloride (0.9 mmol). The resultant solution was warmed to ambient temperature and stirred at ambient temperature for 7 h. The reaction mixture was cooled to -78°C and cold methanol was added to quench the reaction. The resultant solution was concentrated in vacuo and the residue was chased with 2 X 50 mL of methanol. The residue was then dried in vacuo and dissolved in methanol. Methylene chloride and diethyl ether were added to the methanol solution to precipitate 40 mg (40% yield) of the title compound; DCI MS M/Z: 398 (M+H)+, 415 (M +NH₄)+; I.R. (KBr): 3310, 3220, 3030, 1495, 1290, 1185, 810, 700 cm⁻¹; ¹H NMR (CD₃OD) δ 1.84-2.00 (m, 2H), 2.28-2.30 (m, 1H), 2.53-2.65 (m, 1H), 2.94 (dd, 1H, J=15 Hz, 7.5 Hz), 3.26 (m, 1 H), 3.42 (m, 2H), 3.65 (dd, 1 H, J=₇.₅, 2 Hz Hz), 4.19 (d, 1 H, J=14 Hz), 4.28 (d, 1 H, J=14 Hz), 6.72 (d, 1 H, J=8 Hz), 6.78 (d, 1 H, J=8 Hz), 7.02-7.17 (m, 5H). Analysis calculated for C₂₀H₂₃Br₂NO₂+0.5H₂O: C, 50.23; H, 5 06; N, 2.93. Found: C, 50.29; H, 4.96; N, 2 84.

### Example 29

### [1R, 3R] 1-Aminomethyl-5,6-dihydroxy-2-(3'-hydroxy-1'-propyl)-3-phenyl-1,2,3,4-tetrahydronaphthalene formic acid salt

### Step 1: [1R,3R] 1-Aminomethyl-5,6-dimethoxy-2-(3'-hydroxy-1'-propyl)-3-phenyl-1,2,3,4-tetrahydronaphthalene

To a solution of 230 mg (0.58 mmol) of 5-[5',6'-dimethoxy-2'-(3-hydroxy-1-n-propyl)-3'-phenyl-1',2',3',4'-tetrahy- dro-1'-naphthyl]-2-oxo-1,3-oxazolidine, from Step 1 of Example 27, in 50 mL of ethyl alcohol under a nitrogen atmosphere, was added 100 mg of 20% palladium hydroxide on carbon. The reaction flask was purged with hydrogen and the reaction mixture was stirred for 36 h at ambient temperature. The catalyst was removed by filtration and the filtrate was concentrated in vacuo to give 200 mg of a solid. The solid was purified by flash chromatography on silica gel eluted with concentrated ammonium hydroxide/methanol/ethyl acetate/methylene chloride (1:4:50:50) to give 129 mg (63% yield) of the title compound; DCI MS M/Z: 356 (M+H)+; ¹H NMR (CD₃0D) δ 1.30-1.52 (m, 4H), 2.00-2.10 (m, 1H), 2.42-2.53 (m, 1H), 2.57-2.68 (m, 1H), 2.73-2.81 (m, 1H), 2.83-3.04 (m, 2H), 3.15 (dd, 1H, J-15 Hz, 3 Hz), 3.47 (t, 2H J=7.5 Hz), 3.74 (s, 3H), 3.87 (s, 3H), 6 80 (d, 1H, J=9 Hz), 6.96 (d, 1H, J=9 Hz), 7.18-7.37 (m, 5H).

### Step 2: [1R,3R] 1-Aminomethyl-5,6-dihydroxy-2-(3'-hydroxy-1'-propyl)-3-phenyl-1,2,3,4-tetrahydronaphthalene formic acid salt

To a solution of 130 mg (0.37 mmol) of [1R,3R] 1-aminomethyl-5,6-dimethoxy-2-(3'-hydroxy-1'-propyl)-3-phenyl-1,2,3,4-tetrahydronaphthalene, from Step 1, in 4 mL of methylene chloride at -78°C under a nitrogen atmosphere, was added 1.1 mL of a 1 M solution of boron tribromide in methylene chloride (1.1 mmol). The resultant solution was stirred at -78°C for 3.5 h and then it was stirred stirred at 0°C for 2.5 h The reaction mixture was cooled to -78°C and 50 mL of cold methanol was added to quench the reaction. The resultant solution was concentrated in vacuo and the residue was chased with 2 X 50 mL of methanol. The residue was then purified by flash chromatography on silica gel eluted with formic acid/water/ethyl acetate (1:1:18) to give 32 mg (23% yield) of the title compound; DCI MS M/Z: 328 (M+H)⁺; I.R. (KBr): 3320, 2930, 1605, 1350, 1290, 765, 700 cm⁻¹: 1H NMR (CDCl₃) δ 1.26-1.60 (m, 4H), 1.98-2.08 (m, 1 H), 2.50-2.59 (m, 1 H), 2.60-2.70 (m, 1 H), 2.98-3.18 (m, 4H), 3.35-3.46 (m, 2H), 6.63 (d, 1 H, J-9 Hz), 6.73 (d, 1 H, J-9 Hz), 7.19-7.37 (m, 5H), 8.40-8.48 (br s, 2H). Analysis calculated for C₂₁H₂₇BrNO₅+1.3HBr: C, 52.70; H, 5.96; N, 2.93. Found: C, 52.93; H, 5.68; N, 2.91.

### Example 30

### 1-Aminomethyl-5,6-dihydroxy-3-phenylnaphthalene hydrochloride

### Step 1: 1-(N-t-Butoxycarbonyl)aminomethyl-5,6-bis(acetoxy)-3-phenyl-3,4-dihydronaphthalene hydrochloride

Triethylamine (0.33 mL, 2.37 mmol) was added to a cold solution of 1 g (2.58 mmol) of 1-aminomethyl-5,6-bis (acetoxy)-3-phenyl-3,4-dihydronaphthalene hydrochloride, the product of Example 3, in 10 mL of DMF. The resultant solution was added dropwise to a solution of 1.27 mL (5.52 mmol) of trimethylacetic anhydride (commercially available from Aldrich Chemical Co.) in 2 mL of DMF. The solution was allowed to stir at 0°C for approximately 0.5 h and then at ambient temperature for 3 h Water (25 mL) was added and the resultant mixture was extracted with ethyl acetate The combined organic layers were washed successively with 2 X 15 mL of 1 N aqueous hydrochloric acid solution, 2 X 15 mL of water and 15 mL of brine, dried over anhydrous magnesium sulfate and concentrated in vacuo. The residue was purified by flash chromatography on silica gel eluted with hexane/ethyl acetate (3:1) to give 0 84 g (72% yield) of the title compound; DCI MS M/Z: 469 (M+NH4)+; 1 H NMR (CDCl₃) δ 1.45 (s, 9H), 2.25 (s, 3H), 2.28 (s, 3H), 2.63-2.78 (m, 1 H), 2.91-3.03 (m, 1 H), 3.63-3.74 (m, 1 H), 4.08-4.34 (m, 2H), 4.65 (br s, 1 H), 6.02 (d, 1 H), 7.05 (d, 1 H), 7.22-7.37 (m, 6H).

### Step 2: 5,6-Bis(acetoxyl)-1-(N-t-butoxycarbonyl)aminomethyl-3-phenylnaphthalene

To a solution of 1.59 g (3.52 mmol) of 5,6-bis(acetoxy)-1-(N-t-butoxycarbonyl)aminomethyl-3-phenyl-3,4-dihydronaphthalene, from Step 1, in 50 mL of toluene was added 0.80 g (3.52 mmol) of 2,3-dichloro-5,6-dicyano-1,4-benzoquinone as a solid. The reaction mixture was heated to 70°C under a nitrogen atmosphere and allowed to stir overnight. After cooling the reaction mixture to ambient temperature, the orange colored mixture was filtered through a bed of Celite® filter aid. The filtrate was concentrated in vacuo. The residue was taken up in 50 mL of methylene chloride and the methylene chloride solution was washed with 2 X 25 mL of 1 M aqueous phosphoric acid solution, 2 X 25 mL of aqueous sodium bicarbonate solution, 25 mL of water and 25 mL of brine, dried over anhydrous sodium sulfate, filtered and concentrated in vacuo. The residue was purified on a silica gel column eluted with 3:1 hexane/ethyl acetate to give 0.77 g (49% yield) of the title compound as a white solid; ¹H NMR (CDCl₃) δ 1.47 (s, 9H), 2.35 (s, 3H), 2.47 (s, 3H), 4.81 (d, 2H), 4.89 (br s, 1 H), 7.36-7.43 (m, 5H), 7.64-7.71 (m, 2H), 7.94 (d, 1 H), 8.01 (d, 1 H).

### Step 3: 5,6-Bis(acetoxy)-1-aminomethyl-3-phenylnaphthalene hydrochloride

5,6-Bis(acetoxy)-1-(N-t-butoxycarbonyl)aminomethyl-3-phenylnaphthalene (300 mg, 0.67 mmol) from Step 2 was dissolved in 10 mL of dioxane saturated with anhydrous hydrogen chloride. The resultant solution was stirred for 2 h at ambient temperature and concentrated in vacuo. The solid residue was recrystallized from ethanol/hexane to give 130 mg (50% yield) of the title compound as a white solid; DCI MS M/Z: 350 (M+H)⁺, 367 (M+NH4)+; ¹H NMR (dₛ-DMSO) 8 2.36 (s, 3H), 2.51 (m, 3H + DMSO), 4.65 (s, 2H), 7.44-7.51 (m, 1 H), 7.54-7.61 (m, 3H), 7.78 (d, 2H), 8.07 (s, 1 H), 8.13-8.19 (m, 2H), 8.39 (br s, 3H).

### Step 4: 1-Aminomethyl-5,6-dihydroxy-3-phenylnaphthalene hydrochloride

5,6-Bis(acetoxy)-1-aminomethyl-3-phenylnaphthalene hydrochloride (130 mg, 0.34 mmol) from Step 3 was dissolved in 10 mL of methanol saturated with hydrogen chloride. The resultant solution was stirred at ambient temperature for 3 h and then concentrated in vacuo. The solid residue was dissolved in a minimal amount of ethanol. The ethanol solution was added slowly to 30 mL of dry diethyl ether and the precipitate was collected by filtration. The solid was dried at 60°C in vacuo to give 77 mg (76% yield) of the title compound as a white solid, m.p. 205-212°C (dec); DCI MS M/Z: 266 (M+H)⁺, 283 (M+NH₄)⁺; ¹ H NMR (d₆-DMSO) δ 4.50 (d, 2H), 7.28 (d, 1 H), 7.36-7.60 (m, 4H), 7.77 (s, 1 H), 7.83 (d, 2H), 8.35 (s, 1 H), 8.49 (br s, 3H), 9.22 (br s, 1 H), 9.60 (br s, 1 H).

### Example 31

### [1 R,3S)-1-Bromomethyl-3-t-butyl-5,6-cyclohexylidenedioxy-3,4-dihydro-1H-2-benzopyran

### Step 1: Epoxide Synthesis

t-Butyl ethylene oxide, the epoxide used in the synthesis of [1R,3S] 1-bromomethyl-3-t-butyl-5,6-cyclohexylidene- dioxy-3,4-dihydro-1 H-2-benzopyran, is commercially available. Epoxides necessary for the synthesis of other benzopyran derivatives of the present invention which are not commercially available were synthesized by either Method A or Method B described below.

### Method A: 1-Cyclohexyl ethylene oxide

Sodium hydride (4.5 g, 187.5 mmol) and trimethylsulfoxonium iodide (41.25 g, 187.5 mmol) were combined in a 3-neck flask equipped with a mechanical stirrer and an addition funnel Dimethyl sulfoxide (DMSO) was added slowly, over a 30 min period, until 200 mL had been added. Gas was evolved throughout the addition. A solution of cyclohexane carboxaldehyde (21.8 mL, 180 mmol) in 50 mL of DMSO was added dropwise to the reaction mixture over a 15 min period. The reaction mixture was heated to 55°C and stirred at 55°C for 30 min. The reaction mixture was cooled to ambient temperature and poured into 500 mL of water. The aqueous solution was extracted with 3 X 100 mL of diethyl ether The combined ether extracts were washed with water and brine, dried over anhydrous magnesium sulfate and concentrated in vacuo. The crude product was distilled (44°C, 0.1 mm) to give 14 g (62% yield) of 1-cyclohexyl ethylene oxide as a clear colorless liquid.

### Method B: 1-Benzyl ethylene oxide

A solution of m-chloroperbenzoic acid (mCPBA; 17 g, 0.1 mol) in 120 mL of methylene chloride was added (at ambient temperature) dropwise to a solution of allyl benzene (10 g 85 mmol) in 200 mL of methylene chloride. After the reaction mixture was stirred for 5 h with a mechanical stirrer, 5 additional grams of m-CPBA were added and the reaction mixture stirred for another 2 h. The reaction mixture was then diluted with 200 mL of ether, washed with 2 X 100 mL of aqueous sodium bisulfite solution, 1 X 100 mL of aqueous sodium bicarbonate solution and 1 X 100 mL of brine. The organic solution was dried over anhydrous magnesium sulfate, filtered and concentrated in vacuo. The residue was purified by bulb-to-bulb distillation (60°C, 0.1 mm) to give 8.5 g (77% yield) of 1-benzyl ethylene oxide as a clear colorless liquid.

### Step 2:3,3-Dimethyl-1-(spiro-[(1,3-benzodioxole)-2,1'-cyclohexane])-2-butanol

n-Butyl lithium (12.6 mL of 2.5 M solution in hexane, 32 mmol) was added to a solution of spiro[1,3-benzodioxole) -2,1'-cyclohexane] (5 g, 26.3 mmol), prepared as described by Boeckmann and Schill in Chemische Berichte, 110, 703 (1977), in 40 mL of THF at 0°C. After 4 h, 3,3-dimethyl-1,2-epoxybutane (2.5 g, 25 mmol), commercially available from Aldrich Chemical Company, was added dropwise and the reaction mixtures was warmed to 25°C. After 3 h at 25°C, the reaction mixture was poured into saturated aqueous ammonium chloride solution and extracted with 3 X 75 mL of diethyl ether The combined ether extracts were washed with 50 mL of aqueous ammonium chloride solution and 50 mL of brine, dried over anhydrous magnesium sulfate, filtered and concentrated in vacuo. to an oil. The oil was purified on silica gel eluted with 10% ethyl acetate in hexane to give 3.5 g (48% yield) of the title compound as a viscous oil. DCI MS 308 (M+NH4)⁺. ¹H NMR (d₆-DMSO) δ 0.89 (s, 9H), 1.4-1.9 (m, 10H), 2.27 (dd, 1 H, J=14.4, 9.3 Hz), 2.75 (dd, 1 H, J=₁₄.₄, 3.0 Hz), 3.3 (m, 1 H), 4.38 (d, 1 H, J=6.3 Hz), 6.18 (m, 3H).

### Step 3A: [1 R,3S]-1-Bromomethyl-3-t-butyl-5,6-cyclohexylidenedioxy-3,4-dihydro-1H-2-benzopyran

Boron trifluoride etherate (2.88 mL, 23.5 mmol) was added dropwise to a stirred solution of the product of Step 2 (3.4 g, 11.7 mmol) and bromoacetaldehyde dimethyl acetal (1.4 mL, 11.7 mmol) in 15 mL of methylene chloride at -25°C. The reaction mixture was allowed to warm to 0°C. After 1 h at 0°C, the reaction mixture was diluted with 20 mL of diethyl ether and poured into 50 mL of aqueous sodium carbonate solution. The resultant mixture was extracted with 3 X 50 mL of diethyl ether. The combined ether extracts were washed with aqueous sodium bicarbonate solution and brine, dried over anhydrous magnesium sulfate, filtered and concentrated under reduced pressure. The residue was purified by column chromatography on silica gel eluted with 2.5% ethyl acetate in hexane to give 2.85 g (61% yield) of the title compound as a colorless solid, m.p. 113-114°C. DCI MS: 414 (M+NH₄)^{+ 1}H NMR (CDCl₃) 8 1.0 (s, 9H), 1.4-1.95 (m, 10H), 2.6 (m, 2H), 3.28 (dd, 1 H, J=9.3, 5.4 Hz), 3.52 (dd, 1 H, J=₁.2₅, 7.5 Hz), 3.85 (dd, 1 H, J=11.25, 3.0 Hz), 4.87 (m, 1 H), 6.5 (d, 1 H, J=9.0 Hz), 6.6 (d, 1 H, J=9.0 Hz).

### Alternate Step 3B: [1 R,3S]-1-(2-Bromoethyl)-3-t-butyl-5,6-cyclohexylidenedioxy-3,4-dihydro-1H-2-benzopyran

The title compound was prepared following the procedure described in Step 3 above and using 3-bromopropional- dehyde dimethyl acetal instead of bromoacetaldehyde dimethyl acetal.

### Example 32

### [1R,3S] 1-Aminomethyl 3-t-butyl-3,4-dihydro-5,6-dihydroxy-1H-2-benzopyran hydrochloride

### Step 1:[1R,3S] 1-Azidomethyl-3-t-butyl-5,6-cyclohexylidenedioxy-3,4-dihydro-1H-2-benzopyran

Lithium azide (1.6 g, 31 mmol) was added to a solution of the product of Example 31 (2.5 g, 6.35 mmol) in 12 mL of dimethylformamide (DMF) at 25°C. The reaction mixture was heated at 75°C for 2h then cooled and poured into 50 mL of water. The aqueous solution was extracted with 3 X 50 mL of diethyl ether. The combined ether extracts were washed with 50 mL of water, 50 mL of brine, dned over anhydrous magnesium sulfate, filtered and concentrated in vacuo. The residue was purified by column chromatography on silica gel eluted with 2.5% ethyl acetate in hexane to give 1.56 g (69%yield) of the title compound as a colorless syrup; MS DCI: 358 (M+H)⁺, 375 (M+NH₄)⁺; ¹H NMR (CDCl₃) δ 1.1 (s, 9H), 1.4-1 95 (m, 10H), 2.6 (m, 2H), 3.3 (dd, 1 H, J=8.7, 6.0 Hz), 3.42 (dd, 1 H, J=13.5, 7.5 Hz), 3.52 (dd, 1 H, J=₁3.₅, 3.0 Hz), 4.9 (m, 1 H), 6.42 (d, 1 H, J=9.0 Hz), 6.59 (d, 1 H, J=9.0 Hz).

### Step 2: [1R,3S] 1-Aminomethyl-3-t-butyl-5,6-cyclohexylidenedioxy-3,4-dihydro-1H-2-benzopyran hydrochloride

Lithium aluminum hydride (LAH) solution (4.2 mL of 1 M solution in ether, 4.2 mmol) was added dropwise to a solution of [1R,3S] 1-azidomethyl-3-t-butyl-5,6-cyclohexylidenedioxy-3,4-dihydro-1 H-2-benzopyran (1.5 g, 4.2 mmol) in 25 mL of dry diethyl ether at 0°C. After 15 min, the reaction mixture was allowed to warm to 25°C and was stirred at 25°C for 1 h. The reaction mixture was cooled to 0°C and the reaction was quenched by the sequential addition of.0.16 mL of water, 0.16 mL of 15% aqueous sodium hydroxide solution and 0.48 mL of water. The precipitate was removed by filtration and washed with ether. The filtrate was concentrated in vacuo. The crude amine product was dissolved in 15 mL of diethyl ether and diethyl ether saturated with hydrogen chloride was added in excess. The solid was collected by vacuum filtration, washed with diethyl ether and dried to give 1.48 g (96% yield) of the title compound as a colorless solid, m.p. 164-167°C; DCI MS: 332 (M⁺H)⁺; 1H NMR (d₆-DMSO) δ 1.0 (s, 9H), 1.4-1.9 (m, 10H), 2.6 (m, 2H), 2.9 (dd, 1 H, J=14.7, 10.5 Hz), 3.2 (m, 2H), 3.5 (dd, 1 H, J=14.7, 3.0 Hz), 4.82 (br d, 1 H, J=8 Hz), 6.7 (m, 2H), 7.9 (br s, 2H).

### Step 3: [1R,3S] 1-Aminomethyl-3-t-butyl-3,4-dihydro-5,6-dihydroxy-1H-2-benzopyran hydrochloride

A solution of [1R,3S] 1-aminomethyl-3-t-butyl-5,6-cyclohexylidenedioxy-3,4-dihydro-1 H-2-benzopyran hydrochloride, from Step 1, (1 g, 2.72 mmol) in 15 mL of ethanol was saturated with anhydrous hydrogen chloride. The solution was heated to reflux temperature. After 2 h at reflux temperature, the solution was concentrated to approximately 2 mL. A solid was precipitated with diethyl ether, filtered, washed with diethyl ether and dried in a vacuum oven at 80°C to give 630 mg (81% yield) of the title compound as a colorless powder, m.p. 258°C; IR 3200, 1620, 1490, 1300, 1060 cm⁻¹; DCI MS: 252 (M⁺H)⁺; ¹H NMR (d₆-DMSO) δ 1.0 (s, 9H), 2.38 (dd, 1 H, J=16.5, 12 Hz), 2.63 (dd, 1 H, J=16.5, 2.8 Hz), 2.85 (m, 1 H), 3.22 (dd, 1 H, J=12.0, 4.2 Hz), 3.45 (m, 1 H), 4.8 (br d, 1 H, J=₇.₅ Hz), 6.5 (d, 1 H, J=₇.8 Hz), 6.65 (d, 1 H, J=7.8 Hz), 7.9 (br s, 2H), 8.46 (br s, 1 H), 9.22 (br s, 1 H). Analysis calculated for C₁₄H₂₂CINO₃: C, 58.43; H, 7.70; N,-4.9. Found: C, 58.37; H, 7.69; N, 4.77.

### Examples 33-64

Following the synthesis outlined in Examples 31 and 32, using the appreciate epoxide and the appropriate aldehyde diacetal, Examples 33-64 Were made as disclosed in Table 3. The structure of each was confirmed by melting point (m.p), elemental analysis and mass spectra as designated.

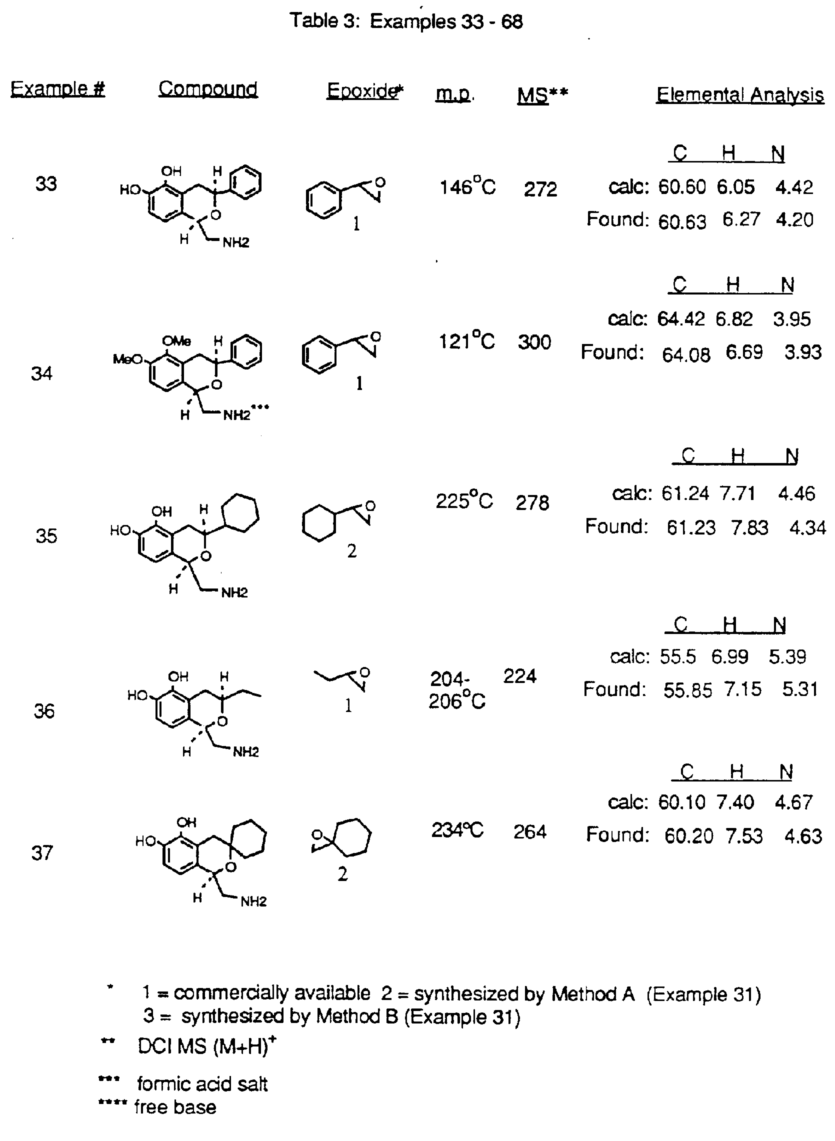

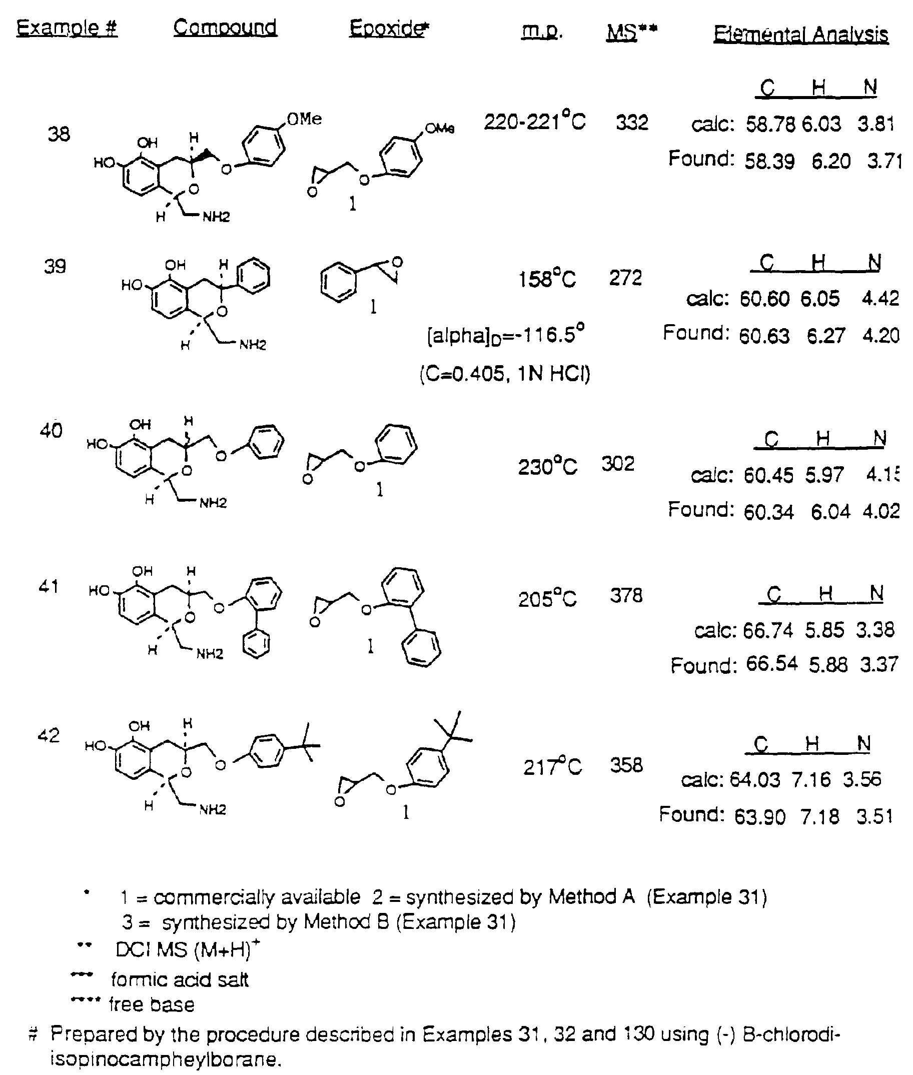

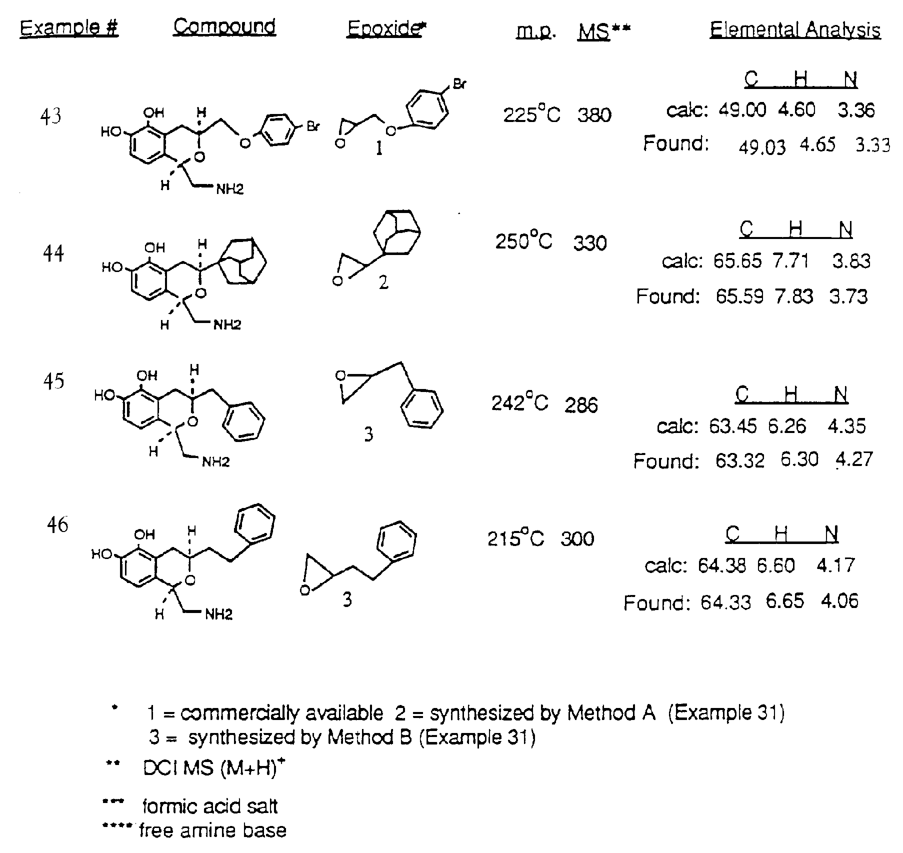

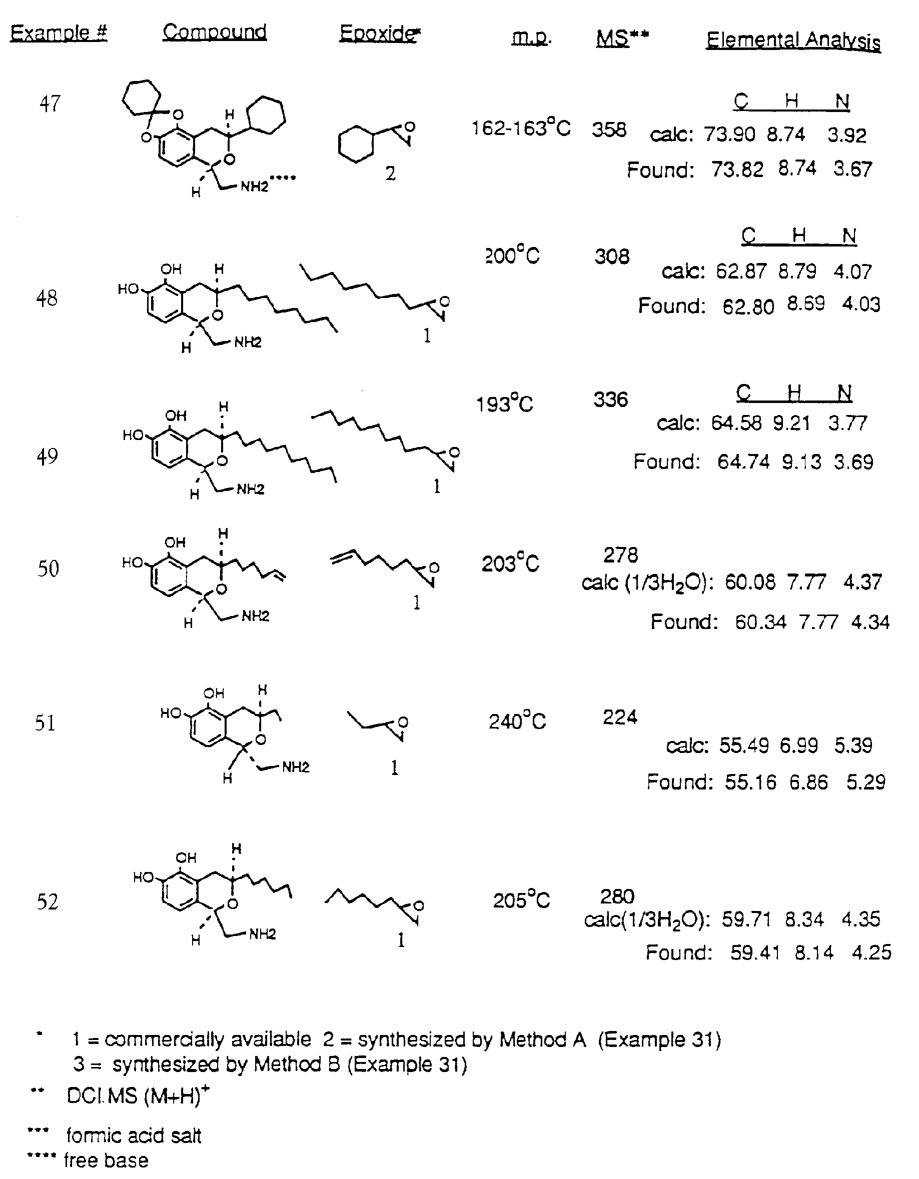

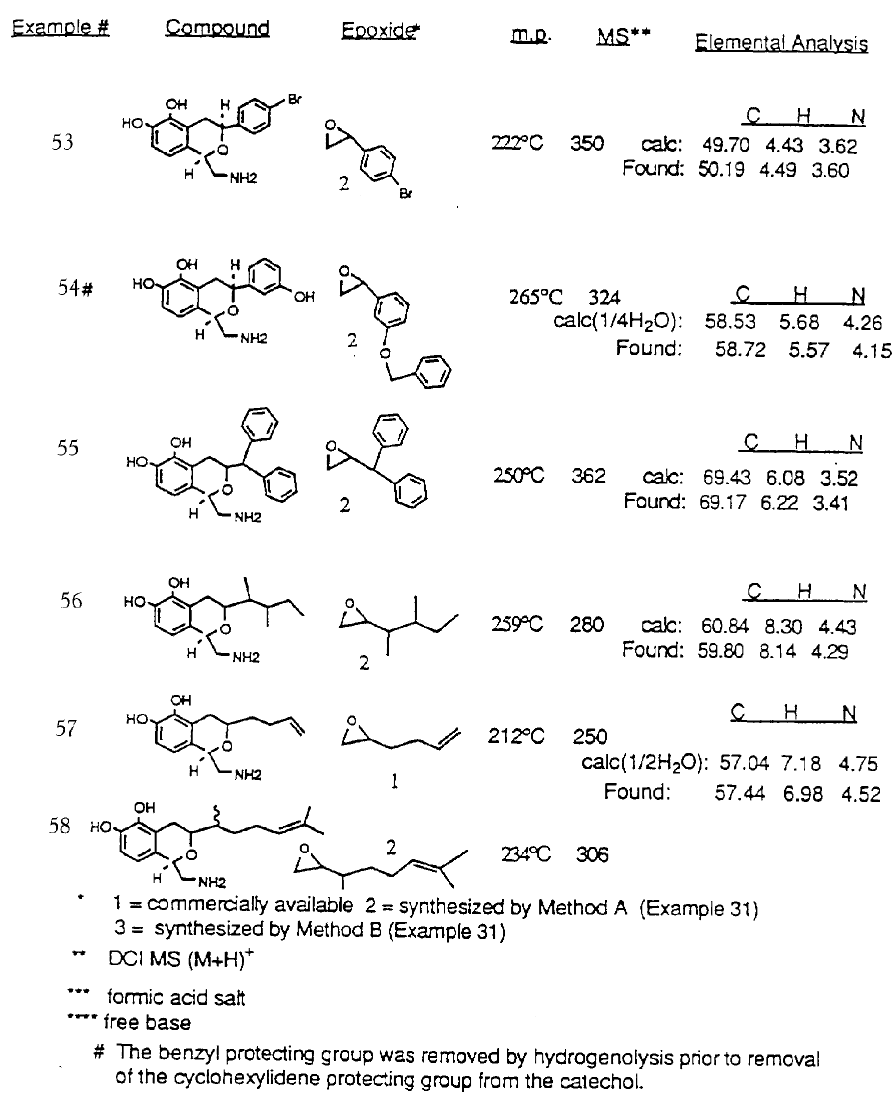

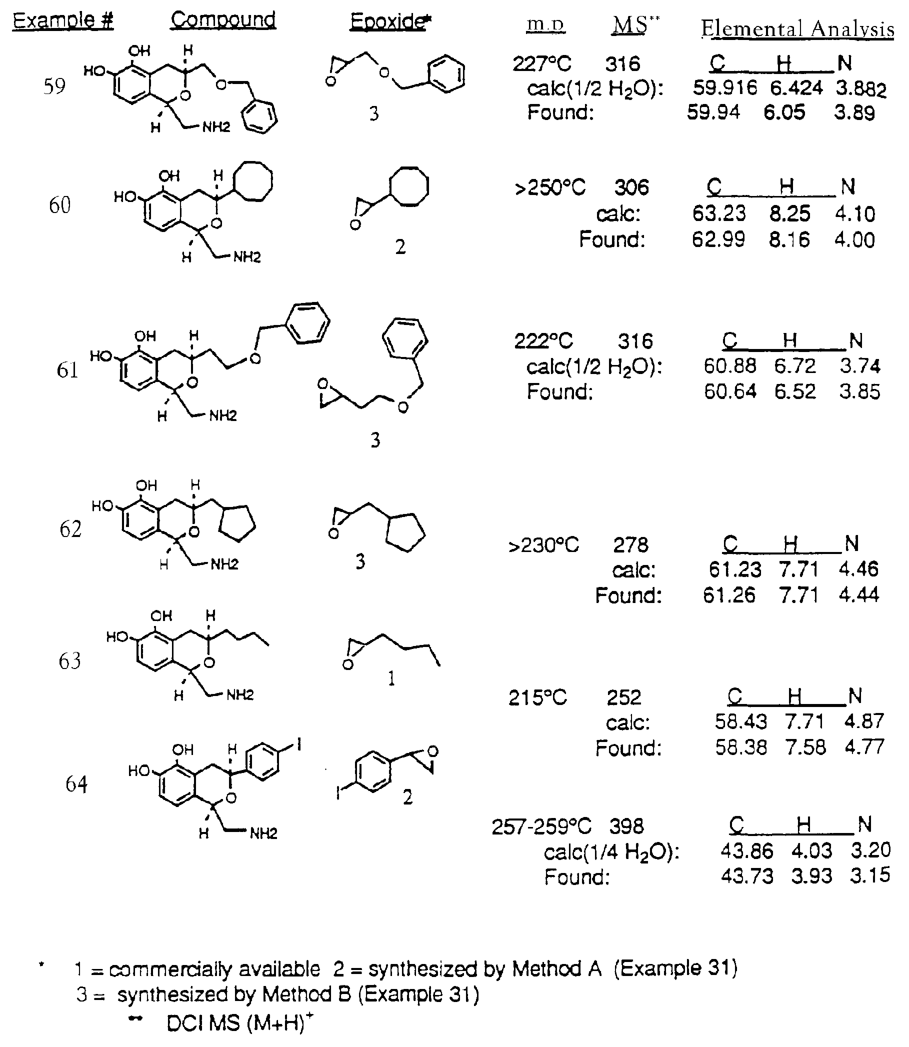

### Example 65

### 1-Aminomethyl-3,4-dihydro-5,6-dihydroxy-3-(2'-tetrahydrofuranyl)-1H-2-benzopyran hydrobromide

### Step 1: 1-(2',3'-Dimethoxyphenyl)-N-methoxy-N-methyl-acetamide

Oxalyl chloride (0.45, 5.1 mmol) and 2 - 3 drops of N,N-dimethylformamide (DMF) were added to a chilled (0°C) solution of 2,3-dimethoxyphenylacetic acid in 25 mL of THF. The resultant solution was allowed to warm to ambient temperature over a 4 h period. The solvent was removed in vacuo and the residue was dissolved in 50 mL of chloroform. N-methoxy-N-methyl-hydroxylamine hydrochloride (0.55 g, 5.61 mmol) was added and the resultant solution was chilled to 0°C. Pyridine (0.91 mL, 11.23 mmol) was added and the solution was stirred for 2 h at 0°C. The solution was then washed twice with brine, dried over anhydrous magnesium sulfate, filtered and concentrated in vacuo to an oil. The oil was purified by column chromatography on silica gel eluted with 20% ethyl acetate in hexane to give 0.65 g (53% yield) of the title compound as an oil; MS DCI: 240 (M+H)⁺, 257 (M+NH4)+; ¹H NMR (CDCl₃) 6 3.21 (s, 3H), 3.68 (s, 3H), 3.80-3.84 (m, 5H), 3.87 (s, 3H), 6.80-6.87 (m, 2H), 6.98-7.4 (m, 1 H).

### Step 2: 2-(2',3'-Dimethoxyphenyl)-1-furanylethanone

n-Butyl lithium (1.87 mL, 3.76 mmol of a 1.75M solution in hexanes) was added to a chilled (0°C) solution of furan (0.2 mL, 2.72 mmol) in 5 mL of THF The mixture was allowed to warm to ambient temperature over a 4 h period. The mixture was then chilled again to 0°C and a solution of 0.65 g (2.72 mmol) of 1-(2',3'-dimethoxyphenyl)-N-methoxy-N-methyl-acetamide, from Step 1, was added. The reaction mixture was allowed to warm to ambient temperature over a 2 h period and was then quenched with a saturated aqueous ammonium chloride solution. The mixture was extracted with ethyl acetate and the extracts were washed once each with saturated aqueous ammonium chloride and brine. The extracts were dried over anhydrous magnesium sulfate, filtered and concentrated in vacuo to give 0.5 g (75% yield) of the title compound as an oil; MS DCI: 247 (M+H)⁺, 264 (M+NH₄)⁺; ¹H NMR (CDCl₃) δ 3.80 (s, 3H), 3.85 (s, 3H), 4.15 (s, 2H), 6.51-6.53 (m, 1 H), 6.82-6.88 (m, 3H), 699-7.05(m, 1 H); 7.25- 7.28 (m, 1 H).

### Step 3: 2-(2',3'-Dimethoxyphenyl)-1-tetrahydrofuranyl ethanol

A solution of 450 mg (1.8 mmol) of 2-(2',3'-dimethoxyphenyl)-1-furanylethanone, from Step 2, and 20% palladium on carbon (225 mg) in 75 mL of methanol was shaken under 4 atmospheres of hydrogen until hydrogen uptake ceased. The solution was filtered and concentrated in vacuo to give 320 mg (69% yield) of the title compound as an oily solid. This product was carried on without purification to the next step.

### Step 4; 1-Aminomethyl-3-(2'-tetrahydrofuranyl)-3,4-dihydro-5,6-dihydroxy-1H-2-benzopyran hydrobromide

2-(2',3'-Dimethoxyphenyl)-1-tetrahydrofuranyl ethanol, from Step 3, was converted to the title compound using the procedures described in Step 3 of Example 31 and Steps 1 and 2 of Example 32. The dimethoxy protecting groups were removed with boron tribromide by the procedures described in Step 3 of Example 2 to afford the title compound, m.p. >250°C ; FAB MS (M/Z): 266 (M+H)⁺; H NMR (d₆-DMSO) δ 1.75-1.93 (m, 4H), 2.37-2.47 (m, 1H), 2.50-2.60 (m, 1H), 2.60-2.68 (m, 1 H), 2.71-2.79 (m, 1 H), 3.65-3.72 (m, 1 H), 3.76-3.84 (m, 2H), 3.90-4.00 (m, 1 H), 4.84-4.91 (m, 1 H), 6.48 (d, 1H), 6.68 (d, 1H), 7.72-7.84 (m, 2H), 8.41-8.49 (m, 1H), 9.20-9.28 (m, 1H). High resolution mass spectral analysis calculated for C₁₄H₂₀NO₄: 266.132. Found: 266.1391

### Example 66

### 1 -Aminomethyl-3,4-dihydro-5,6-dihydroxy-3-(3'-prop-1'-ynyl)-1H-2-benzopyran hydrochloride

### Step 1: 3-(Spiro-[(1,3-benzodioxole)-2.1'cyclohexanel)-propene oxide

n-Butyl lithium (30 mL of 2.5M solution in hexane, 75 mmol) was added dropwise to a solution of spiro(1,3-benzo- dioxole)-2,1'-cyclohexane] (5 g, 26.3 mmol), prepared as described by Boeckmann and Schill in Chemische Berichte, 110, 703 (1977), in 125 mL of anhydrous THF at 0°C. The solution was stirred at 0°C for 2 h and then a solution of 4.8 g (52 mmol) of epichlorohydrin in 10 mL of THF was added via cannula over a 15 minute period. The reaction mixture was heated to ambient temperature and stirred for 60 minutes at ambient temperature and heated at 65° C for 75 minutes. The reaction mixture was cooled to ambient temperature and poured into 150 mL of water. The aqueous layer was extracted with 2 X 75 mL of diethyl ether. The combined ether layers were washed with 75 mL of saturated sodium bicarbonate and brine, dried over anhydrous magnesium suifate, filtered and concentrated in vacuo to an amber colored oil. The oil was purified by flash chromatography on silica gel eluted with 8% ethyl acetate in hexane to give 6.81 g (53% yield) of the title compound as a clear oil.

### Step 2: 1-(Spiro-[(1,3-benzodioxole)-2,1'-cyclohexane))-4-pentyn-2-ol

Crude 3-(spiro-[(1;3-benzodioxole)-2,1'-cyclohexane])-propene oxide (8.29 g, 33.7 mmol), from Step 1, was added to a chilled (0°C) suspension of lithium acetylide-ethylenediamine complex (4.65 g 45.5 mmol of a 90% solid) in 50 mL of methyl sulfoxide. The mixture was allowed to warm to ambient temperature over a 3 h period during which the mixture became a homogeneous solution. The reaction was quenched with 50 mL of water and the aqueous layers were washed with water and brine, dried over anhydrous magnesium sulfate, filtered and concentrated in vacuo to an oil. The oil was purified by column chromatography on silica gel eluted with 15% ethyl acetate in hexane to give 2.08 g (23% yield) of the title compound as an oil; MS DCI: 273 (M+H)⁺, 290 (M+NH4)⁺; ¹H NMR (CDCl₃) δ 1.45-1.55 (m, 2H), 1.67-1.77 (m, 4H), 1.85-1.95 (m, 4H), 2.09 (t, 1 H), 2.28 (d, 1 H), 2.32-2.50 (m, 2H), 2.80-2.93 (m, 2H), 4.00-4.15 (m, 1 H), 6.62-6.77 (m, 3H).

### Step 3: 1-Aminomethyl-3,4-dihydro-5,6-dihydroxy-3-(3'-prop-1'-ynyl)-1H-2-benzopyran hydrochloride

1-(Spiro-[(1,3-benzodioxole)-2,1'-cyclohexane])-4-pentyn-2-ol, from Step 2, was converted to the title compound using the procedures described in Step 3 of Example 31 and Steps 1 - 3 of Example 32 to afford the title compound, m.p. >250°C; DCI MS (M/Z): 234 (M+H)⁺; 1 H NMR (d₆-DMSO) 8 2.34-2.47 (m, 1 H), 2.52-2.57 (m, 1 H), 2.57-2.68 (m, 1 H), 2.84-2.92-(m,-2H), 2.95 (m,1 H), 3.39-3.47 (m, 1 H), 3.71-3.81 (m, 1 H), 4.87-4.91 (m, 1 H), 6.2 (d,1 H), 6.69 (d, 1 H), 7.88 (s, 2H), 8.53 (s, 1H), 9.31 (s, 1H). Analysis calculated for C₁₃H₁₆CINO₃: C, 57.89; H, 5.98; N, 5.19. Found: C, 57.73; H, 6.15; N, 5.09.

### Example 67

### [1R,3S]3-Cyclohexyl-3,4-dihydro-5,6-dihydroxy-1-(N-methyl)aminomethyl-1H-2-benzopyran hydrochloride

[1R,3S] 1 -Aminomethyl-3-cyclohexyl-5,6-cyclohexylidenedioxy-3,4-dihydro-1H-2-benzopyran hydrochloride (synthesized as described in Steps 1 and 2 of Example 32 for [1R,3S] 1-aminomethyl-3-t-butyl-5,6-cyclohexylidenedi- oxy-3,4-dihydro-1 H-2-benzopyran hydrochloride) (0.82 g, 2.3 mmol) was partitioned between methylene chloride and saturated aqueous sodium bicarbonate solution The methylene chloride layer was concentrated under reduced pressure and the residue was dissolved in 25 mL of ethyl formate. The ethyl formate solution was heated to reflux temperature. After 1 h at reflux temperature, the reaction mixture was concentrated to a white solid. The solid was dissolved in 15 mL of THF and 175 mg (4.6 mmol) of lithium aluminum hydride (LAH) was added. The reaction mixture was heated at reflux temperature for 3 h then cooled to 0°C. The reaction was quenched by the sequential addition of 0.175 mL of water, 0.175 mL of 15% aqueous sodium hydroxide solution and 0.525 mL of water. The reaction mixture was filtered and the filter cake washed with diethyl ether. The filtrate was concentrated in vacuo. The residue was dissolved in 20 mL of ethanol and the alcohol solution was saturated with anhydrous hydrogen chloride then heated at reflux temperature for 2 h. The ethanol was evaporated down to approximately 2 mL and ether was added until a solid precipitate was formed. The solid was filtered, washed with diethyl ether and dried to give 504 mg (67% yield) of the title compound as a colorless powder, m.p. 244°C; DCI MS 292 (M⁺H)^{+.} Analysis calculated for C₁₇H₂₆CINO₃: C, 62.28; H, 7.99; N, 4.27. Found: C, 62 24; H, 7.90; N, 4.21.

### Example 68

### [1R,3S] 3-t-Butyl-3,4-dihydro-5,6-dihydroxy-1-(N-methyl)aminomethyl-1H-2-benzopyran hydrochloride

Following the synthesis outlined in Example 67 and starting with [1 R,3S] 1-aminomethyl-3-t-butyl-5,6-cyclohexyli- denedioxy-3,4-dihydro-1 H-2-benzopyran hydrochloride from Step 2 of Example 32, [1R,3S] 3-t-butyl-3,4-dihydro-5,6-dihydroxy-1-(N-methylaminomethyl)-1 H-2-benzopyran hydrochloride was prepared, m p. 246°C; DCI MS 266 (M+H)+. Analysis calculated for C₁₅H₂₃CINO₃: C,59.70; H, 8.00; N, 4.64. Found: C, 59.64; H, 8.10; N, 4.45.

### Example 69

### [1R,3S]1-(N-Allyl)aminomethyl-3-cyclohexyl-3,4-dihydro-5,6-dihydrory-1H-2-benzopyran hydrochloride

### Step 1 : [1R,3S] 1-(N-Allyl)aminomethyl-3-cyclohexyl-3,4-dihydro-5,6-cyclohexylidenedioxy-1H-2-benzopyran

[1R,3S] 1-Bromomethyl-3-cyclohexyl -3,4-dihydro-5,6-dihydroxy-1 H-2-benzopyran (1.1 g, 2.6 mmol) (prepared as described in Example 31, using cyclohexyl ethylene oxide) and dissolved in 10 mL of allyl amine. The reaction mixture was heated at reflux temperature for 5 h then concentrated in vacuo. The residue was dissolved in 50 mL of ethyl acetate. The solution was washed with 2 X 25 mL of aqueous sodium bicarbonate solution and 1 X 25 mL of brine, dried over anhydrous sodium sulfate, filtered and concentrated in vacuo. The residue was purified by column chromatography on silica gel eluted with 30% ethyl acetate in hexane to give 928 mg (90% yield) of the title compound as a colorless oil; DCI MS: 398 (M+H)+; 1 H NMR (CDCI₃) δ 1.0 (m, 20H), 2.05 (br d, 1 H, J=11.0 Hz), 2.4 (br s, 1H), 2.5 (dd, 1H, J=13.5, 9.0 Hz), 2.7 (dd, 1 H, J=₁3.₅, 2.8 Hz), 2.82 (dd, 1 H, J=₁0.0, 7.5 Hz), 3.18 (dd, 1 H, J=₁0.0, 3.0 Hz), 3.48 (m, 3H), 4.7 (br d, 1 H, J=7.5 Hz), 5.2 (m, 2H), 5.95 (m, 1 H), 6.5 (d, 1 H, J=6.3 Hz), 6.58 (d, 1 H, J=6.3 Hz).

### Step 2: [1R,3S] 1-(N-Allyl)aminomethyl-3-cyclohexyl-3,4-dihydro-5,6-dihydroxy-1H-2-benzopyran hydrochloride

[1R,3S] 1-(N-Allyl)aminomethyl-3-cyclohexyl-3,4-dihydro-5,6-cyclohexyli-denedioxy-1H-2-benzopyran (920 mg, 2.3 mmol), from Step 1, was dissolved in 15 mL of ethanol saturated with anhydrous hydrogen chloride. The acidic solution was heated at reflux temperature for 2 h then concentrated to~2 mL. Diethyl ether was added and the precipitate was filtered, washed with diethyl ether and dried to give 590 mg (72% yield) of the title compound as an off-white powder, m.p. 217-219°C; DCI MS: 318 (M+H)+. Analysis calculated for C₁₉H₂₈CINO₃: C, 64.49; H, 7.98; N, 3.96. Found: C, 64 34; H, 8.02; N, 3.82.

### Examples 70-90

Following the syntheses described in Examples 31 and 69 using the appropriate epoxide and the appropriate amine, Examples 70-85 were prepared as disclosed in Table 4. Examples 86-90 were prepared by the procedures described in Examples 31,32 and 67. The structure of each was confirmed by melting point, mass spectra and elemental analysis as designated.

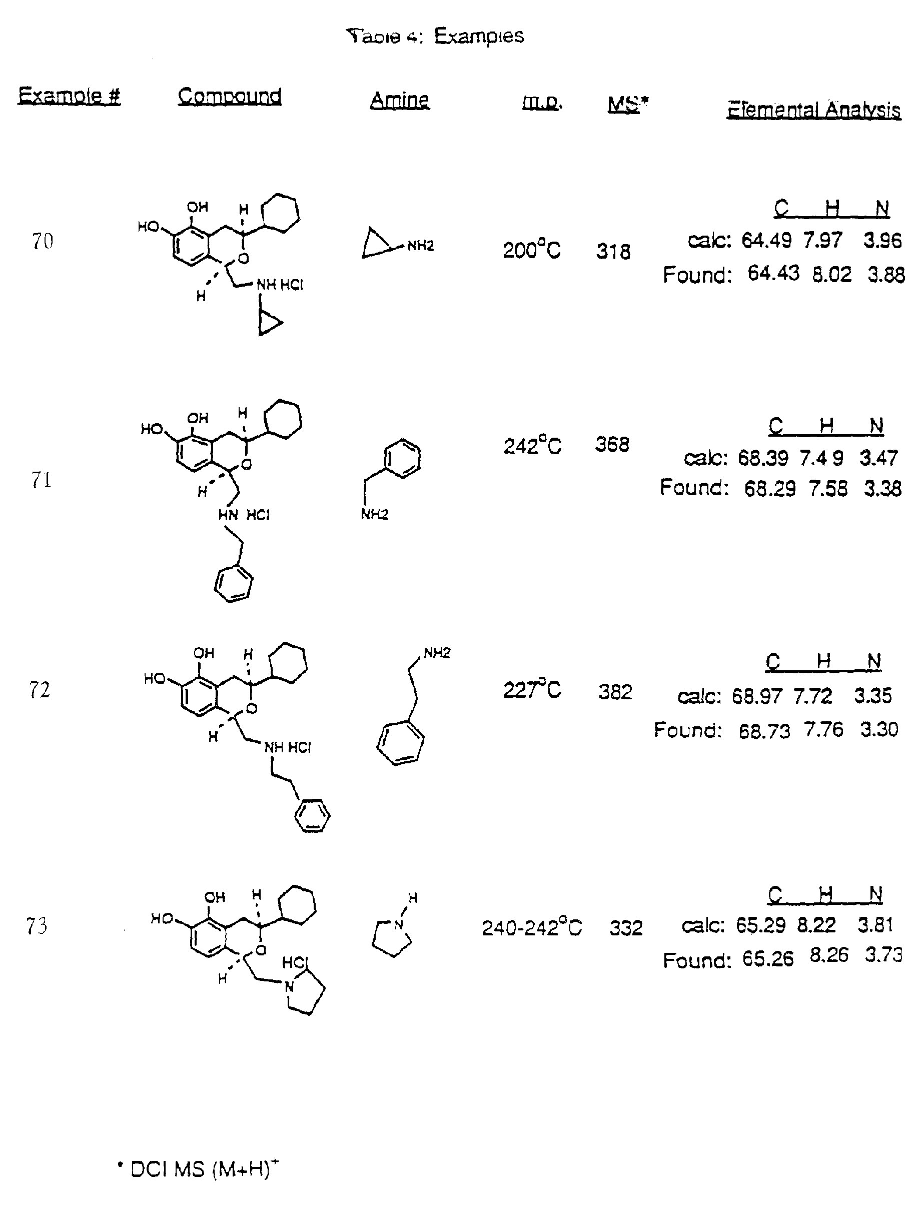

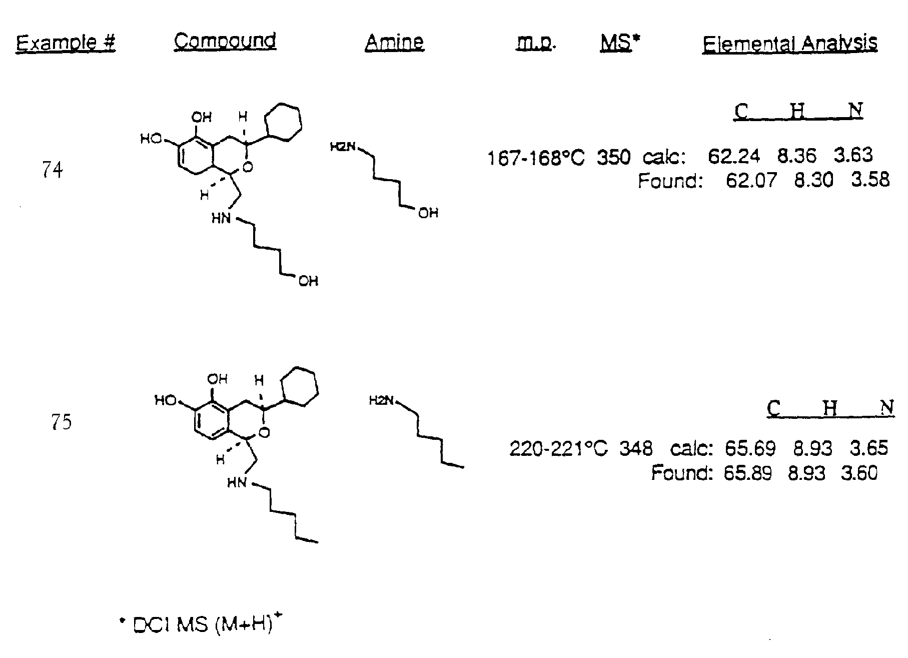

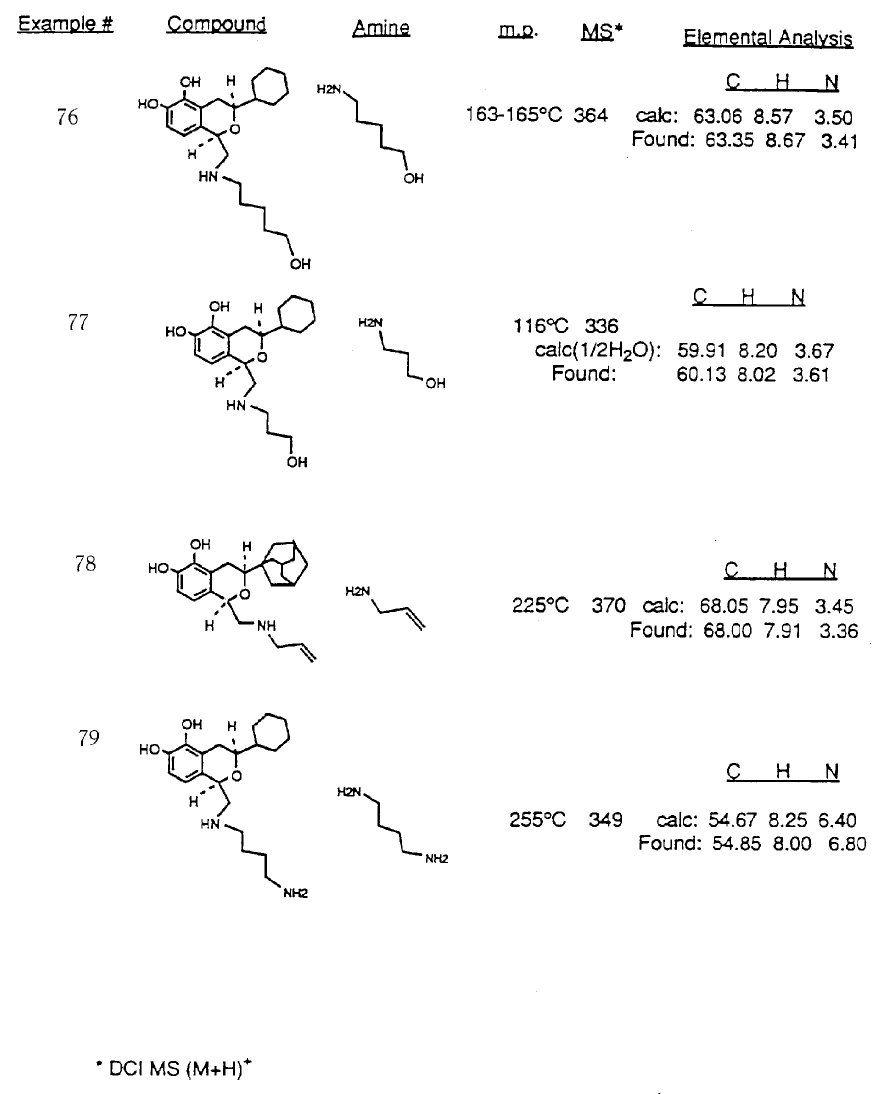

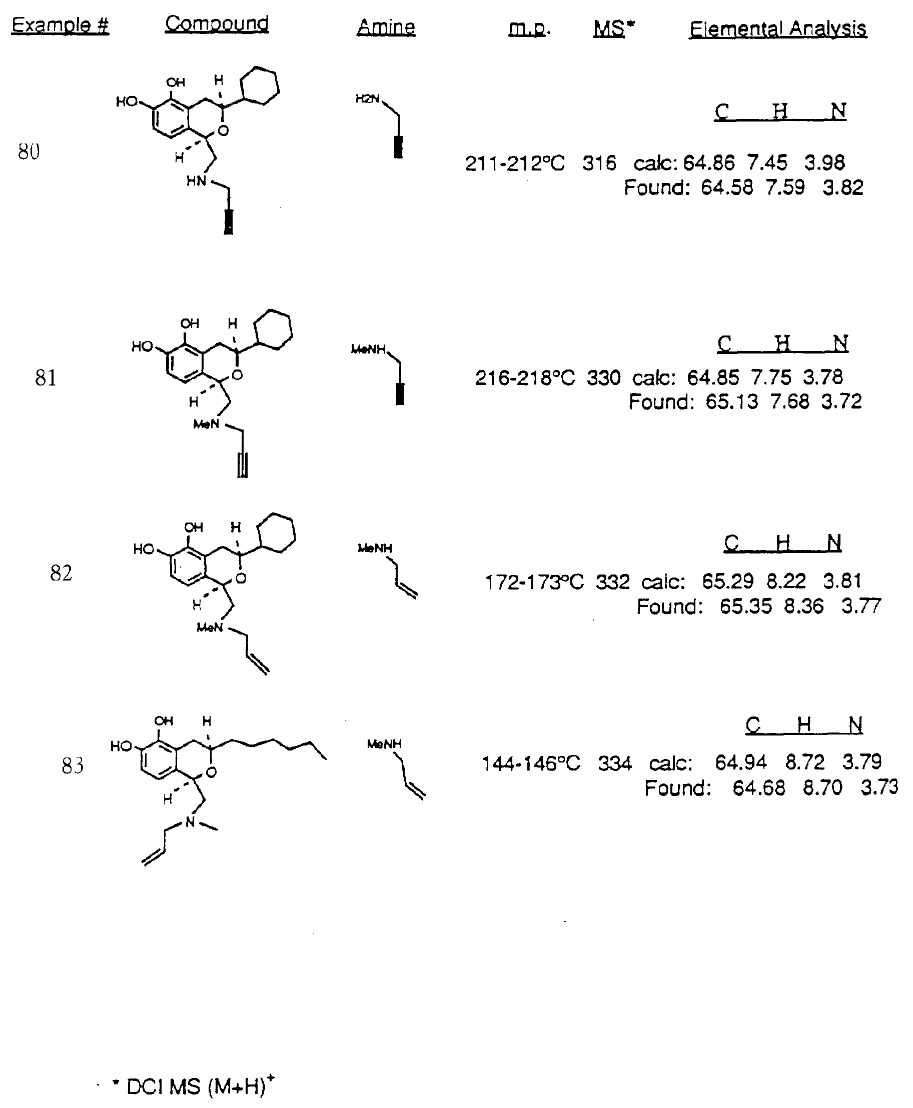

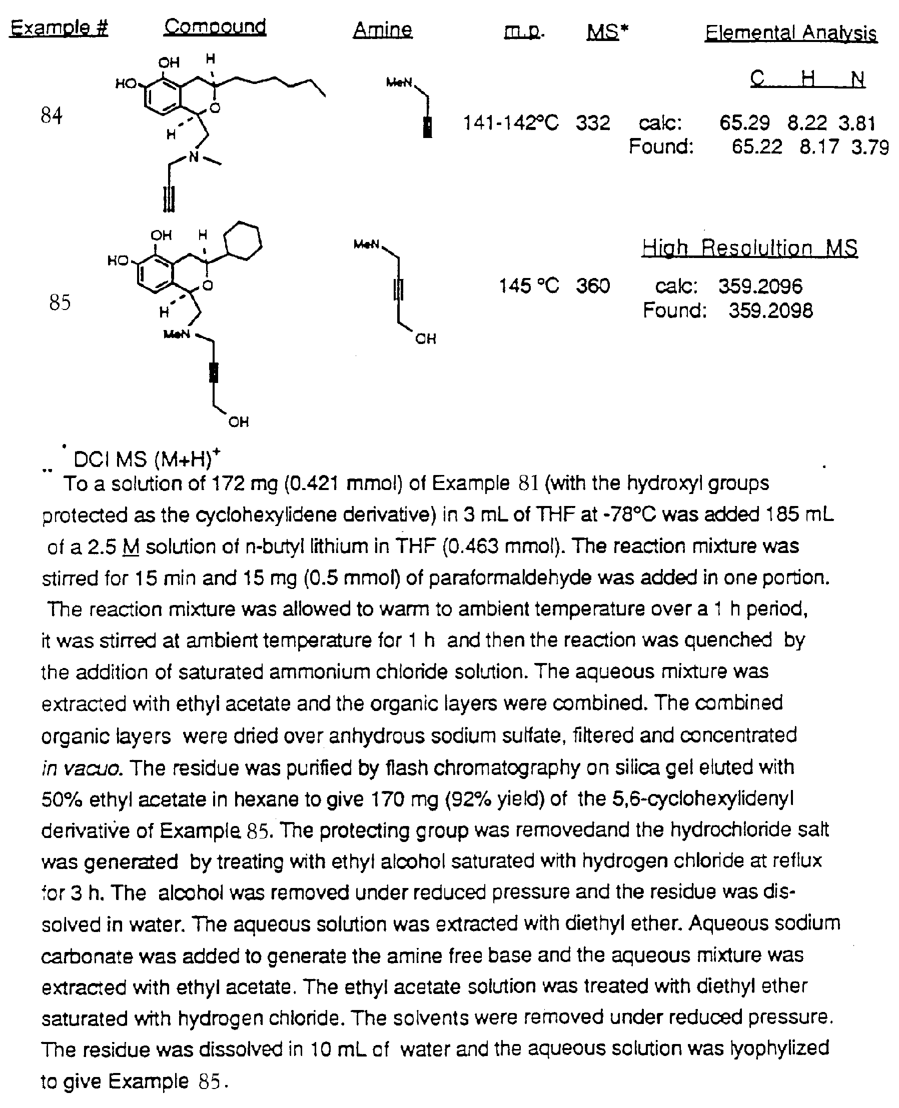

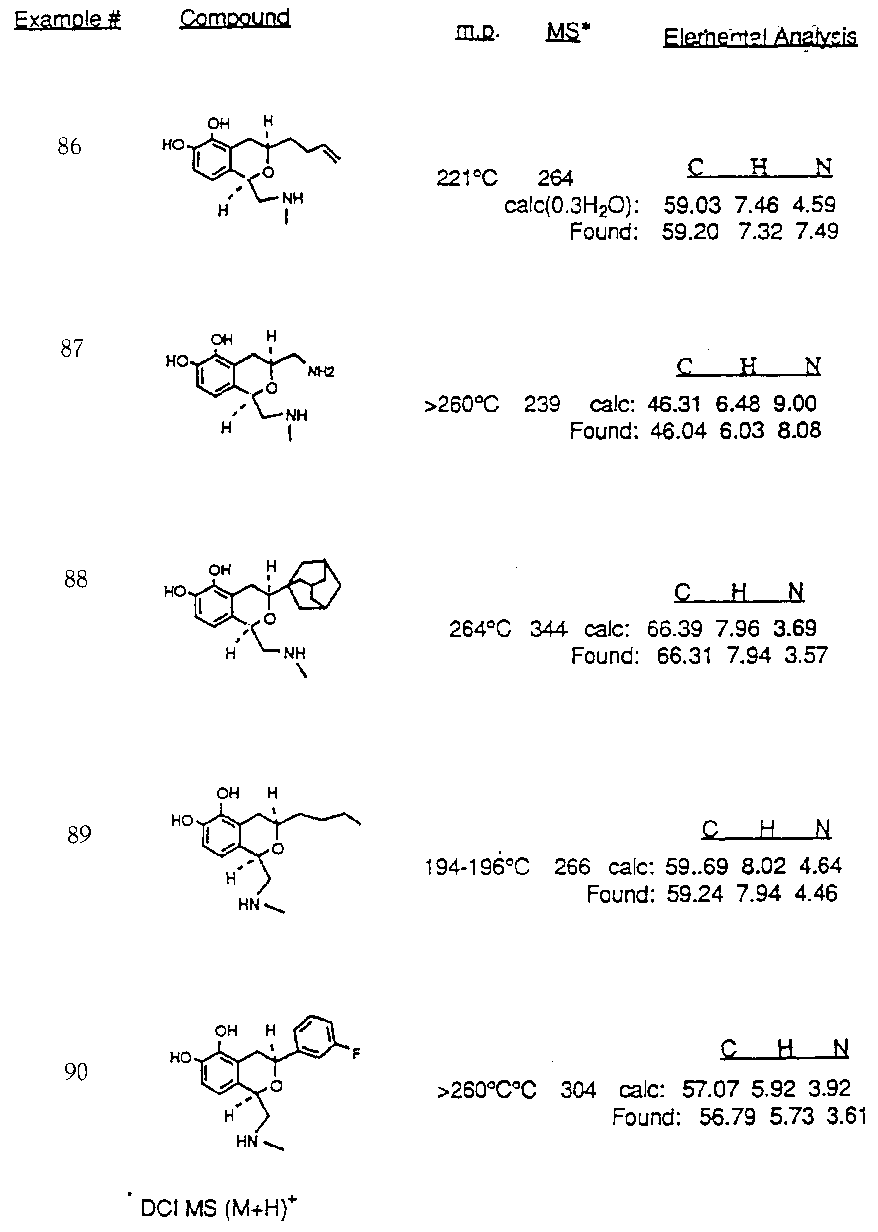

### Example 91

### 5,6-Dihydroxy-1-(N-methyllaminomethyl-3-phenyl-3,4-dihydronaphthalene

1-Aminomethyl-5,6-dimethoxy-3-phenyl-3,4-dihydronaphthalene, the product of Step 2 of Example 2, was N-methylated as described in Example 75 and deprotected as described in Step 4 of Example 2 to give the title compound as its hydrochloride salt, m.p. 131-133°C; DCI MS: 282 (M+H)⁺. Analysis calculated for C₁₈H₂₀CINO₂: C, 68.03; H, 6.34; N, 4.41. Found: C, 67.64; H, 6.54; N, 4.31.

### Example 92

### [1 R, 3S1 5,6-Dihydroxy-1-(N-methyl)aminomethyl-3-phenyl-1,2,3,4-tetrahydro-naphthalene

[1R,3S] 1-Aminomethyl-5,6-dimethoxy-3-phenyl-1,2,3,4-tetrahydronaphthalene, the product of Step 1 of Example 17, was N-methylated as described in Example 67 and deprotected as described in Step 4 of Example 2 to give the title compound as its hydrochloride salt, m.p. 211-213°C; DCI MS: 284 (M+H)^{+.} Analysis calculated for C₁₈H₂₂CINO₂: C, 65.75; H, 7.05; N, 4.26. Found: C, 65.54; H, 6.89; N, 4.04.

### Examples 93-95

Following the synthesis described in Examples 1 A and 2, the 1-aminomethyl precursors to Examples 93 - 95 were prepared with the catechol hydroxyl groups protected as dimethyl ethers. The 1-aminomethyl compounds were N-acylated. The N-acyl derivatives reduced as described in Example 67 and deprotected as described in Step 4 of Example 2, using the appropriate acylating agent and lithium aluminum hydride (LAH) as the reducing agent to give Example 93-95 as their hydrochloride salts unless otherwise noted. In the case of Example 94, the acylation/reduction sequence was repeated to give the dialkylamino derivative. Examples 93-95 are disclosed in Table 5. The structure of each was confirmed by melting point (m.p.), elemental analysis and mass spectra, as designated.

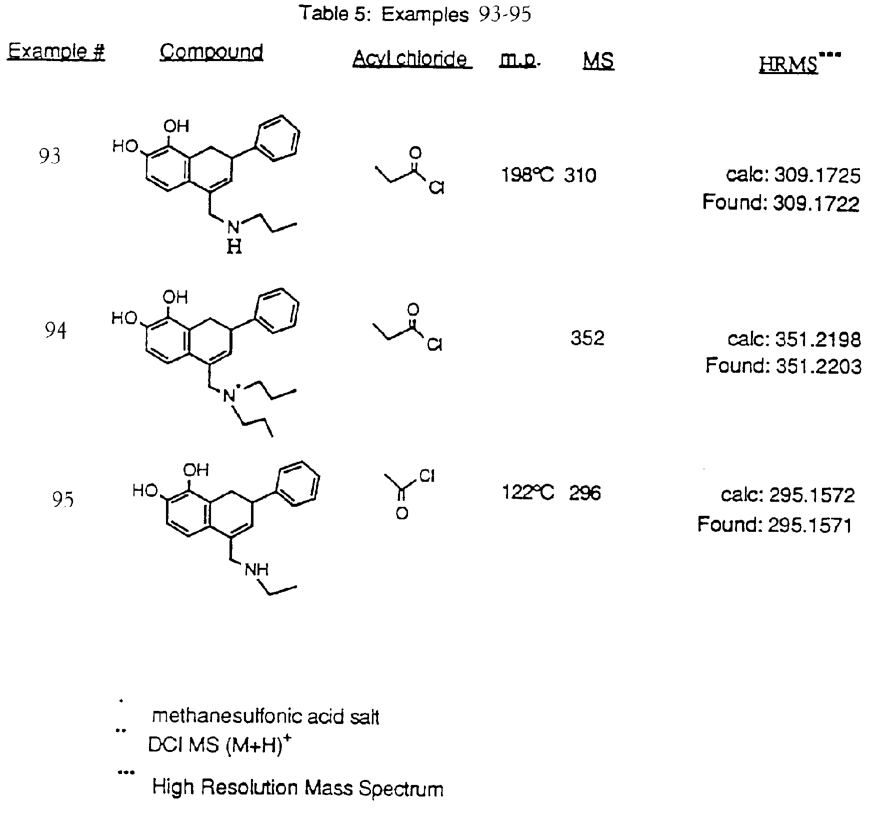

### Example 96

### [1 R,3S] 1,3-Bis(aminomethyl)-3,4-dihydro-5,6-dihydroxy-1H-2-benzopyran dihydrochloride

### Step 1: 1-Benzyloxy-3-(Spiro-[(1,3-benzodioxole)-2,1'-cyclohexane])-2-propanol

Glycidol (3.1 g, 42 mmol) was added dropwise to a suspension of sodium hydride (1.0 g, 42 mmol) in 25 mL of dry dimethyl formamide (DMF) at 0°C. After stirring the suspension for 30 min at 0°C, 7.1 g (42 mmol) of benzyl bromide was added dropwise and the reaction mixture was stirred at 0°C for 40 min. The reaction mixture was then diluted with 75 mL of diethyl ether, transferred to a separatory funnel and washed with 2 X 30 mL of 2 N aqueous sulfuric acid solution, 2 X 30 mL of water and saturated aqueous sodium bicarbonate solution. The organic solution was dried over anhydrous magnesium sulfate, filtered and concentrated at reduced pressure to give 5.3 g of the protected epoxy alcohol as an oil.

N-Butyl lithium (16.5 mL of 2.5M solution in hexane, 46 mmol) was added to a solution of spiro[(1,3-benzodioxole) -2,1'-cyclohexane] (7.4 g, 39 mmol) in 75 mL of THF at 0°C. After 4 h, the protected glycidol (5.3 g, 32 mmol) in 10 mL of THF was added dropwise and the reaction mixture was allowed to warm to ambient temperature. After 1.5 h, the reaction mixture was poured into 10% aqueous ammonium chloride solution and extracted with 2 X 50 mL of diethyl ether. The combined ether extracts were washed with ammonium chloride solution, dried over anhydrous magnesium sulfate, filtered and concentrated in vacuo. The residue was purified by column chromatography on silica gel eluted with 20% ethyl acetate in hexane to give 4.4 g (38% yield) of the title compound as a coloriess oil. DCI MS: 372 (M+NH₄)⁺, 355 (M+H)⁺. ¹H NMR (CDCl₃) δ 1.4-1.9 (m, 10H), 2.46 (d, 1 H, J=3.9 Hz), 2.79 (d, 2H, J=7.0 Hz), 3.4 (dd, 1H, J=9.9, 7.2 Hz), 3.52 (dd, 1 H, J=9.9, 3.0 Hz), 4.12 (m, 1 H), 4.54 (s, 2H), 6.6-6.73 (m, 3H), 7.34 (m, 5H).

### Step 2: [1R,3S] 3-Benzoxymethyl-1-bromomethyl-5,6-cyclohexylidenedioxy-3,4-dihydro-1H-2-benzopyran

A solution of 1-benzyloxy-3-(spira-[(1,3-benzodioxole)-2,1'-cyclohexane])-2-propanol (4.3 g, 12 mmol), from Step 1, and bromoacetaldehyde dimethyl acetal (1.7 mL, 14 mmol) in 25 mL of methylene chloride was cooled to 0°C. Boron trifluoride etherate (3.6 mL, 29 mmol) was added dropwise and the reaction mixture was stirred for 1.5 h. The resultant dark brown solution was poured into 50 mL of 10% aqueous sodium carbonate solution and the aqueous solution was extracted with 3 X 50 mL of diethyl ether. The combined ether extracts were washed with saturated aqueous sodium bicarbonate solution and brine, dried over anhydrous magnesium sulfate, filtered and concentrated under reduced pressure. The residue was purified by column chromatography on silica gel eluted with 20% ethyl acetate in hexane to give 4.2 g (75%) of the title compound as a coloriess syrup; DCI MS: 476 (M+NH4)+ ¹H NMR (CDCl₃)δ 1.45-1.95 (m, 10H), 2.57 (dd, 1 H,J=16.5,11.4Hz), 2.71 (dd, 1 H J=16.5, 3 Hz), 3.59 (dd, 1 H, J=11.4, 6 Hz), 3.63 (dd, 1 H, J=10.8, 4.2 Hz), 3.73 (dd, 1 H, J=10.8, 6 Hz), 3.87 (dd, 1 H, J=11.4, 2.7), 4.65 (d, 1 H, J=12 Hz), 4.72 (d, 1 H, J=12 Hz); 5.0 (m, 1 H), 6.52 (d, 1 H, J=8.₄ Hz), 6.62 (d, 1 H, J=8.₄ Hz), 7.42-7.25 (m, 5H).

### Step 3: [1R,3S] 1-Bromomethyl-3-hydroxymethyl-5,6-cyclohexylidenedioxy-3,4-dihydro-1H-2-benzopyran

5% Platinum on carbon (1.0 g) was added to a solution of [1R,3S] 3-benzyloxymethyl-1-bromomethyl-5,6-cyclohex- ylidenedioxy-3,4-dihydro-1 H-2-benzopyran (4.0 g, 8.7 mmol), from Step 2, in 150 mL of methanol and 5 mL of ethyl acetate. The reaction mixture was sealed under 4 atmospheres of hydrogen and shaken overnight. The reaction mixture was filtered to remove the catalyst and concentrated to a light brown oil. The oil was purified by column chromatography on silica gel eluted with 30% ethyl acetate in hexane to give 2.2 g (68% yield) of the title compound as a white foam. ¹H NMR (CDCl₃) δ 1.4-1.95 (m, 10H), 2.25 (dd, 1 H, J=8.4, 4.5 Hz), 2.62 (d, 2H, J=7.5 Hz), 3.57 (dd, 1 H, J=11.4, 6.9 Hz), 3.65-3.9 (m, 4H), 4.98 (m, 1 H), 6.52 (d, 1 H, J=8.4 Hz), 6.63 (d, 1 H, J=8.4 Hz).

### Step 4: 1-Azidomethyl-5,6-cyclohexylidenedioxy-3- hydroxymethyl-3,4-dihydro-1 H-2-benzopyran

Lithium azide (1.0 g, 20 mmol) was added to a solution of [1R,3S] 1-bromomethyl-5,6-cyclohexylidenedioxy-3-hydroxymethyl-3,4-dihydro-1H-2-benzopyran (2.17 g, 5.87 mmol), from Step 3, in 20 mL of DMF. The reaction mixture was heated to 70°C for 1.5 h then cooled to ambient temperature and poured into 50 mL of diethyl ether and 50 mL of water. The layers were separated and the aqueous layer was extracted with 2 X 50 mL of diethyl ether. The combined ether layers were washed with saturated aqueous sodium bicarbonate solution, dried over anhydrous magnesium sulfate, filtered and concentrated in vacuo. The residue was purified by column chromatography on silica gel eluted with 25% ethyl acetate in hexane to give 1.38 g (70% yield) of the title compound as a colorless glass. ¹H NMR (CDCl₃) δ 1.45-1.95 (m, 10H), 2.14 (dd, 1 H, J=9.0, 4.8 Hz), 2.63 (d, 2H, 7.5 Hz), 3.5 (dd, 1 H, J=13.5, 7.0 Hz), 3.62 (dd, 1 H, J=13.5, 2.7 Hz), 3.65-3.9 (m, 3H), 5.02 (m, 1 H), 6.45 (d, 1 H, J=8.₄ Hz), 6.61 (d, 1 H, J=8.₄ Hz).

### Step 5: [1R,3S] 1,3-Bis(azidomethyl)-5,6-cyclohexylidenedioxy-3,4-dihydro-1H-2-benzopyran

Methanesulfonyl chloride (0.128 mL, 1.65 mmol) was added dropwise to a solution of 1-azidomethyl-5,6-cyclohex- ylidenedioxy-3-hydroxymethyl-3,4-dihydro-1 H-2-benzopyran (500 mg, 1.5 mmol), from Step 4, and 0.314 mL (2.25 mmol) of triethylamine (TEA) in 15 mL of methylene chloride at 0°C. After stirring for 30 min at 0°C, the reaction mixture was transferred to a separatory funnel and diluted with diethyl ether. The layers were separated and the organic layer was washed with 2 X 20 mL of water, 2 X 15 mL of 1 N aqueous hydrochloric acid solution and brine, dried over magnesium sulfate, filtered and concentrated under reduced pressure to yield a white foam. The foam was dissolved in 20 mL of DMF and 440 mg (9 mmol) of lithium azide was added. The reaction mixture was heated to 80°C and stirred at 80°C for 4 h then cooled and poured into 50 mL of water. The aqueous solution was extracted with 3 X 30 mL of diethyl ether and the combined ether extracts were washed with 30 mL of waterand brine, dried over anhydrous magnesium sulfate and concentrated under reduced pressure The residue was purified on silica gel eluted with diethyl ether to give 450 mg (84% yield) of the title compound as a pale yellow oil; DCI MS: 374 (M+NH4)+ ¹ H NMR (CDCl₃) δ 1.45-1.95 (m, 1 OH), 2.67 (m, 2H), 3.38 (dd, 1H, J=13.5, 3.9 Hz), 3.5 (m, 2H), 3.7 (dd, 1 H, J=13.5, 2.7 Hz), 3.9 (m, 1 H), 5.0 (m, 1 H), 6.47 (d, 1 H, J=8.7 Hz), 6.62 (d, 1 H, J=8.7 Hz)

### Step 6: [1R,3S] 1,3-Bis(aminomethyl)-5,6-cyclohexylidenedioxy-3,4-dihydro-1H-2-benzopyran

Lithium aluminum hydride (2.4 mL of 1.0 M solution in diethyl ether, 2.4 mmol) was added dropwise to a solution of [1R,3S] 1,3-bis(azidomethyl)-5,6-cyclohexylidenedioxy-3,4-dihydro-1 H-2-benzopyran (430 mg, 1.2 mmol), from Step 5, in 10 mL of anhydrous diethyl ether at 0°C. The reaction mixture was allowed to warm to ambient temperature and stirred for 45 min. The reaction was then quenched by the sequential addition of 91 µL of water, 91 µL of 15% aqueous sodium hydroxide solution and 273 µL of water. The solution was dried over anhydrous magnesium sulfate, filtered and concentrated in vacuo to give 212 mg (85% yield) of the title compound as a colorless glass; 1H NMR (CDCl₃) δ 1.4-1.95 (m, 14H), 2.5 (dd, 1 H, J=₁₇.₁ Hz), 2.65 (dd, 1 H, J=₁₇.₁, 3 Hz), 2.9 (m, 2H), 3.0 (dd, 1 H, J=₁3.8, 6 Hz), 3.21 (dd, 1 H, J=13.8, 2.4 Hz), 3.66 (m, 1 H), 4.7 (m, 1 H), 6.51 (d, 1 H, J=8.4 Hz), 6.61 (d, 1 H, J=8.4 Hz).

### Step 7: [1R,3S] 1,3-Bis(aminomethyl)-3,4-dihydro-5,6-dihydroxy-1H-2-benzopyran dihydrochloride

Absolute ethyl alcohol was saturated with anhydrous hydrogen chloride and added to 212 mg (0.96 mmol) of [1 R,3S] 1,3-bis(aminomethyl)-5,6-cyclohexylidenedioxy-3,4-dihydro-1H-2-benzopyran from Step 6. The solution was heated to reflux temperature. After 45 min at reflux temperature, a precipitate formed and the volume of the reaction mixture was reduced to 5 mL. Diethyl ether was added until precipitation was complete and the precipitate was collected by vacuum filtration. The solid was washed with diethyl ether and dried in a vacuum oven at 80°C overnight to give 280 mg (96% yield) of the title compound as a fine white powder, m.p >260°C; IR 3320, 3040, 1590, 1500, 1290 cm⁻¹; DCI MS 225 (M+H)⁺; ¹H NMR (d₆-DMSO) δ 2.38 (dd, 1 H, J=16.5, 12 Hz), 2.76 (m, 2H), 2.97 (m, 1 H), 3.52 (m, 2H), 3.9 (m, 1 H), 4.83 (m, 1 H), 6.54 (d, 1 H, 8.1 Hz), 6.7 (d, 1 H, J=8.1 Hz), 8.25 (br s, 6H), 8.6 (s, 1 H), 9.4 (s, 1 H). Analysis calculated for C₁₁H₁₈Cl₂N₂O₃: C, 44.46; H, 6.11; N, 9.43. Found: C, 44.70; H, 6.04; N, 9.22.

### Example 97

### [1 R,3S] 1-Aminomethyl-3,4-dihydro-5,6-dihydroxy-3-hydroxymethyl-1H-2-benzopyran hydrochloride

### Step 1: [1R,3S] 1-Aminomethyl-5,6-cyclohexylidenedioxy-3,4-dihydro-3-hydroxymethyl-1H-2-benzopyran

Lithium aluminum hydride (1.1 mL of 1.0M solution in diethyl ether, 1.1 mmol) was added dropwise to a solution of 370 mg (1.1 mmol) of 1-azidomethyl-5,6-cyclohexylidenedioxy-3,4-dihydro-3-hydroxymethyl-1 H-2-benzopyran, the product of Step 4 of Example 96, in 10 mL of anhydrous diethyl ether at 0°C. The reaction mixture was allowed to warm to ambient temperature and stirred for 40 min The reaction mixture was cooled to 0°C and quenched by the sequential addition of 42 µL of water, 42 µL of 15% aqueous sodium hydroxide solution and 126 µL of water. The solution was dried over anhydrous magnesium sulfate and filtered. The filtrate was concentrated in vacuo to give 263 mg (77% yield) of the title compound as a white powder; DCI MS: 306 (M+H)+; ¹H NMR (CDCl₃) δ 1.4-1.95 (m, 13H), 2.6 (m, 2H), 3.03 (dd, 1H, J=13.5, 5.7 Hz), 3.23 (dd, 1 H, J=13.5, 2.7 Hz), 3.7 (dd, 1 H, J=11.7, 7.5 Hz), 3.77-3.9 (m, 2H), 6.52 (d, 1 H, J=8.₄ Hz), 6.62 (d, 1 H, J=8.4 Hz).

### Step 2 : [1R,3S] 1-Aminomethyl-3,4-dihydro-5,6-dihydroxy-3-hydroxymethyl-1H-2-benzopyran hydrochloride

Absolute ethyl alcohol was saturated with anhydrous hydrogen chloride and added to a suspension of 256 mg (0.83 mmol) of [1R,3S] 1-aminomethyl-5,6-cyclohexylidenedioxy-3,4-dihydro-3-hydroxymethyl- 1 H-2-benzopyran from Step 1 in 2 mL of ethanol. The reaction mixture was heated to reflux temperature. After 1.5 h at reflux temperature, a precipitate had formed. The solvents were evaporated down under reduced pressure to approximately 5 mL. Diethyl ether was added until the precipitation was complete and the solid was collected by vacuum filtration, washed with diethyl ether and dried in a vacuum oven at 80°C overnight to give 160 mg (73% yield) of the title compound as an off-white powder, m.p. 235°C; DCI MS: 226 (M+H)⁺; IR: 3200, 1590, 1500, 1295, 1050 cm⁻¹; ¹H NMR (d₆-DMSO) δ 2.28 (dd, 1 H, J=16.8, 11.4 Hz), 2.66 (dd, 1 H, J=16.8, 3.0 Hz), 2.83 (dd, 1 H, J=12.3, 9.3 Hz), 3.45-3.7 (m, 4H), 4.8 (m, 2H), 6.51 (d, 1 H, J=8.4 Hz), 6.67 (d, 1 H, J=8.4 Hz), 8.05 (br s, 3H), 8.48 (br s, 1 H), 9.3 (br s, 1 H). Analysis calculated for C₁₁ H₁₆CINO₄; C, 50.48; H, 6.16; N, 5.35. Found: C, 50.64; H, 6.24; N, 5.20.

### Example 98

### [1R,3S]1-Aminomethyl-3,4-dihydro-5.6-dihydroxy-3-pyrrolidinylmethyl-1H-2-benzopyran dihydrochloride

### Step 1: [1R,3S] 1-Azidomethyl-5,6-cyclohexylidenedioxy-3,4-dihydro-3-pyrrolidinylmethyl-H-2-benzopyran

Methanesulfonyl chloride (0.146 mL, 1.89 mmol) was added dropwise to a solution of 0.57 g (1.72 mmol) of [1R,3S] -azidomethyl-5,6-cyclohexylidene-dioxy-3,4-dihydro-3-hydroxymethyl-1 H-2-benzopyran, the product of Step 4 of Example 96, and 0.36 mL (2.58 mmol) of triethylamine in 15 mL of methylene chloride at 0°C. The reaction mixture was stirred for 30 min at 0°C then transferred to a separatory funnel and diluted with 45 mL of diethyl ether. The layers were separated and the organic layer was washed with 2 X 20 mL of water, 2 X 20 mL of 1 N hydrochloric acid and 20 mL of brine, dried over anhydrous magnesium sulfate, filtered and concentrated under reduced pressure to 405 mg of white foam. The foam was dissolved in 20 mL of dimethyl formamide (DMF) and an excess amount of pyrrolidine was added to this solution. The reaction mixture was heated at 95°C for 2.5 h then poured into 75 mL of water. The aqueous solution was extracted with 3 X 40 mL of diethyl ether. The combined ether extracts were washed with 2 X 30 mL of water and brine, dried over anhydrous magnesium sulfate, filtered and concentrated in vacuo. The residue was purified by column chromatography on silica gel eluted with 10% methanol in methylene chloride to give 210 mg (55% yield) of the title compound as a white foam; DCI MS: 385 (M+H)+; 1H NMR (CDCI₃) 8 1.4-1.9 (m, 14H), 2.5-2.9 (m, 8H), 3.45 (dd, 1 H, J=13.2, 6.6 Hz), 3.68 (dd, 1 H, J=13.2; 2.4Hz), 3.9 (m, 1 H), 4.97 (m, 1 H), 6.45 (d, 1 H, J=8.1 Hz), 6.6 (d, 1 H, J=8.1 Hz).

### Step 2: [1R,3S] 1-Aminomethyl-5,6-cyclohexylidenedioxy-3,4-dihydro-3-pyrrolidinylmethyl-1H-2-benzopyran

Lithium aluminum hydride (0.52 mL of a 1.0 M solution, 0.52 mmol) was added dropwise to a solution of 20 mg (0.52 mmol) of 1-azidomethyl-5,6-cyclohexylidenedioxy-3,4-dihydro-3-pyrrolidinylmethyl-1H-2-benzopyran, from Step 1, in 10 mL of anhydrous diethyl ether at 0°C. The reaction mixture was allowed to warm to ambient temperature and it was stirred at ambient temperature for 40 min. The reaction mixture was then cooled to 0°C and quenched by the sequential addition of 20 µL of water, 20 µL of 15% aqueous sodium hydroxide solution and 60 µL of water. The resultant solution was dried over anhydrous magnesium sulfate and the precipitate filtered. Diethyl ether saturated with anhydrous hydrogen chloride was then added dropwise to the filtrate to precipitate the hydrochloride salt of [1R,3S] 1-aminomethyl-5,6-cyclohexylidenedioxy-3,4-dihydro-3-pynrolidinylmethyl-1 H-2-benzopyran which was collected by vacuum filtration yielding 220 mg (98% yield) of the title compound as its hydrochloride salt, a white solid; DCI MS: 359 (M+H)⁺.

### Step 3: [1R,3S] 1-Aminomethyl-5,6-dihydroxy-3-pyrrolidinylmethyl-1H-2-benzopyran dihydrochloride

Absolute ethanol (10 mL) was saturated with anhydrous hydrogen chloride and added to 187 mg (0.44 mmol) of the product of Step 2, [1R,3S] 1-aminomethyl-5,6-cyclohexylidenedioxy-3,4-dihydro-3-pyrrolidinylmethyl-1 H-2-benzopyran. The reaction mixture was heated to reflux temperature. After 2 h at reflux temperature, a precipitate formed and the reaction mixture was cooled to ambient temperature. The volume of the reaction mixture was reduced under reduced pressure to approximately 5 mL. Diethyl ether was added to the concentrate to precipitate the product which was collected by vacuum filtration and washed with diethyl ether. The solid was dried in a vacuum oven at 80°C overnight to give 146 mg (96% yield) of the title compound as a fine white powder, m.p > 280°C; I R 3400, 3200, 2960, 1510, 1295 cm-1; DCI MS 279 (M+H)⁺; ¹H NMR (D₆-DMSO) δ 2.0 (m, 4H), 2.33 (dd. 1 H J=16.2, 10.8 Hz), 3.1 (m, 2H), 3.4 (m, 2H), 3.6 (m, 3H), 4.05 (m, 1 H), 4.93 (m, 1 H), 6.54 (d, 1 H, J=8.7 Hz), 6.7 (d, 1 H, J=8.7 Hz), 8.4 (br s, 3H), 8.6 (s, 1 H), 9.4 (s, 1 H), 10.6 (br s, 1 H). Analysis calculated for C₁₅H₂₄Cl₂N₂O₃: C, 51.29; H, 6.89; N 7.97. Found C,50.94; H,6.82; N,7.76.

### Examples 99-102

Following the synthesis described in Example 98, using 3-(benzyloxy) propylene oxide and the appropriate alkyl or cycloalkyl amine, Examples 99 and 100 were prepared as disclosed in Table 6, as their dihydrochloride salts. Following the procedures described in Examples 96 and 97, using 4-(benzyloxy)butylene oxide, Examples 107 and 102 were prepared as disclosed in Table 6. The structure of each was confirmed by melting point, mass spectra and elemental analysis as designated

### Examples 103-114

Following the synthesis illustrated in Scheme V using the appropriate acylating agent Examples 103-114 were prepared as disclosed in Table 7, as their hydrochloride salts. The structure of each was confirmed by melting point, mass spectra and elemental analysis as designated

### Examples 115-117

Following the synthesis described in Examples 96 and 97, using 4-(benzyloxy) butylene oxide and the procedure for N-methylation described in Example 67, Examples 115- 117 were prepared as disclosed in Table 8, as their hydrochloride salts. The structure of each was confirmed by melting point, mass spectra and elemental analysis as designated.

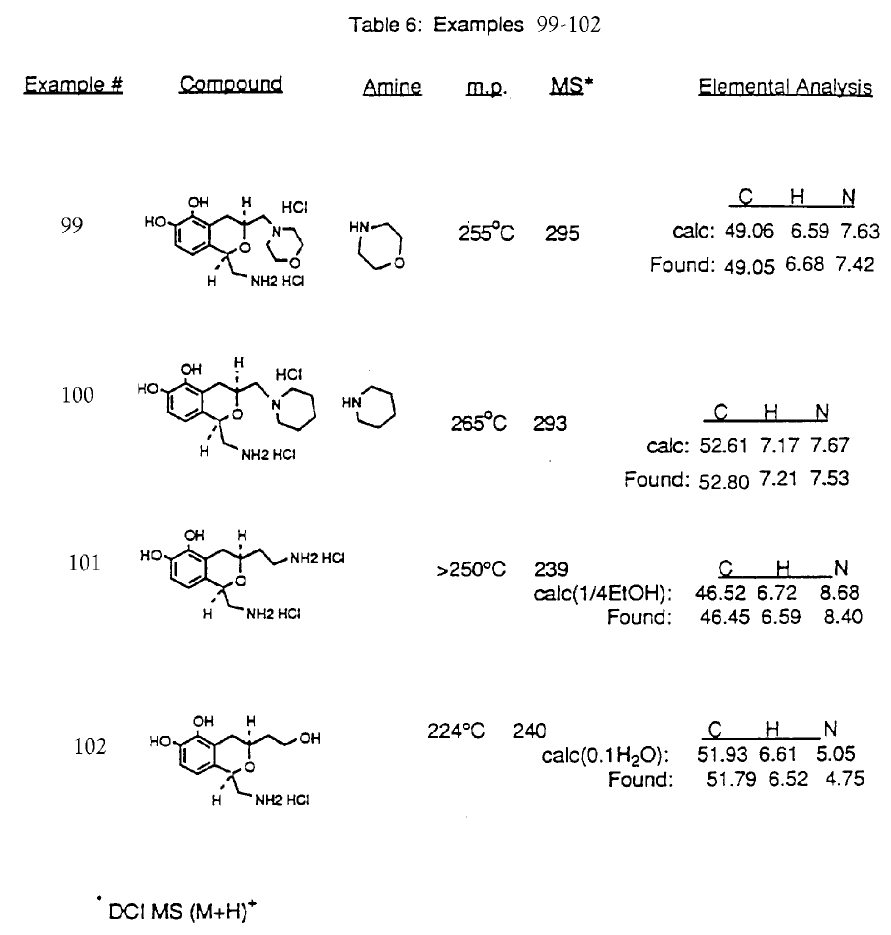

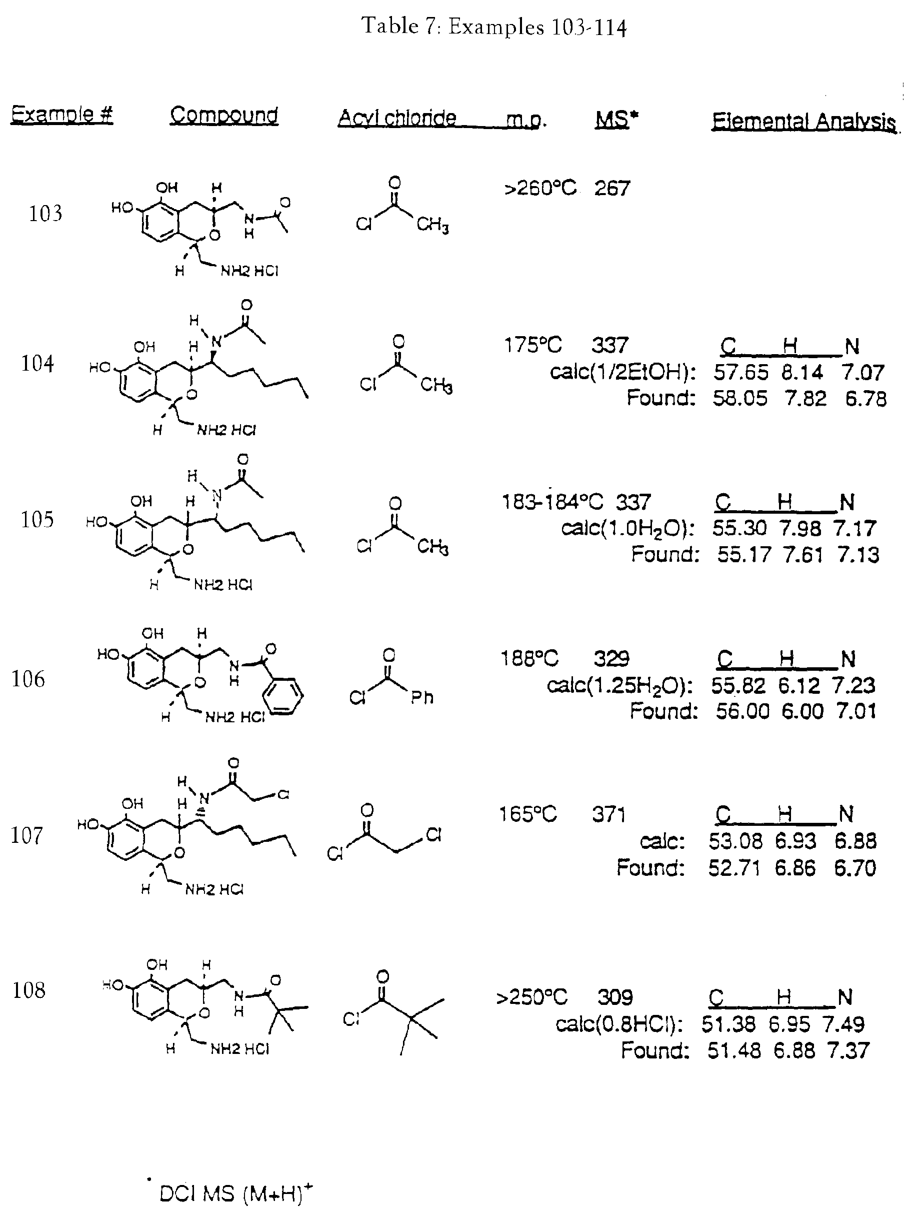

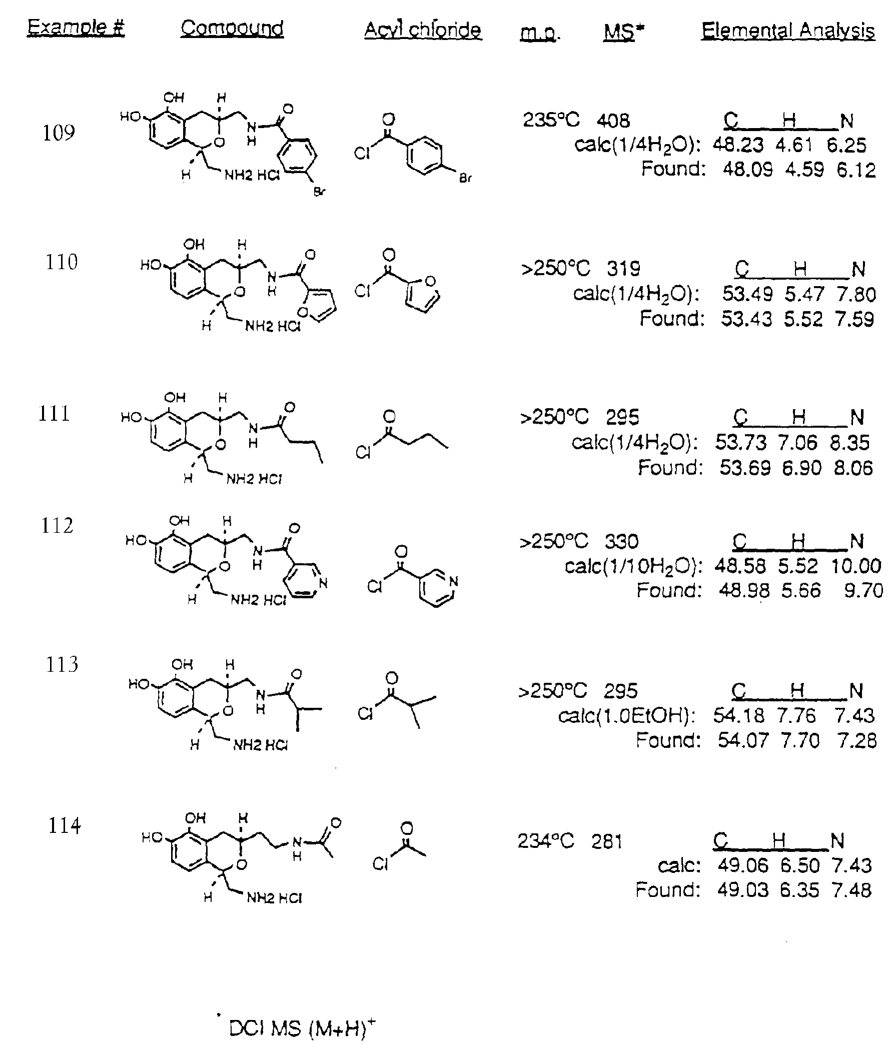

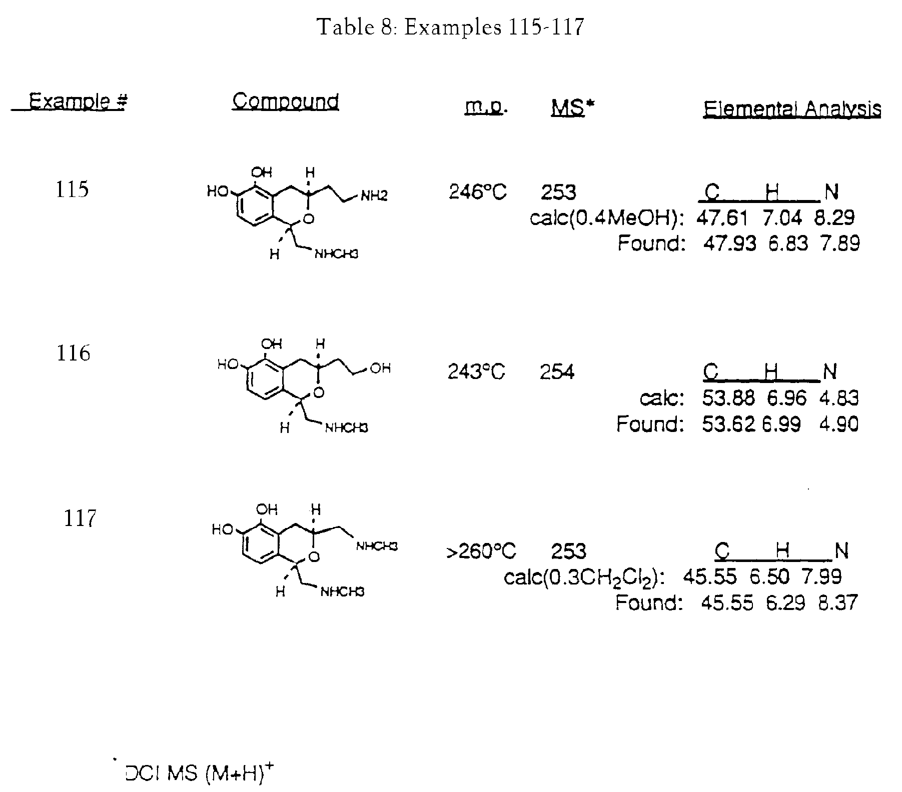

### Example 118

### [1S*,3R*) 1-Aminomethyl-3-phenyl-5,6-dihydroxy-1H-2-benzopyran hydrochloride

### Step 1: 2-(2'-3'-Cyclohexylidenedioxyphenyl)-1-phenylethanone

A solution of 15.5 g (50 mmol) of 2-(2',3'-cyclohexylidenedioxyphenyl)-1-phenylethanol prepared from styrene oxide (commercially available from Aldrich Chemical Company) by the procedure described in Step 2 of Example 31, in 60 mL of methylene chloride was added dropwise to a mixture of 60 g (28 mmol) of pyridinium chlorochromate (PCC) and 35 g of Celite® filter aid in 300 mL of methylene chloride at ambient temperature. After 4 h, the reaction mixture was diluted with 200 mL of diethyl ether and filtered through silica gel. The chromium-containing residue was washed several times with diethyl ether. The filtrate was concentrated under reduced pressure to give 14 g (90% yield) of the title compound as a yellow syrup; DCI MS: 326 (M+NH₄)⁺, 309 (M+H)+; ¹H NMR (CDCl₃) δ1.4-1.9 (m, 10H) 4.2 (s, 2H), 6.7 (m, 3H), 7.42 (m, 2H), 7.53 (m, 1 H), 8.05 (m, 2H).

### Step 2: [1R^{*}] 2-(2',3'-cyclohexylidenedioxyphenyl)-1-phenylethanol

A solution of 754 mg (2.45 mmol) of 2-(2',3'-cyclohexylidenedioxyphenyl)-1-phenylethanone, from Step 1, in 1 mL of THF was added to a solution of 936 mg (2.9 mmol) of (-) B-chlorodiisopinocampheylborane (commercially available from Aldrich Chemical Company) in 3 mL of THF at -20°C. After storing the resultant solution for 12 h at -15°C, the solvent was evaporated, the residue was dissolved in 15 mL of diethyl ether and 565 mg of diethanolamine was added. The mixture was stirred for 30 min The precipitate was removed by filtration through Cetite^{®} filter aid. The filtrate was concentrated and the residue purified by column chromatography on silica gel eluted with methylene chloride/hexane/di- ethyl ether (100:20:1) to give 546 mg (72% yield) of the title compound; DCI MS: 328 (M+NH₄)⁺; ¹H NMR (CDCl₃) δ 1.4-1.9 (m, 10H), 2.3 (br s, 1 H), 3.0 (m, 2H), 4.98 (dd, 1 H, J=7.5, 5.0 Hz), 6.62 (m, 3H), 7.3 (m, 5H).

### Step 3: [1S^{*},3R^{*}] 1-Aminomethyl-3-4-dihydro-5,6-dihydroxy-3-phenyl-1H-2-benzopyran hydrochloride

[1R*] 2-(2',3'-Cyclohexylidenedioxyphenyl)-3-phenylethanol was converted to [1S*, 3R^{*}] 1-aminomethyl-3,4-dihy- dro-5,6-dihydroxy-3-phenyl-1 H-2-benzopyran by the procedures detailed in Step 3 of Example 31 and Steps 1 - 3 of Example 32, m.p. 158-160°C; [alpha]D = +110° (c 0.52, 1N HCl); DCl MS: 272 (M+H)⁺. Analysis calculated for C₁₆H₁₈CINO₃: C, 60.60; H, 6.05; N, 4.42. Found: C,60.71; H,6.2; N,4.31.

### Example 119

### [1R^{*}3S^{*}] 3-Adamantyl-1-aminomethyl-3,4-dihydro-5,6-dihydroxy-1H-2-benzopyran hydrochloride

### Step 1 :1-Aza-2-boro-3-oxa-1,4,4-triphenyl-bicyclo[3,3,0] octane

Diphenyl-(2R^{*-}2'-pyrrolidinyl)methanol (610 mg, 2.41 mmol) and phenylboronic acid (294 mg, 2.41 mmol) were taken up in 25 mL of toluene. The diphenyl-(2R^{*}.2'pyrrolidinyl)methanol was prepared as described by E.J. Corey, et al. in J American Chem Soc. 109: 5551-3 (1987).The reaction mixture was heated at reflux temperature for 4 h under a nitrogen atmosphere using a Dean Stark trap filled with 4 A molecular sieves to remove water. The reaction was then cooled and concentrated in vacuo to afford the title compound as a colorless oil. The product was carried on to the next step without purification.

### Step 2; [1R^{*}] 1-(1'-adamantyl)-2-bromo-1-hydroxyethane

Diborane in THF (5.2 mL of 1.0 M THF solution , 5.2 mmol) was added dropwise over a period of 10 min to a solution of 2.22 g (8.63 mmol) of 1-adamantyl-bromomethyl ketone (commercially available from Aldrich Chemical Co.) and 2.1 mL of a 0.2 M solution in THF (0.43 mmol) of 1-aza-2-boro-3-oxa-1,4,4-triphenyl-bicyclo[3.3.0]octane, from Step 1, in 16 mL of anhydrous THF The reaction mixture was stirred for 10 min at ambient temperature and then cooled to ~0°C in an ice bath and then the reaction was quenched by the careful addition of 3 mL of methanol. Diethyl ether saturated with hydrogen chloride (2 mL) was added and the solution was allowed to warm to ambient temperature. The solution was stirred at ambient temperature for 0.5 h and then it was poured into 100 mL of diethyl ether and 100 mL of water. The organic layer was washed with 1 N aqueous hydrochloric acid solution, aqueous saturated sodium bicarbonate and brine, dried over anhydrous magnesium sulfate, filtered and concentrated under reduced pressure to give 216 mg (96% yield) of the title compound as a white solid.

### Step 3: [1R^{*}) 1-(1'-adamantyl)-ethylene oxide

A 15% aqueous solution of sodium hydroxide was added to a solution of 1.9 g (7.34 mmol) of [1R^{*}] 1-(1'-adamantyl) -2-bromo-1-hydroxyethane, from Step 2, in 50 mL of diethyl ether The mixture was stirred vigorously at ambient temperature for ∿ 18 h. The mixture was then diluted with 100 mL of diethyl ether and 50 mL of water. The aqueous layer was extracted with 50 mL of diethyl ether. The combined organic layers were washed with 2 X 50 mL of water and 50 mL of brine, dried over anhydrous magnesium sulfate, filtered and concentrated under reduced pressure to afford 1.3 g of the title compound. The product was carried on to the next step without purification.

### Step 4: [1S*) 1-(1'-Adamantyl)-2-(spiro-[(1.3-benzodioxole)-2-1'-cyclohexane))-1-ethanol

n-Butyl lithium (6.7 mL of a 1.48 M solution in hexane, 9.9 mmol) was added over a 10 min period to a solution of spiro[(1, 3-benzodioxole)-2,1'-cyclohexane] in 14 mL of THF at 0°C. The reaction mixture was allowed to warm to ambient temperature over a 0.5 h period and then it was stirred for 3.5 h at ambient temperature. The reaction mixture was cooled to -78°C and a solution of 1.2 g (6.74 mmol) of [1 R^{*}] 1-(1'-adamantyl)-ethylene oxide, from Step 3, in 5 mL of THF was added. Boron trifluoride etherate (1.15 mL, 9.44 mmol) was then added dropwise over a 7 min period After 30 minutes, 25 mL of aqueous saturated sodium bicarbonate solution was added, followed by 25 mL of ethyl acetate. The reaction mixture was allowed to warm to ambient temperature and transferred to a separatory funnel. Ethyl acetate (50 mL ) and saturated aqueous sodium bicarbonate solution (25 mL) were added to the funnel and the layers were separated The aqueous layer was extracted with ethyl acetate. The combined organic layers were washed with 2 X 50 mL of aqueous sodium bicarbonate solution and brine, dried over anhydrous magnesium sulfate and concentrated under reduced pressure to an oil. The crude product was dissolved in 20 mL of methanol and the solution was cooled to 0°C. The precipitate was collected by filtration and washed with cold methanol to give 2.21 g (89% yield) of the desired product. The title compound was recrystallized from methanol to give 1.6 g of the title compound, m.p 72-73°C; [alpha]D =-27.75° (c 1.63, CHCl₃); DCI MS: 386 (M+NH4)⁺. 1H NMR (CDCI₃) 8 0.9 (q, 0.25 H, J=6 Hz, MeOH solvate), 1.5-2.1 (m, 25H), 2.5 (dd; 1H, J=13.5, 10.5 Hz), 2.87 (dd, 1 H, J=13.5, 2.0 Hz), 3.3 (m, 1 H), 3.5 (d, 0.75H, J-6 Hz, MeOH solvate). Analysis calculated for C₂₄H₃₂0₃+0.25MeOH: C, 77.35; H, 8.83. Found: C, 77.09; H, 8.77.

### Step 5: [1R*, 3S*] 3-(1'Adamantyl)-5,6-cyclohexylidenedioxy-3,4-dihydro-1-(N-formyl) aminomethyl-1 H-2-benzopyran

Trimethylsilyltriflate (73 µL, 0.38 mmol) was added to a mixture of 3.5 g (9.5 mmol) of [1S^{*}] 1-(1'-adamantyl)-2-(spiro-[(1;3-benzodioxole)-2,1'-cyclohexane])-1-ethanol, from Step 4, and N-formylacetaldehyde dimethyl acetal (2 g, 15.2 mmol) in 20 mL of acetonitrile. The reaction mixture was heated at a gentle reflux for 2 h and an additional 50 µL (0.26 mmol) of trimethylsilyltriflate was added. A precipitate formed and after 4 h the reaction mixture was cooled to 0°C. The precipitate was collected by filtration, washed with cold acetonitrile and dried to afford 2.92 g (70% yield) of the title compound as colorless crystals, m.p. 220-221 °C; [alpha]D =-33,15° (c 1.63, CHCl₃); DCI MS: 438 (M+H)⁺. ¹H NMR (d₆-DMSO) 6 @ 140°C 1.5-2.05 (m, 25H), 2.5 (m, 2H), 2.8 (m, 1H), 3.1 (dd, 1H, J=7.5, 3.0 Hz), 3.32 (m, 1 H) 3.71 (br s, 1 H), 4.65 (br s, 1 H), 6.6 (m, 2H), 8.05 (br s, 1 H).

### Step6: [1R*, 3S*]3-(1'-Adamantyl)-1-aminomethyl-5,6-cyclohexylidenedioxy-3,4-dihydro-iH-2-benzopyran

[1R^{*},3S^{*}] 3-(1'-Adamantyl)-5,6-cyclohexylidenedioxy-3,4-dihydro-1-(N-formyl)aminomethyl-1H-2-benzopyran (2.8 g, 6.41 mmol) was mixed together with 20 mL of 15% aqueous sodium hydroxide solution, 30 mL of methanol and 20 mL of THF The mixture was heated at ∿ 50°C for 3 h and then it was concentrated in vacuoto∿30 mL. The concentrated mixture was diluted with 150 mL of ethyl acetate/methylene chloride (2:1) and 50 mL of water. The organic layer was washed with 2 X 50 mL of water and 50 mL of brine. The combined aqueous layers were extracted with ethyl acetate/methylene chloride (2:1) and discarded. The combined organic layers were dried over anhydrous magnesium sulfate, filtered and concentrated in vacuo to give 2.64 g of the title compound as a light tan colored foam. The product was carried on to the next step without purification.

### Step 7: [1R*,3S*] 3-(1'-Adamantyl)-1-aminomethyl-3,4-dihydro-5,6-dihydroxy-1H-2-benzopyran hydrochloride

A solution of hydrochloric acid in 10:1 1 THF/water (20 mL of a 4 N solution) was added to [1R^{*}_{;} 3S^{*}] 3-(1'-adamantyl) -1-aminomethyl-5,6-cyclohexylidenedioxy-3,4-dihydro-1 H-2-benzopyran (1.1 g, 2.7 mmol) from Step 6, and the reaction mixture was heated at reflux temperature for 3 h. The reaction mixture was cooled to 0°C and 10 mL of diethyl ether was added. At the onset of crystallization an additional 10 mL of diethyl ether was added. After 30 min the precipitate was removed by filtration, washed with diethyl ether and dried to give 0.81 g (82% yield) of the title compound as a colorless solid; DCI MS: 330 (M+H)+, ¹H NMR (CD₃0D) 8 1.65-2.05 (m, 15H), 2.6 (dd, 1H, J=₁6.₅, ₁2.0 Hz), 2.72 (dd, 1 H, J=165, 3.0 Hz), 3.08 (dd, 1 H, J=12.6, 7.5 Hz), 3.15 (dd, 1 H, J=12.0, 3.0) 3.54 (dd, 1 H, J=12.6, 3.2 Hz), 4.85 (m, 1 H), 6.5 (d, 1 H, J=8.₄ Hz), 6.69 (d, 1 H, J=8.₄ Hz).

### Example 120

### [1R,3R] 1- Aminomethyl-3-n-butyl-3,4-dihydro-5,6-dihydroxy-1 H-2-benzothiopyran hydrochloride

### Step 1: 1-Spiro([1,3-benzodioxole)-2,1'-cyclohexanel-2-hexanol

n-Butyl lithium (34.8 mL of a 2.5 M solution in hexane, 87.1 mmol) was added dropwise to a solution of 15.1 g (79.2 mmol) of spiro[(1,3-benzodioxole)-2,1'-cyclohexane] in 100 mL of THF at 0°C. After 4 h, 7.40 g (75.4 mmol) of 1,2-epoxy-5-hexene (commercially available from Aldrich Chemical Co.) was added dropwise. The reaction mixture was warmed to ambient temperature and stirred for 16 h. The reaction mixture was then poured into saturated aqueous ammonium chloride solution and the mixture was extracted with 2 X 50 mL of diethyl ether. The combined organic layers were washed with 50 mL of aqueous ammonium chloride solution and 50 mL of brine, dried over anhydrous magnesium sulfate, filtered and concentrated in vacuo to an oil. The oil was purified by flash chromatography on silica gel eluted with 10% ethyl acetate in hexane to give 12.9 g (59% yield) of the intermediate unsaturated alcohol. The intermediate (6.12 g, 21.2 mmol) was dissolved in 150 mL of methanol and 0.30 g of 10% palladium on carbon was added to the methanol solution. The reaction mixture was sealed under 4 atmospheres of hydrogen ard shaken overnight. The reaction mixture was filtered and concentrated in vacuo to give 5.61 g (91 % yield) of the title compound as a colorless oil; DCI MS: 291 (M+H)⁺, 308 (M+NH4)+ ¹H NMR (CDC1₃) δ 0.9 (t, 3H), 1.24-1.95 (m, 16H), 2.67 (dd, 1H), 2.80 (dd, 1H), 3.88 (m, 1H), 6.7-6.85 (m, 3H).

### Step 2: 1-Spiro[(1,3-benzodioxole)-2,1'-cyclohexane]-2-hexanethiol

A solution of triphenylphosphine (4.32 g, 16.5 mmol) in 70 mL of dry THF was cooled to 0°C. Diisopropylazodicar- boxylate (3.33 g, 16.5 mmol) was added dropwise via syringe and the reaction mixture was stirred at 0°C for 30 min resulting in a yellow suspension. Asolution of 2.39 g (8.23 mmol) of 1-spiro[(1,3-benzodioxoie)-2,1 '-cyclohexane]-2-hexanol, from Step 1, and thiolacetic acid (1.57 g, 20.5 mmol) in 20 mL of THF was then added dropwise to the reaction mixture. The reaction mixture was stirred at 0°C for 2 h, warmed to ambient temperature and stirred for 2 h at ambient temperature, after which the reaction mixture was a homogeneous solution. The solvent was removed under reduced pressure and hexane was added to the residue. The resultant mixture was filtered and the filtrate was concentrated in vacuo. The residue was purified by-flash chromatography on silica gel eluted with 10% ethyl acetate in hexane to give 2.28 g (79% yield) of the intermediate thiolacetate as a yellow oil. The thiolacetate was dissolved in 10 mL of anhydrous diethyl ether. The resultant solution was added dropwise to a solution of lithium aluminum hydride (7.45 mmol) in 30 mL of diethyl ether. After stirring for 20 min at ambient temperature the reaction was quenched by the careful addition of 40 mL of 1 N aqueous hydrochloric acid solution. The diethyl ether layer was separated, dried over anhydrous magnesium sulfate, filtered and concentrated in vacuo to give a colorless oil. The oil was purified by flash chromatography on silica gel eluted with 1% ethyl acetate in hexane to afford 1.2 g (60% yield) of the title compound as a colorless oil; DCI MS: 307 (M+H)⁺, 324 (M+NH4)+; ¹ NMR (CDCl₃) δ 0.9 (t, 3H), 1.2-1.9 (m, 16H), 2.747 (dd, 1 H), 2.92 (dd, 1 H), 3.19 (m, 1 H), 6.6-6.75 (m, 3H).

### Step 3: [1R, 3R] 3-n-Butyl-5,6-cyclohexylidenedioxy-3,4-dihydro-1-(N-formyl)aminomethyl-1 H-2-benzothiopyran

Boron trifluoride etherate (2.40 mL, 19.6 mmol) was added dropwise to a stirred solution of 1.2 g (3.92 mmol) of 1-spiro[(1,3-benzodioxole)-2,1'-cyclohexane]-2-hexanethiol and 1,55 g (11.7 mmol) of N-formylaminoacetaldehyde dimethyl acetal in 25 mL of methylene chloride at 0°C. The reaction mixture was allowed to warm to ambient temperature and stirred at ambient temperature for 5.5 h. The reaction mixture was then diluted with 100 mL of diethyl ether and the reaction was quenched by pouring the reaction mixture into 100 mL of 10% aqueous sodium carbonate solution. The aqueous layer was extracted with 2 X 50 mL of diethyl ether. The combined organic layers were washed with 2 X 50 mL of saturated aqueous sodium bicarbonate solution, dried over anhydrous magnesium sulfate, filtered and concentrated in vacuo to a white foam. The foam was purified by flash chromatography on silica gel eluted with 5% methanol in methylene chloride to give 1.1 g (75% yield) of the title compound as a white foam which was found to be a 4:1 mixture of the cis and trans diastereomers; DCI MS: 376 (M+H)⁺, 393 (M+NH₄)⁺.

### Step 4: [1R,3R] 1-Aminomethyl-3-n-butyl-3,4-dihydro-5,6-dihydroxy-1H-2-benzothiopyran hydrochloride

[1R,3R] 3-n-Butyl-5,6-cyclohexylidenedioxy-3,4-dihydro-1-(N-formyl)aminomethyl-1H-2-benzothiopyran (1.1 g) was dissolved in 5 mL of absolute ethyl alcohol. To the ethanol solution was added 20 mL of ethyl alcohol saturated with anhydrous hydrogen chloride. The solution was heated at reflux temperature for 2 h and then cooled and concentrated to approximately 5 mL. Diethyl ether was added and a precipitate formed. The precipitate was collected by filtration, washed with diethyl ether and dried under vacuum to give 281 mg (65% yield) of the title compound as a crystalline mixture of diastereomers (8:1 cis:trans); DC MS: 268 (M+H)⁺, 285 (M+NH₄)⁺; IR (KBr) 3420, 3200, 1500, 1290 cm-1 ; 1H NMR (CDC1₃) δ 0.93 (t, 3H), 1.3-1.8 (m, 6H), 2.32 (dd, 1 H, J=15 Hz, 11.4Hz), 2.97 (m, 1 H), 3.1 (dd, 1 H, J=12.3 Hz, 9.0 Hz)), 3.21 (dd, 1 H, J=12.3 Hz, 6.9 Hz), 3.56 (dd, 1 H, J=15 Hz, 3.9 Hz), 4.13 (dd, 1 H, J=9.0 Hz, 6.9 Hz), 6.6 (d, 1 H; J=7.8 Hz), 6.7 (d, 1H; J=₇.8 Hz).

### Example 121

### [1R,3S] 5,6-Dihydroxy-3-phenyl-1-(2'R-pyrrolidinyl)-1,2,3,4-tetrahydronaphthalene hydrobromide

### Step 1:1-Cyano-5,6-dimethoxy-3-phenyl-3,4-dihydronaphthalene

To a suspension of 10 g (35 mmol) of 5,6-dimethoxy-3-phenyl-1,2,3,4-tetrahydronaphthalen-1-one, the product of Example 1, was added 7.5 g (75.6 mmol) of trimethylsilyl cyanide (commercially available from Aldrich Chemical Company) and approximately 50 mg of anhydrous aluminum chloride (AlCl₃). The reaction mixture was heated at 60°C for 3 h then cooled to ambient temperature and diluted with 150 mL of toluene. The volume of the reaction mixture was reduced in vacuo to approximately 50 mL. The resultant trimethylsilyl adduct was dehydrated by treatment with 15 mL of trifluoroacetic acid and 100 mg of p-toluenesulfonic acid in 200 mL of toluene at reflux temperature for 1 h. The reaction mixture was cooled to ambient temperature, the layers separated and the organic layer washed with water, aqueous sodium bicarbonate solution and brine, dried over anhydrous magnesium sulfate, filtered and concentrated under reduced pressure to a colorless oil. The oil was purified by column chromatography on silica gel eluted with 20% ethyl acetate in hexane to give 8.5 g (83% yield) of the title compound, m.p. 109-110°C.

### Step 2: 1-Cyano-5,6-dimethoxy-3-phenyl-1,2,3,4-tetrahydronaphthalene

Sodium borohydride (6.8 g) was added to a suspension of 6.8 g (23.3 mmol) of 1-cyano-5,6-dimethoxy-3-phenyl-3,4-dihydronaphthalene, from Step 1, in 100 mL of absolute ethanol and the reaction mixture was heated at reflux temperature for 1.5 h. The solvent was evaporated under reduced pressure and the residue was dissolved in ethyl acetate. The ethyl acetate solution was washed with 1 N aqueous hydrochloric acid solution, aqueous sodium bicarbonate solution and brine, dried over anhydrous magnesium sulfate, filtered and concentrated to an oil. The oil was triturated with heptane to give 5.63 g (82% yield) of the title compound as a white crystalline solid, m p. 118-121°C.

### Step 3: 5,6-Dimethoxy-3-phenyl-1,2,3,4-tetrahydronaphthalene-1-carboxylic acid

A mixture of 5.6 g (19.1 mmol) of 1-cyano-5,6-dimethoxy-3-phenyl-1,2,3,4-tetrahydronaphthalene; from Step 2, 40 mL of 5% aqueous potassium hydroxide solution and 90 mL of ethylene glycol was heated at reflux temperature for 8 h. The reaction mixture was then cooled to -20°C and made acidic by the addition of cold concentrated aqueous hydrochloric acid solution. The acidic solution was extracted with methylene chloride and the organic extracts were washed with brine, dried over anhydrous magnesium sulfate, filtered and evaporated to give 5 g (84% yield) of the title compound which was used in the next step without purification.

### Step 4:N-Methoxy-N-methyl-5,6-dimethoxy-3-phenyl-1,2,3,4-tetrahydro-naphthalene-1-carboxamide

5,6-Dimethoxy-3-phenyl-1,2,3,4-tetrahydronaphthalene-l-carboxylic acid (5 g, 16 mmol), from Step 3, was suspended in 100 mL of toluene and 5 mL of oxalyl chloride was added The reaction mixture was heated at reflux temperature for 1.5 h under a nitrogen atmosphere. The solvent was evaporated and excess reagents removed from the residue as an azeotrope with toluene (2X40 mL). The acid chloride and 2g (20 mmol) of N,O-dimethylhydroxylamine hydrochloride was dissolved in 80 mL of ethanol-free chloroform The solution was cooled to 0°C and 3.3 mL of pyridine was added slowly. The reaction mixture was allowed to warm to ambient temperature and stirred at ambient temperature for approximately 4 h then evaporated to dryness. The residue was dissolved in a 1:1 mixture of diethyl ether and methylene chloride and washed with brine. The layers were separated and the organic layer dried over anhydrous magnesium sulfate, filtered and concentrated under reduced pressure to give the title compound as an oil in 98% yield. The product of Step 4 was used in the next step without purification.

### Step 5: 5,6-Dimethoxy-3-phenyl-1-(2'-pyrrolidinyl)-1.2,3,4-tetrahydronaphthalene hydrochloride

N-Methoxy-N-methyl-5,6-dimethoxy-3-phenyl-1,2,3,4-tetrahydronaph-thalene-1 -carboxamide (3.3 g), from Step 4, was dissolved in 80 mL of dry THF and the solution was cooled to 0°C. An excess (3-4 equivalents) of 2,2,5,5-tetramethyl-1 -aza-2,5-disilacyclopentane-1 -propyl magnesium bromide was added and stirred overnight. The 2,2,5,5-tetramethyl-1-aza-2,5-disilacyclo-pentane-1-propyl magnesium bromide was prepared as described by Basha and DeBer- nardis in Tetrahedron Letters, 25, 5271-5274 (1984). The reaction mixture was cooled to 0°C, 10% hydrochloric acid solution in ethanol was added slowly, and it was allowed to warm to ambient temperature again. The reaction mixture was stirred at ambient temperature for 3 h and the solvent was evaporated. The residue was dissolved in 50 mL of methanol, cooled to 0°C and treated with an excess of sodium cyanoborohydride. The reaction mixture was allowed to warm to ambient temperature and stirred at ambient temperature for 2 h. The solvent was removed in vacuo and the residue was redissolved in diethyl ether and washed with water. The layers were separated and the acidic aqueous layer was made basic and extracted with methylene chloride The methylene chloride extract was dried over anhydrous magnesium sulfate, filtered and concentrated in vacuo. The residue was purified by column chromatography on silica gel eluted with ethyl acetate/formic acid/water (18: 1: 1) to give a total yield, after concentration in vacuo, of 2.42 g (42% yield) of the title compound as individual diastereomers as their formate salts. Each diastereomer was converted to its hydrochloride salt as follows: the formate salt was dissolved in water and the aqueous solution was made basic with sodium hydroxide. The free base was extracted with methylene chloride and the organic layer was washed with brine, dried over anhydrous magnesium sulfate, filtered and concentrated under reduced pressure. The residue was dissolved in diethyl ether and a saturated solution of hydrogen chloride gas in methanol was added to precipitate the hydrochloride salt. The first compound to elute from the column gave 274 mg (7% yield) of the [1R, 3S, 2'R] isomer, m.p. 105-106°C. The structure was confirmed by NMR and X-ray crystallographic analysis (after recrystallization from acetone by slow evaporation).

The final product to elute from the column gave 400 mg (11% yield) of the [1R, 3R, 2'R] isomer, m.p. 150-152°C. The structure was confirmed by NMR and X-ray crystallographic analysis after recrystallization from acetone by slow evaporation.

### Step 6:[1 R,3S] 5,6-Dihydroxy-3-phenyl-1-(2'R-pyrrolidinyl)-1.2,3,4-tetrahydronaphthalene hydrobromide

[1R, 3S] 5,6-Dimethoxy-3-phenyl-1-(2'-pyrrolidinyl)-1,2,3,4-tetrahydronaphthalene hydrochloride (200 mg, m.p. 105-106°C), from Step 5, was dissolved in 10 mL of methylene chloride and the solution was cooled to -78°C under a nitrogen atmosphere. Boron tribromide (0.25 mL of a 1 M solution in methylene chloride) was added and the reaction mixture was stirred for 3 h at -78°C. The reaction mixture was then allowed to warm to -20°C for 1 h, cooled to -78°C and quenched with 10 mL of methanol. The solution was evaporated to dryness and distilled with methanol three times to azeotrope methyl borate from the residue. The solid residue was crystallized from methanol/ethyl acetate to give 130 mg (67% yield) of the title compound, m.p. 265°C (with decomposition). Analysis calculated for C₂₀H₂₄BrNO₂+1/2H₂0: C, 60.16; H; 6.31; N, 3.51. Found: C, 60.06; H, 6.17; N, 3.42.

### Example 122

### [1 R,3R] 5,6-Dihydroxy-3-phenyl-1-(2'R-pyrrolidinyl)-1,2,3,4-tetrahydronaphthalene hydrobromide

According to the procedures described in Step 6 of Example 121, [1R,3R] 5,6-dimethoxy-3-phenyl-1-(2'-pyrrolidinyl) -1,2,3_{;}4-tetrahydronaphthalene hydrochloride (350 mg), from Step 5 of Example 121, in 10 mL of methylene chloride at -78°C, was treated with 472 µL of a 1 M solution of boron tribromide in methylene chloride. The title compound was obtained (213 mg) in 61% yield after crystallization from methanol/ethyl acetate, m.p. 250°C (with decomposition). Analysis calculated for C₂ₒH₂₄BrN0₂⁺1/2H₂0: C, 60.16; H, 6.31; N, 3.51. Found: C, 60.23; H, 6.24; N, 3.38.

### Example 123

### [1R,8S,9aR 1-Amino-5,6-dihydroxy-8-phenyl-2,3,7,8,9,9a-hexahydro-phenalene hydrobromide

### Step 1 : 1-(3'-(3'-Carbomethoxypropanoic acid)-5,6-dimethoxy-3-phenyl-3,4-dihydronaphthalene

To a suspension of 4.0 g. (14.2 mmol) of 5,6-dimethoxy-3-phenyl-1,2,3,4-tetrahydronaphthalen-1-one, the product of Example 1, in 5 mL of t-butyl alcohol was added dropwise a mixture of 13 mL (99.4 mmol) of dimethyl succinate, 9.6 g (86 mmol) of potassium t-butoxide, and 65 mL of t-butyl alcohol. After 10 mL of the mixture was added, the reaction was heated to 55°C and maintained at this temperature for the duration of the reaction. When the addition was complete, the reaction was heated for an addition 60 minutes and then cooled and poured into 50 mL of ice cold 2 N aqueous hydrochloric acid solution. The aqueous phase was extracted with 5 X 100 mL of diethyl ether The combined organic layers were extracted with 5 X 100 mL of aqueous saturated sodium bicarbonate solution. The combined aqueous layers were acidified to pH 3 with 6 N aqueous hydrochloric acid solution and the product was extracted with 2 X 200 mL of 1:1 diethyl ether/ethyl acetate. The organic phase was dried over anhydrous magnesium sulfate, filtered, and concentrated in vacuo. The title compound (5.0 g, 86% yield) was obtained as an oil; MS DCI: 397 (M+H)⁺; ¹ NMR (CDC1₃) δ 2.6-2.8 (m, 4H), 3.1-3.3 (m, 1 H), 3.69 (s, 3H), 3.71 (s, 3H), 3.87 (s, 3H), 4.1-4.25 (m, 1 H), 5.9-6.0 (m, 1H), 6.7-6.8 (m, 1 H), 7.0-7.5 (m, 6H).

### Step 2: 1-(3'-(3'-Calbomethoxypropanoic acid)-5,6-dimethoxy-3-phenyl-1,2,3,4-tetrahydronaphthalene

To a solution of 15.8 g (39.9 mmol) of 1-(3'-(3'-carbomethoxypropanoic acid)-5,6-dimethoxy-3-phenyl-3,4-dihydronaphthalene, from step 1, in 200 mL of ethyl acetate was added 3.16 g of 10% palladium supported on carbon. The reaction mixture was shaken under 4 atmospheres of hydrogen until hydrogen uptake ceased. The reaction mixture was filtered and concentrated under reduced pressure to give 12.2 g (74% yield) of the title compound as an oil. The product was carried on without further purification to the next step.

### Step 3: 1-Carbomethoxy-5,6-dimethoxy-3-hydroxy-8-phenyl-7,8,9,9a-tetrahydrophenalene

1-(3'-(3'-Carbomethoxypropanoic acid)-5,6-dimethoxy-3-phenyl-1,2,3,4-tetrahydronaphthalene (3.5 g, 8.5 mmol), from Step 2, was added to 11 g of polyphosphoric acid at 0°C. The ice bath was removed and the mixture was stirred at ambient temperature for 3 hours. The aqueous solution was extracted with 3 X 50 mL of 1:1 ethyl acetate/diethyl ether. The combined organic layers were washed with 50 mL of saturated aqueous sodium bicarbonate solution, dried over anhydrous magnesium sulfate, filtered and concentrated under reduced pressure. The product was purified by chromatography on silica gel, eluted with 20% ethyl acetate in. hexanes. Four diastereomeric products were obtained of which two were characterized.

The first diastereomer, [1R;8S,9aR]-1-carbomethoxy-5,6-dimethoxy-3-hydroxy-8-phenyl-7,8,9,9a-tetrahydrophen- alene (135-3A), was obtained in 18% yield (0.59 g) as a solid, m.p 170-172°C; DCI MS: 381 (M+H)⁺, 398 (M+NH4)+; ¹H NMR (CDC1₃) δ 1.6-1.7 (m, 1 H), 2.1-2.2 (m, 1 H), 2.6-2.7 (m, 1H); 2.9-3.1 (m, 4H), 3.2-3.4 (m, 2H), 3.71 (m, 3H), 3.87 (s, 3H), 3.91 (s, 3H), 7.2-7.4 (m, 5H), 7.53 (s, 1 H).

The second diastereomer, [1S,8S,9aR]-1-carbomethoxy-5,6-dimethoxy-3-hydroxy-8-phenyl-7,8,9,9a-tetrahy- drophenalene (135-3B) was obtained in 18% yield (0.60 g) as a solid, m.p 160-161°C; DCI MS 381 (M+H)⁺, 398 (M+NH4)+; 1H NMR (CDCI₃) δ 2.0-2.1 (m, 1 H), 2.15-2.25 (m, 1 H), 2.6-2.8 (m, 2H), 3.0-3.1 (m, 2H), 3.2-3.3 (m, 2H), 3.4-3.5 (m, 1 H), 3.7 (s, 3H), 3.83 (s, 3H), 3.91 (s, 3H), 7.2-7.4 (m, 5H), 7.54 (s, 1 H).

### Step 4: [1R,8S,9aR] 1-Carbomethoxy-5,6-dimethoxy-8-phenyl-2,3,7,8,9,9a-hexahydrophenalene

To a solution of 0.5 g (1.3 mmol) of [1R,8S,9aR]-1-carbomethoxy-5,6-dimethoxy-3-hydroxy-8-phenyl-7,8,9,9a-tet- rahydrophenalene (135-3A) in 50 mL of methanol, 50 mL of ethyl acetate, and 0.1 mL of concentrated aqueous hydrochloric acid was solution was added 0.2 g of 5% palladium supported on carbon and the mixture was shaken under 4 atmospheres of hydrogen until the gas uptake had ceased. The palladium catalyst was removed by filtration through Celite® filter aid and the filtrate concentrated to give a white solid, which was carried on to the next step without purification.

### Step 5: [1R,8S,9aR] 1-5,6-Dimethoxy-8-phenyl-2,3,7,8,9,9a-hexahydrophenalene-1-carboxylic acid

Crude [1R,8S,9aR] 1-Carbomethoxy-5,6-dimethoxy-8-phenyl-2;3,7,8,9,9a-hexahydrophenalene (0.8 g., 2.1 mmol), from Step 4, was dissolved in 100 mL of methanol and 8 mL of 1 N aqueous sodium hydroxide solution was added. After stirring for 3 days at ambient temperature, the methanol was removed under reduced pressure. The residue was partitioned between 50 mL of diethyl ether and 75 mL of water. The aqueous phase was acidified to pH 2 with 6 M aqueous hydrochloric acid solution and extracted with 3X25 mL of 1:1 ethyl acetate/diethyl ether. The combined organic layers were washed with brine, dried over anhydrous magnesium sulfate, filtered and concentrated in vacuo to give 0.74 g (100% yield) of the title compound as an oil; DCI MS: 253 (M+H)⁺. ¹H NMR (CDCl₃) 6 2.0-2.3 (m, 2H), 2.65-2.85 (m, 2H), 2.9-3.1 (m, 6H), 3.2-3.3 (m, 1 H), 3.73 (s, 3H), 3.83 (s, 3H), 6.56 (s, 1 H), 7.2-7.4 (m, 5H).

### Step 6: [1R,8S,9aR1 1-Carbobenzyloxyamino-5,6-dimethoxy-8-phenyl-2,3,7,8,9,9a-hexahydrophenalene

[1R,8S,9aR] 5,6-Dimethoxy-8-phenyl-2,3,7,8,9,9a-hexahydrophenalene-1-carboxylic acid (0.8 g 2.3 mmol), from Step 5, and triethylamine (0.32 mL, 2.3 mmol) were dissolved in 16 mL of toluene and 0.55 mL (2.5 mmol) of diphenylphosphoryl azide was added. The reaction mixture was heated at 80°C for 2.5 h then 0.5 mL (4.8 mmol) of benzyl alcohol was added and heating was continued at 80°C for an additional 3 h and at 65°C for 15 h. The reaction mixture was cooled and concentrated under reduced pressure. The residue was dissolved in 25 mL of diethyl ether and the ether solution was washed with 10 mL of 1 N aqueous sodium hydroxide solution and brine, dried over anhydrous magnesium sulfate, filtered and concentrated in vacuo. The product was purified by chromatography on silica gel eluted with 20% ethyl acetate in hexanes to give 0.4 g (39% yield) of the title compound as a white solid DCI MS: 475 (M+NH₄)⁺, 458 (M+H)⁺, 367 (M-benzyl+H)⁺, 324 (M-benzyloxy-carbonyl+2H)⁺. 1H NMR (CDCI₃) 8 1.6-1.7 (m, 3H), 2.2-2.35 (m, 2H), 2.6-2.75 (m, 2H), 2.9-3.0 (m, 3H), 3.2-3.3 (m, 1 H), 3.73 (s, 3H), 3.82 (s, 3H), 4.65-4.7 (m, 1 H), 5.08 (s, 2H), 6.54 (s, 1 H), 7.2-7.4 (m, 10H).

### Step 7: [1R,8S,9aR] 1-Amino-5,6-dimethoxy-8-phenyl-2,3,7,8,9,9a-hexahydrophenalene

A suspension of 0.65 g (1.4 mmol) of [1R,8S,9aR] 1-carbobenzyloxy-amino-5,6-dimethoxy-8-phenyl-2,3,7,8,9,9a-hexahydrophenalene, from Step 6, in 50 mL of methanol and 0.1 g of 10% palladium supported on carbon was stirred under 1 atmosphere of hydrogen for 1 hour. The solid dissolved as the reaction proceeded. The catalyst was removed by filtration and the solution was concentrated under reduced pressure to give 0,4 g ( 87% yield) of crude title compound which was carried on to the next step without further purification.

### Step 8: [1R,8S,9aR] 1-Amino-5,6-dihydroxy-8-phenyl-2,3,7,8,9,9a-hexahydrophenalene hydrobromide

To a solution of 0.4 g (1.2 mmol) of [1R,8S,9aR] 1-amino-5,6-dimethoxy-8-phenyl-2,3,7,8,9,9a-hexahydrophenalene in 9 mL of methylene chloride at -78°C, was added dropwise 4.4 mL (4.4 mmol) of a 1 M solution of boron tribromide in methylene chloride . The reaction mixture was warmed to ambient temperature for 1 hour and cooled to -78°C and the reaction was quenched by the addition of 5 mL of methanol. The reaction was allowed to warm to ambient temperature and stirred for 1 hour. The solvent was removed in vacuo. Methanol (5ml) was added and the solution was concentrated to remove methyl borate by azeotropic distillation. The title compound was obtained (0.32 g, 69% yield) after recrystallization from ethanol/diethyl ether as a white solid; DCI MS: 279 (M+H)⁺, 296 (M-NH4)⁺. 1 H NMR (d₆-DMSO) δ 1.4-1.6 (m, 1H), 1.7-1.9 (m, 1H); 2.1-2.2 (m, 1H), 2.2-2.3 (m, 1H), 2.4-2.5 (m, 2H), 2.7-3.2 (m, 5H), 6.41 (s, 1H); 7.2-7.4 (m, 5H), 8.0 (br s, 5H). Analysis calculated for then stirred an additional 10 minutes at 0°C. A solution of the above crude oil in 60 mL of THF was added dropwise over 30 minutes and the reaction was then allowed to warm to ambient temperature for 4 h. The solvents were removed in vacuo and the residue was dissolved in a mixture of 250 mL each of methylene chloride and water The organic phase was collected and washed with saturated aqueous sodium chloride, dried over anhydrous sodium sulfate, filtered, and concentrated under reduced pressure. The product was recrystallized from ethyl acetate/hexanes to give 33.15 g (83% yield from the ketone) of 5,6-dimethoxy-3-phenyl-2-thiophenyl-1_{;}2,3,4-tetrahydro-naphthalen-1 -one as a white solid; MS DCI: 391 (M+H)⁺; 1 NMR (CDCI₃) 6 3.35 (dd, 1 H, J=6,18 8 Hz), 3.55 (dd, 1H, J=6,₁8 Hz), 3.71 (q, 1H, J=6 Hz), 3.82 (s, 3H), 3.93 (s, 3H), 4.19 (d, 1H, J=6 Hz), 6.91 (d, 1H, J=9 Hz), 7.1-7.3 (m, 8H), 7.4-7.5 (m, 2H), 7.37 (d,1H, J-9 Hz).

### Step 2: -5,6-Dimethoxy-3-phenyl-2-sulfoxophenyl-3,4-dihydronaphthalene

A solution of 20.96 g (53.7 mmol) of 5;6-dimethoxy-3-phenyl-2-thiophenyl-1;2,3,4-tetrahydronaphthalen-1-one in 320 mL of ethanol was treated with 20.03 g. (0.529 mol) of sodium borohydride. The reaction was heated to reflux temperature for 2 h, then cooled and 500 mL of water was added. The solvents were removed in vacuo and the residue was taken up in 500 mL of 1: diethyl ether/methylene chloride and 500 mL of water. The organic layer was removed and washed with 100 mL each of water and brine, dried over magnesium sulfate, filtered, and concentrated under reduced pressure. The crude resultant alcohol was dehydrated by the addition of 700 mL of toluene and 3.6 g (18.9 mmol) of p-toluenesulfonic acid monohydrate and heating to reflux with azeotropic removal of water for 30 minutes. After cooling, the solution was washed with 3 X 100 mL of saturated aqueous sodium bicarbonate 100 mL of water, dried over anhydrous magnesium sulfate, filtered and concentrated in vacuo. The crude thio-enolether was carried on directly by first, dissolution in 360 mL of methylene chloride. This solution was cooled to -15°C and a solution of 12.1 g of 3-chloroperoxybenzoic acid (mCPBA) in 160 mL of methylene chloride was added dropwise over 30 minutes. After the addition was complete, the reaction was quenched by the addition of 100 mL of aqueous saturated sodium thiosulfate. The organic layer was separated, and C₁₉H₂₂BrNO₂+0.5 H₂0: C, 59.23; H; 5.99; N, 3.64. Found: C, 59.26; H, 5.86; N, 3.59.

### Example 124

### [1S,8S,9aR] 1-Amino-5,6-dihydroxy-8-phenyl-2,3,7,8,9,9a-hexahydrophenalene hydrobromide

[1S,8S,9aR] 1-Amino-5,6-dimethoxy-8-phenyl-2,3,7,8,9,9a-hexahydrophenalene hydrobromide was prepared from the second isomeric product of Step 3 of Example 123 (123-3B) according to the procedures described in Steps 4 through 8 of Example 123; DCI MS 279 (M+H)⁺, 296 (M-NH₄)⁺, ¹H NMR (d₆-DMSO) 8 1.6-1.75 (m, 1H), 1.8-2.05 (m, 2H), 2.25-2.7 (m, 5H), 2.85-3.05 (m, 3H), 6.37 (s, 1H), 7.1-7.4 (m, 5H), 7.7 (br s, 5H). Analysis calculated for C₁₉H₂₂BrN0₂+1 H₂0: C 57.88; H, 6.14; N, 3.55. Found: C, 57.82; H, 5.74; N; 3.56.

### Example 125

### 6,7-Dihydroxy-4-phenyl-2,3,4,5-tetrahydro-1 H-benz[e]isoindole formic acid salt

### Step 1: 5,6-Dimethoxy-3-phenyi-2-thiophenyi-1,2,3,4-tetrahydronaphthaien-l -one

To a solution of 28.9 g (0.102 mol) of 5,6-dimethoxy-3-phenyl-1,2,3_{;}4-tetrahydronaphthalen-1-one, the product of Example 1, in 240 mL of THF was added 40.4 g (0.107 mol) of phenyltrimethylammonium tribromide. After stirring at ambient temperature for 1 h 960 mL of water was added. The solution was extracted with 3 X 250 mL of ethyl acetate. The combined organic phase was washed with 3 X 250 mL of water, 250 mL of saturated aqueous sodium chloride, dried over anhydrous magnesium sulfate, filtered, and concentrated under reduced pressure to give an oil which was carried on without further purification or characterization.

A solution of sodium methoxide was prepared by the addition of 3.28 g (0.143 mol) of sodium metal to 97 mL of methanol with cooling to 0°C. Thiophenol (14.6 mL, 0.143 mol) was added dropwise over 10 minutes and washed with 3 X 100 mL of saturated aqueous sodium bicarbonate, 100 mL of water, dried over anhydrous sodium sulfate, filtered and concentrated under reduced pressure. The product was chromatographed on silica gel eluted with 25% ethyl acetate in hexanes to give 18.65 (89% yield) of 5,6-dimethoxy-3-phenyl-2-sulfoxophenyl-3,4-dihydronaphthalene as a white solid as a mixture of diastereomers; MS DCI: 391 (M+H)+; ¹H 1H NMR (CDCI₃) 8 2.9-3,1 (m, 1H), 3.1-3.3 (m, 1 H), 3.46 and 3.51 (2 x s, 3H total), 3.55 and 3.7 (2 x m, 1 H total), 3.83 and 3.86 (2 x s,3H total), 6.75-7.15 (m, 7H), 7.3-7.6 (m, 6H).

### Step 3: N-Trimethylsilylmethyl benzylamine

A mixture of 264 mL (2.42 mol) of benzylamine and 97.7 g. (0.796 mol) of chloromethyltrimethylsilane was heated to 200°C for 2.5 h then cooled to 10°C. A 0.1 M sodium hydroxide solution (400 mL) was added and the product was extracted with 3 X 200 mL of diethyl ether. The combined organic phase was washed with 100 mL of water, dried over anhydrous magnesium sulfate, filtered and concentrated under reduced pressure. The product was distilled at 115-125°C and 5 mm of Hg to give 125.4 g (81 % yield) of N-trimethylsilylmethyl benzylamine as a clear liquid; 1H NMR (CDCI₃) δ 0.0 (s, 9H), 1 1 (br s,1 H), 2.01 (s, 2H), 3.76 (s, 2H), 7.1-7.3 (m, 5H).

### Step 4: N-Methoxymethyl-N-trimethylsilylmethyl benzylamine

N-Trimethylsilylmethyl benzylamine (125.4 g, 0.649 mol), from Step 3, was added dropwise over a 10 minute period to a solution of 69.5 mL of 37% aqueous formaldehyde at 0°C. After an additional 10 minutes, 75.2 mL of methanol was added. The solution was then saturated with solid potassium carbonate and stirred at 0°C for 1 h. The layers were separated and the organic phase was stirred over solid potassium carbonate at ambient temperature for 18 h. The solution was filtered and fractionally distilled at 20 mm of Hg to give a 145-155°C, fraction as a viscous oil, identified as N-methoxymethyl-N-trimethylsilylmethyl benzylamine. ¹H NMR (CDCl₃) 8 0.0 (s, 9H), 2.13 (s, 2H), 3.18 (s, 3H), 3.71 (s, 2H), 3.96 (s, 2H), 7.1-7.3 (m, 5H).

### Step 5: 2-Benzyl-6,7-dimethoxy-4-phenyl-2,3,4,5-tetrahydro-1 H-benz[e]lisoindole

To a solution of 1.22 g (3.13 mmol) of 5,6-dimethoxy-3-phenyl-2-sulfoxophenyl-3,4-dihydronaphthalene in 10 mL of methylene chloride was added 1 g (4.21 mmol) of N-methoxymethyl-N-trimethylsilylmethyl benzylamine, from Step 4, and 0.1 mL of trifluoroacetic acid. At 12 h intervals, the amine and acid additions were repeated 7 more times. The solvent was then removed under reduced pressure with heating to 100°C and the product was chromatographed on silica gel, eluted with 25% ethyl acetate in hexanes to give 0.14 g (11% yield) of 2-benzyl-6,7-dimethoxy-4-phenyl-2,3,4,5-tetrahydrobenzo[e]isoindole; MS DCI: 398 (M+H)⁺; ¹H NMR (CDCl₃) δ 3.0-3.15 (m, 1H)_{;} 3.25-3.35 (m, 1 H, 3.45-3.55 (m, 3H), 3.62 (s, 3H), 3.65-3.7 (m, 2H), 3.8-3.9 (m, 2H), 3.82 (s, 3H), 6.68 (m, 1 H), 7.1-7.4 (m, 11 H).

### Step 6: 6,7-Dimethoxy-4-phenyl-2,3,4,5-tetrahydro-1 H-benz[e]isoindole hydrochloride

To a solution of 1.0 g (2.52 mmol) of 2-benzyl-6,7-dimethoxy-4-phenyl-2,3,4,5-tetrahydro-1 H-benz[e]isoindole, from Step 5, in 22 mL of 1,2-dichloroethane was added 0.11 g (0.05 mmol) of 1_{;}8-bis(dimethylamino)-naphthalene and 0.33 mL (3.15 mmol) of 1-chloroethyl chloroformate at 0°C. The solution was heated to reflux for 2 h and the solvent removed in vacuo. The residue was filtered through silica gel eluted with 25% ethyl acetate in hexanes. After concentration under reduced pressure, methanol (20 mL) was added and the solution was heated to reflux for 30 minutes, before the solvent was removed in vacuo. The product was crystallized from ethanol/ether to give 0.46 g. (75% yield) of 6,7-dimethoxy-4-phenyl-2,3,4,5-tetrahydro-1 H-benz[e] isoindole hydrochloride as a white solid.; MS DCI: 308 (M+H)⁺; ¹ H NMR (d₆-DMSO) 8 3.05-3.25 (m, 2H), 3.55 (s, 3H), 3.80 (s, 3H), 3.88 (m, 1 H), 4.0-4.15 (m, 2H), 4.25-4.45 (m, 2H), 6.91 (m,1 H), 7.15-7.3 (m, 3H), 7.4-7.6 (m, 3H).

### Step 7: 6,7-Dihydroxy-4-phenyl-2,3,4,5-tetrahydro-1 H-benz[e]isoindole formic acid salt

A suspension of 54.5 mg (0.159 mmol) of 6,7-dimethoxy-4-phenyl-2,3,4,5-tetrahydro-1 H-benz[e]isoindole hydrochloride, from Step 6, in 2 mL of methylene chloride was cooled to -78°C and 0.64 mL of a 1 M solution of boron tribromide in methylene chloride was added. The reaction was warmed to ambient. temperature for 1 h and cooled to -78°C before 1 mL of methanol was added. After warming to ambient temperature for 1 h, the solvents were removed in vacuo. Additional methanol (5 mL) was added and removed in vacuo. The product was chromatographed on silica gel eluted with ethyl acetate/formic acid/ water (18:1:1) to give 29.8 mg (58% yield) of the title compound as an off-white powder, m.p. 144°C; MS DCI: 279 (M+H)+; 1 H NMR (d₆-DMSO) δ 2.95-3.15 (m, 2H), 3.6-3.9 (m, 3H), 4.1-4.3 (m, 2H), 6.43 (d, 1 H, J=7.5 Hz), 6.62 (d, 1 H, J=7.5 Hz), 7.1-7.3 (m, 5H), 8.3 (s, 1 H).

### Example 126

### [1 R, 3S] 3-(1'-Adamantyl)-5,6-cyclohexylidenedioxy-3,4-dihydro-1 -[N-formyl₎aminomethy!-1 H-2-benzopyran (Method A)

A mixture of 185 mg (0.5 mmol) of 1-(1'-adamantyl)-2-(spiro-[(1;3-benzodioxole)-2,1'-cyclohexane])-1-ethanol, 110 mg (0.83 mmol) N-formyl-acetaldehyde dimethyl acetal and 46 mg (0.13 mmol) of zinc triflate in 3.0 mL of dry 1,2-dichloroethane was heated at reflux under a nitrogen atmosphere for 18 h. The 1-(1'-adamantyl)-2-(spiro-[(1,3-benzodioxole) -2,1'-cyclohexane])-1-ethanol was preapred from 1-(1-adamantyl)-ethylene oxide by the procedures described in Step 2 of Example 74. The reaction mixture was allowed to cool to ambient temperature and the reaction was quenched with∿30 mL ethyl acetate and saturated aqueous sodium bicarbonate solution The organic layer was washed with water and brine, dried over anhydrous magnesium sulfate, filtered and concentrated to an oil. The oil was triturated with methanol to give 191 mg (87% yield) of the title compound as a solid, m.p. 182-184°C.

### Example 127

### [1 R, 3S] 3-(1'-Adamantyl)-5,6-cvclohexylidenedioxt-3,4-dihydro-1 -N-formyl)aminomethyi-1 H-2-benzopyran

### (Method B)

To a mixture of 94 mg (0.26 mmol) of 1-(1'-adamantyl)-2-(spiro-[(1,3-benzodioxole)-2,1'-cyclohexane])-1-ethanol and 100 mg (0.75 mmol) of N-formylacetaldehyde dimethyl acetal in 2.0 mL of dry acetonitrile was added 3 µL ∿0.1 equivalent) of methanesulfonic acid and the reaction mixture was heated at reflux for 12 h. The mixture was cooled to 0°C. The solid was removed by filtration, washed with cold acetonitrile and dried to give 79 mg (70% yield) of the title compound as a beige solid, m.p. 185-187°C.

### Example 128

### [1 R, 3S] 3-(1'-Adamantyl)-5,6-cyclohexylidenedioxy-3,4-dihydro-1-(N-formyl)aminomethyl-1 H-2-benzopyran

### (Method C)

To a mixture of 50 mg (0.14 mmol) of 1-(1'-adamantyl)-2-(spiro-[(1,3-benzodioxole)-2,1'-cyclohexane])-1-ethanol and 60 mg (0.45 mmol) of N-formylacetaldehyde dimethyl acetal was added a solution of 3 mg of polyphosphoric acid in ∿0.5 mL of acetonitrile. The reaction mixture was heated at reflux for 48 h and then was cooled to 0°C for 0.5 h and filtered. The solid was washed with cold acetonitrile and dried to give 10.9 mg (18% yield) of the title compound as an off-white solid, m.p. 185-187°C.

### Example 129

### [1 R, 3S] 3-(1'-Adamantyl)-5,6-cyclohexylidenedioxy-3,4-dihydro-1-(N-formyl)aminomethyl-1 H-2-benzopyran

### (Method D)

To a mixture of 181 mg (0.5 mmol) of 1-(1'-adamantyl)-2-(spiro-[(1,3-benzodioxole)-2,1'-cyclohexane])-1-ethanol and 107 mg (0.83 mmol) of N-formylacetaldehyde dimethyl acetal was added 25 µL of a 1.0 M a solution of trimethylsilyl triflate in methylene chloride. The reaction mixture was heated at reflux for 4 h and then was cooled to 0°C for 0.5 h and filtered through a sintered glass funnel. The solid was washed with cold acetonitrile and dried to give 198 mg (92% yield) of the title compound as a white solid, m.p. 187-189°C.

### Competitive Binding

### D-1 and D-2 Receptor Binding Assays

Homogenized rat caudate was incubated in the presence of [¹²⁵]SCH-23982 (a selective antagonist of the dopamine D-1 receptor) and the compounds of this invention, according to procedures described by A. Sidhu, et al. in European J Pharmacoloqy, 113: 437 (1985) and in European J Pharmacology, 128: 213 (1986). The compounds compete with the radiolabeled ligand for occupancy of the receptors and the molar potency of each compound was quantified. The affinity of the compound for the receptor (Ki) was calculated as descnbed by YC. Cheng and W.H. Prusoff in Biochemical Pharmacoloqy,22: 3099 (1973) from the relationship Ki = IC₅₀(1+[L]/Kp) where IC_{SO} is the concentration of test compound which produces a 50% inhibition in the specific binding of the radioligand, L; [L] is the concentration of radioligand; and KD is the affinity of the radioligand for the receptor.

The procedure for the dopamine D-2 receptor binding assay was similar to that used for the D-1 receptor assay. Homogenized rat caudate was the source of the D-2 receptors. The tissue homogenate was incubated in the presence of [125]-p-aminophenylethyl spiroperidol (a selective antagonist of the dopamine D-2 receptor) and the compounds being evaluated, according to the protocol described by T Agui, N. Amlaiky, M.G. Caron and J.W. Kebabian in Molecular Pharmacoloqy, 33: 163 (1988). The molar affinity of the compound for the receptor binding site was calculated by the same method used for the D-1 receptor assay, assuming a competitive interaction between the compound and the radiolabeled ligand.

The competitive binding data (Ki values) from the D-1 and D-2 receptor binding assays are shown in Table 9. The Ki values are inversely proportional to the affinity of the compound for the receptor.

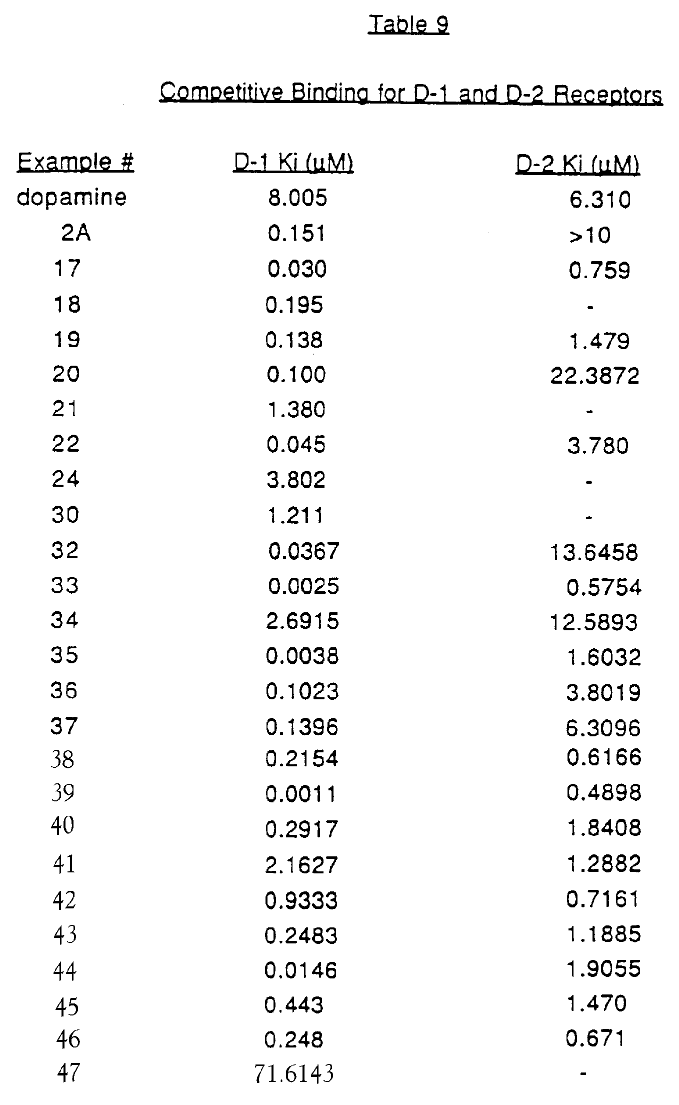

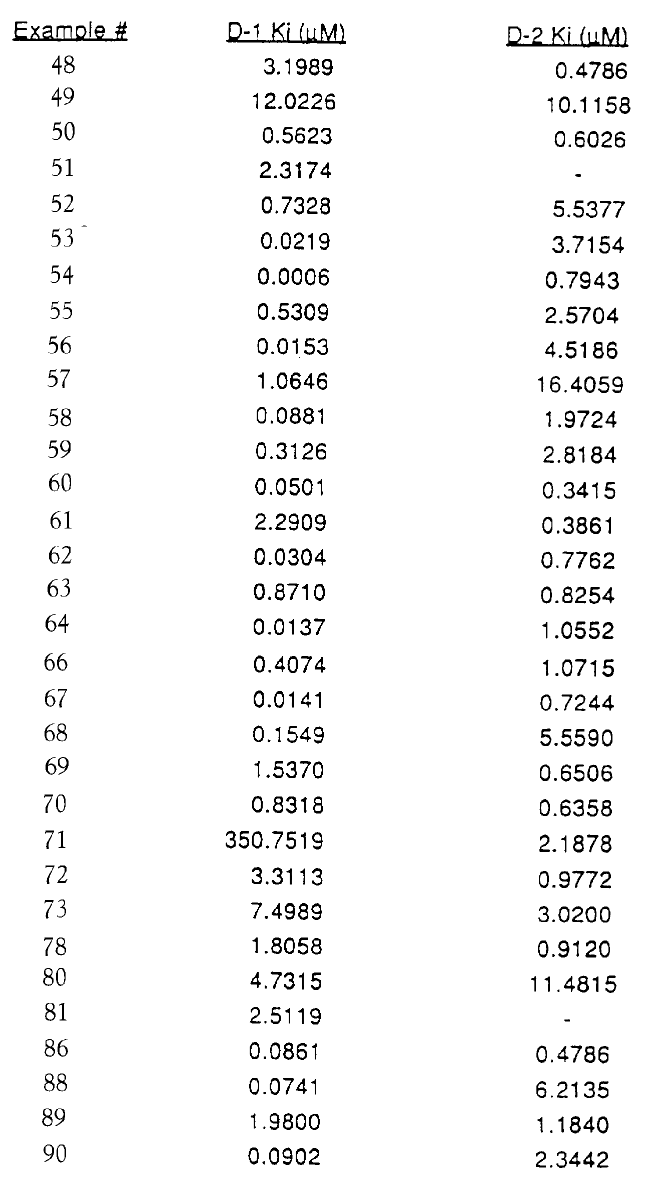

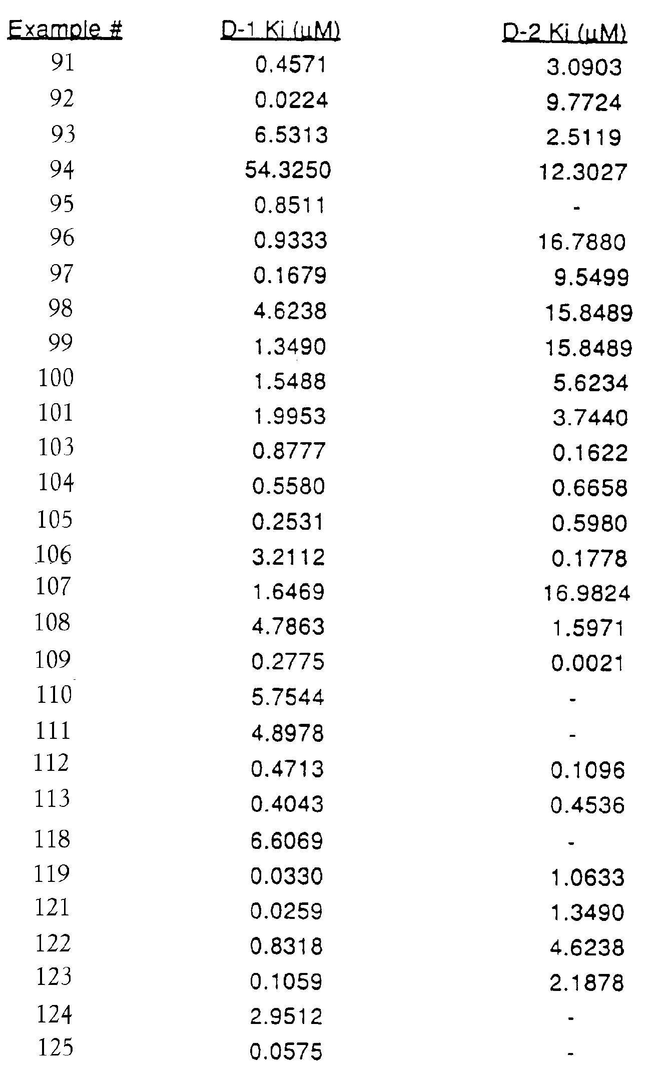

### Intrinsic Activity

The interaction of dopamine or a dopamine D-1 receptor agonist with the D-1 receptor causes a dose-dependent increase in the adenylate cyclase-catalyzed conversion of adenosine triphosphate (ATP) to cyclic adenosine monophosphate (cAMP). The functional activity of the compounds of the invention was determined by assaying, in vitro, their ability to either stimulate the enzyme adenylate cyclase to produce more CAMP (agonist activity) or to antagonize a dopamine-induced increase in cAMP levels. The protocol for the adenylate cyclase assays was described by K.J. Watling and J.E.Dowling in J Neurochemistry, 36: 559 (1981) and by J W. Kebabian, et al. in Proc Natl Acad Sci. USA, 69: 2145 (1972). In order to determine agonist activity, cell-free tissue homogenates are incubated in an ionic buffer solution containing ATP and the compound being evaluated. The tissue was obtained from either goldfish retina or rat striatum.

Table 10 shows the intrinsic activity in an adenylate cyclase assay indicating that the compounds of the present invention are dopamine agonists

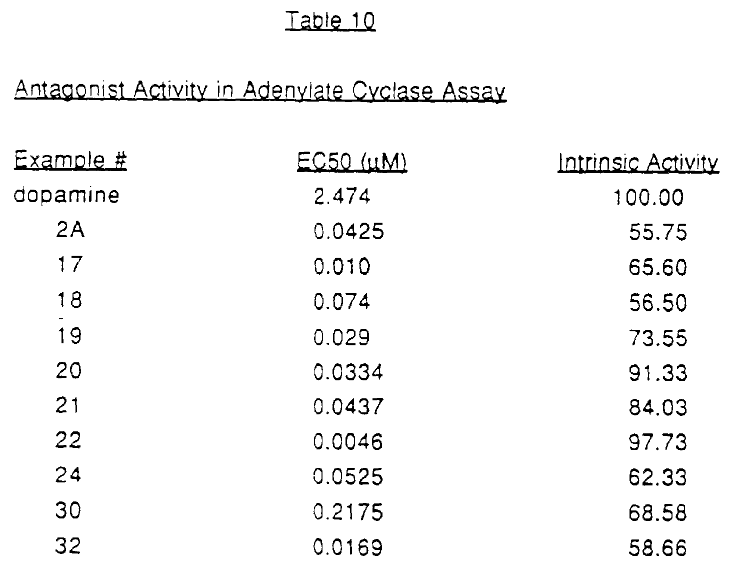

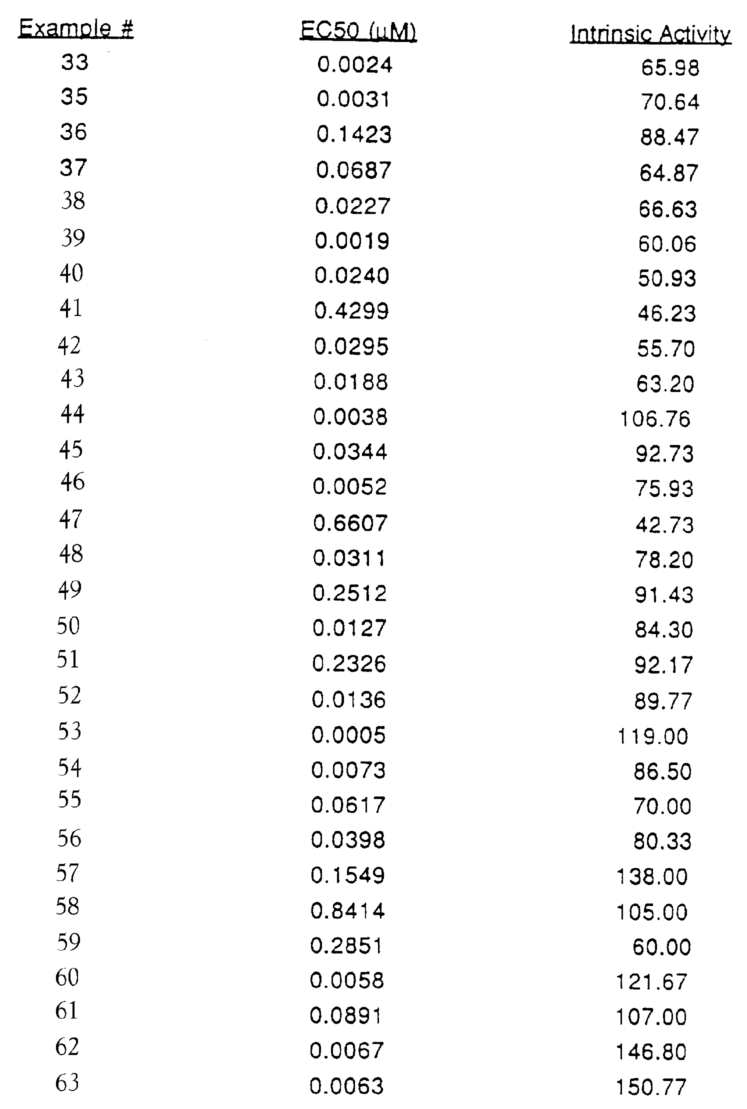

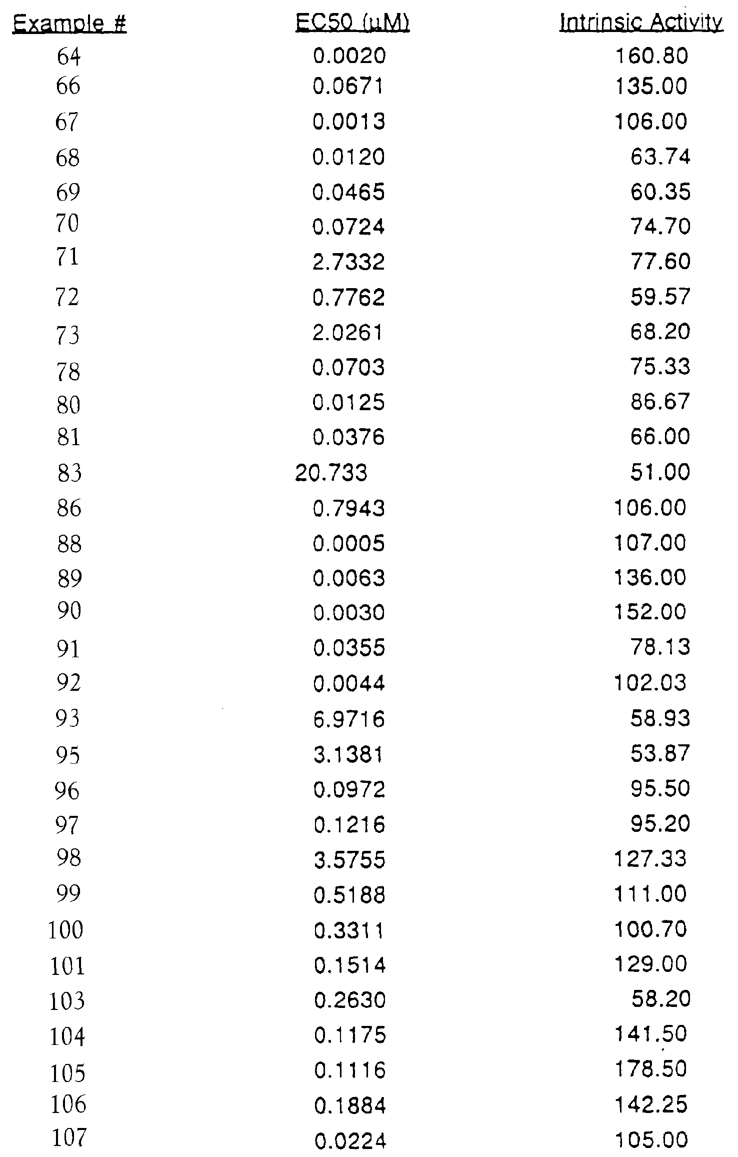

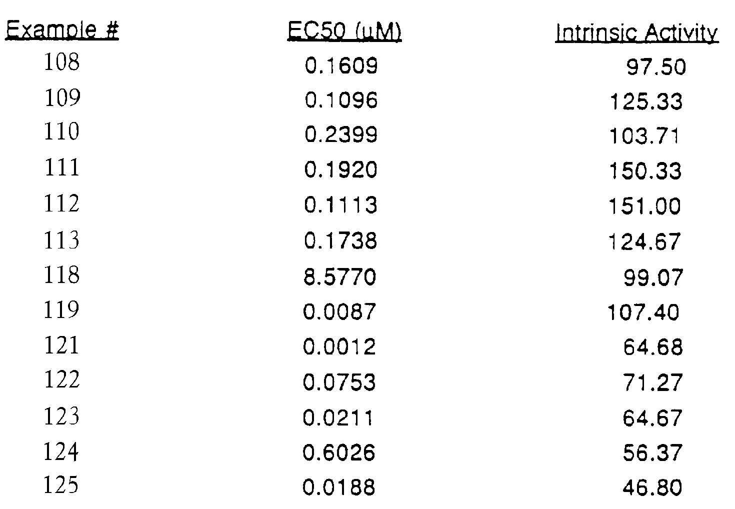

### Rotation Behavior

The behavioral assay used was based on the rat rotational model. Striatal dopamine was depleted by the intracranial injection of 6-hydroxydopamine, a neurotoxin which specifically destroys catecholaminergic neurons. The intracranial injection was conducted on anesthetized animals using standard stereotaxic techniques (U. Ungerstedt and G.W. Ar- buthnott, Brain Research, 24: 485, 1970 and U. Ungerstedt, Acta Physiol. Scand. Suppl. 367 69: 1973). This unilateral lesioning of dopamine-containing neurons causes the post synaptic dopamine receptors to become supersensitive to dopaminergic stimulation in behavioral assays. When these striatal dopamine receptors are stimulated by the test compounds, the rats rotate or physically turn, in a direction that is away from the side of their body that receives the greater dopaminergic activation due to the receptor supersensitivity. Agonist activity was measured by the ability of the test compound to induce rotation.

Tables 11 and 12 show the rotation behavior of selected compounds of the present invention.

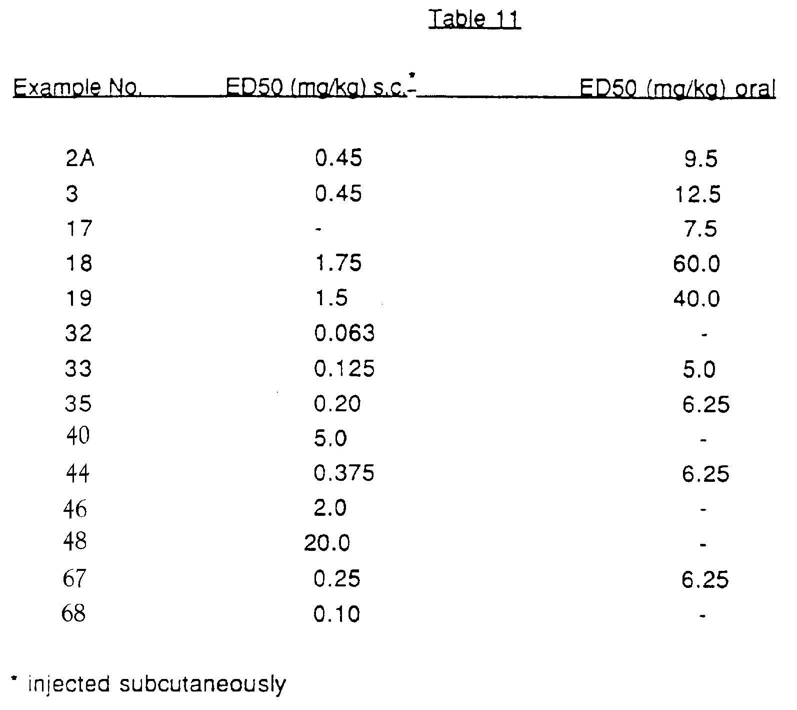

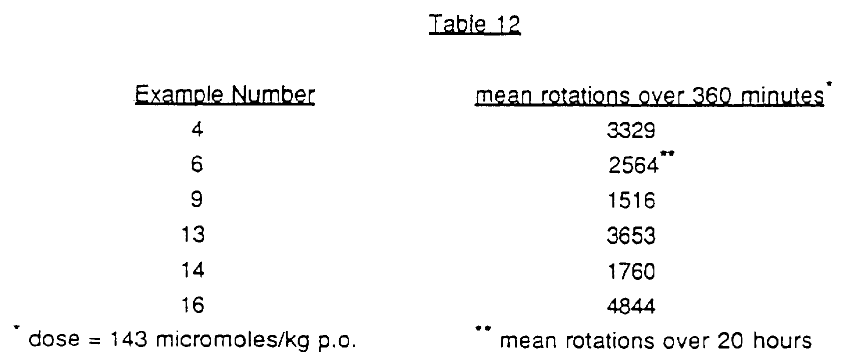

### CARDIOVASCULAR PHARMACOLOGY

### Hemodynamic Studies in Anesthetized Doqs:

Male Beagle dogs were anesthetized with pentobarbital (30 mg/kg. i.v.) and maintained with i.v. infusion (Ab- bott/Shaw Life Care Pump, Model II/D) to maintain stable cardiovascular function. The dogs were incubated with a cuffed endotracheal tube and ventilated with room air by means of a positive pressure respiratory pump. Expired respiratory C0₂ was monitored with a Beckman LB-2 gas analyzer and maintained at 5% by appropriate pump adjustments. The dogs were maintained at a body temperature of 37.5 _ 1.0°C with a thermostatically controlled animal table. Polyethylene catheters were placed in the abdominal aorta via the femoral and carotid arteries for blood pressure and left ventricular pressure recordings. A Swan-Ganz thermodilution catheter with a 15 cm proximal port was placed in the jugular vein for central venous and pulmonary arterial recordings and for determination of cardiac output (American Edwards Cardiac Output Computer, Model COM-1). Heart rate and electrocardiogram (ECG) recordings were made from a Lead 11 ECG connection. With the dog on its right side the abdominal cavity was surgically entered laterally, immediate inferior to the rib cage, to expose the left renal artery. A calibrated electromagnetic flow probe (Carolina Medical Electronics) was positioned around the renal artery. The abdominal cavity is closed with wound clips. Recordings were made on a Grass polygraph.

An additional small polyethylene catheter was insened into a branch of the left femoral artery and the tip positioned in the aorta above the renal arteries. Compounds were continuously infused intrarterially (Harvard Infusion Pump, Model 975) for approximately 5 minutes per dose. A thirty-fold dose-response-curve was administered by varying flow rate from 0.01 to 0.30 mL/minute.

Table 13 shows the effects of selected compounds of the present invention on cardiovascular pharmacology.

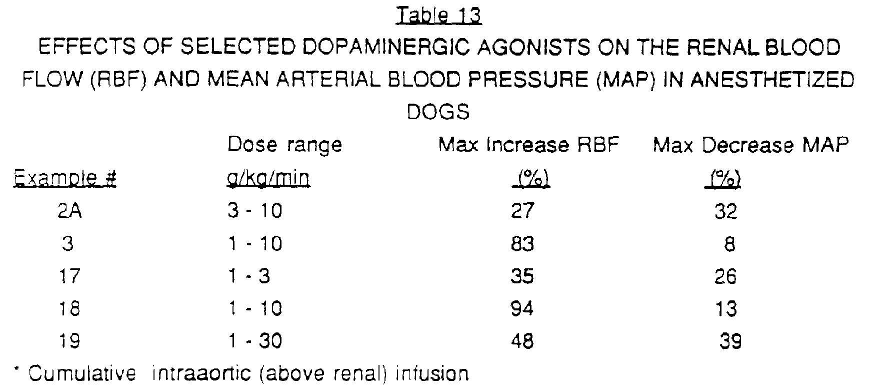

### DIURETIC EFFECTS OF EXAMPLE 2A IN SPONTANEOUSLY HYPERTENSIVE RATS

Male, spontaneously hypertensive rats (SHR), weighing 285-350 grams were used. Following an overnight fasting period with free access to drinking water, the rats received an intragastric fluid load of 0.9 saline at 5% of their body weight Simultaneously with the load, the rats were dosed with a test compound or vehicle and placed individually in stainless steel metabolic cages where they had access to drinking water throughout the duration of the experiment. For intravenous administration, the rats were instrumented with indwelling cannulas placed into the jugular vein at least one week prior to the experiment.

Urine was collected at 2 and 4 hours following drug administration. The volume of excreted urine at each collection interval was measured accurately and the samples were analyzed for sodium, potassium and chloride ions. Sodium and potassium were measured using a Digital Readout Flame Photometer (Instrumentation Labs). Chloride was measured by the method of Shales and Shales,JBiol Chem, Q:879 (1941). The statistical analysis of the data was computed by an off-line computer program. In this program, a companson test is made between the vehicle (control) group and each treatment group for all variables at each time interval of the experiment. The test of statistical significance is based on the Student's t-test, where the calculated t is a measure of the probability density function.

The compound of Example 2A was administered to six rats intravenously at a dose of 0.3, 1.0 and 3.0 mg/kg. A control group of six rats received 0.1 mg/kg of saline solution acidified by ascorbic acid (0.3 mL). This solution was also the vehicle for the test compound.

Table 14 shows the diuretic and saliuretic effects of the compound of Example ₂A.

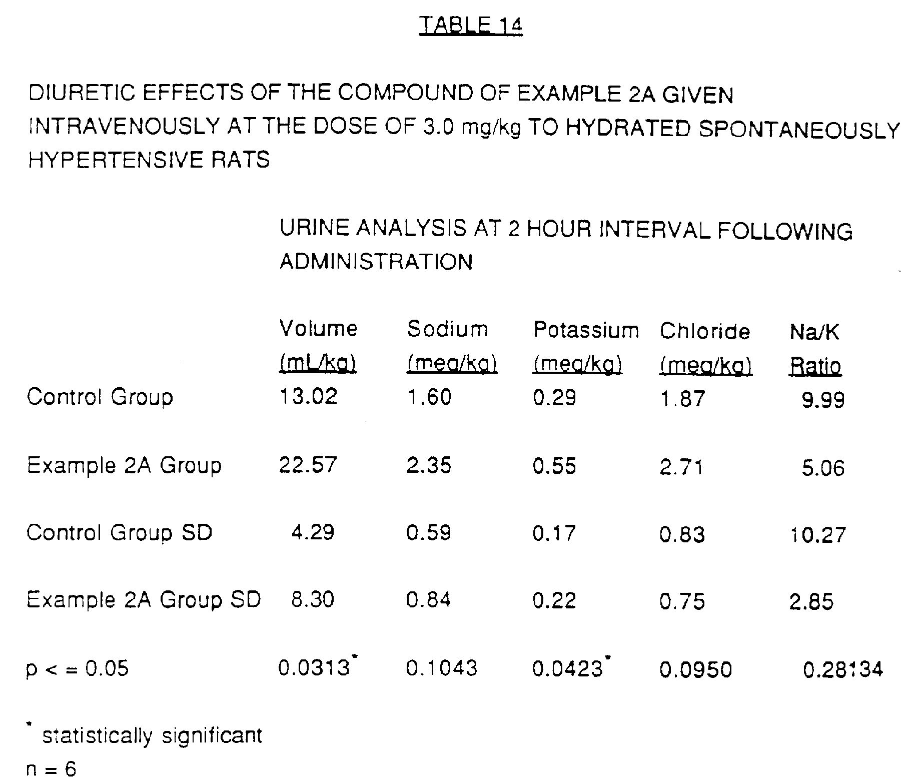

## Claims (Claims for the following Contracting State(s) : AT, BE, CH, LI, DE, DK, FR, GB, IT, LU, NL, SE)

1. A compound having the formula: 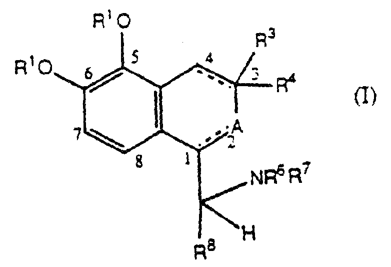 or a pharmaceutically acceptable salt, ester or amide thereof, wherein A is O, S, CHR², CR² or C when A and R⁶ taken together form a nitrogen-containing 5-, 6- or 7-membered ring;
the dotted lines represent optional double bonds;
R¹ is selected from hydrogen and a group which is readily cleavable in vivo;
R² is selected from hydrogen, C₁-C₁₂ alkyl and substituted C₁-C₆ alkyl;
R³ is selected from C₁-C₁₂ alkyl, substituted C₁₋C₆ alkyl as defined below, C₂-C₁₂ alkenyl, substituted C₂-C₁₂ alkenyl as defined below, C₂-C₁₂ alkynyl, substituted C₂-C₁₂ alkynyl as defined below, C₃-C₁₂ cycloalkyl, substituted C₃-C₁₂ cycloalkyl as defined below, carbocyclic C₅-C₁₀ aryl, substituted carbocyclic C₅-C₁₀ aryl as defined below, heterocycle as defined below, and substituted heterocycle as defined below,
R⁴ is selected from hydrogen and C₁-C12 alkyl or, taken together with R³ and the carbon atom to which they are attached, forms a spirocycloalkyl ring of from 3 to 7 carbons;
R⁶ is selected from hydrogen, C₁-C2 alkyl, substituted C₁-C₆ alkyl as defined below, C₂-C₁₂ alkenyl, substituted C₂-C₁₂ alkenyl as defined below, C₂-C₁₂ alkynyl, substituted C₂-C₁₂ alkynyl as defined below, C₃-C₁₂ cycloalkyl, substituted C₃-C₁₂ cycloalkyl as defined below, alkanoyl of from 1 to 8 carbons, naturally occurring amino acid and dipeptide formed from naturally occurring amino acids or, taken together with A when A is C, forms a nitrogen-containing 5-, 6- or 7-membered ring;
R⁷ is hydrogen or C₁ -C₁₂ alkyl or, taken together with R⁶ or R⁸_{;} forms a nitrogen-containing 5-, 6-or 7-membered ring, provided that when R⁶ is carbocyclic arylalkyl, R⁷ is not alkyl;
R⁸ is hydrogen or C₁ -C₁₂ alkylor, taken together with R⁶ or R⁷; forms a nitrogen-containing 5-, 6- or 7-membered ring or taken together with the catechol ring at the 8-position and the carbon atoms to which they are attached forms a 5-, 6- or 7-membered ring;
wherein substituted C₁-C₆ alkyl is a C₁-C₆ alkyl group bearing 1-2 substituents selected from halogen, hydroxy, C₁-C₆ alkoxy, amino, C₁-C₆ alkylamino, C₃-C₁₂ cycloalkyl, heterocycle as defined below, substituted heterocycle as defined below; carbocyclic C₅-C₁₀ aryl, substituted carbocyclic C₅-C₁₀ aryl as defined below, carbocyclic C₅-Cₗₒ aryloxy, substituted carbocyclic C₅-C₁ aryloxy wherein substituted carbocyclic C₅-C₁ aryl is as defined below, or -NC(O)R¹¹ wherein R¹¹ is selected from hydrogen, C₁-C₆ alkyl, halo-(C₁-C₆ alkyl), heterocycle as defined below, substituted heterocycle as defined below, carbocyclic C₅-C₁₀ aryl, and substituted carbocyclic C₅-C₁₀ aryl as defined below;
wherein substituted C₂-C₁₂ alkenyl is a C₂-C₁₂ alkenyl group bearing a substituent selected from C₁-C₆ alkyl, C₃-C₁₂ cycloalkyl, carbocyclic C₅-C₁₀ aryl, benzyl, phenylethyl, amino, hydroxy, C₁-C₆ alkoxy and heterocycle as defined below,
wherein substituted C₂-C₁₂ alkynyl is a C₂-C12 alkynyl bearing a substituent selected from C₁-C₆ alkyl, C₃-C₁₂ cycloalkyl, carbocyclic C₅-C₁₀ aryl, benzyl, phenylethyl amino, hydroxy, C₁-C₁₂ alkylamino, C₁-C₆ alkoxy, and heterocycle as defined below;
wherein substituted C₃-C₁₂ cycloalkyl is a C₃-Cₗ₂ cycloalkyl group substituted by one or more C₁-Cₗ₂ alkyl, hydroxy, amino, C₁-C₁₂ alkylamino, Cᵢ-Cᵢ₂ aminoalkyl, mercapto, C₁-C12 alkylthio or halogen groups,
wherein substituted carbocyclic C₅-C₁₀. aryl is a carbocyclic C₅-C₁₀ aryl substituted by one or more hydroxy, halogen, C₁-C₆ alkyl or C₁-C₆ alkoxy groups or by a phenyl group;
wherein heterocycle is a monocyclic 5- or 6-membered saturated or unsaturated group containing one or two ring heteroatoms selected from N O and S, the remaining atoms being carbon, such as, for example, furyl, tetrahydrofuryl, thienyl, pyrryl, pyrrolidyl, pyridyl, piperidyl, pyrazinyl, pyrimidyl, piperazinyl, morpholinyl, thiomorpholinyl, pyrazolyl, imidazolyl, oxazolyl, oxazolidyl, isoxazolyl, isoxazolidyl and thiazolyl; and
wherein substituted heterocycle is a heterocycle as defined above substituted by halogen, C₁-C₆ alkyl or C₁-C₆ alkoxy.

2. A compound according to claim 1 in which A is CR² and the dotted line between A and ring atom number 1 represents a bond, having the formula: 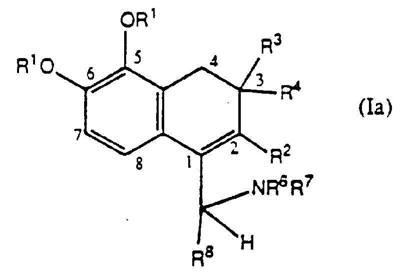 wherein R¹, R², R³, R⁴, R⁶, R⁷ and R⁸ are as defined in claim 1.

3. A compound according to claim 1 in which A is CHR² having the formula: 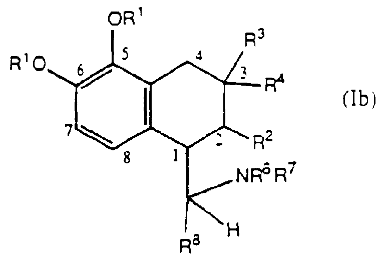 wherein R¹, R², R³, R⁴, R⁶, R⁷ and R⁸ are as defined in claim 1.

4. A compound according to claim 1 in which A is O.

5. A compound according to claim 1 in which n is 0, and R⁶ and A taken together form a nitrogen-containing 5-, 6- or 7-membered ring having the formula: 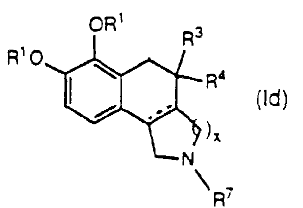 wherein x is 1, 2 or 3 and R¹, R³, R⁴ and R⁷ are as defined in claim 1.

6. A compound according to claim 1 in which A is CHR², R² is H and R⁸ taken together with position 8 of the catechol ring form a 5-, 6- or 7-membered ring, having the formula: 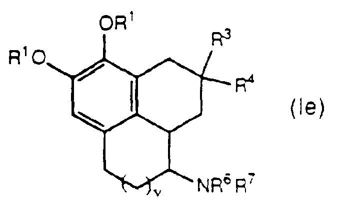 wherein y is 0,1 or 2, R¹ and R³, R⁴_{;} R⁶ and R⁷ are as defined in claim 1.

7. A compound according to claim 1 in which R³ is C₁-C₁₂ alkyl, substituted C₁-C₆ alkyl, C₃-C₁₂ cycloalkyl, substituted C₃-C₁₂ cycloalkyl, carbocyclic C5-C₁₀ aryl or substituted carbocyclic C₅-C₁₀ aryl and R⁴ is hydrogen.

8. A compound according to claim 1 in which R₇ and R₈ are hydrogen.

9. A compound according to claim 1 selected from the group consisting of:
1-Aminomethyl-5,6-dihydroxy-3-phenyl-3,4.dihydronaphthalene;
1-Aminomethyl-5,6-bis(acetoxy)-3-phenyl-3,4-dihydronaphthalene;
1-Aminomethyl-5,6-bis(trimethylacetoxy)-3-phenyl-3,4-dihydronaphthalene;
[1R,3S] 1-Aminomethyl-5,6-dihydroxy-3-phenyl-1,2,3,4-tetrahydronaphthalene;
1-Aminomethyl-3-cyclohexyl-5,6-dihydroxy-3,4-dihydronaphthalene;
[1R,3S] 1 -Aminomethyl-3-cyclohexyl-5,6-dihydroxy-1,2,3,4-tetrahydronaphtha!ene:
[1R,3S] 1-Aminomethyl-3-t-butyl-3,4-dihydro-5,6-dihydroxy-1 H-2-benzopyran;
[1R,3S] 1 -Aminomethyl-3,4-dihydro-5,6-d!hydroxy-3-pheny!-1 H-2-benzopyran;
[1R,3S] 1-Aminomethyl-3-cyclohexyl-3,4-dihydro-5,6-dihydroxy-1 H-2-benzopyran;
[1R,3R] 1-Aminomethyl-3,4-dihydro-5,6-dihydroxy-3-ethyl-1 H-2-benzopyran;
Spiro[(1-aminomethyl-3,4-dihydro-5,6-dihydroxy-1 H-2-benzopyran)-3,1'-cyclohexane];
[1R,3S] 1 -Aminomethy!-3,4-d!hydro-5,6-d!hydroxy-3-(4'-methoxyphenoxy)methyl-1 H-2-benzopyran;
[1R*,3S*] 1-Aminomethyl-3,4-dihydro-5,6-dihydroxy-3-phenyl-1 H-2-benzopyran;
[1R,3S] 1-Aminomethyl-3,4-dihydro-5,6-dihydroxy-3-phenoxymethyl-1 H-2-benzopyran;
[1R,3S] 1 -Aminomethyl-3,4-dihydro-5,6-dihydroxy-3-(2'-phenylphenoxy)methyl-1 H-2-benzopyran;
[1R,3S] 1-Aminomethyl-3-(4'-t-butylphenoxy)methyl-3,4-dihydro-5,6-dihydroxy-1 H-2-benzopyran;
[1R,3S] 1-Aminomethyl-3-(4'-bromophenoxy)methyl-3,4-dihydro-5,6-dihydroxy-1 H-2- benzopyran;
[1R,3R] 1-Aminomethyl-3-benzyl-3,4-dihydro-5,6-dihydroxy-1 H-2-benzopyran;
[1R,3R] 1-Aminomethyl-3,4-dihydro-5,6-dihydroxy-3-(2'phenyl)ethyl-1 H-2-benzopyran;
[1R,3S] 1 -Amlnomethyl-8-bromo-3,4-d!hydro-5,6-d!hydroxy-3-pheny!-1 H-2-benzopyran;
[1R,3R] 1 -Amonomethyh-3,4-d!hydro-5,6-dihydroxy-3-n-octyl-1 H-2-benzopyran:
[1R,3R] 1 -Aminomethyl-3,4-dihydro-5,6-dihydroxy-3-(1'-hex-5'-ene)-1 H-2-benzopyran;
[1R,3S] 1-Aminomethyl-3,4-dihydro-5,6-dihydroxy-3-ethyl-1 H-2-benzopyran;
[1R,3R] 1-Aminomethyl-3,4-dihydro-5,6-dihydroxy-3-n-hexyl-1 H-2-benzopyran;
[1R,3S] 1-Aminomethyl-3-(4'-bromophenyl)-3,4-dihydro-5,6-dihydroxy-1 H-2-benzopyran;
[1R,3S] 1-Aminomethyl-3,4-dihydro-5,6-dihydroxy-3-(3'-hydroxyphenyl)-1 H-2-benzopyran;
[1R,3S] 3-Cyclohexyl-3,4-dihydro-5,6-dihydroxy-1-(N-methyl)aminomethyl-1 H-2- benzopyran;
[1R,3S] 3-t-Butyl-3,4-dihydro-5,6-dihydroxy-1-(N-methyl)aminomethyl-1 H-2-benzopyran;
[1R,3S] 1-(N-Allyl)aminomethyl-3-cyclohexyl-3,4-dihydro-5,6-dihydroxy-1 H-2-benzopyran;
[1R,3] 3-Cyclohexyl-1-(N-cyclopropyl)aminomethyl-3,4-dihydro-5,6-dihydroxy-1 H-2- benzopyran;
[1R,3S] 1 -(N.benzyl)aminomethyl-3-cyclohexyl-3,4-dihydro-5,6-dihydroxy-1 H-2-benzopyran;
[1R,3S] 1,3-Bis(aminomethyl)-3,4-dihydro-5,6-dihydroxy-1 H-2-benzopyran;
[1R,3S] 1-Aminomethyl-3,4-dihydro-5,6-dihydroxy-3-hydroxymethyl-1 H-2-benzopyran;
[1R,3S] 1-Aminomethyl-3,4-dihydro-5,6-dihydroxy-3-(N-piperidinyl)methyl-1 H-2- benzopyran;
[1R,3S] 5,6-Dihydroxy-3-phenyl-1 -(2'R-pyrrolidunyl)-1,2,3,4-tetrahydronaphthalene;
[1R,3R] 5,6-Dihydroxy-3-pheny!-1 -(2'R-pyrrolidinyl)-1,2,3,4-tetrahydronaphtha!ene:
5;6-Dihydroxy-1-(N-methyl)aminomethyl-3-phenyl-3,4-dihydronaphthalene;
[1R,3S] 5,6-Dihydroxy-1-(N-methy!)aminomethy!-3-pheny!-1,2,3,4-tetrahydronaphthalene;
1-Aminomethyl-5,6-dihydroxy-3-(3'-hydroxyphenyl)-3,4-dihydronaphthalene;
1-Aminomethyl-5,6-dihydroxy-3-(4'-hydroxyphenyl)-3,4-dihydronaphthalene;
[1R,3S] 1-Aminomethyl-5,6-dihydroxy-3-(3'-hydroxyphenyl)-1,2,3,4-tetrahydronaphthalene;
5;6-Bis(acetoxy)-1-(alanyl-alanyl)aminomethyl-3-phenyl-3,4-dihydronaphthalene;
5;6-Bis(acetoxy)-1-(γ-glutamyl)aminomethyl-3-phenyl-3;4-dihydronaphthalene;
5;6-Bis(acetoxy)-1-(alanyl)aminomethyl-3-phenyl-3,4-dihydronaphthalene;
5;6-Bis(acetoxty)-1-(methionyl)aminomethyl-3-phenyl-3,4-dihydronaphthalene;
1-(Alanyl-alanyl)aminomethyl-5,6-bis(benzoyloxy)-3-phenyl-3,4-dihydronaphthalene;
[1 R,2S] 1-Aminomethyl-5,6-dihydroxy-2-(2'-hydroxy-1'-ethyl)-3-phenyl-1,2,3,4-tetrahydronaphthalene; 1-Aminomethyl-5,6-dihydroxy-3-phenylnaphthalene;
[1R,3S] 1-Aminomethyl-3,4-dihydro-5,6-dihydroxy-3-(diphenyl)methyl-1 H-2-benzopyran;
[1R,3S] 1 -Aminomethyl-3,4-dihydro-5,6-dihydroxy-3-(3'-methyl-2'-n-pentyl)-1 H-2-benzopyran;
[1R,3S] 1 -Aminomethyl-3,4-dihydro-5,6-dihydroxy-3-(1 '-but-3'-ene)-1 H-2-benzopyran;
[1R,3S] 1-Aminomethyl-3,4-dihydro-5,6-dihydroxy-3-(6'-methyl-2'-hep-5'-ene)-1 H-2- benzopyran;
[1 R,3S] 1 -Aminomethyl-3-benzyloxymethyl-3,4-dihydro-5,6-dihydroxy-1 H-2-benzopyran;
[1R, 8S, 9aR] 1-Amino-5,6-dihydroxy-2,3,7,8,9,9a-hexahydro-8-phenyl-phenalene;
[1S, 8S, 9aR] 1-Amino-5,6-dihydroxy-2,3,7,8,9,9a-hexahydro-8-phenyl-phenalene;
6;7-Dihydroxy-4-phenyl-2,3,4,5-tetrahydro-1 H-benz[e]isoindole; and
1-Aminomethyl-5,6-bis(benzoyloxy)-3-phonyl-3,4-dihydronaphthalene, or a pharmaceutically acceptable salt thereof.

10. A compound selected from the group consisting of: [1R,3S] 3-(1'-Adamantyl)-1 -aminomethyl-3,4-dihydro-5,6-dihydroxy-1 H-2-benzopyran:
[1 R^{*},3S^{*}] 3-(1 '-Adamantyl)-1 -aminomethyl-3,4-dihydro-5,6-dihydroxy-1 H-2-benzopyran;
[1 R,3S] 3-(1'-Adamantyl)-3,4-dihydro-5,6-dihydroxy-1-(N-methyl)aminomethyl-1 H-2- benzopyran;
[1R,3R] 1-Aminomethyl-3-cyclopentylmethyl-3;4-dihydro-5,6-dihydroxy-1 H-2-benzopyran;
[1R,3S] 1-Aminomethyl-3-cyclooctyl-3,4-dihydro-5,6-dihydroxy-1H-2-benzopyran;
[1R,3R] 1-Aminomethyl-3-n-butyl-3,4-dihydro-5,6-dihydroxy-1H-2-benzopyran;
[1R,3R] 3-n-Butyl-3,4-dihydro-5,6-dihydroxy-1-(N-methyl)aminomethyl-1H-2-benzopyran,
or a pharmaceutically acceptable salt thereof.

11. A pharmaceutical composition for selectively acting on dopaminergic receptors comprising a pharmaceutically acceptable carrier and a therapeutically effective amount of a compound of claim 1.

12. A pharmaceutical composition for treating dopamine-related neurological disorders comprising a pharmaceutically acceptable carrier and a therapeutically effective amount of a compound of claim 1.

13. A pharmaceutical comDosition for treating dopamine-related psychological disorders comprising a pharmaceutically acceptable carrier and a therapeutically effective amount of a compound of claim 1.

14. A pharmaceutical composition for treating dopamine-related cardiovascular disorders comprising a pharmaceutically acceptable carrier and a therapeutically effective amount of a compound of claim 1.

15. A pharmaceutical composition for treating substance abuse and addictive behavior disorders comprising a pharmaceutically acceptable carrier and a therapeutically effective amount of a compound of claim 1.

16. A pharmaceutical composition for treating cognitive and attention disorders comprising a pharmaceutically acceptable carrier and a therapeutically effective amount of a compound of claim 1.

17. A compound according to claim 1 for use as a therapeutic agent.

18. Use of a compound of Claim 1 for manufacturing a medicament for selectively acting on dopaminergic receptors in a patient in need of such treatment

19. Use of a compound of Claim 1 for manufacturing a medicament for treating dopamine-related neurological disorders characterized by abnormal dopaminergic activity in a patient in need of such treatment

20. Use of a compound of Claim 1 for manufacturing a medicament for treating dopamine-related psychological disorders characterized by abnormal dopaminergic activity in a patient in need of such treatment.

21. Use of a compound of Claim 1 for manufacturing a medicament for treating dopamine-related cardiovascular disorders in a patient in need of such treatment.

22. Use of a compound of Claim 1 for manufacturing a medicament for treating addictive behavior disorders in a patient in need of such treatment.

23. Use of a compound of Claim 1 for manufacturing a medicament for treating cognitive and attention disorders in a patient in need of such treatment.

24. A compound of the formula: 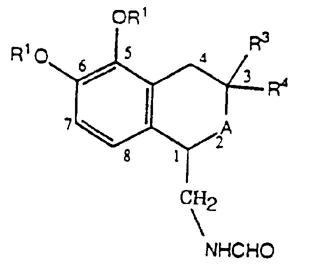
wherein A is O or S;
R¹ is a catechol protecting group;
R³ is selected from C₁-C₁₂ alkyl, substituted C₁-C₆ alkyl as defined below, C₂-C₁₂ alkenyl, substituted C₂-C₁₂ alkenyl as defined below, C₂-C₁₂ alkynyl, substituted C₂-Cᵢ₂ alkynyl as defined below, C₃-C₁₂ cycloalkyl, substituted C₃-C₁₂ cycloalkyl as defined below, carbocyclic C₅-Cₗₒ aryl, substituted carbocyclic C5-C₁₀ aryl as defined below, heterocycle as defined below, and substituted heterocycle as defined below,
R⁴ is selected from hydrogen and C₁-C₁₂ alkyl or, taken together with R³ and the carbon atom to which they are attached, forms a spirocycloalkyl ring of from 3 to 7 carbons;
wherein substituted C₁-C₆ alkyl is a C₁ -C₆ alkyl group bearing 1-2 substituents selected from halogen, hydroxy, C₁-C₆ alkoxy, amino, C₁-C₆ alkylamino, C₃-C₁₂ cycloalkyl, heterocycle as defined below, substituted heterocycle as defined below carbocyclic C₅-C₀ aryl, substituted carbocyclic C₅-C₁₀ aryl as defined below, carbocyclic C₅-C₁₀ aryloxy, substituted carbocyclic C₅-C₁₀ aryloxy wherein substituted carbocyclic C₅-C₁₀ aryl is as defined below, or -NC(O)R¹¹ wherein R¹¹ is selected from hydrogen, C₁-C₆ alkyl, halo-(C₁-C₆ alkyl), heterocycle as defined below, substituted heterocycle as defined below, carbocyclic C₅-C₁₀ aryl, and substituted carbocyclic C₅-C₁₀ aryl as defined below;
wherein substituted C₂-C₁₂ alkenyl is a C₂-C₁₂ alkenyl group bearing a substituent selected from C₁-C₆ alkyl, C₃-C₁₂ cycloalkyl, carbocyclic C5-C₁₀ aryl, benzyl, phenylethyl, amino, hydroxy, C₁-C₆ alkoxy and heterocycle as defined below,
wherein substituted C₂-C₁₂ alkynyl is a C₂-C₁₂ alkynyl bearing a substituent selected from C₁-C₆ alkyl, C₃-C₁₂ cycloalkyl, carbocyclic C₅-C₁₀ aryl, benzyl, phenylethyl, amino, hydroxy C₁-C₁₂ alkylamino, C₁-C6 alkoxy, and heterocycle as defined below;
wherein substituted C₃-C₁₂ cycloalkyl is a C₃-C₁₂ cycloalkyl group substituted by one or more C₁-C₁₂ alkyl, hydroxy amino, C₁-C₁₂ alkylamino, C₁-C₁₂ aminoalkyl, mercapto, C₁-C₁₂ alkylthio or halogen groups,
wherein substituted carbocyclic C₅-C₁₀ aryl is a carbocyclic C5-C10 aryl substituted by one or more hydroxy, halogen, C₁-C₆ alkyl or C₁-C₆ alkoxy groups or by a phenyl group;
wherein heterocycle is a monocyclic 5- or 6-membered saturated or unsaturated group containing one or two ring heteroatoms selected from N O and S, the remaining atoms being carbon, such as, for example, furyl, tetrahydrofuryl, thienyl, pyrryl, pyrrolidyl, pyridyl, piperidyl, pyrazinyl, pyrimidyl, piperazinyl, morpholinyl, thiomorpholinyl, pyrazolyl, imidazolyl, oxazolyl, oxazolidyl, isoxazolyl, isoxazolidyl and thiazolyl; and
wherein substituted heterocycle is a heterocycle as defined above substituted by halogen, C₁-C₆ alkyl or C₁-C₆ alkoxy.

25. A process for preparing a compound of claim 24 comprising: reacting a compound having the Formula: 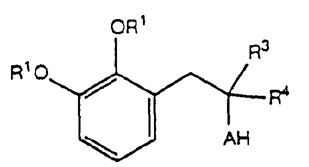
wherein R¹, R³ and R⁴ are as defined therein,
with N-formylaminoacetaldehyde dimethyl acetal or with 3-(N-formylamino)-propionaldehyde dimethyl acetal in the presence of an acid catalyst.

26. A process according to claim 24 wherein the catalyst is selected from boron trifluoride etherate, zinc triflate, trimethylsilyl triflate and methanesulfonic acid.

## Claims (Claims for the following Contracting State(s) : ES)

1. A process for preparing a compound having the formula: 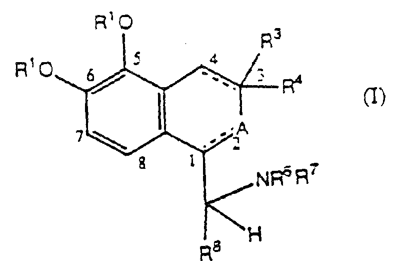 or a pharmaceutically acceptable salt, ester or amide thereof, wherein A is O, S, CHR², CR² or C when A and R⁶ taken together form a nitrogen-containing 5-, 6- or 7-membered ring;
the dotted lines represent optional double bonds;
R¹ is selected from hydrogen and a group which is readily cleavable in vivo;
R² is selected from hydrogen, C₁-C₁₂ alkyl and substituted C₁-C₆ alkyl;
R³ is selected from C₁-C₁₂ alkyl, substituted C₁-C₆ alkyl as defined below, C₂-Cᵢ₂ alkenyl, substituted C₂-C₁₂ alkenyl as defined below, C₂-C₁₂ alkynyl, substituted C₂-C₁₂ alkynyl as defined below, C₃-C₁₂ cycloalkyl, substituted C₃-C₁₂ cycloalkyl as defined below, carbocyclic C₅-Cₗₒ aryl, substituted carbocyclic C₅-C₁₀ aryl as defined below, heterocycle as defined below, and substituted heterocycle as defined below,
R⁴ is selected from hydrogen and C₁-C₁₂ alkyl or, taken together with R³ and the carbon atom to which they are attached, forms a spirocycloalkyl ring of from 3 to 7 carbons;
R⁶ is selected from hydrogen, C₁-C₁₂ alkyl, substituted C₁-C₆ alkyl as defined below, C₂-C₁₂ alkenyl, substituted C₂-C₁₂ alkenyl as defined below, C₂-C₁₂ alkynyl, substituted C₂-C₁₂ alkynyl as defined below, C₃-C₁₂ cycloalkyl, substituted C₃-C12 cycloalkyl as defined below, alkanoyl of from 1 to 8 carbons, naturally occurring amino acid and dipeptide formed from naturally occurring amino acids or, taken together with A when A is C, forms a nitrogen-containing 5-, 6- or 7-membered ring;
R⁷ is hydrogen or C₁-C₁₂ alkyl or, taken together with R⁶ or R⁸, forms a nitrogen-containing 5-, 6-_{or 7-mem}bered ring, provided that when R⁶ is carbocyclic arylalkyl, R⁷ is not alkyl;
R⁸ is hydrogen or Cₗ -C₁₂ alkyl or, taken together with R⁶ or R⁷, forms a nitrogen-containing 5-, 6- or 7-membered ring or taken together with the catechol ring at the 8-position and the carbon atoms to which they are attached forms a 5-, 6- or 7-membered ring;
wherein substituted C₁-C₆ alkyl is a C₁-C₆ alkyl group bearing 1-2 substituents selected from halogen, hydroxy, C₁-C₆ alkoxy, amino, C₁-C₆ alkylamino, C₃-C₁₂ cycloalkyl, heterocycle as defined below, substituted heterocycle as defined below carbocyclic C₅-C₁₀ aryl, substituted carbocyclic C₅-C10 aryl as defined below, carbocyclic C₅-C₁₀ aryloxy, substituted carbocyclic C₅-C₁₀ aryloxy wherein substituted carbocyclic C₅-C₁₀ aryl is as defined below, or -NC(O)R¹¹ wherein R¹¹ is selected from hydrogen, C₁-C₆ alkyl, halo-(C₁-C₆ alkyl), heterocycle as defined below, substituted heterocycle as defined below, carbocyclic C₅-C₁₀ aryl, and substituted carbocyclic C₅-C₁₀ aryl as defined below;
wherein substituted C₂-C₁₂ alkenyl is a C2-C₁₂ alkenyl group bearing a substituent selected from C₁-C₆ alkyl, C₃-C₁₂ cycloalkyl, carbocyclic C₅-C₁₀ aryl, benzyl, phenylethyl, amino, hydroxy, C₁-C₆ alkoxy and heterocycle as defined below,
wherein substituted C₂-C₁₂ alkynyl is a C₂-C₁₂ alkynyl bearing a substituent selected from C₁-C₆ alkyl, C₃-C₁₂ cycloalkyl, carbocyclic C₅-C₁₀ aryl, benzyl, phenylethyl, amino, hydroxy C₁-C₁₂ alkylamino, C₁-C₆ alkoxy, and heterocycle as defined below;
wherein substituted C₃-C₁₂ cycloalkyl is a C₃-C₁₂ cycloalkyl group substituted by one or more C₁-C₁₂ alkyl, hydroxy amino, C₁-C₁₂ alkylamino, C₁-C₁₂ aminoalkyl, mercapto, C₁-C₁₂ alkylthio or halogen groups,
wherein substituted carbocyclic C₅-C₁₀ aryl is a carbocyclic C₅-Cₗₒ aryl substituted by one or more hydroxy, halogen, C₁-C₆ alkyl or C₁-C₆ alkoxy groups or by a phenyl group;
wherein heterocycle is a monocyclic 5- or 6-membered saturated or unsaturated group containing one or two ring heteroatoms selected from N O and S, the remaining atoms being carbon, such as, for example, furyl, tetrahydrofuryl, thienyl, pyrryl, pyrrolidyl, pyridyl, piperidyl, pyrazinyl, pyrimidyl, piperazinyl, morpholinyl, thiomorpholinyl, pyrazolyl, imidazolyl, oxazolyl, oxazolidyl, isoxazolyl, isoxazolidyl and thiazolyl; and
wherein substituted heterocycle is a heterocycle as defined above substituted by halogen, C₁-C₆ alkyl or C₁-C₆ alkoxy, comprising the steps of:
(a) reacting a suitably-protected catechol starting material of the formula 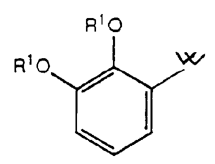 wherein R₁ is selected from hydrogen and a group which is readily clarable in vivo; and W is selected from the group comprising H, Li or a ring forming group, with an appropriately-substituted condensing molecule and a condensing reagent under proper condensing conditions to give a first intermediate compound;
(b) converting the first intermediate compound into a suitably-protected, appropriately-substituted desired second intermediate compound;
(c) cyclizing the second intermediate compound to give a suitably-protected, appropriately-substituted third intermediate compound;
(d) adding the desired amine-containing grouping by one or more derivatizing reactions to prepare a suitably-protected final intermediate compound; and
(e) deprotecting the final intermediate compound with the appropriate deprotecting reagent and isolating the desired product.

2. A process according to claim 1, wherein said ring forming group is selected from the group comprising:

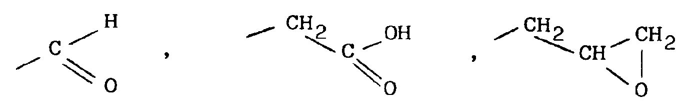

3. A process according to claims 1-2 in which A is CR² and the dotted line between Aand ring atom number 1 represents a bond, having the formula: 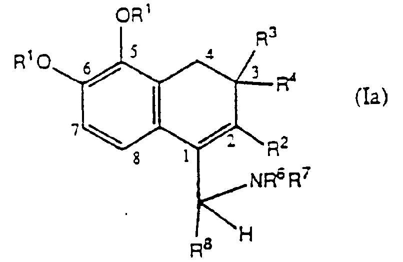 wherein R¹, R², R³, R⁴, R⁶, R⁷ and R⁸ are as defined in claim 1.

4. A process according to claims 1-2 in which A is CHR² having the formula: 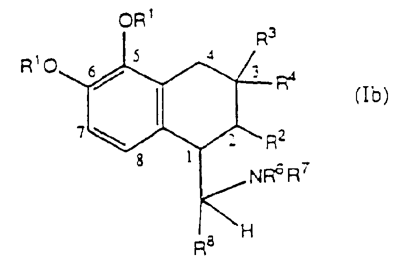 wherein R¹, R², R³, R⁴, R⁶, R⁷ and R⁸ are as defined in claim 1.

5. A process according to claims 1-2 in which A is O.

6. A process according to claims 1-2 in which n is 0, and R⁶ and A taken together form a nitrogen-containing 5-,6- or 7-membered rina havina the formula: 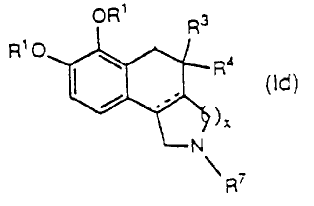 wherein x is 1, 2 or 3 and R¹, R³, R⁴ and R⁷ are as defined in claim 1.

7. A process according to claims 1-2 in which A is CHR², R² is H and R⁸ taken together with position 8 of the catechol ring form a 5-, 6- or 7-membered ring, having the formula: 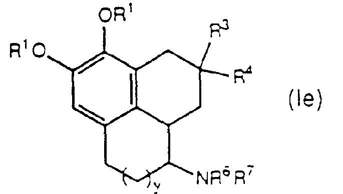 wherein y is 0, 1 or 2, R¹ and R³, R⁴, R⁶ and R⁷ are as defined in claim 1.

8. A process according to claims 1-2 in which R³ is C₁-C₁₂ alkyl, substituted C₁-C₆ alkyl, C₃-C₁₂ cycloalkyl, substituted C₃-C₁₂ cycloalkyl, carbocyclic C₅-C₁₀ aryl or substituted carbocyclic C₅-C₁₀ aryl and R⁴ is hydrogen.

9. A process according to claims 1-2 in which R₇ and R₈ are hydrogen.

10. A process according to claims 1-2 for preparing a compound selected from the group consisting of:
1-Aminomethyl-5,6-dihydroxy-3-phenyl-3,4-dihydronaphthalene;
1-Aminomethyl-5,6-bis(acetoxy)-3-phenyl-3,4-dihydronaphthalene;
1-Aminomethyl-5,6-bis(trimethylacetoxy)-3-phenyl-3,4-dihydronaphthalene;
[1R,3S] 1-Aminomethyl-5,6-dihydroxy-3-phenyl-1,2,3,4-tetrahydronaphthalene;
1-Aminomethyl-3-cyclohexyl-5,6-dihydroxy-3,4-dihydronaphthalene;
[1R,3S] 1-Aminomethyl-3-cyclohexyl-5,6-dihydroxy-1,2,3,4-tetrahydronaphthalene;
[1R,3S] 1 -Aminomethyl-3-t-butyl-3,4-dihydro-5,6-dihydroxy-1 H-2-benzopyran;
[1R,3S] 1-Aminomethyl-3,4-dihydro-5,6-dihydroxy-3-phenyl-1H-2-benzopyran;
[1R,3S] 1-Aminomethyl-3-cyclohexyl-3,4-dihydro-5,6-dihydroxy-1H-2-benzopyran;
[1R,3R] 1-Aminomethyl-3,4-dihydro-5,6-dihydroxy-3-ethyl-1 H-2-benzopyran;
Spiro[(1-aminomethyl-3,4-dihydro-5,6-dihydroxy-1 H-2-benzopyran)-3,1'-cyclohexane];
[1R,3S] 1 -Aminomethyl-3,4-dihydro-5,6-dihydroxy-3-(4'-methoxyphenoxy)methyl-1H-2-benzopyran;
[1R*,3S*] 1-Aminomethyl-3,4-dihydro-5,6-dihydroxy-3-phenyl-1H-2-benzopyran;
[1R,3S] 1-Aminomethyl-3,4-dihydro-5,6-dihydroxy-3-phenoxymethyl-1 H-2-benzopyran;
[1R,3S] 1-Aminomethyl-3,4-dihydro-5,6-dihydroxy-3-(2'-phenylphenoxy)methyl-1 H-2- benzopyran;
[1R,3S] 1-Aminomethyl-3-(4'-t-butylphenoxy)methyl-3,4-dihydro-5,6-dihydroxy-1 H-2- benzopyran;
[1R,3S] 1-Aminomethyl-3-(4'-bromophenoxy)methyl-3,4-dihydro-5,6-dihydroxy-1 H-2- benzopyran;
[1R,3R] 1-Aminomethyl-3-benzyl-3,4-dihydro-5,6-dihydroxy-1 H-2-benzopyran;
[1R,3R] 1-Aminomethyl-3,4-dihydro-5,6-dihydroxy-3-(2'phenyl)ethyl-1H-2-benzopyran;
[1R,3S] 1-Aminomethyl-8-bromo-3,4-dihydro-5,6-dihydroxy-3-phenyl-1H-2-benzopyran;
[1R,3R] 1-Aminomethyl-3,4-dihydro-5,6-dihydroxy-3-n-octyl-1H-2-benzopyran;
[1R,3R] 1-Aminomethyl-3,4-dihydro-5,6-dihydroxy-3-(1'-hex-5'-ene)-1H-2-benzopyran;
[1R,3S] 1-Aminomethyl-3,4-dihydro-5,6-dihydroxy-3-ethyl-1H-2-benzopyran;
[1R,3R] 1-Aminomethyl-3,4-dihydro-5,6-dihydroxy-3-n-hexyl-1 H-2-benzopyran;
[1R,3S] 1-Aminomethyl-3-(4'-bromophenyl)-3,4-dihydro-5,6-dihydroxy-1H-2-benzopyran;
[1R,3S] 1 -Aminomethyl-3,4-dihydro-5, 6-dihydroxy-3-(3'-hydroxyphenyl)-1H-2-benzopyran;
[1R,3S] 3-Cyclohexyl-3,4-dihydro-5,6-dihydroxy-1-(N-methyl)aminomethyl-1 H-2- benzopyran;
[1R,3S] 3-t-Butyl-3,4-dihydro-5,6-dihydroxy-1-(N-methyl)aminomethyl-1H-2-benzopyran;
[1R,3S] 1-(N-Allyl)aminomethyl-3-cyclohexyl-3,4-dihydro-5,6-dihydroxy-1H-2-benzopyran;
[1R,3S] 3-Cyclohexyl-1-(N-cyclopropyl)aminomethyl-3,4-dihydro-5,6-dihydroxy-1H-2- benzopyran;
[1 R,3S] 1 -(N-benzyl)aminomethyl-3-cyclohexyl-3,4-dihydro-5,6-dihydroxy-1H-2-benzopyran;
[1R,3S] 1-,3-Bis(aminomethyl)-3,4-dihydro-5,6-dihydroxy-1H-2-benzopyran;
[1R,3S] 1-Aminomethyl-3,4-dihydro-5,6-dihydroxy-3-hydroxymethyl-1 H-2-benzopyran;
[1R,3S] 1-Aminomethyl-3,4-dihydro-5,6-dihydroxy-3-(N-piperidinyl)methyl-1 H-2- benzopyran;
[1R,35] 5,6-Dihydroxy-3-pheny!-1-(2'R-pyrrolidinyl)-1,2,3,4-tetrahydronaphthalene;
[1R,3R] 5,6-Dihydroxy-3-pheny!-1 -(2'R-pyrrolidyl)-1,2,3,4-tetrahydronaphtha!ene;
5;6-Dihydroxy-1-(N-methyl)aminomethyl-3-phenyl-3,4-dihydronaphthalene;
[1R,3S] 5,6-Dihydroxy-1 -(N-methy!)aminomethy!-3-pheny!-1,2,3,4-tetrahydronaphthalene;
1-Aminomethyl-5,6-dihydroxy-3-(3'-hydroxyphenyl)-3,4-dihydronaphthalene;
1-Aminomethyl-5,6-dihydroxy-3-(4'-hydroxyphenyl)-3,4-dihydronaphthalene;
[1R,3S] 1-Aminomethyl-5,6-dihydroxy-3-(3'-hydroxyphenyl)-1,2,3;4-tetrahydronaphthalene;
5;6-Bis(acetoxy)-1-(alanyl-alanyl)aminomethyl-3-phenyl-3,4-dihydronaphthalene;
5;6-Bis(acetoxy)-1-(γ-glutamyl)aminomethyl-3-phenyl-3;4-dihydronaphthalene;
5;6-Bis(acetoxy)-1-(alanyl)aminomethyl-3-phenyl-3,4-dihydronaphthalene;
5;6-Bis(acetoxy)-1-(methionyl)aminomethyl-3-phenyl-3,4-dihydronaphthalene;
1-(Alanyl-alanyl)aminomethyl-5,6-bis(benzoyloxy)-3-phenyl-3,4-dihydronaphthalene;
[1R,2S] 1-Aminomethy!-5,6-dihydroxy-2-(2'-hydroxy-1 '-ethyl)-3-phenyl-1,2,3,4-totrahydronaphthalene;
1-Aminomethyl-5,6-dihydroxy-3-phenylnaphthalene;
[1R,3S] 1 -Aminomethyl-3,4-dihydro-5,6-dihydroxy-3-(diphenyl)methyl-1 H-2-benzopyran;
[1R,3S] 1-Aminomethyl-3,4-dihydro-5,6-dihydroxy-3-(3'-methyl-2'-n-pentyl)-1 H-2-benzopyran;
[1R,3S] 1-Aminomethy!-3,4-dihydro-5,6-dihydroxy-3-(1 '-but-3'-ene)-1 H-2-benzopyran;
[1R,3S] 1-Aminomethyl-3,4-dihydro-5,6-dihydroxy-3-(6'-methyl-2'-hep-5'-ene)-1 H-2- benzopyran;
[1R,3S] 1-Aminomethyl-3-benzyloxymethyl-3,4-dihydro-5,6-dihydroxy-1 H-2-benzopyran;
[1R, 8S9aR] 1-Amino-5,6-dihydroxy-2,3,7,8,9,9a-hexahydro-8-phenylphenalene;
[1S, 8S 9aR] 1-Amino-5,6-dihydroxy-2,3,7,8,9,9a-hexahydro-8-phenylphenalene;
6;7-Dihydroxy-4-phenyl-2,3,4,5-tetrahydro-1 H-benz[e]isoindole; and
1-Aminomethyl-5,6-bis(benzoyloxy)-3-phenyl-3,4-dihydronaphthalene, or a pharmaceutically acceptable salt thereof.

11. A process according to claims 1-2 for preparing a compound selected from the group consisting of:
[1R,3S] 3-(1 '-Adamanty!)-1 -aminomethyl-3,4-dihydro-5,6-dihydroxy-1 H-2-benzopyran;
[1R,3S] 3-(1 '-Adamantyl)-1 -am!nomethyl-3,4-dihydro-5,6-dihydroxy-1H-2-benzopyran;
[1R,3S] 3-(1'-Adamantyl)-3,4-dihydro-5,6-dihydroxy-1-(N-methyl)aminomethyl-1 H-2- benzopyran;
[1R,3R] 1-Aminomethyl-3-cyclopentylmethyl-3;4-dihydro-5,6-dihydroxy-1 H-2-benzopyran;
[1R,3S] 1-Aminomethyl-3-cyclooctyl-3;4-dihydro-5,6-dihydroxy-1H-2 benzopyran;
[1R,3R] 1-Aminomethyl-3-n-butyl-3,4-dihydro-5,6-dihydroxy-1H-2-benzopyran;
[1R,3R] 3-n-Butyl-3,4-dihydro-5,6-dihydroxy-1-(N-methyl)aminomethyl-1H-2-benzopyran,
or a pharmaceutically acceptable salt thereof.

12. A process according to claims 1-2 for preparing a compound of the formula: 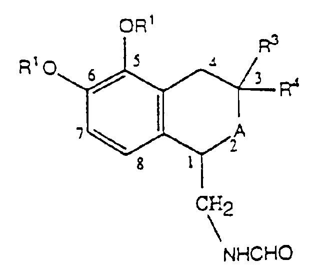
wherein A is 0 or S;
R¹ is a catechol protecting group;
R³ is selected from C₁-C₂ alkyl, substituted C₁-C₆ alkyl as defined below, C₂-C₁₂ alkenyl, substituted C₂-C₁₂ alkenyl as defined below, C₂-C₁₂ alkynyl, substituted C₂-C₁₂ alkynyl as defined below, C₃-C₁₂ cycloalkyl, substituted C₃-C₁₂ cycloalkyl as defined below, carbocyclic C₅-C₁₀ aryl, substituted carbocyclic C₅-C₁₀ aryl as defined below, heterocycle as defined below, and substituted heterocycle as defined below,
R⁴ is selected from hydrogen and C₁-C₁₂ alkyl or, taken together with R³ and the carbon atom to which they are attached, forms a spirocycloalkyl ring of from 3 to 7 carbons;
wherein substituted C₁-C₆ alkyl is a C₁-C₆ alkyl group bearing 1-2 substituents selected from halogen, hydroxy, C₁-C₆ alkoxy, amino, C₁-C₆ alkylamino, C₃-C₁₂ cycloalkyl, heterocycle as defined below, substituted heterocycle as defined below carbocyclic C₅-C₁₀ aryl, substituted carbocyclic C₅-C₁₀ aryl as defined below, carbocyclic C₅-C₁₀ aryloxy, substituted carbocyclic C₅₋C₁₀ aryloxy wherein substituted carbocyclic C₅-C₁ aryl is as defined below, or -NC(O)R¹¹ wherein R¹¹ is selected from hydrogen, C₁-C₆ alkyl, halo-(C₁-C₆ alkyl), heterocycle as defined below, substituted heterocycle as defined below, carbocyclic C₅-C₁₀ aryl, and substituted carbocyclic C₅-C₁₀ aryl as defined below;
wherein substituted C₂-C₁₂ alkenyl is a C₂-C₁₂ alkenyl group bearing a substituent selected from C₁-C₆ alkyl, C₃-C₁₂ cycloalkyl, carbocyclic C₅-C₁₀ aryl, benzyl, phenylethyl, amino, hydroxy, C₁-C₆ alkoxy and heterocycle as defined below,
wherein substituted C₂-C₁₂ alkynyl is a C₂-C₁₂ alkynyl bearing a substituent selected from C₁-C₆ alkyl, C₃-C₁₂ cycloalkyl, carbocyclic C₅-C₁₀ aryl, benzyl, phenylethyl amino, hydroxy C₁-C₁₂ alkylamino, C₁-C₆ alkoxy, and heterocycle as defined below;
wherein substituted C₃-C₁₂ cycloalkyl is a C₃-C₁₂ cycloalkyl group substituted by one or more C₁-C₁₂ alkyl, hydroxy amino, C₁-C₁₂ alkylamino, C₁-C₁₂ aminoalkyl, mercapto, C₁-C₁₂ alkylthio or halogen groups,
wherein substituted carbocyclic C₅-C₁₀ aryl is a carbocyclic C₅-C₁₀ aryl substituted by one or more hydroxy, halogen, C₁-C₆ alkyl or C₁-C₆ alkoxy groups or by a phenyl group;
wherein heterocycle is a monocyclic 5- or 6-membered saturated or unsaturated group containing one or two ring heteroatoms selected from N. O and S, the remaining atoms being carbon, such as, for example, furyl, tetrahydrofuryl, thienyl, pyrryl, pyrrolidyl, pyridyl, piperidyl, pyrazinyl, pyrimidyl, piperazinyl, morpholinyl, thiomorpholinyl, pyrazolyl, imidazolyl, oxazolyl, oxazolidyl, isoxazolyl, isoxazolidyl and thiazolyl; and
wherein substituted heterocycle is a heterocycle as defined above substituted by halogen, C₁-C₆ alkyl or C₁-C₆ alkoxy.

13. A process for preparing a compound of the formula: 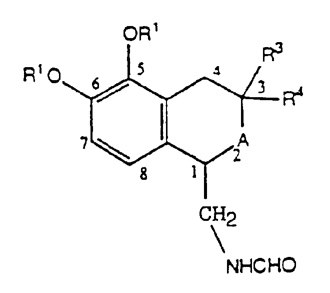
wherein A is 0 or S;
R¹ is a catechol protecting group;
R³ is selected from C₁-C₂ alkyl, substituted C₁-C₆ alkyl as defined below, C₂-C₁₂ alkenyl, substituted C₂-C₁₂ alkenyl as defined below, C₂-C₁₂ alkynyl, substituted C₂-C₁₂ alkynyl as defined below, C₃-C₁₂ cycloalkyl, substituted C₃-Cₗ₂ cycloalkyl as defined below, carbocyclic C₅-Cₗₒ aryl, substituted carbocyclic C₅-C₁₀ aryl as defined below, heterocycle as defined below, and substituted heterocycle as defined below,
R⁴ is selected from hydrogen and C₁-C₁₂ alkyl or, taken together with R³ and the carbon atom to which they are attached, forms a spirocycloalkyl ring of from 3 to 7 carbons;
wherein substituted C₁-C₆ alkyl is a C₁-C₆ alkyl group bearing 1-2 substituents selected from halogen, hydroxy, C₁-C₆ alkoxy, amino, C₁-C₆ alkylamino, C₃-Cₗ₂ cycloalkyl, heterocycle as defined below, substituted heterocycle as defined below, carbocyclic C₅-C₁₀ aryl, substituted carbocyclic C₅-C₁₀ aryl as defined below, carbocyclic C₅-C₁₀ aryloxy, substituted carbocyclic C₅-C₁₀aryloxy wherein substituted carbocyclic C₅-C₁₀ aryl is as defined below, or -NC(O)R¹¹ wherein R¹¹ is selected from hydrogen, C₁-C₆ alkyl, halo-(C₁-C₆ alkyl), heterocycle as defined below, substituted heterocycle as defined below, carbocyclic C₅-C₁₀ aryl, and substituted carbocyclic C₅-C₁₀ aryl as defined below;
wherein substituted C₂-C₁₂ alkenyl is a C₂-C₁₂ alkenyl group bearing a substituent selected from C₁-C₆ alkyl, C₃-C₁₂ cycloalkyl, carbocyclic C₅-C₁₀ aryl, benzyl, phenylethyl, amino, hydroxy, C₁-C₆ alkoxy and heterocycle as defined below,
wherein substituted C₂-C₁₂ alkynyl is a C₂-C₁₂ alkynyl bearing a substituent selected from C₁-C₆ alkyl, C₃-C₁₂ cycloalkyl, carbocyclic C₅-C₁₀ aryl, benzyl, phenylethyl amino, hydroxy C₁-C₁₂ alkylamino, C₁-C₆ alkoxy, and heterocycle as defined below;
wherein substituted C₃-C₁₂ cycloalkyl is a C₃-C₁₂ cycloalkyl group substituted by one or more C₁-C₁₂ alkyl, hydroxy amino, C₁-C₁₂ alkylamino, C₁-C₁₂ aminoalkyl, mercapto, C₁-C₁₂ alkylthio or halogen groups,
wherein substituted carbocyclic C₅-C₁₀ aryl is a carbocyclic C₅-C₁₀ aryl substituted by one or more hydroxy, halogen, C₁-C₆ alkyl or C₁-C₆ alkoxy groups or by a phenyl group;
wherein heterocycle is a monocyclic 5- or 6-membered saturated or unsaturated group containing one or two ring heteroatoms selected from N. O and S, the remaining atoms being carbon, such as, for example, furyl, tetrahydrofuryl, thienyl, pyrryl, pyrrolidyl, pyridyl, piperidyl, pyrazinyl, pyrimidyl, piperazinyl, morpholinyl, thiomorpholinyl, pyrazolyl, imidazolyl, oxazolyl, oxazolidyl, isoxazolyl, isoxazolidyl and thiazolyl; and
wherein substituted heterocycle is a heterocycle as defined above substituted by halogen, C₁-C₆ alkyl or C₁-C₆ alkoxy, comprising reacting a compound having the formula: 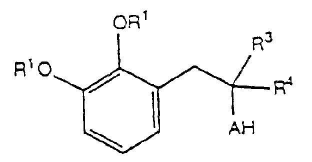
wherein R¹, R³ and R⁴ are as defined therein, with N-formylaminoacetaldehyde dimethyl acetal or with 3-(N-formylamino)-propionaldehyde dimethyl acetal in the presence of an acid catalyst.

14. A process according to claim 13 wherein the catalyst is selected from boron trifluoride etherate, zinc triflate, trimethylsilyl triflate and methanesulfonic acid.

## Patentansprüche (Patentansprüche für folgende(n) Vertragsstaat(en) : AT, BE, CH, LI, DE, DK, FR, GB, IT, LU, NL, SE)

1. Verbindung nach folgender Formel: 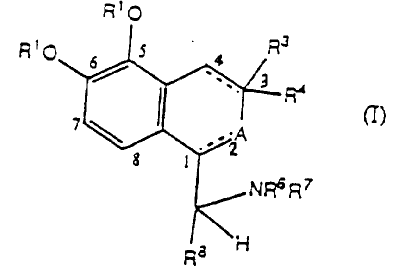 oder ein pharmazeutisch verträgliches Salz, ein Esters oder ein Amid dieser, worin A gleich O, S, CHR², CR² oder C ist, wenn A und R⁶ zusammen genommen einen stickstoffhaltigen 5-, 6- oder 7-gliedrigen Ring bilden;
worin die gestrichelten Linien mögliche Doppelbindungen darstellen;
worin R¹ aus Wasserstoff und aus einer Gruppe gewählt ist, die leicht in vivo abspaltbar ist;
worin R² aus Wasserstoff, C₁-C₁₂-Alkyl und aus substituiertem C₁-C₆-Alkyl gewählt ist;
worin R³ aus C₁-C₁₂-Alkyl, aus substituiertem C₁-C₆-Alkyl, wie unten definiert, aus C₂-C₁₂-Alkenyl, aus substituiertem C₂-C₁₂-Alkenyl, wie unten definiert, aus C₂-C₁₂-Alkinyl, aus substituiertem C₂-C₁₂-Alkinyl, wie unten definiert, aus C₃-C₁₂-Cycloalkyl, aus substituiertem C₃-C₁₂-Cycloalkyl, wie unten definiert, aus carbocyclischem C₅-C₁₀-Aryl, aus substituiertem carbocyclischem C₅-C₁₀-Aryl, wie unten definiert, aus einem Heterocyclus, wie unten definiert, und aus einem substituierten Hetrocyclus, wie unten definiert, gewählt ist;
worin R⁴ aus Wasserstoff und aus C₁-C₁₂-Alkyl gewählt ist, oder worin R⁴ zusammen mit R³ und dem Kohlenstoffatom, an das sie gebunden sind, einen Spirocycloalkylring von 3 bis 7 Kohlenstoffatomen ausbildet;
worin R⁶ aus Wasserstoff, aus C₁-C₁₂-Alkyl, aus substituiertem C₁-C₆-Alkyl, wie unten definiert, aus C₂-C₁₂-Alkenyl, aus substituiertem C₂-C₁₂-Alkenyl, wie unten definiert, aus C₂-C₁₂-Alkinyl, aus substituiertem C₂-C₁₂-Alkinyl, wie unten definiert, aus C3-C12-Cycloalkyl, aus substituiertem C₃-C₁₂-Cycloalkyl, wie unten definiert, aus Alkanoyl von 1 bis 8 Kohlenstoffatomen, aus einer natürlich vorkommenden Aminosäure und aus einem Dipeptid, das aus natürlich vorkommenden Aminosäuren gebildet ist, gewählt ist, oder worin R⁶ zusammen genommen mit A, wenn A gleich C ist, einen stickstoffhaltigen 5-, 6- oder 7-gliedrigen Ring ausbildet;
worin R⁷ gleich Wasserstoff oder C₁-C₁₂-Alkyl ist, oder worin R⁷ zusammen genommen mit R⁶ oder R⁸ einen stickstoffhaltigen 5-, 6-oder 7-gliedrigen Ring ausbildet, vorausgesetzt, daß wenn R⁶ gleich carbocyclischem Arylalkyl ist, R⁷ nicht gleich Alkyl ist;
worin R⁸ gleich Wasserstoff oder gleich C₁-C₁₂-Alkyl ist, oder worin R⁸ zusammen genommen mit R⁸ oder R⁷ einen stickstoffhaltigen 5-, 6-oder 7-gliedrigen Ring ausbildet oder zusammen genommen mit dem Katecholring
in der 8-Position und den Kohlenstoffatomen, an die sie gebunden sind, einen 5-, 6- oder 7-gliedrigen Ring ausbildet;
worin substituiertes C₁-C₆-Alkyl gleich einer C₁-C₆-Alkylgruppe ist, die 1-2 Substituenten trägt, die aus Halogen, Hydroxy, C₁-C₆-Alkoxy, Amino, C₁-C₆-Alkylamino, C₃-C₁₂-Cycloalkyl, aus einem Heterocyclus; wie unten definiert, aus einem substituierten Heterocyclus, wie unten definiert, aus carbocyclischem C₅-C₁₀-Aryl, aus substituiertem carbocyclischem C₅-C₁₀-Aryl, wie unten definiert, aus carbocyclischem C₅-C₁₀-Aryloxy, aus substituiertem carbocyclischem C₅-C₁₀-Arylox, worin das substituierte carbocyclische C₅-C₁₀-Aryl wie unten definiert ist, oder aus - NC(O)R¹¹» gewählt sind, worin R¹¹» aus Wasserstoff, aus C₁-C₆-Alkyl, aus Halogen-(C₁-C₆-Alkyl), aus einem Heterocyclus, wie unten definiert, aus einem substituierten Heterocyclus, wie unten definiert, aus carbocyclischem C₅-C₁₀-Aryl, und aus substituiertem carbocyclischem C₅-C₁₀-Aryl, wie unten definiert, gewählt ist;
worin substituiertes C₂-C₁₂-Alkenyl gleich einer C₂-C₁₂-Alkenylgruppe ist, die einen Substituenten trägt, der aus C₁-C₆-Alkyl, C₃-C₁₂-Cycloalkyl, carbocyclischem C₅-C₁₀-Aryl, Benzyl, Phenylethyl, Amino, Hydroxy, C₁-C₆-Alkoxy und aus einem Heterocyclus, wie unten definiert, gewählt ist;
worin substituiertes C₂-C₁₂-Alkinyl gleich einem C₂-C₁₂-Alkinyl ist, das einen Substituenten trägt, der aus C₁-C₆-Alkyl, aus C₃-C₁₂-Cycloalkyl, carbocyclischem C₅-C₁₀-Aryl, Benzyl, Phenylethyl, Amino, Hydroxy, aus C₁-C₁₂-Alkylamino, aus C₁-C₆-Alkox, und aus einem Heterocyclus; wie unten definiert, gewählt ist;
worin substituiertes C₃-C₁₂-Cycloalkyl gleich einer C₃-C₁₂-Cycloalkylgruppe ist, die mit einer oder mehreren C₁-C₁₂-Alkyl-, Hydroxy-, Amino-, C₁-C₁₂-Alkylamino-, C₁-C₁₂-Aminoalkyl-, Mercapto-, C₁-C₁₂-Alkylthio-oder Halogengruppe(n) substituiert ist;
worin substituiertes carbocyclisches C₅-C₁₀-Aryl ein carbocyclisches C₅-C₁₀-Aryl ist, das durch eine oder mehrere Hydroxy-, Halogen-, C₁-C₆-Alkyl oder C₁-C₆-Alkoxygruppe(n) oder durch eine Phenylgruppe substituiert ist;
worin der Heterocyclus eine monocyclische 5- oder 6-gliedrige gesättigte oder ungesättigte Gruppe ist, die ein oder zwei Ringheteroatome enthält, die aus N, O und S gewählt sind, wobei die verbleibenden Atome Kohlenstoff sind, wie zum Beispiel Furyl, Tetrahydrofuryl, Thienyl, Pyrryl, Pyrrolidyl, Pyridyl, Piperidyl, Pyrazinyl, Pyrimidyl, Piperazinyl, Morpholinyl, Thiomorpholinyl, Pyrazolyl, Imidazolyl, Oxazolyl, Oxazolidyl, Isoxazolyl, Isoxazolidyl und Thiazolyl; and
worin der substituierte Heterocyclus ein Heterocyclus ist, wie oben definiert, der mit Halogen, C₁-C₆-Alkyl oder C₁-C₆-Alkoxy substituiert ist.

2. Verbindung nach Anspruch 1, bei der A gleich CR² ist, und die gestrichelte Linie zwischen A und dem Ringatom mit der Nummer 1 eine Bindung darstellt, wobei die Verbindung folgende Formel aufweist: 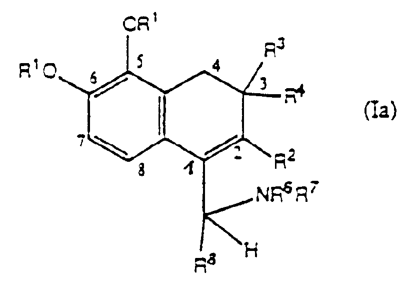 worin R¹, R², R³, R⁴, R⁶, R⁷ und R⁸ die in Anspruch 1 definierte Bedeutung haben.

3. Verbindung nach Anspruch 1, bei der A gleich CHR² ist, die folgende Formel aufweist: 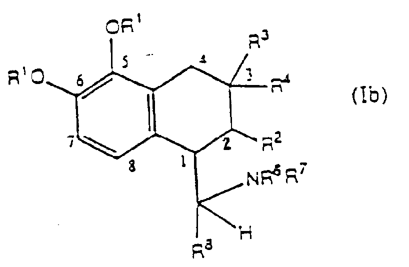 worin R¹, R², R³, R⁴, R⁶, R⁷ und R⁸ die in Anspruch 1 definierte Bedeutung haben.

4. Verbindung nach Anspruch 1, bei der A gleich O ist.

5. Verbindung nach Anspruch 1, bei der n gleich 0 ist, und bei der R⁶ und A zusammen genommen einen stickstoffhaltigen 5-, 6-oder 7-gliedrigen Ring bilden, die folgende Formel aufweist: 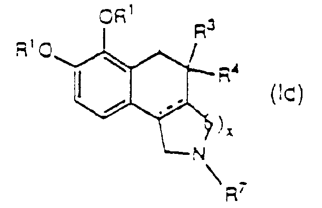 worin x gieich 1, 2 oder 3 ist und worin R¹, R³, R⁴ und R⁷ die in Anspruch 1 definierte Bedeutung haben.

6. Verbindung nach Anspruch 1, bei der A gleich CHR² ist, R² gleich H ist, und bei der R⁸ zusammen genommen mit der 8-Position des Katecholrings einen 5-, 6- oder 7-gliedrigen Ring ausbildet, die folgende Formel aufweist: 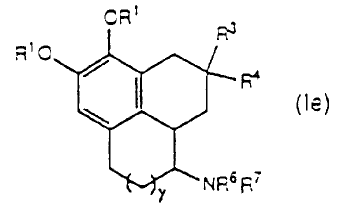 worin y gleich 0, 1 oder 2 ist und worin R¹ und R³, R⁴, R⁸ und R⁷ die in Anspruch 1 definierte Bedeutung haben.

7. Verbindung nach Anspruch 1, bei der R³ gleich C₁-C₁₂-Alkyl, substituiertem C₁-C₆-Alkyl, C₃-C₁₂-Cycloalkyl, substituiertem C₃-C₁₂-Cycloalkyl, carbocyclischem C₅-C₁₀-Aryl oder gleich substituiertem carbocyclischem C₅-C₁₀-Aryl ist, und bei der R⁴ gleich Wasserstoff ist.

8. Verbindung nach Anspruch 1 bei der R⁷ und R⁸ gleich Wasserstoff sind.

9. Verbindung nach Anspruch 1, die aus der Gruppe gewählt ist, die aus folgendem besteht:
1-Aminomethyl-5,6-dihydroxy-3-phenyl-3,4-dihydronaphthalin;
1-Aminomethyl-5,6-bis(acetoxy)-3-phenyl-3,4-dihydronaphthalin;
1-Aminomethyl-5,6-bis(trimethylacetoxy)-3-phenyl-3,4-dihydronaphthalin;
[1R,3S]-1-Aminomethyl-5,6-dihydroxy-3-phenyl-1,2,3,4-tetrahydronaphthalin;
1-Aminomethyl-3-cyclohexyl-5,6-dihydroxy-3,4-dihydronaphthalin;
[1R,3S]-1-Aminomethyl-3-cyclohexyl-5,6-dihydroxy-1,2,3,4-tetrahydronaphthalin;
[1R;3S]-1-Aminomethyl-3-t-butyl-3,4-dihydro-5,6-dihydroxy-1 H-2-benzopyran;
[1R,3S]-1-Aminomethyl-3,4-dihydro-5,6-dihydroxy-3-phenyl-1 H-2-benzopyran;
[1R,3]_{-1-Aminomethyl-3-c}yclohexyl-3,4-dihydro-5,6-dihydroxy-1 H-2-benzopyran;
[1R,3R]-1-Aminomethyl-3,4-dihydro-5,6-dihydroxy-3-ethyl-1 H-2-benzopyran;
Spiro[(1-aminomethyl-3,4-dihydro-5,6-dihydroxy-1 H-2-benzopyran)-3, '-cyclo hexan];
[1R,3S]-1-Aminomethyl-3,4-dihydro-5,6-dihydroxy-3-(4'-methoxyphenoxy)methyl-1 H-2-benzopyran;
[1R,*3S*]-1 -Aminomethvi-3,4-dihvdro-5,6-dihvdroxv-3-phenyi-1 H-2-benzopyran;
[1R,3S]-1-Aminomethyl-3,4-dihydro-5,6-dihydroxy-3-phenoxymethyl-1 H-2-benzopyran;
[1R,3S]-1-Aminomethyl-3,4-dihydro-5,6-dihydroxy-3-(2'-phenylphenoxy)methyl-1 H-2- benzopyran;
[1R,3S]-1-Aminomethyl-3-(4'-t-butylphenoxy)methyl-3,4-dihydro-5;6-dihydroxy-1 H-2-benzopyran;
[1R,3S]-1-Aminomethyl-3-(4'-bromphenoxy)methyl-3,4-dihydro-5,6-dihydroxy-1 H-2- benzopyran;
[1 R,3R]-1-Aminomethyl-3-benzyl-3,4-dihydro-5,6-dihydroxy-1 H-2-benzopyran;
[1R,3S]-1-Aminomethyl-3,4-dihydro-5,6-dihydroxy-3-(2'phenyl)ethyl-1 H-2-benzopyran;
[1R,3S]-1-Aminomethyl-8-brom-3,4-dihydro-5,6-dihydroxy-3-phenyl-1 H-2-benzopyran;
[1 R,3R]-1-Aminomethyl-3,4-dihydro-5,6-dihydroxy-3-n-octyl-1 H-2-benzopyran;
[1R,3S]-1-Aminomethyl-3,4-dihydro-5,6-dihydroxy-3-(1'-hex-5'-en)-1 H-2-benzopyran;
[1R,3S]-1-Aminomethyl-3,4-dihydro-5,6-dihydroxy-3-ethyl-1 H-2-benzopyran;
[1R,3S]-1-Aminomethyl-3,4-dihydro-5,6-dihydroxy-3-n-hexyl-1 H-2-benzopyran;
[1R,3S]-1-Aminomethyl-3-(4'-bromphenyl)-3,4-dihydro-5,6-dihydroxy-1 H-2-benzopyran;
[1R,3S]-1-Aminomethyl-3,4-dihydro-5,6-dihydroxy-3-(3'-hydroxyphenyl)-1 H-2-benzopyran;
[1R,3S]-3-Cyclohexyl-3,4-dihydro-5,6-dihydroxy-1-(N-methyl)aminomethyl-1 H-2-benzopyran;
[1R,3S]-3-t-Butyl-3,4-dihydro-5,6-dihydroxy-1-(N-methyl)aminomethyl-1 H-2-benzopyran;
[1R,3S]-1-(N-Allyl)aminomethyl-3-cyclohexyl-3,4-dihydro-5,6-dihydroxy-1 H-2-benzopyran;
[1R,3S]-3-Cyclohexyl-1-(N-cyclopropyl)aminomethyl-3,4-dihydro-5,6-dihydroxy-1 H-2- benzopyran;
[1R,3S]-1-(N-Benzyl)aminomethyl-3-cyclohexyl-3,4-dihydro-5,6-dihydroxy-1H-2-benzopyran;
[1R,3S]-1,3-Bis(aminomethyl)-3,4-dihydro-5,6-dihydroxy-1 H-2-benzopyran;
[1R,3S]-1-Aminomethyl-3,4-dihydro-5,6-dihydroxy-3-hydroxymethyl-1 H-2-benzopyran;
[1R,3S]-1-Aminomethyl-3,4-dihydro-5,6-dihydroxy-3-(N-piperidinyl)methyl-1 H-2-benzopyran;
[1R,3S]-5,6-Dihydroxy-3-phenyl-1-(2'_R-pyrrolidinyl)-1,2,3,4-tetrahydronaphthalin;
[1R,3R]-5,6-Dihydroxy-3-phenyl-1-(2'_R-pyrrolidinyl)-1,2;3,4-tetrahydronaphthalin;
5;6-Dihydroxy-1-(N-methyl)aminomethyl-3-phenyl-3,4-dihydronaphthalin;
[1R,3S]-5,6-Dihydroxy-1-(N-methyl)aminomethyl-3-phenyl -1,2, 3,4-tetrahydronaphthalin;
1-Aminomethyl-5,6-dihydroxy-3-(3'-hydroxyphenyl)-3,4-dihydronaphthalin;
1-Aminomethyl-5,6-dihydroxy-3-(4'-hydroxyphenyl)-3,4-dihydronaphthalin;
[1R,3S]-1-Aminomethyl-5,6-dihydroxy-3-(3'-hydroxyphenyl)-1,2,3,4-tetrahydronaphthalin;
5;6-Bis(acetoxy)-1-(alanyl-alanyl)aminomethyl-3-phenyl-3,4-dihydronaphthalin;
5;6-Bis(acetoxy)-1-(γ-glutamyl)aminomethyl-3-phenyl-3;4-dihydronaphthalin;
5;6-Bis(acetoxy)-1-(alanyl)aminomethyl-3-phenyl-3,4-dihydronaphthalin;
5;6-Bis(acetoxy)-1-(methionyl)aminomethyl-3-phenyl-3,4-dihydronaphthalin;
1-(Alanyl-alanyl)aminomethyl-5,6-bis(benzoyloxy)-3-phenyl-3,4-dihydronaphthalin;
[1R,2S]-1-Aminomethyl-5,6-dihydroxy-2-(2'-hydroxy-1'-ethyl)-3-phenyl-1,2,3,4-tetrahydronaphthalin;
1-Aminomethyl-5,6-dihydroxy-3-phenylnaphthalin;
[1R,3S]-1-Aminomethyl-3,4-dihydro-5,6-dihydroxy-3-(diphenyl)methyl-1 H-2benzopyran;
[1R,3S]-1-Aminomethyl-3,4-dihydro-5,6-dihydroxy-3-(3'-methyl-2'-n-pentyl)-1 H-2-benzopyran;
[1R,3S]-1-Aminomethyl-3,4-dihydro-5,6-dihydroxy-3-(1'-but-3'-en)-1 H-2-benzopyran;
[1R,3S]-1-Aminomethyl-3,4-dihydro-5,6-dihydroxy-3-(6'-methyl-2'-hept-5'-en)-1 H-2- benzopyran;
[1R,3S]-1-Aminomethyl-3-benzyloxymethyl-3,4-dihydro-5,6-dihydroxy-1 H-2-benzopyran;
[1R,8S;9a_R]-1-Amino-5,6-dihydroxy-2,3,7,8;9,9a-hexahydro-8-phenylphenalen;
[1S,8S,9aR]-1-Amino-5,6-dihydroxy-2,3,7,8,9,9a-hexahydro-8-phenylphenalen;
6;7-Dihydroxy-4-phenyl-2,3,4,5-tetrahydro-1H-benz[e]isoindol; und
1-Aminomethyl-5,6-bis(benzoyloxy)-3-phenyl-3_{;}4-dihydronaphthalin oder aus einem pharmazeutisch verträglichen Salz dieser

10. Verbindung, die aus der Gruppe gewählt ist, die aus folgendem besteht:
[1R,3S]-3-(1'-Adamantyl)-1-aminomethyl-3,4-dihydro-5,6-dihydroxy-1H-2-benzopyran;
[1R*;3S*]-3-(1'-Adamantyl)-1-aminomethyl-3;4-dihydro-5,6-dihydroxy-1H-2benzopyran;
[1R,3S]-3-(1 '-Adamantyi)-3,4-dihydro-5,6-d!hydroxy-1 -(N-methyi)aminomethyi-l H-2-benzopyran;
[1R,3R]-1-Aminomethyl-3-cyclopentylmethyl-3,4-dihydro-5;6-dihydroxy-1 H-2benzopyran;
[1R,3S]1-Aminomethyl-3-cyclooctyl-3,4-dihydro-5;6-dihydroxy-1 H-2-benzopyran;
[i R,3R]-1-Aminomethyl-3-n-butyl-3,4-dihydro-5,6-dihydroxy-1 H-2-benzopyran;
[1 R,3R]-3-n-Butyl-3,4-dihydro-5,6-dihydroxy-1 (N-methyl)aminomethyl-1 H-2-benzopyran_{;} oder aus einem pharmazeutisch verträglichen Salz dieser

11. Pharmazeutische Zusammensetzung zur selektiven Einwirkung auf die dopaminergen Rezeptoren, die einen pharmazeutisch verträglichen Träger und eine therapeutisch wirksame Menge einer Verbindung nach Anspruch 1 umfaßt.

12. Pharmazeutische Zusammensetzung zur Behandlung von im Zusammenhang mit Dopamin stehenden neurologischen Störungen, die einen pharmazeutisch verträglichen Träger und eine therapeutisch wirksame Menge einer Verbindung nach Anspruch 1 umfaßt.

13. Pharmazeutische Zusammensetzung zur Behandlung von im Zusammenhang mit Dopamin stehenden psychologischen Störungen, die einen pharmazeutisch verträglichen Träger und eine therapeutisch wirksame Menge einer Verbindung nach Anspruch 1 umfaßt.

14. Pharmazeutische Zusammensetzung zur Behandlung von im Zusammenhang mit Dopamin stehenden kardiovaskulären Störungen, die einen pharmazeutisch verträglichen Träger und eine therapeutisch wirksame Menge einer Verbindung nach Anspruch 1 umfaßt.

15. Pharmazeutische Zusammensetzung zur Behandlung von Störungen, die auf Substanzmißbrauch und Suchtverhalten beruhen, die einen pharmazeutisch verträglichen Träger und eine therapeutisch wirksame Menge einer Verbindung nach Anspruch 1 umfaßt.

16. Pharmazeutische Zusammensetzung zur Behandlung von Wahrnehmungs- und Aufmerksamkeitsstörungen, die einen pharmazeutisch verträglichen Träger und eine therapeutisch wirksame Menge einer Verbindung nach Anspruch 1 umfaßt.

17. Verbindung nach Anspruch 1 zur Verwendung als therapeutischer Wirkstoff.

18. Verwendung einer Verbindung nach Anspruch 1 zur Herstellung eines Medikamentes zur selektiven Einwirkung auf die dopaminergen Rezeptoren eines Patienten, der einer solchen Behandlung bedarf.

19. Verwendung einer Verbindung nach Anspruch 1 zur Herstellung eines Medikamentes zur Behandlung von im Zusammenhang mit Dopamin stehenden neurologischen Störungen, die durch abnormale dopaminerge Aktivität gekennzeichnet sind, bei einem Patienten, der einer solchen Behandlung bedarf.

20. Verwendung einer Verbindung nach Anspruch 1 zur Herstellung eines Medikamentes zur Behandlung von im Zusammenhang mit Dopamin stehenden psychologischen Störungen, die durch abnormale dopaminerge Aktivität gekennzeichnet sind, bei einem Patienten, der einer solchen Behandlung bedarf.

21. Verwendung einer Verbindung nach Anspruch 1 zur Herstellung eines Medikamentes zur Behandlung von im Zusammenhang mit Dopamin stehenden kardiovaskulären Störungen bei einem Patienten, der einer solchen Behandlung bedarf.

22. Verwendung einer Verbindung nach Anspruch 1 zur Herstellung eines Medikamentes zur Behandlung von Störungen aufgrund von Suchtverhalten bei einem Patienten, der einer solchen Behandlung bedarf.

23. Verwendung einer Verbindung nach Anspruch 1 zur Herstellung eines Medikamentes zur Behandlung von Wahrnehmungs- und Aufmerksamkeitsstörungen bei einem Patienten, der einer solchen Behandlung bedarf.

24. Verbindung nach folgender Formel: 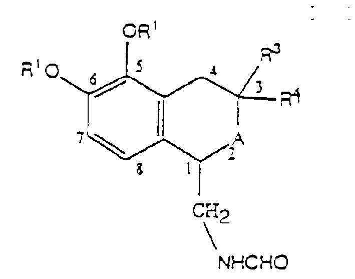
worin A gleich O oder S ist;
worin R¹ gleich einer Katechol-Schutzgruppe ist;
worin R³ aus C₁-C₁₂-Alkyl, aus substituiertem C₁-C₆-Alkyl, wie unten definiert, aus C₂-C₁₂-Alkenyl, aus substituiertem C₂-C₁₂-Alkenyl, wie unten definiert, aus C₂-C₁₂-Alkinyl, aus substituiertem C₂-C₁₂-Alkinyl, wie unten definiert, aus C₃-C₁₂-Cycloalkyl, aus substituiertem C₃-Cᵢ₂-Cycloalkyl, wie unten definiert, aus carbocyclischem C₅-C₁₀-Aryl, aus substituiertem carbocyclischem C_{S}-C₁₀-Aryl, wie unten definiert, aus einem Heterocyclus, wie unten definiert, und aus einem substituierten Hetrocyclus, wie unten definiert, gewählt ist;
worin R⁴ aus Wasserstoff und aus C₁-C₁₂-Alkyl gewählt ist, oder worin R⁴ zusammen mit R³ und dem Kohlenstoffatom, an das sie gebunden sind, einen Spirocycloalkylring von 3 bis 7 Kohlenstoffatomen ausbildet;
worin substituiertes Cᵢ-C₆-Alkyl gleich einer C₁-C₆-Alkylgruppe ist, die 1-2 Substituenten trägt, die aus Halogen, Hydroxy, C₁-C₆-Alkoxy, Amino, C₁-C₆-Alkylamino, C₃-C₁₂-Cycloalkyl, aus einem Heterocyclus, wie unten definiert, aus einem substituierten Heterocyclus, wie unten definiert, aus carbocyclischem C₅-C₁₀-Aryl, aus substituiertem carbocyclischem C₅-C₁₀-Aryl, wie unten definiert, aus carbocyclischem C₅-C₁₀-Aryloxy, aus substituiertem carbocyclischem C₅-C₁₀ Aryloxy, worin das substituierte carbocyclische C₅-C₁₀ Aryl wie unten definiert ist, oder aus - NC(O)R¹¹ gewählt sind, worin R¹¹ aus Wasserstoff, aus C₁-C₆-Alkyl, aus Halogen-(C₁-C₆-Alkyl), aus einem Heterocyclus, wie unten definiert, aus einem substituierten Heterocyclus, wie unten definiert, aus carbocyclischem C₅-C₁₀-Aryl, und aus substituiertem carbocyclischem C₅-C₁₀-Aryl, wie unten definiert, gewählt ist;
worin substituiertes C₂-C₁₂-Alkenyl gleich einer C₂-C₁₂-Alkenylgruppe ist, die einen Substituenten trägt, der aus C₁-C₆-Alkyl, C₃-C₁₂-Cycloalkyl, carbocyclischem C₅-C₁₀-Aryl, Benzyl, Phenylethyl, Amino, Hydroxy, C₁-C₆-Alkoxy und aus einem Heterocyclus, wie unten definiert, gewählt ist;
worin substituiertes C₂-C₁₂ Alkinyl gleich einem C₂-C₁₂-Alkinyl ist, das einen Substituenten trägt, der aus C₁-C₆-Alkyl, aus C₃-C₁₂-Cycloalkyl, carbocyclischem C₅-C₁₀-Aryl, Benzyl, Phenylethyl, Amino, Hydroxy, aus C₁-C₁₂-Alkylamino, aus C₁-C₆-Alkoxy, und aus einem Heterocyclus, wie unten definiert, gewählt ist;
worin substituiertes C₃-Cᵢ₂-Cycloalkyl gleich einer C₃-C₁₂-Cycloalkylgruppe ist, die mit einer oder mehreren C₁-C₁₂-Alkyl-, Hydroxy-, Amino-, C₁-C₁₂-Alkylamino-, C₁-C₁₂-Aminoalkyl-, Mercapto-, C₁-C₁₂-Alkylthio-oder Halogengruppe(n) substituiert ist;
worin substituiertes carbocyclisches C₅-C₁₀-Aryl ein carbocyclisches C₅-C₁₀-Aryl ist, das durch eine oder mehrere Hydroxy-, Halogen-, C₁-C₆-Alkyl oder C₁-C₆-Alkoxygruppe(n) oder durch eine Phenylgruppe substituiert ist;
worin der Heterocyclus eine monocyclische 5- oder 6-gliedrige gesättigte oder ungesättigte Gruppe ist, die ein oder zwei Ringheteroatome enthält, die aus N, O und S gewählt sind, wobei die verbleibenden Atome Kohlenstoff sind, wie zum Beispiel Furyl, Tetrahydrofuryl, Thienyl, Pyrryl, Pyrrolidyl, Pyridyl, Piperidyl, Pyrazinyl, Pyrimidyl, Piperazinyl, Morpholinyl, Thiomorpholinyl, Pyrazolyl, Imidazolyl, Oxazolyl, Oxazolidyl, Isoxazolyl, Isoxazolidyl und Thiazolyl; and
worin der substituierte Heterocyclus ein Heterocyclus ist, wie oben definiert, der mit Halogen, C₁-C₆-Alkyl oder C₁-C₆-Alkoxy substituiert ist.

25. Verfahren zur Herstellung einer Verbindung nach Anspruch 24, wobei das Verfahren die Umsetzung einer Verbindung nach folgender Formel: 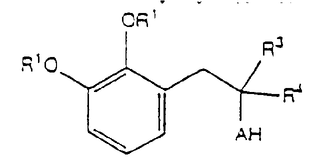 worin R¹, R³ und R⁴ die hierin definierte Bedeutung haben, mit N-Formylaminoacetaldehyddimethylacetal oder mit 3-(N-Formylamino)propionaldehyddimethylacetal in Gegenwart eines sauren Katalysators umfaßt.

26. Verfahren nach Anspruch 24, worin der Katalysator aus Bortrifluoridetherat, Zinktriflat, Trimethylsilyltriflat und Methansulfonsäure ausgewählt ist

## Patentansprüche (Patentansprüche für folgende(n) Vertragsstaat(en) : ES)

1. Verfahren zur Herstellung einer Verbindung nach folgender Formel 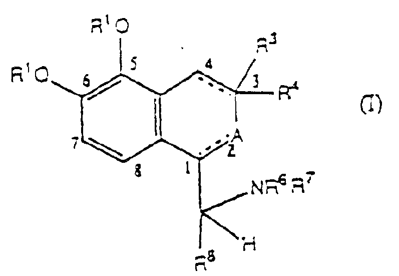 oder eines pharmazeutisch verträglichen Salzes, eines Esters oder eines Amides dieser, worin A gleich O, S, CHR², CR² oder C ist, wenn A und R⁶ zusammen genommen einen stickstoffhaltigen 5-, 6- oder 7-gliedrigen Ring bilden;
worin die gestrichelten Linien mögliche Doppelbindungen darstellen;
worin R¹ aus Wasserstoff und aus einer Gruppe gewählt ist, die leicht in vivo abspaltbar ist;
worin R² aus Wasserstoff, C₁-C₁₂-Alkyl und aus substituiertem C₁-C₆-Alkyl gewählt ist;
worin R³ aus C₁-C₁₂-Alkyl, aus substituiertem C₁-C₆-Alkyl, wie unten definiert, aus C₂-C₁₂-Alkenyl, aus substituiertem C₂-C₁₂-Alkenyl, wie unten definiert, aus C₂-C₁₂-Alkinyl, aus substituiertem C₂-C₁₂-Alkinyl, wie unten definiert, aus C₃-C₁₂-Cycloalkyl; aus substituiertem C₃-C₁₂-Cycloalkyl, wie unten definiert, aus carbocyclischem C₅-C₁₀-Aryl, aus substituiertem carbocyclischem C₅-C₁₀-Aryl, wie unten definiert, aus einem Heterocyclus, wie unten definiert, und aus einem substituierten Hetrocyclus, wie unten definiert, gewählt ist;
worin R⁴ aus Wasserstoff und aus C₁-C₁₂-Alkyl gewählt ist, oder worin R⁴ zusammen mit R³ und dem Kohlenstoffatom, an das sie gebunden sind, einen Spirocycloalkylring von 3 bis 7 Kohlenstoffatomen ausbildet;
worin R⁶ aus Wasserstoff, aus C₁-C₁₂-Alkyl, aus substituiertem C₁-C₆-Alkyl, wie unten definiert, aus C₂-C₁₂-Alkenyl, aus substituiertem C₂-C₁₂-Alkenyl, wie unten definiert, aus C₂-C₁₂-Alkinyl, aus substituiertem C₂-C₁₂-Alkinyl, wie unten definiert, aus C3-C12-Cycloalkyl, aus substituiertem C₃-Cᵢ₂-Cycloalkyl, wie unten definiert, aus Alkanoyl von 1 bis 8 Kohlenstoffatomen, aus einer natürlich vorkommenden Aminosäure und aus einem Dipeptid, das aus natürlich vorkommenden Aminosäuren gebildet ist, gewählt ist, oder worin R⁶ zusammen genommen mit A, wenn A gleich C ist, einen stickstoffhaltigen 5-, 6- oder 7-gliedrigen Ring ausbildet;
worin R⁷ gleich Wasserstoff oder C₁-C₁₂-Alkyl ist, oder worin R⁷ zusammen genommen mit R⁶ oder R⁸ einen stickstoffhaitigen 5-, 6-oder 7-gliedrigen Ring ausbildet, vorausgesetzt, daß wenn R⁶ gleich carbocyclischem Arylalkyl ist, R⁷ nicht gleich Alkyl ist;
worin R⁸ gleich Wasserstoff oder gleich C₁-C₁₂-Alkyl ist, oder worin R⁸ zusammen genommen mit R⁸ oder R⁷ einen stickstoffhaltigen 5-, 6-oder 7-gliedrigen Ring ausbildet oder zusammen genommen mit dem Katecholring in der 8-Position und den Kohlenstoffatomen, an die sie gebunden sind, einen 5-, 6- oder 7-gliedrigen Ring ausbildet;
worin substituiertes C₁-C₆-Alkyl gleich einer C₁-C₆-Alkylgruppe ist, die 1-2 Substituenten trägt, die aus Halogen, Hydroxy, C₁-C₆-Alkoxy, Amino, C₁-C₆-Alkylamino, C₃-C₁₂-Cycloalkyl, aus einem Heterocyclus; wie unten definiert, aus einem substituierten Heterocyclus, wie unten definiert, aus carbocyclischem C₅-C₁₀-Aryl, aus substituiertem carbocyclischem C₅-C₁₀-Aryl, wie unten definiert, aus carbocyclischem C₅-C₁₀-Aryloxy, aus substituiertem carbocyclischem C₅-C₁₀-Aryloxy, worin das substituierte carbocyclische C₅-C₁₀-Aryl wie unten definiert ist, oder aus - NC(O)R¹¹ gewählt sind, worin R¹¹ aus Wasserstoff, aus C₁-C₆-Alkyl; aus Halogen-(C₁-C₆-Alkyl), aus einem Heterocyclus, wie unten definiert, aus einem substituierten Heterocyclus, wie unten definiert, aus carbocyclischem C₅-C₁₀-Aryl, und aus substituiertem carbocyclischem C₅-C₁₀-Aryl, wie unten definiert, gewählt ist;
worin substituiertes C₂-C₁₂-Alkenyl gleich einer C₂-C₁₂-Alkenylgruppe ist, die einen Substituenten trägt, der aus C₁-C₆-Alkyl, C₃-C₁₂-Cycloalkyl, carbocyclischem C₅-Cₗₒ-Aryl, Benzyl, Phenylethyl, Amino, Hydroxy, C₁-C₆-Alkoxy und aus einem Heterocyclus, wie unten definiert, gewählt ist;
worin substituiertes C₂-C₁₂-Alkinyl gleich einem C₂-C₁₂-Alkinyl ist, das einen Substituenten trägt, der aus C₁-C₆-Alkyl, aus C₃-C₁₂-Cycloalkyl, carbocyclischem C₅-C₁₀-Aryl, Benzyl, Phenylethyl, Amino, Hydroxy, aus C₁-C₁₂-Alkylamino, aus C₁-C₆-Alkoxy, und aus einem Heterocyclus, wie unten definiert, gewählt ist;
worin substituiertes C₃-C₁₂-Cycloalkyl gleich einer C₃-C₁₂-Cycloalkylgruppe ist, die mit einer oder mehreren C₁-C₁₂-Alkyl-, Hydroxy-, Amino-, C₁-C₁₂-Alkylamino-, C₁-C₁₂-Aminoalkyl-, Mercapto-, C₁-C₁₂-Alkylthio-oder Halogengruppe(n) substituiert ist;
worin substituiertes carbocyclisches C₅-C₁₀-Aryl ein carbocyclisches C₅-C₁₀-Aryl ist, das durch eine oder mehrere Hydroxy-, Halogen-, C₁-C₆-Alkyl oder C₁-C₆-Alkoxygruppe(n) oder durch eine Phenylgruppe substituiert ist;
worin der Heterocyclus eine monocyclische 5- oder 6-gliedrige gesättigte oder ungesättigte Gruppe ist, die ein oder zwei Ringheteroatome enthält, die aus N, O und S gewählt sind, wobei die verbleibenden Atome Kohlenstoff sind, wie zum Beispiel Furyl, Tetrahydrofuryl, Thienyl, Pyrryl, Pyrrolidyl, Pyridyl, Piperidyl, Pyrazinyl, Pyrimidyl, Piperazinyl, Morpholinyl, Thiomorpholinyl, Pyrazolyl, Imidazolyl, Oxazolyl, Oxazolidyl, Isoxazolyl, Isoxazolidyl und Thiazolyl; and
worin der substituierte Heterocyclus ein Heterocyclus ist, wie oben definiert, der mit Halogen, C₁-C₆-Alkyl oder C₁-C₆-Alkoxy substituiert ist; wobei das Verfahren folgende Schritte umfaßt:
(a) Umsetzung eines geeignet geschützten Katechol-Ausgangsmaterials nach folgender Formel 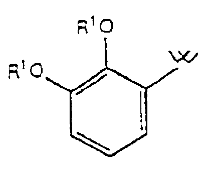
worin R₁ aus Wasserstoff und aus einer Gruppe gewählt ist, die in vivo leicht abspaltbar ist, und
worin W aus der Gruppe gewählt ist, die H, Li oder eine ringbildende Gruppe umfaßt;
mit einem geeignet substituierten kondensierenden Molekül und einem kondensierenden Reagenz unter geeigneten Kondensationsbedingungen unter Erzeugung einer ersten Zwischenstufen-Verbindung;
(b) Überführen der ersten Zwischenstufen-Verbindung in eine geeignet geschützte, geeignet substituierte, gewünschte zweite Zwischenstufen-Verbindung;
(c) Cyclisieren der zweiten Zwischenstufenverbindung unter Erzeugung einer geeignet geschützten, geeignet substituierten, dritten Zwischenstufen- Verbindung;
(d) Addieren der gewünschten aminhaltigen Gruppierung durch eine oder mehrere derivatisierende Reaktionen unter Herstellung einer geeignet geschützten End-Zwischenstufen-Verbindung; und
(e) Entschützen der End-Zwischenstufen-Verbindung mit dem geeigneten Entschützungreagenz und Isolieren des gewünschten Produkts.

2. Verfahren nach Anspruch 1, wobei die ringbildende Gruppe aus der Gruppe gewählt ist, die folgendes umfaßt:

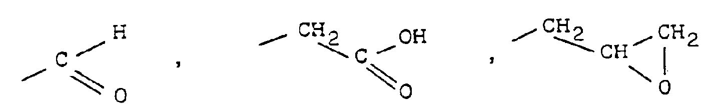

3. Verfahren nach einem oder mehreren der Ansprüche 1-2, bei dem A gleich CR² ist und die gestrichelte Linie zwischen A und dem Ringatom mit der Nummer 1 eine Bindung darstellt, nach folgender Formel: 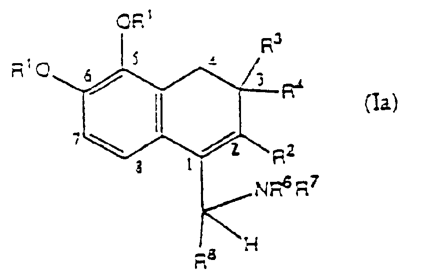 worin R¹, R², R³, R⁴, R⁶, R⁷ und R⁸ die in Anspruch 1 definierte Bedeutung haben.

4. Verfahren nach einem oder mehreren der Ansprüche 1-2, bei dem A gleich CHR² ist nach folgender Formel: 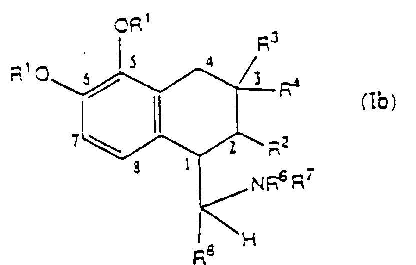 worin R¹, R², R³, R⁴, R⁶, R⁷ und R⁸ die in Anspruch 1 definierte Bedeutung haben.

5. Verfahren nach einem oder mehreren der Ansprüche 1-2, bei dem A gleich O ist.

6. Verfahren nach einem oder mehreren der Ansprüche 1-2, bei dem n gleich 0 ist, und bei dem R⁶ und A zusammen genommen einen stickstoffhaltigen 5-, 6- oder 7-gliedrigen Ring bilden, nach folgender Formel: 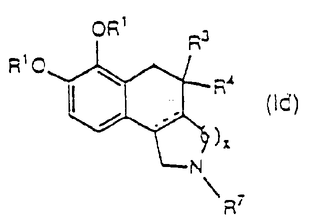 worin x gleich 1, 2 oder 3 ist und worin R¹, R³, R⁴ und R⁷ die in Anspruch 1 definierte Bedeutung haben.

7. Verfahren nach einem oder mehreren der Ansprüche 1-2, bei dem A gleich CHR² ist, R² gleich H ist, und bei dem R⁸ zusammen genommen mit der 8-Position des Katecholrings einen 5-, 6- oder 7-gliedrigen Ring ausbildet, nach folgender Formel: 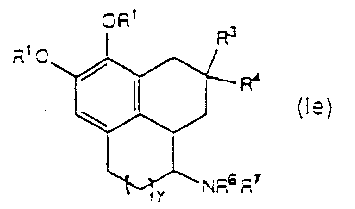 worin y gleich 0, 1 oder 2 ist, und worin R¹ und R³,R⁴,R⁶ und R⁷ die in Anspruch 1 definierte Bedeutung haben.

8. Verfahren nach einem oder mehreren der Ansprüche 1-2, bei dem R³ gleich C₁-C₁₂-Alkyl, substituiertem C₁-C₆-Alkyl, C₃-C₁₂₋Cycloalkyl, substituiertem C₃-C₁₂-Cycloalkyl, carbocyclischem C₅-C₁₀-Aryl oder gleich substituiertem carbocyclischem C₅-C₁₀-Aryl ist, und bei dem R⁴ gleich Wasserstoff ist.

9. Verfahren nach einem oder mehreren der Ansprüche 1-2, bei dem R⁷ und R⁸ gleich Wasserstoff sind.

10. Verfahren nach einem oder mehreren der Ansprüche 1-2 zur Herstellung einer Verbindung, die aus der Gruppe gewählt ist, die aus folgendem besteht:
1-Aminomethyl-5,6-dihydroxy-3-phenyl-3,4-dihydronaphthalin;
1-Aminomethyl-5,6-bis(acetoxy)-3-phenyl-3,4-dihydronaphthalin;
1-Aminomethyl-5,6-bis(trimethylacetoxy)-3-phenyl-3,4-dihydronaphthalin;
[1R,3S]-1-Aminomethyl-5,6-dihydroxy-3-phenyl-1,2,3,4-tetrahydronaphthalin;
1-Aminomethyl-3-cyclohexyl-5,6-dihydroxy-3,4-dihydronaphthalin;
[1R,3S]-1-Aminomethyl-3-cyclohexyl-5,6-dihydroxy-1,2,3,4-tetrahydronaphthalin;
[1R,3S]-1-Aminomethyl-3-t-butyl-3,4-dihydro-5,6-dihydroxy-1H-2-benzopyran;
[1R,3S]-1-Aminomethyl-3,4-dihydro-5,6-dihydroxy-3-phenyl-1H-2-benzopyran;
[4R,3S]-1-Aminomethyl-3-cyclohexyl-3,4-dihydro-5,6-dihydroxy-1H-2-benzopyran;
[1R,3R]-1-Aminomethyl-3,4-dihydro-5,6-dihydroxy-3-ethyl-1H-2-benzopyran;
Spiro[(1-aminomethyl-3,4-dihydro-5,6-dihydroxy-1 H-2-benzopyran)--3,1'-cyclo hexan];
[1R,3S]-1-Aminomethyl-3,4-dihydro-5,6-dihydroxy-3-(4'-methoxyphenoxy)methyl-1H-2-benzopyran;
[1 R^{*},3S^{*}]-1-Aminomethyl-3,4-dihydro-5,6-dihydroxy-3-phenyl-1H-2-benzopyran;
[1R,3S]-1-Aminomethyl-3,4-dihydro-5,6-dihydroxy-3-phenoxymethyl-1H-2-benzopyran;
[1R,3S]-1-Aminomethyl-3,4-dihydro-5,6-dihydroxy-3-(2'-phenylphenoxy)methyl-1H-2- benzopyran;
[1R,3S]-1-Aminomethyl-3-(4'-t-butylphenoxy)methyl-3,4-dihydro-5;6-dihydroxy-IH-2-benzopyran;
[1R,3S]-1-Aminomethyl-3-(4'-bromphenoxy)methyl-3,4-dihydro-5,6-dihydroxy-1H-2- benzopyran;
[1 R,3R]-1-Aminomethyl-3-benzyl-3,4-dihydro-5,6-dihydroxy-1 H-2-benzopyran;
[1R,3R]-1-Aminomethyl-3,4-dihydro-5,6-dihydroxy-3-(2'phenyl)ethyl-1H-2-benzopyran;
[1R,3S]-1-Aminomethyl-8-brom-3,4-dihydro-5,6-dihydroxy-3-phenyl-1H-2-benzopyran;
[1 R,3R]-1-Aminomethyl-3,4-dihydro-5,6-dihydroxy-3-n-octyl-1 H-2-benzopyran;
[1R,3R]-1-Aminomethyl-3,4-dihydro-5,6-dihydroxy-3-(1'-hex-5'-en)-1 H-2-benzopyran;
[1R,3S]-1-Aminomethyl-3,4-dihydro-5,6-dihydroxy-3-ethyl-1H-2-benzopyran;
[1R,3R]-1-Aminomethyl-3,4-dihydro-5,6-dihydroxy-3-n-hexyl-1H-2-benzopyran;
[1R,3S]-1-Aminomethyl-3-(4'-bromphenyl)-3,4-dihydro-5,6-dihydroxy-1H-2-benzopyran;
[1R,3S]-1-Aminomethyl-3,4-dihydro-5,6-dihydroxy-3-(3'-hydroxyphenyl)-1H-2-benzopyran;
[1R,3S]-3-Cyclohexyl-3,4-dihydro-5,6-dihydroxy-1-(N-methyl)aminomethyl-1H-2-benzopyran;
[1R,3S]-3-t-Butyl-3,4-dihydro-5,6-dihydroxy-1-(N-methyl)aminomethyl-1H-2-benzopyran;
[1R,3S]-1-(N-Allyl)aminomethyl-3-cyclohexyl-3,4-dihydro-5,6-dihydroxy-1H-2-benzopyran;
[1R,3S]-3-Cyclohexyl-1-(N-cyclopropyl)aminomethyl-3,4-dihydro-5,6-dihydroxy-1H-2- benzopyran;
[1R,3S]-1-(N-Benzyl)aminomethyl-3-cyclohexyl-3,4-dihydro-5,6-dihydroxy-1H-2-benzopyran;
[1R,3S]-1,3-Bis(aminomethyl)-3,4-dihydro-5,6-dihydroxy-1H-2-benzopyran;
[1R,3S]-1-Aminomethyl-3,4-dihydro-5,6-dihydroxy-3-hydroxymethyl-1H-2-benzopyran;
[1R,3S]-1-Aminomethyl-3,4-dihydro-5,6-dihydroxy-3-(N-piperidinyl)methyl-1H-2-benzopyran;
[1R,3S]-5,6-Dihydroxy-3-pheny1-1-(2'R-pyrrolidinyl)-1,2,3,4-tetrahydronaphthalin;
[1R,3R]-5,6-Dihydroxy-3-pheny1-1-(2'R-pyrrolidinyl)-1,2,3,4-tetrahydronaphthalin;
5,6-Dihydroxy-1-(N-methyl)aminomethyl-3-phenyl-3,4-dihydronaphthalin;
[1R,3S]-5,6-Dihydroxy-1-(N-methyl)aminomethyl-3-phenyl-1,2,3,4-tetrahydronaphthalin;
1-Aminomethyl-5,6-dihydroxy-3-(3'-hydroxyphenyl)-3,4-dihydronaphthalin;
1-Aminomethyl-5,6-dihydroxy-3-(4'-hydroxyphenyl)-3,4-dihydronaphthalin;
[1R,3S]-1-Aminomethyl-5,6-dihydroxy-3-(3'-hydroxyphenyl)-1,2,3,4-tetrahydronaphthalin;
5,6-Bis(acetoxy)-1-(alanyl-alanyl)aminomethyl-3-phenyl-3,4-dihydronaphthalin;
5,6-Bis(acetoxy)-1-(γ(-glutamyl)aminomethyl-3-phenyl-3,4-dihydronaphthalin;
5,6-Bis(acetoxy)-1-(alanyl)aminomethyl-3-phenyl-3,4-dihydronaphthalin;
5,6-Bis(acetoxy)-1-(methionyl)aminomethyl-3-phenyl-3,4-dihydronaphthalin;
1-(Alanyl-alanyl)aminomethyl-5,6-bis(benzoyloxy)-3-phenyl-3,4-dihydronaphthalin;
[1R,2S]-1-Aminomethyl-5,6-dihydroxy-2-(2'-hydroxy-1'-ethyl)-3-phenyl-1,2,3,4-tetrahydronaphthalin;
1-Aminomethyl-5,6-dihydroxy-3-phenylnaphthalin;
[1R,3S]-1-Aminomethyl-3,4-dihydro-5,6-dihydroxy-3-(diphenyl)methyl-1H-2benzopyran;
[1R,3S]-1-Aminomethyl-3,4-dihydro-5,6-dihydroxy-3-(3'-methyl-2'-n-pentyl)-1H-2-benzopyran;
[1R,3S]-1-Aminomethyl-3;4-dihydro-5,6-dihydroxy-3-(1'-but-3'-en)-1H-2-benzopyran;
[1R,3S]-1-Aminomethyl-3,4-dihydro-5,6-dihydroxy-3-(6'-methyl-2'-hept-5'-en)-1H-2- benzopyran;
[1R,3S]-1-Aminomethyl-3-benzyloxymethyl-3,4-dihydro-5,6-dihydroxy-1H-2-benzopyran;
[1R,8S;9aR]-1-Amino-5,6-dihydroxy-2,3,7,8;9,9a-hexahydro-8-phenylphenalen;
[1S,8S,9aR]-1-Amino-5,6-dihydroxy-2,3,7,8,9,9a-hexahydro-8-phenylphenalen;
6,7-Dihydroxy-4-phenyl-2,3,4,5-tetrahydro-1H-benz[e]isoindol; und
1-Aminomethyl-5,6-bis(benzoyloxy)-3-phonyl-3,4-dihydronaphthalin oder aus einem pharmazeutisch verträglichen Salz dieser

11. Verfahren nach einem oder mehreren der Ansprüche 1-2 zur Herstellung einer Verbindung, die aus der Gruppe gewählt ist, die aus folgendem besteht:
[1R,3S]-3-(1'-Adamantyl)-1-aminomethyl-3,4-dihydro-5,6-dihydroxy-1H-2-benzopyran,
[1 R*;3S*]-3-(1'-Adamantyl)-1-aminomethyl-3,4-dihydro-5,6-dihydroxy-1H-2benzopyran;
[1 R,3S]-3-(1 '-Adamantyl)-3,4-dihydro-5,6-dihydroxy-1-(N-methyl)aminomethyl-1H-2-benzopyran;
[1R,3R]-1-Aminomethyl-3-cyclopentylmethyl-3,4-dihydro-5,6-dihydroxy-1H-2benzopyran;
[1R,3S]1-Aminomethyl-3-cyclooctyl-3,4-dihydro-5,6-dihydroxy-1H-2-benzopyran;
[1 R,3R]-1-Aminomethyl-3-n-butyl-3,4-dihydro-5,6-dihydroxy-1 H-2-benzopyran;
[1R,3R]-3-n-Butyl-3,4-dihydro-5,6-dihydroxy-1-(N-methyl)aminomethyl-1H-2-benzopyran, oder aus einem pharmazeutisch verträglichen Salz dieser.

12. Verfahren nach einem oder mehreren der Ansprüche 1-2 zur Herstellung einer Verbindung nach folgender Formel: 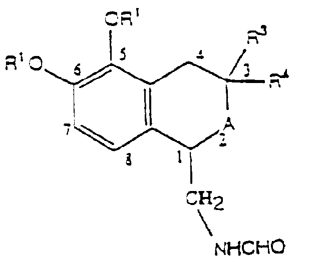 worin A gleich O oder S ist;
worin R¹ gleich einer Katechol-Schutzgruppe ist;
worin R³ aus C₁-C₁₂-Alkyl, aus substituiertem C₁-C₆-Alkyl, wie unten definiert, aus C₂-C₁₂-Alkenyl, aus substituiertem C₂-C₁₂-Alkenyl, wie unten definiert, aus C₂-C₁₂-Alkinyl, aus substituiertem C₂-C₁₂-Alkinyl, wie unten definiert, aus C₃-C₁₂-Cycloalkyl; aus substituiertem C₃-C₁₂-Cycloalkyl, wie unten definiert, aus carbocyclischem C₅-C₁₀-Aryl, aus substituiertem carbocyclischem C₅-C₁₀-Aryl, wie unten definiert, aus einem Heterocyclus, wie unten definiert, und aus einem substituierten Hetrocyclus, wie unten definiert, gewählt ist;
worin R⁴ aus Wasserstoff und aus C₁-C₁₂-Alkyl gewählt ist, oder worin R⁴ zusammen mit R³ und dem Kohlenstoffatom, an das sie gebunden sind, einen Spirocycloalkylring von 3 bis 7 Kohlenstoffatomen ausbildet;
worin substituiertes C₁-C₆-Alkyl gleich einer C₁-C₆-Alkylgruppe ist, die 1-2 Substituenten trägt, die aus Halogen, Hydroxy, C₁-C₆-Alkoxy, Amino, C₁-C₆-Alkylamino, C₃-C₁₂-Cycloalkyl, aus einem Heterocyclus; wie unten definiert, aus einem substituierten Heterocyclus, wie unten definiert, aus carbocyclischem C₅-C₁₀-Aryl, aus substituiertem carbocyclischem C₅-C₁₀-Aryl, wie unten definiert, aus carbocyclischem C₅-C₁₀-Arylox, aus substituiertem carbocyclischem C₅-C₁₀-Aryloxy worin das substituierte carbocyclische C₅-C₁₀-Aryl wie unten definiert ist, oder aus - NC(O)R¹¹ gewählt sind, worin R¹¹ aus Wasserstoff, aus C₁-C₆-Alkyl, aus Halogen-(C₁-C₆-Alkyl) , aus einem Heterocyclus, wie unten definiert, aus einem substituierten Heterocyclus, wie unten definiert, aus carbocyclischem C₅-C₁₀-Aryl, und aus substituiertem carbocyclischem C₅-C₁₀-Aryl, wie unten definiert, gewählt ist;
worin substituiertes C₂-C₁₂-Alkenyl gleich einer C₂-C₁₂-Alkenylgruppe ist, die einen Substituenten trägt, der aus C₁-C₆-Alkyl, C₃-C₁₂-Cycloalkyl, carbocyclischem C₅-C₁₀-Aryl, Benzyl, Phenylethyl, Amino, Hydroxy, C₁-C₆-Alkoxy und aus einem Heterocyclus, wie unten definiert, gewählt ist;
worin substituiertes C₂-C₁₂-Alkinyl gleich einem C₂-C₁₂-Alkinyl ist, das einen Substituenten trägt, der aus C₁-C₆-Alkyl, aus C₃-C₁₂-Cycloalkyl, carbocyclischem C₅-C₁₀-Aryl, Benzyl, Phenylethyl, Amino, Hydroxy, aus C₁-C₁₂-Alkylamino, aus C₁-C₆-Alkoxy, und aus einem Heterocyclus, wie unten definiert, gewählt ist;
worin substituiertes C₃-C₁₂-Cycloalkyl gleich einer C₃-C₁₂-Cycloalkylgruppe ist, die mit einer oder mehreren C₁-C₁₂-Alkyl-, Hydroxy-, Amino-, C₁-C₁₂-Alkylamino-, C₁-C₁₂-Aminoalkyl-, Mercapto-, C₁-C₁₂-Alkylthio- oder Halogengruppe(n) substituiert ist;
worin substituiertes carbocyclisches C₅-C₁₀-Aryl ein carbocyclisches C₅-C₁₀-Aryl ist, das durch eine oder mehrere Hydroxy-, Halogen-, C₁-C₆-Alkyl oder C₁-C₆-Alkoxygruppe(n) oder durch eine Phenylgruppe substituiert ist;
worin der Heterocyclus eine monocyclische 5- oder 6-gliedrige gesättigte oder ungesättigte Gruppe ist, die ein oder zwei Ringheteroatome enthält, die aus N, O und S gewählt sind, wobei die verbleibenden Atome Kohlenstoff sind, wie zum Beispiel Furyl, Tetrahydrofuryl, Thienyl, Pyrryl, Pyrrolidyl, Pyridyl, Piperidyl, Pyrazinyl, Pyrimidyl, Piperazinyl, Morpholinyl, Thiomorpholinyl, Pyrazolyl, Imidazolyl, Oxazolyl, Oxazolidyl, Isoxazolyl, Isoxazolidyl und Thiazolyl; and
worin der substituierte Heterocyclus ein Heterocyclus ist, wie oben definiert, der mit Halogen, C₁-C₆-Alkyl oder C₁-C₆-Alkoxy substituiert ist.

13. Verfahren zur Herstellung einer Verbindung nach folgender Formel 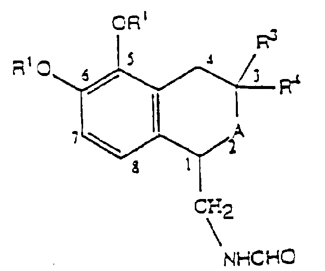 worin A gleich O oder S ist;
worin R¹ gleich einer Katechol-Schutzgruppe ist;
worin R³ aus C₁-C₁₂-Alkyl, aus substituiertem C₁-C₆-Alkyl, wie unten definiert, aus C₂-C₁₂-Alkenyl, aus substituiertem C₂-C₁₂-Alkenyl, wie unten definiert, aus C₂-C₁₂-Alkinyl, aus substituiertem C₂-C₁₂-Alkinyl, wie unten definiert, aus C₃-C₁₂-Cycloalkyl, aus substituiertem C₃-C₁₂-Cycloalkyl, wie unten definiert, aus carbocyclischem C₅-Cₗₒ-Aryl, aus substituiertem C₅-Cₗₒ-Aryl, wie unten definiert, aus einem Heterocyclus, wie unten definiert, und aus einem substituierten Hetrocyclus, wie unten definiert, gewählt ist;
worin R⁴ aus Wasserstoff und aus C₁-C₁₂-Alkyl gewählt ist, oder worin R⁴ zusammen mit R³ und dem Kohlenstoffatom, an das sie gebunden sind, einen Spirocycloalkylring von 3 bis 7 Kohlenstoffatomen ausbildet;
worin substituiertes C₁-C₆-Alkyl gleich einer C₁-C₆-Alkylgruppe ist, die 1-2 Substituenten trägt, die aus Halogen, Hydroxy, C₁-C₆-Alkoxy, Amino, C₁-C₆-Alkylamino, C₃-C₁₂-Cycloalkyl, aus einem Heterocyclus, wie unten definiert, aus einem substituierten Heterocyclus, wie unten definiert, aus carbocyclischem C₅-Cₗₒ-Aryl, aus substituiertem carbocyclischem C₅-C₁₀-Aryl, wie unten definiert, aus carbocyclischem C₅-C₁₀-Aryloxy, aus substituiertem carbocyclischem C₅-C₁₀-Aryloxy, worin das substituierte carbocyclische C₅-C₁₀-Aryl wie unten definiert ist, oder aus - NC(O)R¹¹ gewählt sind, worin R¹¹ aus Wasserstoff, aus C₁-C₆-Alkyl, aus Halogen-(C₁-C₆-Alkyl), aus einem Heterocyclus, wie unten definiert, aus einem substituierten Heterocyclus, wie unten definiert, aus carbocyclischem C₅-C₁₀-Aryl, und aus substituiertem carbocyclischem C₅-C₁₀-Aryl, wie unten definiert, gewählt ist;
worin substituiertes C₂-C₁₂-Alkenyl gleich einer C₂-C₁₂-Alkenylgruppe ist, die einen Substituenten trägt, der aus C₁-C₆-Alkyl, C₃-C₁₂-Cycloalkyl, carbocyclischem C₅-C₁₀-Aryl, Benzyl, Phenylethyl, Amino, Hydroxy, C₁-C₆-Alkox und aus einem Heterocyclus, wie unten definiert, gewählt ist;
worin substituiertes C₂-C₁₂-Alkinyl gleich einem C₂-C₁₂-Alkinyl ist, das einen Substituenten trägt, der aus C₁-C₆-Alkyl, aus C₃-C₁₂-Cycloalkyl, carbocyclischem C₅-C₁₀)-Aryl, Benzyl, Phenylethyl, Amino, Hydroxy, aus C₁-C₁₂-Alkylamino, aus C₁-C₆-Alkoxy, und aus einem Heterocyclus, wie unten definiert, gewählt ist;
worin substituiertes C₃-C₁₂-Cycloalkyl gleich einer C₃-C₁₂-Cycloalkylgruppe ist, die mit einer oder mehreren C₁-C₁₂-Alkyl-, Hydroxy-, Amino-, C₁-C₁₂-Alkylamino-, C₁-C₁₂-Aminoalkyl-, Mercapto-, C₁-C₁₂-Alkylthio- oder Halogengruppe(n) substituiert ist;
worin substituiertes carbocyclisches C₅-C₁₀-Aryl ein carbocyclisches C₅-C₁₀-Aryl ist, das durch eine oder mehrere Hydroxy-, Halogen-, C₁-C₆-Alkyl oder C₁-C₆-Alkoxygruppe(n) oder durch eine Phenylgruppe substituiert ist;
worin der Heterocyclus eine monocyclische 5- oder 6-gliedrige gesättigte oder ungesättigte Gruppe ist, die ein oder zwei Ringheteroatome enthält, die aus N, O und S gewählt sind, wobei die verbleibenden Atome Kohlenstoff sind, wie zum Beispiel Furyl, Tetrahydrofuryl, Thienyl, Pyrryl, Pyrrolidyl, Pyridyl, Piperidyl, Pyrazinyl, Pyrimidyl, Piperazinyl, Morpholinyl, Thiomorpholinyl, Pyrazolyl, Imidazolyl, Oxazolyl, Oxazolidyl, Isoxazolyl, Isoxazolidyl und Thiazolyl; and
worin der substituierte Heterocyclus ein Heterocyclus ist, wie oben definiert, der mit Halogen, C₁-C₆-Alkyl oder C₁-C₆-Alkoxy substituiert ist; wobei das Verfahren die Umsetzung einer Verbindung nach folgender Formel: 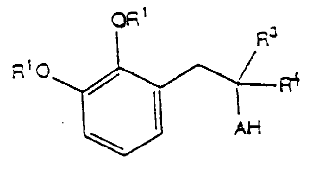
worin R¹, R³ und R⁴ die hierin definierte Bedeutung haben, mit N-Formylaminoacetaldehyddimethylacetal oder mit 3-(N-Formylamino)propionaldehyddimethylacetal in Gegenwart eines sauren Katalysators umfaßt.

14. Verfahren nach Anspruch 13, worin der Katalysator aus Bortrifluoridetherat, Zinktriflat, Trimethylsilyltriflat und Methansulfonsäure ausgewählt ist.

## Revendications (Revendications pour l'(les) Etat(s) contractant(s) suivant(s) : AT, BE, CH, LI, DE, DK, FR, GB, IT, LU, NL, SE)

1. Composé présentant la formule : 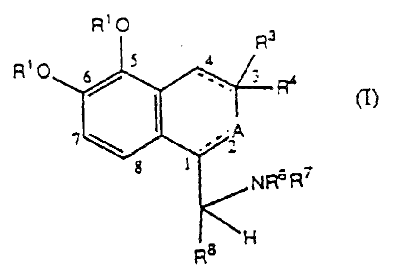 ou sel, ester ou amide pharmaceutiquement acceptable de celui-ci, dans lequel A est O, S, CHR², CR² ou C lorsque A et R⁶ pris ensemble forment un cycle azoté contenant 5, 6 ou 7 chaînons ;
les pointillés représentent des doubles liaisons éventuelles;
R¹ est choisi parmi l'hydrogène et un groupe qui est aisément clivable in vivo ;
R² est choisi parmi l'hydrogène, alkyle en C₁-C₁₂ et alkyle en C₁-C₆ substitué;
R³ est choisi parmi alkyle en C₁-C_{12'} alkyle en C₁-C₆ substitué comme défini ci-dessous, alcényle en C₂-C₁₂, alcényle en C₂-Cᵢ₂ substitué comme défini ci-dessous, alcynyle en C₂-Cᵢ₂, alcynyle en C₂-Cᵢ₂ substitué comme défini ci-dessous, cycloalkyle en C₃-C₁₂, cycloalkyle en C₃-C₁₂ substitué comme défini ci-dessous, aryle carbocyclique en C₅-C₁₀, aryle carbocyclique en C₅-C₁₀ substitué comme défini ci-dessous, hétérocycle comme défini ci-dessous et hétérocycle substitué comme défini ci-dessous;
R⁴ est choisi parmi l'hydrogène et alkyle en C₁-C₁₂ ou, pris ensemble avec R³ et l'atome de carbone auquel ils sont liés, forme un cycle spirocycloalkyle de 3 à 7 atomes de carbone ;
R⁶ est choisi parmi l'hydrogène, alkyle en C₁-C₁₂, alkyle en C₁-C₆ substitué comme défini ci-dessous, alcényle en C₂-C₁₂, alcényle en C₂-C₁₂ substitué comme défini ci-dessous, alcynyle en C₂-C₁₂, alcynyle en C₂-C₁₂ substitué comme défini ci-dessous, cycloalkyle en C₃-C₁₂, cycloalkyle en C₃-C₁₂ substitué comme défini ci-dessous, alcanoyle de 1 à 8 atomes de carbone, acide aminé naturel et dipeptide formé d'acides aminés naturels ou, pris ensemble avec A lorsque A est C, forme un cycle azoté à 5, 6 ou 7 chaînons ;
R⁷ est l'hydrogène ou alkyle en C₁-C₁₂ ou, pris ensemble avec R⁶ ou R⁸, forme un cycle azoté à 5, 6 ou 7 chaînons, à condition que, lorsque R⁶ est arylalkyle carbocyclique, R⁷ ne soit pas alkyle ;
R⁸ est l'hydrogène ou alkyle en C₁-C₁₂ ou, pris ensemble avec R⁶ ou R⁷, forme un cycle azoté à 5, 6 ou 7 chaînons ou pris ensemble avec le cycle catéchol en position 8 et les atomes de carbone auxquels ils sont liés forme un cycle à 5, 6 ou 7 chaînons ;
dans lequel alkyle en C₁-C₆ substitué est un groupe alkyle en C₁-C₆ portant 1-2 substituants choisis parmi halogène, hydroxyle, alcoxy en C₁-C₆, amino alkylamino en C₁-C₆, cycloalkyle en C₃-C₁₂, hétérocycle comme défini ci-dessous, hétérocycle substitué comme défini ci-dessous, aryle carbocyclique en C₅-C₁₀, aryle carbocyclique en C₅-C₁₀ substitué comme défini ci-dessous, aryloxy carbocyclique en C₅-C₁₀, aryloxy carbocyclique en C₅-C₁₀ substitué dans lequel aryle carbocyclique en C₅-C₁₀ substitué et comme défini ci-dessous, ou -NC (O)R¹¹ où R¹¹ est choisi parmi l'hydrogène, alkyle en C₁-C₆, halogéno-(alkyle en C₁-C₆), hétérocycle comme défini ci-dessous, hétérocycle substitué comme défini ci-dessous, aryle carbocyclique en C₅-C₁₀ et aryle carbocyclique en C₅-C₁₀ substitué comme défini ci-dessous;
dans lequel alcényle en C₂-C₁₂ substitué est un groupe alcényle en C₂-C₁₂ portant un substituant choisi parmi alkyle en C₁-C₆, cycloalkyle en C₃-C₁₂, aryle carbocyclique en C₅-C₁₀, benzyle, phényléthyle, amino, hydroxyle, alcoxy en Ci -C₆ et hétérocycle comme défini ci-dessous;
dans lequel alcynyle en C₂-C₁₂ substitué est un alcynyle en C₂-C₁₂ portant un substituant choisi parmi alkyle en C₁-C₆, cycloalkyle en C₃-C₁₂, aryle carbocyclique en C₅-C₁₀, benzyle, phényléthyle, amino, hydroxyle, alkylamino en C₁-C₁₂, alcoxy en C₁-C₆ et hétérocycle comme défini ci-dessous;
dans lequel cycloalkyle en C₃-C₁₂ substitué est un groupe cycloalkyle en C₃-C₁₂ substitué par un ou plusieurs groupes alkyles en C₁-C₁₂, hydroxyle, amino, alkylamino en C₁-C₁₂, aminoalkyle en C₁-C₁₂, mercapto, alkylthio en C₁-C₁₂ ou halogène,
dans lequel aryle carbocyclique en C₅-C₁₀ substitué est un aryle carbocyclique en C₅-C₁₀ substitué par un ou plusieurs groupes hydroxyle, halogène, alkyle en C₁-C₆ ou alcoxy en C₁-C₆ ou par un groupe phényle ;
dans lequel hétérocycle est un groupe monocyclique à 5 ou 6 chaînons, saturé ou insaturé contenant 1 ou 2 hétéroatomes cycliques choisis parmi N, O et S, les atomes restants étant des atomes de carbone, comme par exemple furyle, tétrahydrofuryle, thiényle, pyrryle, pyrrolidyle, pyridyle, pipéridyle, pyrazinyle, pyrimidyle, pipé- razinyle, morpholinyle, thiomorpholinyle, pyrazolyle, imidazolyle, oxazolyle, oxazolidyle, isoxazolyle, isoxazo- lidyle et thiazolyle ; et
dans lequel hétérocycle substitué est un hétérocycle comme défini ci-dessus substitué par halogène, alkyle en C₁-C₆ ou alcoxy en C₁-C₆.

2. Composé selon la revendication 1, dans lequel A est CR² et les pointillés entre A et l'atome cyclique numéro 1 représentent une liaison, présentant la formule: 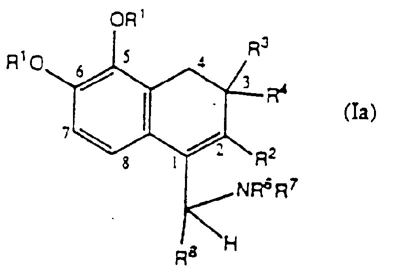 dans laquelle R¹, R², R³, R⁴, R⁶, R⁷ et R⁸ sont tels que définis dans la revendication 1.

3. Composé selon la revendication 1, dans lequel A est CHR², présentant la formule: 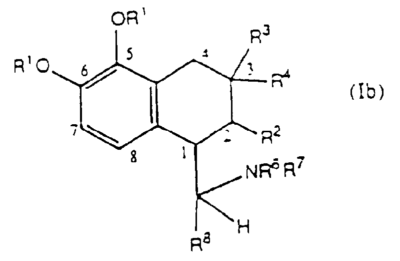 dans laquelle R¹, R², R³, R⁴, R⁶, R⁷ et R⁸ sont tels que définis dans la revendication 1.

4. Composé selon la revendication 1, dans lequel A est O.

5. Composé selon la revendication 1, dans lequel n est 0 et R⁶ et A pris ensemble forment un cycle azoté contenant 5, 6 ou 7 chaînons présentant la formule : 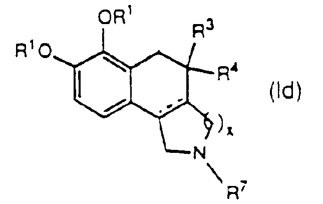 dans laquelle x est 1, 2 ou 3 et R¹, R³, R⁴ et R⁷ sont tels que définis dans la revendication 1.

6. Composé selon la revendication 1, dans lequel A est CHR², R² est H et R⁸ pris ensemble avec la position 8 du cycle catéchol forme un cycle à 5, 6 ou 7 chaînons, présentant la formule : 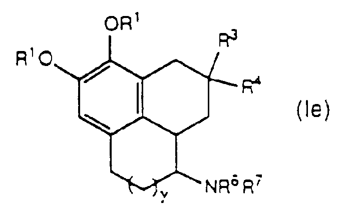 dans laquelle y est 0, 1 ou 2, R¹ et R³, R⁴, R⁶ et R⁷ sont tels que définis dans la revendication 1.

7. Composé selon la revendication 1, dans lequel R³ est alkyle en C₁-C₁₂, alkyle en C₁-C₆ substitué, cycloalkyle en C₃-C₁₂, cycloalkyle en C₃-C₁₂ substitué, aryle carbocyclique en C₅-C₁₀ ou aryle carbocyclique en C₅-C₁₀ substitué et R⁴ est l'hydrogène.

8. Composé selon la revendication 1, dans lequel R⁷ et R⁸ sont l'hydrogène.

9. Composé selon la revendication 1, choisi dans le groupe consistant en
1-aminométhyl-5,6-dihydroxy-3-phényl-3,4-dihydronaphtalène;
1-aminométhyl-5,6-bis(acétoxy)-3-phényl-3,4-dihydronaphtalène;
1-aminométhyl-5,6-bis(triméthylacétoxy)-3-phényl-3,4-dihydronaphtalène;
[1R,3S]1-aminométhyl-5,6-dihydroxy-3-phényl-1,2,3,4-tétrahydronaphtalène;
1-aminométhyl-3-cyclohexyl-5,6-dihydroxy-3,4-dihydronaphtalène;
[1R,3S]1-aminométhyl-3-cyclohexyl-5,6-dihydroxy-1,2,3,4-tétrahydronaphtalène;
[1R,3S]1-aminométhyl-3-t-butyl-3,4-dihydro-5,6-dihydroxy-1H-2-benzopyrane;
[1R,3S]1-aminométhyl-3,4-dihydro-5,6-dihydroxy-3-phényl-1 H-2-benzopyrane;
[1R,3S]1 -aminométhyl-3-cyclohexyl-3,4-dihydro-5,6-dihydroxy-1H-2-benzopyrane;
[1R,3R]1-aminométhyl-3,4-dihydro-5,6-dihydroxy-3-éthyl-1H-2-benzopyrane;
Spiro[(1-aminométhyl-3,4-dihydro-5,6-dihydroxy-1H-2-benzopyrane)-3,1'-cyclohexane];
[1R,3S]1-aminométhyl-3,4-dihydro-5,6-dihydroxy-3-(4'méthoxyphénoxy)-méthyl-1H-2-benzopyrane;
[1R,3S]1-aminométhyl-3,4-dihydro-5,6-dihydroxy-3-phényl-1 H-2-benzopyrane;
[1R,3S]1-aminométhyl-3,4-dihydro-5,6-dihydroxy-3-phénoxyméthyl-1H-2-benzopyrane;
[1R,3S]1-aminométhyl-3,4-dihydro-5,6-dihydroxy-3-(2'-phénylphénoxy)-méthyl-1H-2-benzopyrane;
[1R,3S]1-aminométhyl-3-(4'-t-butylphénoxy)méthyl-3,4-dihydro-5,6-dihydroxy-1H-2-benzopyrane;
[1R,3S]1-aminométhyl-3-(4'-bromophénoxy)méthyl-3,4-dihydro-5,6-dihydroxy-1H-2-benzopyrane;
[1 R,3R] 1-aminométhyl-3-benzyl-3,4-dihydro-5,6-dihydroxy-1 H-2-benzopyrane;
[1R,3R]1-aminométhyl-3,4-dihydro-5,6-dihydroxy-3-(2'-phényl)éthyl-1 H-2-benzopyrane;
[1R,3S]1-aminométhyl-8-bromo-3,4-dihydro-5,6-dihydroxy-3-phényl-1 H-2-benzopyrane;
[1R,3R] 1-aminométhyl-3,4-dihydro-5,6-dihydroxy-3-n-octyl-1H-2-benzopyrane;
[1R,3R]1-aminométhyl-3,4-dihydro-5,6-dihydroxy-3-(1'-hex-5'-ène)-1H-2-benzopyrane;
[1R,3S]1-aminométhyl-3,4-dihydro-5,6-dihydroxy-3-éthyl-1H-2-benzopyrane;
[1R,3R]1-aminométhyl-3,4-dihydro-5,6-dihydroxy-3-n-hexyl-1H-2-benzopyrane;
[1R,3S]1-aminométhyl-3-(4'-bromophényl)-3,4-dihydro-5,6-dihydroxy-1H-2-benzopyrane;
[1R,3S]1-aminométhyl-3,4-dihydro-5,6-dihydroxy-3-(3'-hydroxyphényl)-1H-2-benzopyrane;
[1R,3S]3-cyclohexyl-3,4-dihydro-5,6-dihydroxy-1-(N-méthyl)aminométhyl-1H-2-benzopyrane;
[1R,3S]3-t-butyl-3,4-dihydro-5,6-dihydroxy-1-(N-méthyl)aminométhyl-1H-2-benzopyrane;
[1R,3S]1-(N-allyl)aminométhyl-3-cyclohexyl-3,4-dihydro-5,6-dihydroxy-1H-2-benzopyrane;
[1R,3S]3-cyclohexyl-1-(N-cyclopropyl)aminométhyl-3,4-dihydro-5,6-dihydroxy-1H-2-benzopyrane;
[1R,3S]1-(N-benzyl)aminométhyl-3-cyclohexyl-3,4-dihydro-5,6-dihydroxy-1H-2-benzopyrane;
[1R,3S]1,3-bis(aminométhyl)-3,4-dihydro-5,6-dihydroxy-1 H-2-benzopyrane;
[1R,3S]1-aminométhyl-3,4-dihydro-5,6-dihydroxy-3-hydroxyméthyl-1H-2-benzopyrane;
[1R,3S]1-aminométhyl-3,4-dihydro-5,6-dihydroxy-3-(N-pipéridinyl)methyl-1H-2-benzopyrane;
[1R,3S]5,6-dihydroxy-3-phényl-1-(2'R-pyrrolidinyl)-1,2,3,4-tétrahydronaphtalène;
[1 R,3R] 5,6-dihydroxy-3-phényl-1-(2'-R-pyrrolidinyl)-1,2,3,4-tétrahydronaphtalène;
5,6-dihydroxy-1-(N-méthyl)aminométhyl-3-phényl-3,4-dihydronaphtalène;
[1R,3S]5,6-dihydroxy-1-(N-méthyl)aminométhyl-3-phényl-1,2,3,4-tétrahydronaphtalène;
1-aminométhyl-5,6-dihydroxy-3-(3'-hydroxyphényl)-3,4-dihydronaphtalène;
1-aminométhyl-5,6-dihydroxy-3-(4'-hydroxyphényl)-3,4-dihydronaphtalène;
[1R,3S] 1-aminométhyl-5,6-dihydroxy-3-(3'-hydroxyphényl)-1,2,3,4-tétrahydronaphtalène;
5,6-bis(acétoxy)-1-(alanyl-alanyl)aminométhyl-3-phényl-3,4-dihydronaphtalène;
5,6-bis(acétoxy)-1-(γ-glutamyl)aminométhyl-3-phényl-3,4-dihydronaphtalène;
5,6-bis(acétoxy)-1-(alanyl)aminométhyl-3-phényl-3,4-dihydronaphtalène;
5,6-bis(acétoxy)-1-(méthionyl)aminométhyl-3-phényl-3,4-dihydronaphtalène;
1-(alanyl-alanyl)aminométhyl-5,6-bis(benzoyloxy)-3-phényl-3,4-dihydronaphtalène;
[1R,2S] 1-aminométhyl-5,6-dihydroxy-2-(2'-hydroxy-1'-éthyl)-3-phényl-1,2,3,4-tétrahydronaphtalène;
1-aminométhyl-5,6-dihydroxy-3-phénylnaphtalène;
[1R,3S] 1-aminométhyl-3,4-dihydro-5,6-dihydroxy-3-(diphényl)méthyl-1H-2-benzopyrane;
[1R,3S]1-aminométhyl-3,4-dihydro-5,6-dihydroxy-3-(3'-méthyl-2'-n-pentyl)-1H-2-benzopyrane;
[1R,3S]1-aminométhyl-3,4-dihydro-5,6-dihydroxy-3-(1'-but-3'-ène)-1H-2-benzopyrane;
[1R,3S]1-aminométhyl-3,4-dihydro-5,6-dihydroxy-3-(6'-méthyl-2'-hept-5'-ene)-1 H-2-benzopyrane ;
[1R,3S]1-aminométhyl-3-benzyloxyméthyl-3,4-dihydro-5,6-dihydroxy-1H-2-benzopyrane;
[1R, 8S, 9aR] 1-amino-5,6-dihydroxy-2,3,7,8,9,9a-hexahydro-8-phénylphénalène;
[1S, 8S 9aR] 1-amino-5,6-dihydroxy-2,3,7,8,9,9a-hexahydro-8-phénylphénalène;
6,7-dihydroxy-4-phényl-2,3,4,5-tétrahydro-1H-benzo[e]isoindole; et
1-aminométhyl-5,6-bis(benzoyloxy)-3-phényl-3,4-dihydronaphtalène ou sel pharmaceutiquement acceptable de celui-ci.

10. Composé choisi dans le groupe consistant en :
[1 R,3S] 3-(1'-adamantyl)-1-aminométhyl-3,4-dihydro-5,6-dihydroxy-1H-2-benzopyrane;
[1R^{*},3S^{*}]3-(1'-adamantyl)- 1-aminométhyl-3,4-dihydro-5,6-dihydroxy-1H-2-benzopyrane;
[1 R,3S] 3-(1'-adamantyl)-3,4-dihydro-5,6-dihydroxy-1-(N-méthyl)aminométhyl-1H-2-benzopyrane;
[1R,3R]1-aminométhyl-3-cyclopentylméthyl-3,4-dihydro-5,6-dihydroxy-1H-2-benzopyrane;
[1R,3S]1-aminométhyl-3-cyclooctyl-3,4-dihydro-5,6-dihydroxy-1 H-2-benzopyrane;
[1R,3R]1-aminométhyl-3-n-butyl-3,4-dihydro-5,6-dihydroxy-1H-2-benzopyrane;
[1 R,3R] 3-n-butyl-3,4-dihydro-5,6-dihydroxy-1-(N-méthyl)aminométhyl-1H-2-benzopyrane,
ou un sel pharmaceutiquement acceptable de celui-ci.

11. Composition pharmaceutique pour agir sélectivement sur les récepteurs dopaminergiques comprenant un support pharmaceutiquement acceptable et une quantité thérapeutiquement efficace d'un composé selon la revendication 1.

12. Composition pharmaceutique pour traiter les troubles neurologiques liés à la dopamine comprenant un support pharmaceutiquement acceptable et une quantité thérapeutiquement efficace d'un composé selon la revendication 1.

13. Composition pharmaceutique pour traiter les troubles psychologiques liés à la dopamine comprenant un support pharmaceutiquement acceptable et une quantité thérapeutiquement efficace d'un composé selon la revendication 1.

14. Composition pharmaceutique pour traiter les troubles cardiovasculaires liés à la dopamine comprenant un support pharmaceutiquement acceptable et une quantité thérapeutiquement efficace d'un composé selon la revendication 1.

15. Composition pharmaceutique pour traiter l'usage de drogues et les troubles du comportement entraînant une accoutumance comprenant un support pharmaceutiquement acceptable et une quantité thérapeutiquement efficace d'un composé selon la revendication 1.

16. Composition pharmaceutique pour traiter les troubles cognitifs et de l'attention comprenant un support pharmaceutiquement acceptable et une quantité thérapeutiquement efficace d'un composé selon la revendication 1.

17. Composé selon la revendication 1, destiné à être utilisé comme agent thérapeutique.

18. Utilisation d'un composé selon la revendication 1 pour fabriquer un médicament pour agir sélectivement sur les récepteurs dopaminergiques chez un patient nécessitant un tel traitement.

19. Utilisation d'un composé selon la revendication 1 pour fabriquer un médicament pour traiter les troubles neurologiques liés à la dopamine caractérisés par une activité dopaminergique anormale chez un patient nécessitant un tel traitement.

20. Utilisation d'un composé selon la revendication 1 pour fabriquer un médicament pour traiter les troubles psychologiques liés à la dopamine caractérisés par une activité dopaminergique anormale chez un patient nécessitant un tel traitement.

21. Utilisation d'un composé selon la revendication 1 pour fabriquer un médicament pour traiter les troubles cardiovasculaires liés à la dopamine chez un patient nécessitant un tel traitement.

22. Utilisation d'un composé selon la revendication 1, pour fabriquer un médicament pour traiter les troubles du comportement entraînant à une accoutumance chez un patient nécessitant un tel traitement.

23. Utilisation d'un composé selon la revendication 1, pour fabriquer un médicament pour traiter les troubles cognitifs et de l'attention chez un patient nécessitant un tel traitement.

24. Composé présentant la formule : 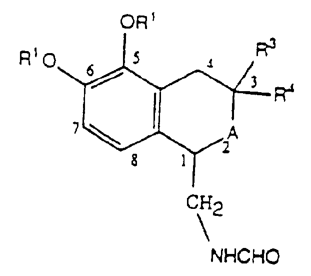 dans laquelle A est O ou S;
R¹ est un groupe protecteur de catéchol ;
R³ est choisi parmi alkyle en C₁-C₁₂, alkyle en C₁-C₆ substitué comme défini ci-dessous, alcényle en C₂-Cᵢ₂, alcényle en C₂-C₁₂ substitué comme défini ci-dessous, alcynyle en C₂-C₁₂, alcynyle en C₂-C₁₂ substitué comme défini ci-dessous, cycloalkyle en C₃-C₁₂, cycloalkyle en C₃-C₁₂ substitué comme défini ci-dessous, aryle carbocyclique en C₅-C₁₀, aryle carbocyclique en C₅-C₁₀ substitué comme défini ci-dessous, hétérocycle comme défini ci-dessous et hétérocycle substitué comme défini ci-dessous ;
R⁴ est choisi parmi l'hydrogène et alkyle en C₁-C₁₂ ou, pris ensemble avec R³ et l'atome de carbone auquel ils sont liés, forme un cycle spirocycloalkyle de 3 à 7 atomes de carbone ;
dans lequel alkyle en C₁-C₆ substitué est un groupe alkyle en C₁-C₆ portant 1-2 substituants choisis parmi halogène, hydroxyle, alcoxy en C₁-C₆, amino alkylamino en C₁-C₆, cycloalkyle en C₃-C₁₂, hétérocycle comme défini ci-dessous, hétérocycle substitué comme défini ci-dessous, aryle carbocyclique en C₅-C₁₀, aryle carbocyclique en C₅-C₁₀ substitué comme défini ci-dessous, aryloxy carbocyclique en C₅-C₁₀, aryloxy carbocyclique en C₅-C₁₀ substitué dans lequel aryle carbocyclique en C₅-C₁₀ substitué est comme défini ci-dessous, ou -NC (O)R¹¹ où R¹¹ est choisi parmi l'hydrogène, alkyle en C₁-C₆, halogéno-(alkyle en C₁-C₆), hétérocycle comme défini ci-dessous, hétérocycle substitué comme défini ci-dessous, aryle carbocyclique en C₅-C₁₀, et aryle carbocyclique en C₅-C₁₀ substitué comme défini ci-dessous ;
dans lequel alcényle en C₂-C₁₂ substitué est un groupe alcényle en C₂-C₁₂ portant un substituant choisi parmi alkyle en C₁-C₆; cycloalkyle en C₃-C₁₂, aryle carbocyclique en C₅-C₁₀, benzyle, phényléthyle, amino, hydroxyle, alcoxy en C₁-C₆ et hétérocycle comme défini ci-dessous,
dans lequel alcynyle en C₂-C₁₂ substitué est un alcynyle en C₂-C₇₂ portant un substituant choisi parmi alkyle en C₁-C₆, cycloalkyle en C₃-C₁₂, aryle carbocyclique en C₅-C₁₀, benzyle, phényléthyle, amino, hydroxyle, alkylamino en C₁-C₁₂, alcoxy en C₁-C₆ et hétérocycle comme défini ci-dessous ;
dans lequel cycloalkyle en C₃-C₁₂ substitué est un groupe cycloalkyle en C₃-C₁₂ substitué par un ou plusieurs groupes alkyles en C₁-C₁₂, hydroxyle, amino, alkylamino en C₁-C₁₂, aminoalkyle en C₁-C₁₂, mercapto, alkylthio en C₁-C₁₂ ou halogène,
dans lequel aryle carbocyclique en C₅-C₁₀ substitué est un aryle carbocyclique en C₅-C₁₀ substitué par un ou plusieurs groupes hydroxyle, halogène, alkyle en C₁-C₆ ou alcoxy en C₁-C₆ ou par un groupe phényle ;
dans lequel hétérocycle est un groupe monocyclique à 5 ou 6 chaînons, saturé ou insaturé, contenant 1 ou 2 hétéroatomes cycliques choisis parmi N, O et S, les atomes restants étant des atomes de carbone, comme par exemple furyle, tétrahydrofuryle, thiényle, pyrryle, pyrrolidyle, pyridyle, pipéridyle, pyrazinyle, pyrimidyle, pipé- razinyle, morpholinyle, thiomorpholinyle, pyrazolyle, imidazolyle, oxazolyle, oxazolidyle, isoxazolyle, isoxazo- lydyle et thiazolyle ; et
dans lequel hétérocycle substitué est un hétérocycle comme défini ci-dessus substitué par halogène, alkyle en C₁-C₆ ou alcoxy en C₁-C₆.

25. Procédé pour préparer un composé selon la revendication 24, comprenant : la réaction d'un composé présentant la formule : 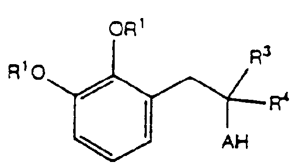
dans laquelle R¹, R³ et R⁴ sont tels que définis ici,
avec l'acétal diméthylique du N-formylaminoacétaldéhyde ou avec l'acétal diméthylique du 3-(N-formylamino) -propionaldéhyde en présence d'un catalyseur acide.

26. Procédé selon la revendication 24, dans lequel le catalyseur est choisi parmi l'éthérate de trifluorure de bore, le triflate de zinc, le triflate de triméthylsilyle et l'acide méthanesulfonique.

## Revendications (Revendications pour l'(les) Etat(s) contractant(s) suivant(s) : ES)

1. Procédé pour préparer un composé présentant la formule : 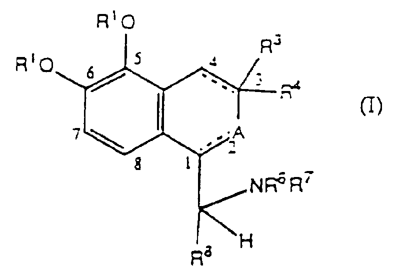 ou sel, ester ou amide pharmaceutiquement acceptable de celui-ci, dans lequel A est O, S, CHR², CR² ou C lorsque A et R⁶ pris ensemble forment un cycle azoté contenant 5, 6 ou 7 chaînons ;
les pointillés représentent des doubles liaisons éventuelles ;
R¹ est choisi parmi l'hydrogène et un groupe qui est aisément clivable in vivo ;
R² est choisi parmi l'hydrogène, alkyle en C₁-C₁₂ et alkyle en C₁-C₆ substitué ;
R³ est choisi parmi alkyle en C₁-C₁₂, alkyle en C₁-C₆ substitué comme défini ci-dessous, alcényle en C₂-C₁₂, alcényle en C₂-C₁₂ substitué comme défini ci-dessous, alcynyle en C₂-C₁₂, alcynyle en C₂-C₁₂ substitué comme défini ci-dessous, cycloalkyle en C₃-C₁₂, cycloalkyle en C₃-C₇₂ substitué comme défini ci-dessous, aryle carbocyclique en C₅-C₁₀, aryle carbocyclique en C₅-C₁₀ substitué comme défini ci-dessous, hétérocycle comme défini ci-dessous et hétérocycle substitué comme défini ci-dessous ;
R⁴ est choisi parmi l'hydrogène et alkyle en C₁-C₁₂ ou, pris ensemble avec R³ et l'atome de carbone auquel ils sont liés, forme un cycle spirocycloalkyle de 3 à 7 atomes de carbone ;
R⁶ est choisi parmi l'hydrogène, alkyle en C₁-C₁₂, alkyle en C₁-C₆ substitué comme défini ci-dessous, alcényle en C₂-C₁₂, alcényle en C₂-C₁₂ substitué comme défini ci-dessous, alcynyle en C₂-C₁₂, alcynyle en C₂-C₁₂ substitué comme défini ci-dessous, cycloalkyle en C₃-Cᵢ₂, cycloalkyle en C₃-C₁₂ substitué comme défini ci-des-
sous, alcanoyle de 1 à 8 atomes de carbone, acide aminé naturel et dipeptide formé d'acides aminés naturels ou, pris ensemble avec A lorsque A est C, forme un cycle azoté à 5, 6 ou 7 chaînons ;
R⁷ est l'hydrogène ou alkyle en C₁-C₁₂ ou, pris ensemble avec R⁶ ou R⁸, forme un cycle azoté à 5, 6 ou 7 chaînons, à condition que, lorsque R⁶ est arylalkyle carbocyclique, R⁷ ne soit pas alkyle ;
R⁸ est l'hydrogène ou alkyle en C₁-C₁₂ ou, pris ensemble avec R⁶ ou R⁷, forme un cycle azoté à 5, 6 ou 7 chaînons ou pris ensemble avec le cycle catéchol en position 8 et les atomes de carbone auxquels ils sont liés forme un cycle à 5, 6 ou 7 chaînons ;
dans lequel alkyle en C₁-C₆ substitué est un groupe alkyle en C₁-C₆ portant 1-2 substituants choisis parmi halogène, hydroxyle, alcoxy en C₁-C₆, amino alkylamino en C₁-C₆, cycloalkyle en C₃-C₁₂, hétérocycle comme défini ci-dessous, hétérocycle substitué comme défini ci-dessous, aryle carbocyclique en C₅-C₁₀, aryle carbocyclique en C₅-C₁₀ substitué comme défini ci-dessous, aryloxy carbocyclique en C₅-C₁₀, aryloxy carbocyclique en C₅-C₁₀ substitué dans lequel aryle carbocyclique en C₅-C₁₀ substitué et comme défini ci-dessous, ou -NC (O)R¹¹ où R¹¹ est choisi parmi l'hydrogène, alkyle en C₁-C₆, halogéno-(alkyle en C₁-C₆), hétérocycle comme défini ci-dessous, hétérocycle substitué comme défini ci-dessous, aryle carbocyclique en C₅-C₁₀ et aryle carbocyclique en C₅-C₁₀ substitué comme défini ci-dessous;
dans lequel alcényle en C₂-Cᵢ₂ substitué est un groupe alcényle en C₂-Cᵢ₂ portant un substituant choisi parmi alkyle en C₁-C₆, cycloalkyle en C₃-C₁₂, aryle carbocyclique en C₅-C₁₀, benzyle, phényléthyle, amino, hydroxyle, alcoxy en C₁ -C₆ et hétérocycle comme défini ci-dessous ;
dans lequel alcynyle en C₂-Cᵢ₂ substitué est un alcynyle en C₂-C₁₂ portant un substituant choisi parmi alkyle en C₁-C₆, cycloalkyle en C₃-C₁₂, aryle carbocyclique en C₅-C₁₀, benzyle, phényléthyle, amino, hydroxyle, alkylamino en C₁-C₁₂, alcoxy en C₁-C₆ et hétérocycle comme défini ci-dessous ;
dans lequel cycloalkyle en C₃-Cᵢ₂ substitué est un groupe cycloalkyle en C₃-C₁₂ substitué par un ou plusieurs groupes alkyles en C₁-C₁₂, hydroxyle, amino, alkylamino en C₁-C₁₂, aminoalkyle en C₁-C₁₂, mercapto, alkylthio en C₁-C₁₂ ou halogène,
dans lequel aryle carbocyclique en C₅-C₁₀ substitué est un aryle carbocyclique en C₅-C₁₀ substitué par un ou plusieurs groupes hydroxyle, halogène, alkyle en C₁-C₆ ou alcoxy en C₁-C₆ ou par un groupe phényle ;
dans lequel hétérocycle est un groupe monocyclique à 5 ou 6 chaînons, saturé ou insaturé, contenant 1 ou 2 hétéroatomes cycliques choisis parmi N, O et S, les atomes restants étant des atomes de carbone, comme par exemple furyle, tétrahydrofuryle, thiényle, pyrryle, pyrrolidyle, pyridyle, pipéridyle, pyrazinyle, pyrimidyle, pipé- razinyle, morpholinyle, thiomorpholinyle, pyrazolyle, imidazolyle, oxazolyle, oxazolidyle, isoxazolyle, isoxazo- lidyle et thiazolyle ; et
dans lequel hétérocycle substitué est un hétérocycle comme défini ci-dessus substitué par halogène, alkyle en C₁-C₆ ou alcoxy en C₁-C₆, comprenant les étapes suivantes :
(a) réaction d'un produit de départ catéchol protégé de manière appropriée, de formule : 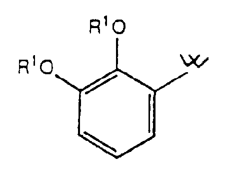 dans laquelle R¹ est choisi parmi l'hydrogène et un groupe qui est aisément clivable in vivo ; et
W est choisi dans le groupe comprenant H, Li ou un groupe formateur de cycle, avec une molécule de condensation substituée de manière appropriée et un réactif de condensation dans des conditions de condensation appropriées pour donner un premier composé intermédiaire ;
(b)conversion du premier composé intermédiaire en un second composé intermédiaire voulu substitué de manière appropriée, protégé de manière appropriée ;
(c)cyclisation du second composé intermédiaire pour donner un troisième composé intermédiaire substitué de manière appropriée, protégé de manière appropriée ;
(d) addition du groupement aminé voulu par une ou plusieurs réactions de transformation en dérivé pour préparer un composé intermédiaire final protégé de manière appropriée ; et
(e)déprotection du composé intermédiaire final avec le réactif de déprotection approprié et isolement du produit voulu.

2. Procédé selon la revendication 1, dans lequel ledit groupe formateur de cycle est choisi dans le groupe consistant en

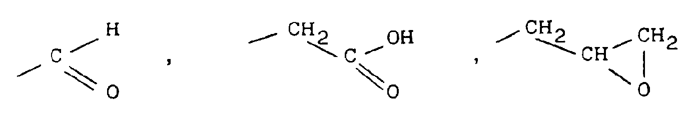

3. Procédé selon les revendications 1-2, dans lequel A est CR² et les pointillés entre A et l'atome cyclique numéro 1 représentent une liaison, présentant la formule : 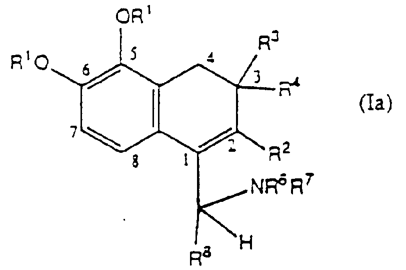 dans laquelle R¹, R², R³, R4 R⁶, R⁷ et R⁸ sont tels que définis dans la revendication 1.

4. Procédé selon les revendications 1-2 dans lequel A est CHR² présentant la formule : 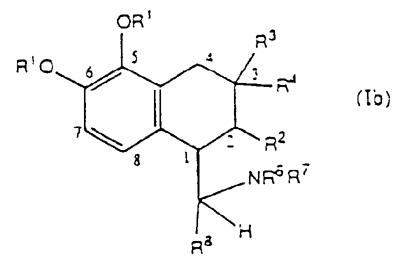 dans laquelle R¹, R², R³, R4 R⁶, R⁷ et R⁸ sont tels que définis dans la revendication 1.

5. Procédé selon les revendications 1-2, dans lequel A est O.

6. Procédé selon les revendications 1-2, dans lequel n est 0 et R⁶ et A pris ensemble forment un cycle azoté à 5, 6 ou 7 chaînons présentant la formule : 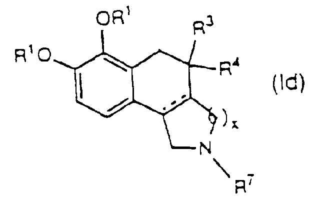 dans laquelle x est 1, 2 ou 3 et R¹, R³, R⁴ et R⁷ sont tels que définis dans la revendication 1.

7. Procédé selon les revendications 1-2 dans lequel A est CHR², R² est H et R⁸ pris ensemble avec la position 8 du cycle catéchol forme un cycle à 5, 6 ou 7 chaînons, présentant la formule : 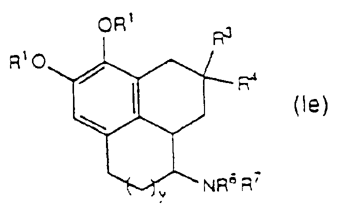 dans laquelle y est 0, 1 ou 2, R¹ et R³, R⁴, R⁶ et R⁷ sont tels que définis dans la revendication 1

8. Procédé selon les revendications 1-2 dans lequel R³ est alkyle en C₁-C₁₂, alkyle en C₁-C₆ substitué, cycloalkyle en C₃-C₁₂, cycloalkyle en C₃-C₁₂ substitué, aryle carbocyclique en C₅-C₁₀ ou aryle carbocyclique en C₅-C₁₀ substitué et R⁴ est l'hydrogène.

9. Procédé selon les revendications 1-2, dans lequel R⁷ et R⁸ sont l'hydrogène.

10. Procédé selon les revendications 1-2 pour préparer un composé choisi dans le groupe consistant en
1-aminométhyl-5,6-dihydroxy-3-phényl-3,4-dihydronaphtalène;
1-aminométhyl-5,6-bis(acétoxy)-3-phényl-3,4-dihydronaphtalène;
1-aminométhyl-5,6-bis(triméthylacétoxy)-3-phényl-3,4-dihydronaphtalène;
[1R,3S] 1-aminométhyl-5,6-dihydroxy-3-phényl-1,2,3,4-tétrahydronaphtalène;
1-aminométhyl-3-cyclohexyl-5,6-dihydroxy-3,4-dihydronaphtalène;
[1R,3S] 1-aminométhyl-3-cyclohexyl-5,6-dihydroxy-1,2,3,4-tétrahydronaphtalène;
[1R,3S] 1-aminométhyl-3-t-butyl-3,4-dihydro-5;6-dihydroxy-1 H-2-benzopyrane ;
[1R,3S] 1-aminométhyl-3,4-dihydro-5,6-dihydroxy-3-phényl-1 H-2-benzopyrane;
[1R,3S] 1-aminométhyl-3-cyclohexyl-3,4-dihydro-5,6-dihydroxy-1 H-2-benzopyrane;
[1R,3R] 1-aminométhyl-3,4-dihydro-5,6-dihydroxy-3-éthyl-1 H-2-benzopyrane;
Spiro[(1-aminométhyl-3,4-dihydro-5,6-dihydroxy-1H-2-benzopyrane)-3,1'-cyclohexane];
[1R,3S] 1-aminométhyl-3,4-dihydro-5,6-dihydroxy-3-(4'méthoxyphénoxy)-méthyl-1 H-2-benzopyrane;
[1R,3S] 1-aminométhyl-3,4-dihydro-5,6-dihydroxy-3-phényl-1 H-2-benzopyrane;
[1R,3S]1-aminométhyl-3,4-dihydro-5,6-dihydroxy-3-phénoxyméthyl-1H-2-benzopyrane;
[1R,3S]1-aminométhyl-3,4-dihydro-5,6-dihydroxy-3-(2'-phénylphénoxy)-méthyl-1 H-2-benzopyrane;
[1R,3S] 1-aminométhyl-3-(4'-t-butylphénoxy)méthyl-3,4-dihydro-5,6-dihydroxy-1 H-2-benzopyrane;
[1R,3S] 1-aminométhyl-3-(4'-bromophénoxy)méthyl-3,4-dihydro-5,6-dihydroxy-1 H-2-benzopyrane;
[1 R,3R] 1-aminométhyl-3-benzyl-3,4-dihydro-5,6-dihydroxy-1 H-2-benzopyrane;
[1 R,3R] 1-aminométhyl-3,4-dihydro-5,6-dihydroxy-3-(2'-phényl)éthyl-1 H-2-benzopyrane ;
[1R,3S] 1-aminométhyl-8-bromo-3,4-dihydro-5,6-dihydroxy-3-phényl-1 H-2-benzopyrane ;
[1R,3R] 1-aminométhyl-3,4-dihydro-5,6-dihydroxy-3-n-octyl-1 H-2-benzopyrane;
[1R,3R] 1-aminométhyl-3,4-dihydro-5,6-dihydroxy-3-(1'-hex-5'-ène)-1H-2-benzopyrane;
[1R,3S] 1-aminométhyl-3,4-dihydro-5,6-dihydroxy-3-éthyl-1 H-2-benzopyrane;
[1R,3R] 1-aminométhyl-3,4-dihydro-5,6-dihydroxy-3-n-hexyl-1 H-2-benzopyrane;
[1R,3S] 1-aminométhyl-3-(4'-bromophényl)-3,4-dihydro-5,6-dihydroxy-1 H-2-benzopyrane ;
[1R,3S] 1-aminométhyl-3,4-dihydro-5,6-dihydroxy-3-(3'-hydroxyphényl)-1 H-2-benzopyrane ;
[1R,3S] 3-cyclohexyl-3,4-dihydro-5,6-dihydroxy-1-(N-méthyl)aminométhyl-1 H-2-benzopyrane ;
[1R,3S] 3-t-butyl-3,4-dihydro-5,6-dihydroxy-1-(N-méthyl)aminométhyl-1 H-2-benzopyrane ;
[1R,3S] 1-(N-allyl)aminométhyl-3-cyclohexyl-3;4-dihydro-5,6-dihydroxy-1 H-2-benzopyrane ;
[1R,3S] 3-cyclohexyl-1-(N-cyclopropyl)aminométhyl-3,4-dihydro-5,6-dihydroxy-1 H-2-benzopyrane;
[1R,3S] 1-(N-benzyl)aminométhyl-3-cyclohexyl-3,4-dihydro-5,6-dihydroxy-1 H-2-benzopyrane ;
[1R,3S] 1,3-bis(aminométhyl)-3,4-dihydro-5,6-dihydroxy-1 H-2-benzopyrane;
[1R,3S] 1-aminométhyl-3,4-dihydro-5,6-dihydroxy-3-hydroxyméthyl-1 H-2-benzopyrane ;
[1R,3S] 1-aminométhyl-3,4-dihydro-5,6-dihydroxy-3-(N-pipéridinyl)mélhyl-1H-2-benzopyrane ;
[1R,3S] 5,6-dihydroxy-3-phényl-1-(2'R-pyrrolidinyl)-1,2,3,4-tétrahydronaphtalène
[1R,3R] 5,6-dihydroxy-3-phényl-1-(2'-R-pyrrolidinyl)-1;2,3,4-tétrahydronaphtalène;
5,6-dihydroxy-1-(N-méthyl)aminomélhyl-3-phényl-3,4-dihydronaphialène,
[1R,3S]5,6-dihydroxy-1-(N-méthyl)aminométhyl-3-phényl-1,2,3,4-tétrahydronaphtalène;
1-aminométhyl-5,6-dihydroxy-3-(3'-hydroxyphényl)-3,4-dihydronaphtalène;
1-aminométhyl-5,6-dihydroxy-3-(4'-hydroxyphényl)-3,4-dihydronaphtalène;
[1R,3S] 1 -aminométhyl-5,6-dihydroxy-3-(3'-hydroxyphényl)-1,2,3,4-létrahydronaphtalène,
5,6-bis(acétoxy)-1-(alanyl-alanyl)aminométhyl-3-phényl-3,4-dihydronaphtalène,
5,6-bis(acétoxy)-1-(γ-glutamyl)aminométhyl-3-phényl-3,4-dihydronaphtalène
5,6-bis(acétoxy)-1-(alanyl)aminométhyl-3-phényl-3,4-dihydronaphtalène;
5,6-bis(acétoxy)-1-(méthionyl)aminométhyl-3-phényl-3,4-dihydronaphialène;
1-(alanyl-alanyl)aminométhyl-5,6-bis(benzoyloxy)-3-phényl-3;4-dihydronaphtalène;
[1R,2S] 1-aminométhyl-5,6-dihydroxy-2-(2'-hydroxy-1'-éthyl)-3-phényl-1,2,3,4-tétrahydronaphtalène;
1-aminométhyl-5,6-dihydroxy-3-phénylnaphtalène;
[1R,3S] 1-aminométhyl-3,4-dihydro-5,6-dihydroxy-3-(diphényl)méthyl-1 H-2-benzopyrane;
[1R,3S] 1-aminométhyl-3,4-dihydro-5,6-dihydroxy-3-(3'-méthyl-2'-n-pentyl)-1 H-2-benzopyrane;
[1R,3S] 1-aminométhyl-3,4-dihydro-5,6-dihydroxy-3-(1'-but-3'-ène)-1H-2-benzopyrane ;
[1R,3S] 1-aminométhyl-3,4-dihydro-5,6-dihydroxy-3-(6'-méthyl-2'-hept-5'-ene)-1H-2-benzopyrane;
[1R,3S] 1-aminométhyl-3-benzyloxyméthyl-3,4-dihydro-5;6-dihydroxy-1 H-2-benzopyrane;
[1R, 8S, 9aR] 1-amino-5,6-dihydroxy-2,3,7,8,9,9a-hexahydro-8-phénylphénalène;
[1S, 8S, 9aR] 1-amino-5,6-dihydroxy-2,3,7,8,9,9a-hexahydro-8-phénylphénalène;
6,7-dihydroxy-4-phényl-2,3,4,5-tétrahydro-1 H-benzo[e]isoindole;et
1-aminométhyl-5,6-bis(benzoyloxy)-3-phényl-3,4-dihydronaphtalène ou sel pharmaceutiquement acceptable de celui-ci.

11. Procédé selon les revendications 1-2 pour préparer un composé choisi dans le groupe consistant en
[1R,3S]3-(1'-adamantyl)-1-aminométhyl-3,4-dihydro-5,6-dihydroxy-1H-2-benzopyrane ;
[1R*,3S*] 3-(1'-adamantyl)-1-aminométhyl-3,4-dihydro-5,6-dihydroxy-1 H-2-benzopyrane;
[1 R,3S] 3-(1'-adamantyl)-3,4-dihydro-5,6-dihydroxy-1-(N-méthyl)aminométhyl-1H-2-benzopyrane;
[1 R,3R] 1-aminométhyl-3-cyclopentylméthyl-3,4-dihydro-5,6-dihydroxy-1 H-2-benzopyrane ;
[1R,3S] 1-aminométhyl-3-cyclooctyl-3,4-dihydro-5,6-dihydroxy-1H-2-benzopyrane ;
[1 R,3R] 1-aminométhyl-3-n-butyl-3,4-dihydro-5;6-dihydroxy-1H-2-benzopyrane ;
[1R,3R] 3-n-butyl-3,4-dihydro-5,6-dihydroxy-1-(N-méthyl)aminométhyl-1H-2-benzopyrane,
ou un sel pharmaceutiquement acceptable de celui-ci.

12. Procédé selon les revendications 1-2 pour préparer un composé présentant la formule : 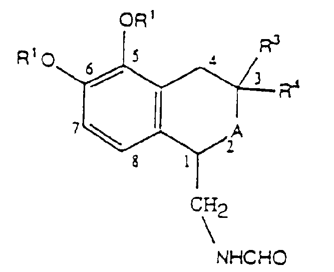 dans laquelle A est O ou S ;
R¹ est un groupe protecteur de catéchol ;
R³ est choisi parmi alkyle en C₁-C₁₂, alkyle en C₁-C₆ substitué comme défini ci-dessous, alcényle en C₂-C₁₂, alcynyle en C₂-C₁₂ substitué comme défini ci-dessous, alcényle en C₂-C₁₂, alcynyle en C₂-Cₗ₂ substitué comme défini ci-dessous, cycloalkyle en C₃-C_{12'} cycloalkyle en C₃-C₁₂ substitué comme défini ci-dessous, aryle carbocyclique en C₅-C₁₀, aryle carbocyclique en C₅-C₁₀ substitué comme défini ci-dessous, hétérocycle comme défini ci-dessous et hétérocycle substitué comme défini ci-dessous ;
R⁴ est choisi parmi l'hydrogène et alkyle en C₁-C₁₂ ou, pris ensemble avec R³ et l'atome de carbone auquel ils sont liés, forme un cycle spirocycloalkyle de 3 à 7 atomes de carbone ;
dans lequel alkyle en C₁-C₆ substitué est un groupe alkyle en C₁-C₆ portant 1-2 substituants choisis parmi halogène, hydroxyle, alcoxy en C₁-C₆, amino alkylamino en C₁-C₆, cycloalkyle en C₃-C_{12;} hétérocycle comme défini ci-dessous, hétérocycle substitué comme défini ci-dessous, aryle carbocyclique en C₅-C_{10'} aryle carbocyclique en C₅-C₁₀ substitué comme défini ci-dessous, aryloxy carbocyclique en C₅-C₁₀, aryloxy carbocyclique en C₅-C₁₀ substitué dans lequel aryle carbocyclique en C₅ C₁₀ substitué est comme défini ci-dessous, ou -NC (O)R¹¹ où R¹¹ est choisi parmi l'hydrogène, alkyle en C₁-C₆, halogéno-(alkyle en C₁-C₆)_{'} hétérocycle comme défini ci-dessous, hétérocycle substitué comme défini ci-dessous, aryle carbocyclique en C₅-C₁₀, et aryle carbocyclique en C₅-C₁₀ substitué comme défini ci-dessous ;
dans lequel alcényle en C₂-C₁₂ substitué est un groupe alcényle en C₂-C₁₂ portant un substituant choisi parmi alkyle en C₁-C₆, cycloalkyle en C₃-C₁₂, aryle carbocyclique en C₅-C₁₀, benzyle, phényléthyle, amino, hydroxyle, alcoxy en C₁-C₆ et hétérocycle comme défini ci-dessous,
dans lequel alcynyle en C₂-C₁₂ substitué est un alcynyle en C₂-C₇₂ portant un substituant choisi parmi alkyle en C₁-C₆, cycloalkyle en C₃-C₁₂, aryle carbocyclique en C₅-C₁₀, benzyle, phényléthyle, amino, hydroxyle, alkylamino en C₁-C₁₂, alcoxy en C₁-C₆ et hétérocycle comme défini ci-dessous ;
dans lequel cycloalkyle en C₃-C₁₂ substitué est un groupe cycloalkyle en C₃-C₁₂ substitué par un ou plusieurs groupes alkyles en C₁-C₁₂, hydroxyle, amino, alkylamino en C₁-C₁₂, aminoalkyle en C₁-C_{12'} mercapto, alkylthio en C₁-C₁₂ ou halogène,
dans lequel aryle carbocyclique en C₅-C₁₀ substitué est un aryle carbocyclique en C₅-C₁₀ substitué par un ou plusieurs groupes hydroxyle, halogène, alkyle en C₁-C₆ ou alcoxy en C₁-C₆ ou par un groupe phényle ;
dans lequel hétérocycle est un groupe monocyclique à 5 ou 6 chaînons, saturé ou insaturé, contenant 1 ou 2 hétéroatomes cycliques choisis parmi N, O et S, les atomes restants étant des atomes de carbone, comme par exemple furyle, tétrahydrofuryle, thiényle, pyrryle, pyrrolidyle, pyridyle, pipéridyle, pyrazinyle, pyrimidyle, pipé- razinyle, morpholinyle, thiomorpholinyle, pyrazolyle, imidazolyle, oxazolyle, oxazolidyle, isoxazolyle, isoxazo- lidyle et thiazolyle ; et
dans lequel hétérocycle substitué est un hétérocycle comme défini ci-dessus substitué par halogène, alkyle en C₁-C₆ ou alcoxy en C₁-C₆.

13. Procédé pour préparer un composé présentant la formule : 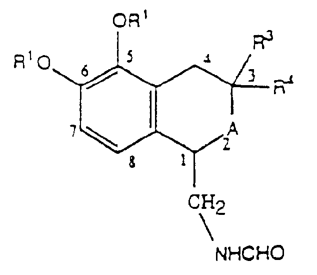
dans laquelle A est O ou S ;
R⁷ est un groupe protecteur de catéchol ;
R³ est choisi parmi alkyle en C₁-C_{12'} alkyle en C₁-C₆ substitué comme défini ci-dessous, alcényle en C₂-C_{12'} alcényle en C₂-C₁₂ substitué comme défini ci-dessous, alcynyle en C₂-Cᵢ₂, alcynyle en C₂-C₁₂ substitué comme défini ci-dessous, cycloalkyle en C₃-C_{12'} cycloalkyle en C₃-C₁₂ substitué comme défini ci-dessous, aryle carbocyclique en C₅-C₁₀, aryle carbocyclique en C₅-C_{1O} substitué comme défini ci-dessous, hétérocycle comme défini ci-dessous et hétérocycle substitué comme défini ci-dessous ;
R⁴ est choisi parmi l'hydrogène et alkyle en C₁-C₁₂ ou, pris ensemble avec R³ et l'atome de carbone auquel ils sont liés, forme un cycle spirocycloalkyle de 3 à 7 atomes de carbone ;
dans lequel alkyle en C₁-C₆ substitué est un groupe alkyle en C₁-C₆ portant 1-2 substituants choisis parmi halogène, hydroxyle, alcoxy en C₁-C_{6'} amino alkylamino en C₁-C_{6'} cycloalkyle en C₃-C₁₂, hétérocycle comme défini ci-dessous, hétérocycle substitué comme défini ci-dessous, aryle carbocyclique en C₅-C₁₀, aryle carbocyclique en C₅-C₁₀ substitué comme défini ci-dessous, aryloxy carbocyclique en C₅-C₁₀, aryloxy carbocyclique en C₅-C₁₀ substitué dans lequel aryle carbocyclique en C₅C₁₀ substitué est comme défini ci-dessous, ou -NC (O)R¹¹ où R¹¹ est choisi parmi l'hydrogène, alkyle en C₁-C₆, halogéno-(alkyle en C₁-C₆), hétérocycle comme défini ci-dessous, hétérocycle substitué comme défini ci-dessous, aryle carbocyclique en C₅-Cₗₒ, et aryle carbocyclique en C₅-C₁₀ substitué comme défini ci-dessous ;
dans lequel alcényle en C₂-Cᵢ₂ substitué est un groupe alcényle en C₂-C₁₂ portant un substituant choisi parmi alkyle en C₁-C₆, cycloalkyle en C₃-C₁₂, aryle carbocyclique en C₅-C₁₀, benzyle, phényléthyle, amino, hydroxyle, alcoxy en C₁-C₆ et hétérocycle comme défini ci-dessous,
dans lequel alcynyle en C₂-C₁₂ substitué est un alcynyle en C₂-C₁₂ portant un substituant choisi parmi alkyle en C₁-C₆, cycloalkyle en C₃-C_{12'} aryle carbocyclique en C₅-C₁₀, benzyle, phényléthyle, amino hydroxyle, alkylamino en C₁-C₁₂, alcoxy en C₁-C₆ et hétérocycle comme défini ci-dessous ;
dans lequel cycloalkyle en C₃-C₁₂ substitué est un groupe cycloalkyle en C₃-C₁₂ substitué par un ou plusieurs groupes alkyles en C₁-C₁₂, hydroxyle, amino, alkylamino en C₁-C₁₂, aminoalkyle en C₁-C_{12'} mercapto, alkylthio en C₁-C₁₂ ou halogène,
dans lequel aryle carbocyclique en C₅-C₁₀ substitué est un aryle carbocyclique en C₅-C₁₀ substitué par un ou plusieurs groupes hydroxyle, halogène, alkyle en C₁-C₆ ou alcoxy en C₇-C₆ ou par un groupe phényle ;
dans lequel hétérocycle est un groupe monocyclique à 5 ou 6 chaînons, saturé ou insaturé, contenant 1 ou 2 hétéroatomes cycliques choisis parmi N, O et S, les atomes restants étant des atomes de carbone, comme par exemple furyle, tétrahydrofuryle, thiényle, pyrryle, pyrrolidyle, pyridyle, pipéridyle, pyrazinyle, pyrimidyle, pipé- razinyle, morpholinyle, thiomorpholinyle, pyrazolyle, imidazolyle, oxazolyle, oxazolidyle, isoxazolyle, isoxazo- lidyle et thiazolyle ; et
dans lequel hétérocycle substitué est un hétérocycle comme défini ci-dessus substitué par halogène, alkyle en C₁-C₆ ou alcoxy en C₁-C₆,
comprenant la réaction d'un composé de formule : 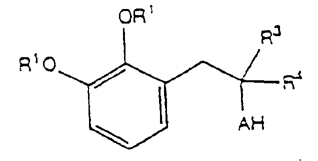 dans laquelle R¹, R³ et R⁴ sont tels que définis ici, avec l'acétal diméthylique du N-formylaminoacétaldéhyde ou avec l'acétal diméthylique du 3-(N-formylamino)-propionaldéhyde en présence d'un catalyseur acide.

14. Procédé selon la revendication 13, dans lequel le catalyseur est choisi parmi l'éthérate de trifluorure de bore, le triflate de zinc, le triflate de triméthylsilyle et l'acide méthanesulfonique.
